(19)

Europäisches Patentamt
European Patent Office
Office européen des brevets

(11) **EP 4 629 247 A1**

(12) **EUROPEAN PATENT APPLICATION**

(43) Date of publication:
**08.10.2025 Bulletin 2025/41**

(21) Application number: **25168546.7**

(22) Date of filing: **04.04.2025**

(51) International Patent Classification (IPC):
***G16B 20/20*** (2019.01)

(52) Cooperative Patent Classification (CPC):
**G16B 20/20**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **04.04.2024 US 202463574758 P
26.03.2025 US 202519091587**

(71) Applicant: **Tempus AI, Inc.
Chicago, IL 60654 (US)**

(72) Inventors:
• **DRIESSEN, Terri, M.**
**Chicago, 60654 (US)**
• **ZHU, Wei**
**Chicago, 60654 (US)**
• **LEE, Christine, Y.**
**Chicago, 60654 (US)**

(74) Representative: **Grund, Martin et al
Grund Intellectual Property Group
Patentanwälte und Solicitor PartG mbB
Steinsdorfstraße 2
80538 München (DE)**

(54) **METHODS AND SYSTEMS FOR TUMOR INFORMED CIRCULATING TUMOR FRACTION ESTIMATION**

(57)     Methods, systems, and software for estimating circulating tumor fraction are provided. A first plurality of nucleic acid sequences for a plurality of loci in genomic DNA from a solid tumor sample is obtained. A second plurality of nucleic acid sequences for a plurality of cell-free DNA fragments obtained from a liquid biopsy sample from the same subject is obtained. One or more somatic mutations is identified in the first plurality of nucleic acid sequences. A variant allele frequency (VAF) is determined for each somatic mutation based on a frequency of the respective somatic mutation in the liquid biopsy sample and a frequency of the corresponding wild type allele in the liquid biopsy sample, thereby determining a set of VAFs. An estimate of the circulating tumor fraction for the test subject is determined based on the set of VAFs for the one or more somatic mutations.

500

**(502)** Obtain a first plurality of nucleic acid sequences including a corresponding nucleic acid sequence for each respective locus in a plurality of loci in genomic DNA from a solid tumor sample from the test subject.

**(504)** The first plurality of nucleic acid sequences is determined from a second panel-enriched sequencing reaction using a second plurality of probes including, for each respective locus in the plurality of loci a corresponding probe, in a second plurality of probes, that hybridizes to the respective locus.

**(506)** The plurality of loci is sequenced at an average sequence depth of at least 1000X in the second panel-enriched sequencing reaction.

**(508)** The second plurality of probes enriches for loci from at least 50 genes.

A

Fig. 5A

**EP 4 629 247 A1**

A

(509) Obtain a second plurality of nucleic acid sequences including a corresponding nucleic acid sequence for each cell-free DNA fragment in a plurality of cell-free DNA fragments obtained from a liquid biopsy sample from a first panel-enriched sequencing assay using a first plurality of probes including, for each respective locus in the plurality of loci, a corresponding probe that hybridizes the respective locus.

(510) The first plurality of probes and the second plurality of probes are different.

(512) The plurality of loci is sequenced at an average sequence depth of at least 1000X in the first panel-enriched sequencing reaction.

(514) The first plurality of probes enriches for loci from at least 50 genes.

(516) The identity of the first plurality of probes is non-bespoke for the test subject.

(518) The solid tumor sample is collected prior to collecting the liquid biopsy sample.

(520) The solid tumor sample and the liquid biopsy sample are collected within 6 months of each other.

(522) The liquid biopsy sample is blood.

(524) The liquid biopsy sample includes blood, whole blood, peripheral blood, plasma, serum, or lymph of the subject.

B

Fig. 5B

2

B

**(525)** Identify, in the first plurality of nucleic acid sequences, one or more somatic mutations, where each respective somatic mutations in the one or more somatic mutations is at a corresponding one or more nucleotide positions in a corresponding loci in the plurality of one or more loci.

**(526)** The identifying includes identifying a plurality of candidate somatic mutations by comparing respective nucleic acid sequences in the first plurality of nucleic acid sequences to nucleic acid sequences in a third plurality of nucleic acid sequences obtained from a sequencing reaction of genomic DNA from a non-cancerous tissue of the subject.

**(528)** The identifying further includes excluding one or more respective candidate somatic mutations in the plurality of candidate somatic mutations determined to have outlying variant allele fractions in the first plurality of sequences.

**(530)** The excluding includes fitting VAFs for each respective candidate somatic mutation in the plurality of candidate somatic mutations in the first plurality of sequences to a distribution and excluding candidate somatic mutations with corresponding VAFs outside of a statistical measure of variation for the distribution.

**(532)** The distribution is a normal distribution, a beta distribution, a beta prime distribution, a log normal distribution, or a gamma distribution.

**(534)** The probability distribution is a normal distribution.

**(536)** The statistical measure of variation is a multiple of a standard deviation for the probability distribution.

**(538)** The identifying further includes excluding one or more respective candidate somatic mutations in the plurality of candidate somatic mutations having a nucleotide position that does not correspond to any probe in the first plurality of probes.

C

Fig. 5C

(C)

**(539)** Determine, for each respective somatic mutation in the one or more somatic mutations, a corresponding variant allele frequency (VAF) in the liquid biopsy sample, where the corresponding VAF is determined from (i) a frequency of the respective somatic mutation in the second plurality of nucleic acid sequences and (ii) a frequency of a wild type allele at the corresponding one or more nucleotide positions for the respective somatic variant in the second plurality of nucleic acid sequences, thereby determining a set of VAFs for the one or more somatic mutations in the liquid biopsy sample.

**(540)** Determine an estimate of the circulating tumor fraction for the test subject based on the set of VAFs.

**(542)** The estimate of the circulating tumor fraction is a measure of central tendency for the set of VAFs for the one or more somatic mutations.

**(544)** The measure of central tendency is a median.

Fig. 5D

**Description**

**CROSS REFERENCE TO RELATED PATENT APPLICATION**

This application claims priority to United States Provisional Patent Application No. 63/574,758, entitled "Methods and Systems for Tumor Informed Circulating Tumor Fraction Estimation," filed April 4, 2024, which is hereby incorporated by reference.

**TECHNICAL FIELD**

The present disclosure relates generally to the use of tumor informed liquid biopsy data to estimate the circulating tumor fraction for a test subject in order to provide clinical support for personalized treatment of cancer.

**BACKGROUND**

Precision oncology is the practice of tailoring cancer therapy to the unique genomic, epigenetic, and/or transcriptomic profile of an individual's cancer. Personalized cancer treatment builds upon conventional therapeutic regimens used to treat cancer based only on the gross classification of the cancer, *e.g.*, treating all breast cancer patients with a first therapy and all lung cancer patients with a second therapy. This field was borne out of many observations that different patients diagnosed with the same type of cancer, *e.g.*, breast cancer, responded very differently to common treatment regimens. Over time, researchers have identified genomic, epigenetic, and transcriptomic markers that improve predictions as to how an individual cancer will respond to a particular treatment modality.

There is growing evidence that cancer patients who receive therapy guided by their genetics have better outcomes. For example, studies have shown that targeted therapies result in significantly improved progression-free cancer survival. See, *e.g.,* Radovich M. et al., Oncotarget, 7(35):56491-500 (2016). Similarly, reports from the IMPACT trial-a large (n = 1307) retrospective analysis of consecutive, prospectively molecularly profiled patients with advanced cancer who participated in a large, personalized medicine trial-indicate that patients receiving targeted therapies matched to their tumor biology had a response rate of 16.2%, as opposed to a response rate of 5.2% for patients receiving non-matched therapy. Tsimberidou et al., ASCO 2018, Abstract LBA2553 (2018).

In fact, therapy targeted to specific genomic alterations is already the standard of care in several tumor types, *e.g.,* as suggested in the National Comprehensive Cancer Network (NCCN) guidelines for melanoma, colorectal cancer, and non-small cell lung cancer. In practice, implementation of these targeted therapies requires determining the status of the diagnostic marker in each eligible cancer patient. While this can be accomplished for the few, well-known mutations associated with treatment recommendations in the NCCN guidelines using individual assays or small next generation sequencing (NGS) panels, the growing number of actionable genomic alterations and increasing complexity of diagnostic classifiers necessitates a more comprehensive evaluation of each patient's cancer genome, epigenome, and/or transcriptome.

For instance, some evidence suggests that use of combination therapies where each component is matched to an actionable genomic alteration holds the greatest potential for treating individual cancers. To this point, a retroactive study of cancer patients treated with one or more therapeutic regimens revealed that patients who received therapies matched to a higher percentage of their genomic alterations experienced a greater frequency of stable disease (*e.g.,* a longer time to recurrence), longer time to treatment failure, and greater overall survival. Wheeler et al., 2016, Cancer Res., 76:3690-701. Thus, comprehensive evaluation of each cancer patient's genome, epigenome, and/or transcriptome should maximize the benefits provided by precision oncology, by facilitating more fine-tuned combination therapies, use of novel off-label drug indications, and/or tissue agnostic immunotherapy. See, for example, Schwaederle et al., 2015, J Clin Oncol., 33(32):3817-25; Schwaederle et al., 2016, JAMA Oncol., 2(11):1452-59; and Wheler et al., 2016, Cancer Res., 76(13):3690-701. Further, the use of comprehensive next generation sequencing analysis of cancer genomes facilitates better access and a larger patient pool for clinical trial enrollment. Coyne et al., 2017, Curr. Probl. Cancer, 41(3):182-93; and Markman, Oncology, 31(3):158, 168.

The use of large NGS genomic analysis is growing in order to address the need for more comprehensive characterization of an individual's cancer genome. See, for example, Fernandes et al., Clinics, 72(10):588-94. Recent studies indicate that of the patients for which large NGS genomic analysis is performed, 30-40% then receive clinical care based on the assay results, which is limited by at least the identification of actionable genomic alterations, the availability of medication for treatment of identified actionable genomic alterations, and the clinical condition of the subject. See, Ross et al., 2015, JAMA Oncol., 1(1):40-49; Ross et al., 2015, Arch. Pathol. Lab Med., 139:642-49; Hirshfield KM et al., Oncologist, 2016, 21(11):1315-25; and Groisberg et al., 2017, Oncotarget, 8:39254-67.

However, these large NGS genomic analyses are conventionally performed on solid tumor samples. For instance, each of the studies referenced in the paragraph above performed NGS analysis of FFPE tumor blocks from patients. Solid tissue biopsies remain the gold standard for diagnosis and identification of predictive biomarkers because they represent well-known and validated methodologies that provide a high degree of accuracy. Nevertheless, there are significant limitations to the use of solid tissue material for large NGS genomic analyses of cancers. For example, tumor biopsies are subject to sampling bias caused by spatial and/or temporal genetic heterogeneity, e.g., between two regions of a single tumor and/or between different cancerous tissues (such as between primary and metastatic tumor sites or between two different primary tumor sites). Such intertumor or intratumor heterogeneity can cause sub-clonal or emerging mutations to be overlooked when using localized tissue biopsies, with the potential for sampling bias to be exacerbated over time as sub-clonal populations further evolve and/or shift in predominance.

Additionally, the acquisition of solid tissue biopsies often requires invasive surgical procedures, e.g., when the primary tumor site is located at an internal organ. These procedures can be expensive, time consuming, and carry a significant risk to the patient, *e.g.,* when the patient's health is poor and may not be able to tolerate invasive medical procedures and/or the tumor is located in a particularly sensitive or inoperable location, such as in the brain or heart. Further, the amount of tissue, if any, that can be procured depends on multiple factors, including the location of the tumor, the size of the tumor, the fragility of the patient, and the risk of comorbidities related to biopsies, such as bleeding and infections. For instance, recent studies report that tissue samples in a majority of advanced non-small cell lung cancer patients are limited to small biopsies and cannot be obtained at all in up to 31% of patients. Ilie and Hofman, Transl. Lung Cancer Res., 5(4):420-23 (2016). Even when a tissue biopsy is obtained, the sample may be too scant for comprehensive testing.

Further, the method of tissue collection, preservation (e.g., formalin fixation), and/or storage of tissue biopsies can result in sample degradation and variable quality DNA. This, in turn, leads to inaccuracies in downstream assays and analysis, including next-generation sequencing (NGS) for the identification of biomarkers. Ilie and Hofman, Transl Lung Cancer Res., 5(4):420-23 (2016).

In addition, the invasive nature of the biopsy procedure, the time and cost associated with obtaining the sample, and the compromised state of cancer patients receiving therapy render repeat testing of cancerous tissues impracticable, if not impossible. As a result, solid tissue biopsy analysis is not amenable to many monitoring schemes that would benefit cancer patients, such as disease progression analysis, treatment efficacy evaluation, disease recurrence monitoring, and other techniques that require data from several time points.

Cell-free DNA (cfDNA) has been identified in various bodily fluids, *e.g.,* blood serum, plasma, urine, *etc.* Chan et al., 2003, Ann. Clin. Biochem., 40(Pt 2):122-30. This cfDNA originates from necrotic or apoptotic cells of all types, including germline cells, hematopoietic cells, and diseased (e.g., cancerous) cells. Advantageously, genomic alterations in cancerous tissues can be identified from cfDNA isolated from cancer patients. See, *e.g.,* Stroun et al., 1989, Oncology, 46(5):318-22; Goessl et al., 2000, Cancer Res., 60(21):5941-45; and Frenel et al., 2015, Clin. Cancer Res. 21(20):4586-96. Thus, one approach to overcoming the problems presented by the use of solid tissue biopsies described above is to analyze cell-free nucleic acids (e.g., cfDNA) and/or nucleic acids in circulating tumor cells present in biological fluids, *e.g.,* via a liquid biopsy.

Specifically, liquid biopsies offer several advantages over conventional solid tissue biopsy analysis. For instance, because bodily fluids can be collected in a minimally invasive or non-invasive fashion, sample collection is simpler, faster, safer, and less expensive than solid tumor biopsies. Such methods require only small amounts of sample (*e.g.,* 10 mL or less of whole blood per biopsy) and reduce the discomfort and risk of complications experienced by patients during conventional tissue biopsies. In fact, liquid biopsy samples can be collected with limited or no assistance from medical professionals and can be performed at almost any location. Further, liquid biopsy samples can be collected from any patient, regardless of the location of their cancer, their overall health, and any previous biopsy collection. This allows for analysis of the cancer genome of patients from which a solid tumor sample cannot be easily and/or safely obtained. In addition, because cell-free DNA in the bodily fluids arise from many different types of tissues in the patient, the genomic alterations present in the pool of cell-free DNA are representative of various different clonal sub-populations of the cancerous tissue of the subject, facilitating a more comprehensive analysis of the cancerous genome of the subject than is possible from one or more sections of a single solid tumor sample.

Liquid biopsies also enable serial genetic testing prior to cancer detection, during the early stages of cancer progression, throughout the course of treatment, and during remission, e.g., to monitor for disease recurrence. The ability to conduct serial testing via non-invasive liquid biopsies throughout the course of disease could prove beneficial for many patients, e.g., through monitoring patient response to therapies, the emergence of new actionable genomic alterations, and/or drug-resistance alterations. These types of information allow medical professionals to more quickly tailor and update therapeutic regimens, e.g., facilitating more timely intervention in the case of disease progression. See, *e.g.,* Ilie and Hofman, 2016, Transl. Lung Cancer Res., 5(4):420-23.

Nevertheless, while liquid biopsies are promising tools for improving outcomes using precision oncology, there are significant challenges specific to the use of cell-free DNA for evaluation of a subject's cancer genome. For instance, one challenge associated with liquid biopsies is the accurate determination of tumor fraction in a sample. This difficulty arises from at least the heterogeneity of cancers and the increased frequency of large chromosomal duplications and deletions found in cancers. As a result, the frequency of genomic alterations from cancerous tissues varies from locus to locus based on at least (i) their prevalence in different sub-clonal populations of the subject's cancer, and (ii) their location within the genome, relative to large chromosomal copy number variations. The difficulty in accurately determining the tumor fraction of liquid biopsy samples affects accurate measurement of various cancer features shown to have diagnostic value for the analysis of solid tumor biopsies. These include allelic ratios, copy number variations, overall mutational burden, frequency of abnormal methylation patterns, *etc.,* all of which are correlated with the percentage of DNA fragments that arise from cancerous tissue, as opposed to healthy tissue.

The information disclosed in this Background section is only for enhancement of understanding of the general background of the invention and should not be taken as an acknowledgement or any form of suggestion that this information forms the prior art already known to a person skilled in the art.

## SUMMARY

Estimating quantitative circulating tumor fraction in liquid biopsy samples is a promising area of clinical development for monitoring therapeutic molecular response and correlates with patient outcomes. However, given the above background, there is a need in the art for improved methods and systems for supporting clinical decisions in precision oncology using liquid biopsy assays. In particular, there is a need in the art for improved methods and systems for determining accurate circulating tumor fraction estimates (ctFEs) in liquid biopsy assays. The present disclosure solves this and other needs in the art by providing methods and systems for estimating the circulating tumor fraction of a liquid biopsy sample using a combination of tissue-informed, comprehensive genomic profiling (CGP) and non-bespoke blood-based profiling.

For example, in one aspect, the present disclosure provides methods, systems programed to execute such methods, and computer readable medium storing instructions for performing such methods, for estimating a circulating tumor fraction for a test subject.

The method includes obtaining a first plurality of nucleic acid sequences including a corresponding nucleic acid sequence for each respective locus in a plurality of loci in genomic DNA from a solid tumor sample from the test subject.

In some such embodiments, the first plurality of nucleic acid sequences is determined from a second panel-enriched sequencing reaction using a second plurality of probes including, for each respective locus in the plurality of loci a corresponding probe, in a second plurality of probes, that hybridizes to the respective locus.

In some such embodiments, the plurality of loci is sequenced at an average sequence depth of at least 50X, 75X, 100X, 125X, 500X, or 1000X in the second panel-enriched sequencing reaction.

In some such embodiments, the second plurality of probes enriches for loci from at least 50 genes.

The method also includes obtaining a second plurality of nucleic acid sequences including a corresponding nucleic acid sequence for each cell-free DNA fragment in a plurality of cell-free DNA fragments obtained from a liquid biopsy sample from a first panel-enriched sequencing assay using a first plurality of probes including, for each respective locus in the plurality of loci, a corresponding probe that hybridizes the respective locus.

In some such embodiments, the first plurality of probes and the second plurality of probes are different.

In some such embodiments, the plurality of loci is sequenced at an average sequence depth of at least 300X, 400X, 500X, 700X, or 1000X in the first panel-enriched sequencing reaction.

In some such embodiments, the first plurality of probes enriches for loci from at least 50 genes.

In some such embodiments, the identity of the first plurality of probes is non-bespoke for the test subject.

In some such embodiments, the solid tumor sample is collected prior to collecting the liquid biopsy sample.

In some such embodiments, the solid tumor sample and the liquid biopsy sample are collected within 6 months of each other.

In some such embodiments, the liquid biopsy sample is blood.

In some such embodiments, the liquid biopsy sample includes blood, whole blood, peripheral blood, plasma, serum, or lymph of the subject.

The method also includes, identifying, in the first plurality of nucleic acid sequences, one or more somatic mutations, where each respective somatic mutation in the one or more somatic mutations is at a corresponding one or more nucleotide positions in a corresponding loci in the plurality of one or more loci.

In some such embodiments, the identifying includes identifying a plurality of candidate somatic mutations by comparing respective nucleic acid sequences in the first plurality of nucleic acid sequences to nucleic acid sequences in a third plurality of nucleic acid sequences obtained from a sequencing reaction of genomic DNA from a non-cancerous tissue of the subject.

In some such embodiments, the identifying further includes excluding one or more respective candidate somatic mutations in the plurality of candidate somatic mutations determined to have outlying variant allele fractions in the first plurality of sequences.

In some such embodiments, the excluding includes fitting VAFs for each respective candidate somatic mutation in the plurality of candidate somatic mutations in the first plurality of sequences to a distribution and excluding candidate somatic mutations with corresponding VAFs outside of a measure of dispersion for the distribution.

In some such embodiments, the distribution is a normal distribution, a beta distribution, a beta prime distribution, a log normal distribution, or a gamma distribution.

In some such embodiments, the distribution is a normal distribution.

In some such embodiments, the measure of dispersion is a multiple of a standard deviation for the distribution.

In some such embodiments, the identifying further includes excluding one or more respective candidate somatic mutations in the plurality of candidate somatic mutations having a nucleotide position that does not correspond to any probe in the first plurality of probes.

The method also includes, determining, for each respective somatic mutation in the one or more somatic mutations, a corresponding variant allele frequency (VAF) in the liquid biopsy sample, where the corresponding VAF is determined from (i) a frequency of the respective somatic mutation in the second plurality of nucleic acid sequences and (ii) a frequency of a wild type allele at the corresponding one or more nucleotide positions for the respective somatic mutation in the second plurality of nucleic acid sequences, thereby determining a set of VAFs for the one or more somatic mutations in the liquid biopsy sample.

In some embodiments, determining an estimate of the circulating tumor fraction for the test subject based on the set of VAFs for the one or more somatic mutations.

In some such embodiments, the estimate of the circulating tumor fraction is a measure of central tendency for the set of VAFs for the one or more somatic mutations.

In some such embodiments, the measure of central tendency is a median.

Additional aspects and advantages of the present disclosure will become readily apparent to those skilled in this art from the following detailed description, wherein only illustrative embodiments of the present disclosure are shown and described. As will be realized, the present disclosure is capable of other and different embodiments, and its several details are capable of modifications in various obvious respects, all without departing from the disclosure. Accordingly, the drawings and description are to be regarded as illustrative in nature, and not as restrictive.

## BRIEF DESCRIPTION OF THE DRAWINGS

Figures 1A, 1B, 1C, and 1D collectively illustrate a block diagram of an example computing device for estimating the circulating tumor fraction of a liquid biopsy sample from targeted-panel sequencing data, in accordance with some embodiments of the present disclosure.

Figure 2A illustrates an example workflow for generating a clinical report based on information generated from analysis of one or more patient specimens, in accordance with some embodiments of the present disclosure.

Figure 2B illustrates an example of a distributed diagnostic environment for collecting and evaluating patient data for the purpose of precision oncology, in accordance with some embodiments of the present disclosure.

Figure 3 provides an example flow chart of processes and features for liquid biopsy sample collection and analysis for use in precision oncology, in accordance with some embodiments of the present disclosure.

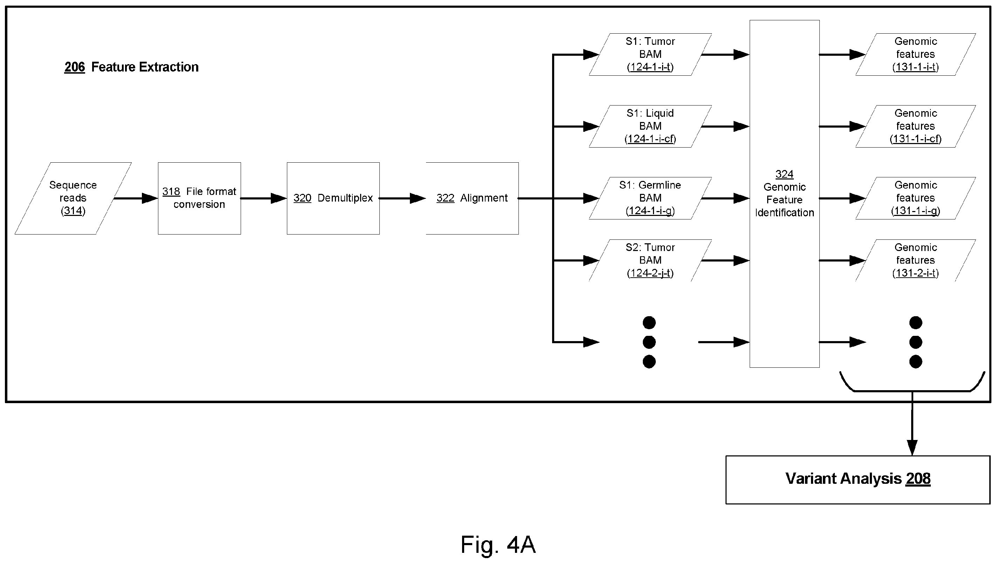

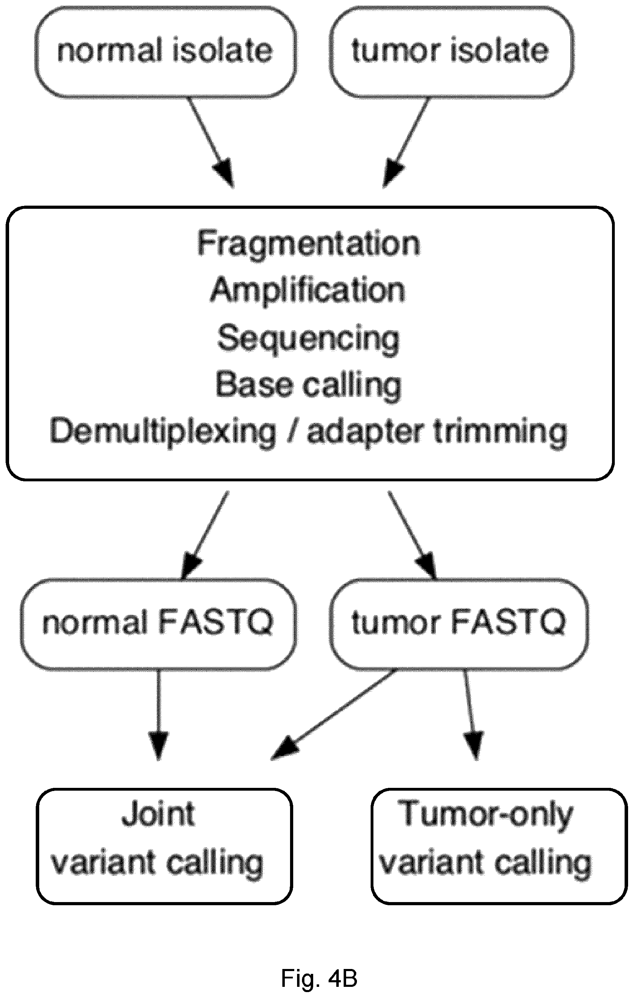

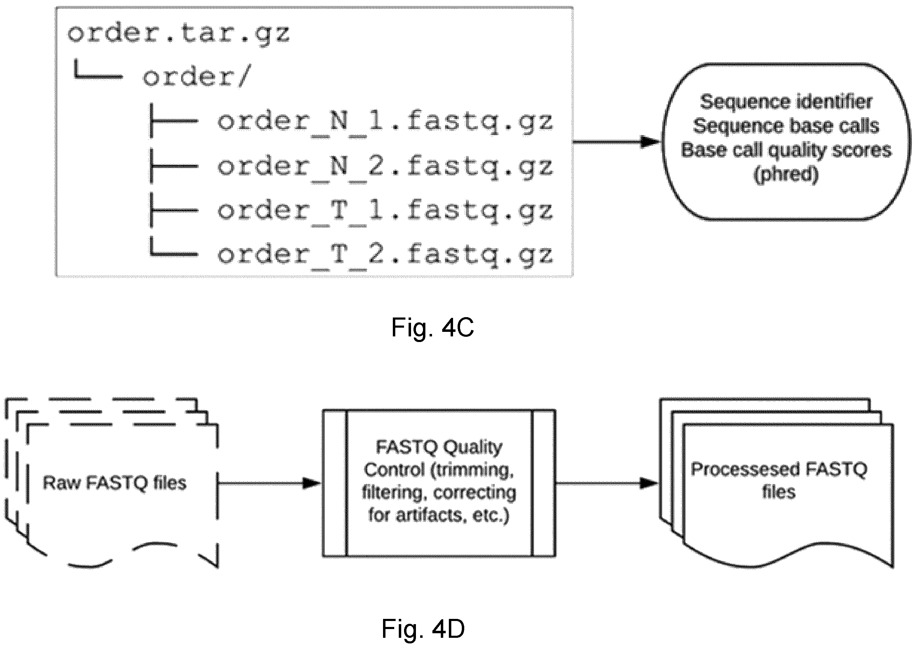

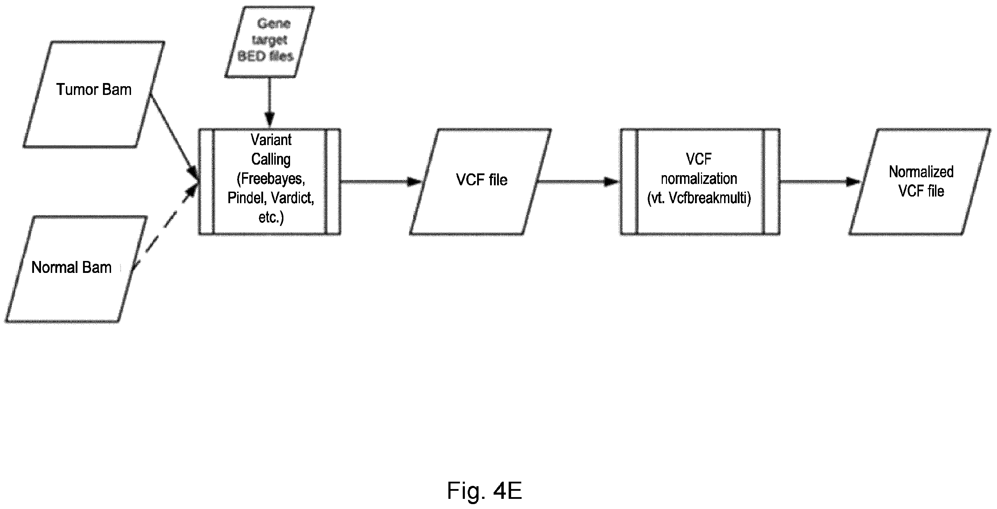

Figures 4A, 4B, 4C, 4D, and 4E collectively illustrate an example bioinformatics pipeline for precision oncology. Figure 4A provides an overview flow chart of processes and features in a bioinformatics pipeline, in accordance with some embodiments of the present disclosure. Figure 4B provides an overview of a bioinformatics pipeline executed with either a liquid biopsy sample alone or a liquid biopsy sample and a matched normal sample. Figure 4C illustrates that paired end reads from tumor and normal isolates are zipped and stored separately under the same order identifier, in accordance with some embodiments of the present disclosure. Figure 4D illustrates quality correction for FASTQ files, in accordance with some embodiments of the present disclosure. Figure 4E illustrates processes for obtaining tumor and normal BAM alignment files, in accordance with some embodiments of the present disclosure.

Figures 5A, 5B, 5C, and 5D collectively provide a flow chart of processes and features for estimating a tumor-informed circulating tumor fraction of a liquid biopsy sample from targeted-panel sequencing data, in which dashed boxes represent optional portions of the method, in accordance with some embodiments of the present disclosure.

Figures 6A and 6B illustrate distributions of Kolmogorov-Smirnov p-values for beta, betaprime (inverted beta), gamma, lognorm (for heavy tails), and normal distributions fit to untransformed (6A) and log2-transformed (6B) VAFs for candidate mutations identified in solid tumor samples, as described in Example 3.

Figures 7A and 7B illustrate a normal distribution fit to solid tumor VAFs for a solid tumor sample (7A) and an example of a distribution of VAFs for identified somatic mutations in a liquid biopsy assay (7B), as described in Example 3.

Figure 8A illustrates that tumor-informed ctDNA tumor fraction (TF) correlates with tumor-naïve ctDNA TF after removing specimens with no tumor-informed mutations, when using a 105 gene liquid biopsy panel-enriched sequencing assay.

Figure 8B illustrates tumor-informed mutation count in tumor specimens.

Figure 9A illustrates that tumor-informed ctDNA TF correlates with tumor-naïve ctDNA TF after removing specimens with no tumor-informed somatic mutations, when using a 523 gene liquid biopsy panel-enriched sequencing assay.

Figure 9B illustrates tumor-informed variant count in tumor specimens.

Figure 10 illustrates accuracy of tumor-informed cfDNA TF estimate in a tumor sample cohort with companion LPWGS using ichorCNA, Mean VAF, and tumor-naive ctDNA TF as the comparators.

Figures 11A and 11B illustrate that tumor-informed ctDNA TF correlates with tumor-naïve ctDNA TF when requiring more than 4 tumor-informed somatic mutations, when using 105 gene (11A) and 523 gene (11B) liquid biopsy panel-enriched sequencing assays.

Figure 12A illustrates performance metrics for a tumor-informed ctDNA fraction estimate. The LOB(95) and LOB(99) were 0%. LOD hit-rate was 100% at the lowest titer evaluated in each assay.

Figures 12B and 12C illustrate the 100x bootstrapped LOB calculated from presumed healthy subjects yields a variant count distribution similar to that observed in 105 gene (12A) and 523 gene (12B) liquid biopsy panel-enriched sequencing assays.

Figures 13A and 13B illustrate the LOD calculated from titered Seraseq ctDNA reference material has low inter-titer variability and strong linear relationship, when using 105 gene (13A) and 523 gene (13B) liquid biopsy panel-enriched sequencing assays.

Figures 14A, 14B, 14C, 14D, 14E, 14F, 14G, 14H, 14I 14J, 14K, 14L, and 14M collectively illustrate example nucleic acids targeted for enrichment and variant detection using one or more probes, in accordance with some embodiments of the present disclosure.

Figures 15A, 15B, and 15C collectively illustrate example nucleic acids targeted for enrichment and variant detection using one or more probes, in accordance with some embodiments of the present disclosure.

Like reference numerals refer to corresponding parts throughout the several views of the drawings.

## DETAILED DESCRIPTION

*Introduction.*

As described above, conventional liquid biopsy assays do not provide accurate determination of circulating tumor fraction estimates (ctFEs). For example, while low-pass, whole-genome sequencing can be used to estimate tumor fractions, somatic variant sequences are poorly identified from low-pass, whole genome sequencing data, particularly from samples having low tumor fractions. Accordingly, conventional liquid biopsy assays typically use targeted-panel sequencing in order to achieve higher sequence coverage required to identify somatic variants present at low levels within the sample. However, targeted-panel sequencing data may not span a large enough portion of the genome to accurately estimate tumor fraction. Rather, tumor fraction estimates obtained using variant allele fractions (VAFs) in targeted-panel sequencing data are noisy, due to variant tissue source and capture bias.

Altogether, these factors result in highly variable concentrations of ctDNA-from patient to patient and possibly from locus to locus-that confound accurate measurement of disease indicators and actionable genomic alterations. Further, the quantity and quality of cfDNA obtained from liquid biopsy samples are highly dependent on the particular methodology for collecting the samples, storing the samples, sequencing the samples, and standardizing the sequencing data. Accurate ctFEs provide several benefits to liquid biopsy applications, including classification of variants as somatic or germline, detection of clinically relevant copy number variations, and/or use of ctFEs as biomarkers.

For example, because up to 30% of breast cancer patients and up to 55% of lung cancer patients relapse after initial treatment, as well as a significant portion of patients in other cancer cohorts, the ability to detect metastasis and disease recurrence earlier in these patients could significantly improve patient outcomes. See, Colleoni et al., 2016, "Annual Hazard Rates of Recurrence for Breast Cancer During 24 Years of Follow-Up: Results From the International Breast Cancer Study Group Trials I to V," J Clin Oncol, (34), pg. 927; Yates et al., 2017, "Genomic Evolution of Breast Cancer Metastasis and Relapse," Cancer Cell, (32), pg. 169; Uramoto et al., 2014, "Recurrence after surgery in patients with NSCLC," Transl Lung Cancer Res, (3), pg. 242; Taunk et al., 2017, "Immunotherapy and radiation therapy for operable early stage and locally advanced non-small cell lung cancer," Transl Lung Cancer Res, (6), pg. 178. Indeed, recent retrospective and prospective studies have shown ctDNA after completion of treatment or surgery can act as a biomarker for disease recurrence in many cancer types, including breast cancer, lung cancer, melanoma, bladder cancer, and colon cancer. See, Coombes et al., 2019, "Personalized Detection of Circulating Tumor DNA Antedates Breast Cancer Metastatic Recurrence," Clin Cancer Res, (25), pg. 4255; Tie et al., 2019, "Circulating Tumor DNA Analyses as Markers of Recurrence Risk and Benefit of Adjuvant Therapy for Stage III Colon Cancer," JAMA Oncol, print; McEvoy et al., 2019, "Monitoring melanoma recurrence with circulating tumor DNA: a proof of concept from three case studies," Oncotarget, (10), pg. 113; Christensen et al. 2019, "Early Detection of Metastatic Relapse and Monitoring of Therapeutic Efficacy by Ultra-Deep Sequencing of Plasma Cell-Free DNA in Patients With Urothelial Bladder Carcinoma," J Clin Oncol, (37), pg. 1547; Isaksson et al., 2019, "Pre-operative plasma cell-free circulating tumor DNA and serum protein tumor markers as predictors of lung adenocarcinoma recurrence," Acta Oncol, (58), pg. 1079. Higher ctFEs are associated with disease progression at radiographic evaluation and an increased metastatic lesion count.

Furthermore, ctFEs correlate with important clinical outcomes, and provide a minimally invasive method to monitor patients for response to therapy, disease relapse, and disease progression. However, conventional methodologies used for determining ctFEs in liquid biopsy samples rely on low-pass, whole-genome sequencing, which often cannot also be used for variant detection (see, for example, Adalsteinsson et al., "Scalable whole-exome sequencing of cell-free DNA reveals high concordance with metastatic tumors," (2017) Nature Communications Nov 6;8(1):1324, doi:10.1038/s41467-017-00965-y; and ichorCNA, the Broad Institute, available on the internet at github.com/broadinstitute/ichorCNA). Other traditional approaches use variant allele fractions (VAFs) to estimate tumor fraction, but such approaches are confounded by variant tissue source and capture bias resulting in high levels of noise. Additionally, conventional methodologies for determining tumor purity estimates in solid tumor biopsy samples rely solely on on-target probe regions, which often cannot be used in conjunction with targeted gene panels containing small numbers of genes.

Advantageously, the present disclosure provides a sensitive and specific tumor-informed, non-bespoke approach for estimating ctDNA TF, in which all patients' samples are analyzed by the same panel/assay, as opposed to bespoke methods in which each patient has a customized set of probes, usually based on the patient's previous sequencing results.

Advantageously, linearity improves with increased panel size and increased variant number, as indicated by the data presented in the Examples herein. These results suggest that a tumor-informed ctDNA TF can be utilized to improve the sensitivity of existing methods for estimating tumor fraction to help in treatment decisions using tissue and liquid comprehensive NGS genomic profiling.

An improved method for obtaining accurate circulating tumor fraction estimates provide several benefits to liquid biopsies. Advantageously, more reliable ctFEs improves the classification accuracy of detected variants as somatic or germline variants (e.g., any variant detected at or below the ctFE can be classified as a somatic variant with high confidence). In addition, accurate ctFEs can greatly improve the sensitivity of detection of clinically relevant copy number variations, including integer copy number calling. Furthermore, in some embodiments, ctFEs are used as biomarkers for tumor burden, metastases, disease progression, or treatment resistance. For example, ctFEs have been shown to correlate with tumor volumes and vary in response to treatment.

As a result, the methods and systems disclosed herein provide a sensitive, cost-effective, and minimally invasive method to monitor patients for response to therapy, disease burden, relapse, progression, and/or emerging resistance mutations, which can translate into better care for patients. When used as part of the course of care, serial ctFE monitoring can predict objective measures of progression in at-risk individuals. Due to cost and convenience of sampling, the methods and systems disclosed herein can be applied at shorter time intervals than radiographic methods and can allow for more timely intervention in the case of disease progression.

Additionally, the methods and systems disclosed herein provide benefits to clinicians by generating more accurate variant calls and/or informative ctFE biomarkers that can aid in the prediction of clinical outcomes in patients and/or the selection of appropriate treatment plans.

The identification of actionable genomic alterations in a patient's cancer genome is a difficult and computationally demanding problem. For instance, the determination of various prognostic metrics useful for precision oncology, such as variant allelic ratio, copy number variation, tumor mutational burden, microsatellite instability status, *etc.,* requires analysis of hundreds of millions to billions, of sequenced nucleic acid bases. An example of a typical bioinformatics pipeline established for this purpose includes at least five stages of analysis: assessment of the quality of raw next generation sequencing data, generation of collapsed nucleic acid fragment sequences and alignment of such sequences to a reference genome, detection of structural variants in the aligned sequence data, annotation of identified variants, and visualization of the data. Each one of these procedures is computationally taxing in its own right.

For instance, the overall temporal and spatial computation complexity of simple global and local pairwise sequence alignment algorithms are quadratic in nature (e.g., second order problems), that increase rapidly as a function of the size of the nucleic acid sequences (n and m) being compared. Specifically, the temporal and spatial complexities of these sequence alignment algorithms can be estimated as O(mn), where O is the upper bound on the asymptotic growth rate of the algorithm, n is the number of bases in the first nucleic acid sequence, and m is the number of bases in the second nucleic acid sequence. See, Baichoo and Ouzounis, BioSystems, 156-157:72-85 (2017), the content of which is hereby incorporated by reference, in its entirety, for all purposes. Given that the human genome contains more than 3 billion bases, these alignment algorithms are extremely computationally taxing, especially when used to analyze next generation sequencing (NGS) data, which can generate more than 3 billion sequence reads per reaction.

This is particularly true when performed in the context of a liquid biopsy assay, because liquid biopsy samples contain a complex mixture of short DNA fragments originating from many different germline (e.g., healthy) and diseased (e.g., cancerous) tissues. Thus, the cellular origins of the sequence reads are unknown, and the sequence signals originating from cancerous cells, which may constitute multiple sub-clonal populations, must be computationally deconvoluted from signals originating from germline and hematopoietic origins, in order to provide relevant information about the subject's cancer. Thus, in addition to the computationally taxing processes required to align sequence reads to a human genome, there is a computation problem of determining whether a particular abnormal signal, *e.g.,* one or more sequence reads corresponding to a genomic alteration, (i) is not an artifact, and (ii) originated from a cancerous source in the subject. This is increasingly difficult during the early stages of cancer-when treatment is presumably most effective-when only small amounts of ctDNA are diluted by germline and hematopoietic DNA.

Advantageously, the present disclosure provides various systems and methods that improve the computational elucidation of actionable genomic alterations from a liquid biopsy sample of a cancer patient. Specifically, the present disclosure improves upon the accuracy of circulating tumor fractions estimated from targeted-panel sequencing. Moreover, because the methods described herein eliminate the need to process data from two different sequencing reactions, the disclosure lowers the computational budget for accurately estimating circulating tumor fractions and identifying actionable variants. As described above, the disclosed methods and systems are necessarily computer-implemented due to their complexity and heavy computational requirements, and thus solve a problem in the computing art.

The methods and systems described herein also improve precision oncology methods for assigning and/or administering treatment because of the improved accuracy of circulating tumor fraction estimations. Accurate ctFEs can be reported as biomarkers and/or used in downstream analysis for identification of therapeutically actionable variants to be included in a clinical report for patient and/or clinician review. Additionally, ctFEs and any therapeutically actionable variants identified using ctFEs can be matched with appropriate therapies and/or clinical trials, allowing for more accurate assignment of treatments. The improved accuracy of biomarker detection increases the chance of efficacy and reduces the risk of patients undergoing unnecessary or potentially harmful regimens due to misdiagnoses.

*Definitions.*

As used herein, the term "subject" refers to any living or non-living organism including, but not limited to, a human (e.g., a male human, female human, fetus, pregnant female, child, or the like), a non-human mammal, or a non-human animal. Any human or non-human animal can serve as a subject, including but not limited to mammal, reptile, avian, amphibian, fish, ungulate, ruminant, bovine (e.g., cattle), equine (e.g., horse), caprine and ovine (e.g., sheep, goat), swine (e.g., pig), camelid (e.g., camel, llama, alpaca), monkey, ape (e.g., gorilla, chimpanzee), ursid (e.g., bear), poultry, dog, cat, mouse, rat, fish, dolphin, whale and shark. In some embodiments, a subject is a male or female of any age (e.g., a man, a woman, or a child).

As used herein, the terms "control," "control sample," "reference," "reference sample," "normal," and "normal sample" describe a sample from a non-diseased tissue. In some embodiments, such a sample is from a subject that does not have a particular condition (e.g., cancer). In other embodiments, such a sample is an internal control from a subject, e.g., who may or may not have the particular disease (e.g., cancer), but is from a healthy tissue of the subject. For example, where a liquid or solid tumor sample is obtained from a subject with cancer, an internal control sample may be obtained from a healthy tissue of the subject, e.g., a white blood cell sample from a subject without a blood cancer or a solid germline tissue sample from the subject. Accordingly, a reference sample can be obtained from the subject or from a database, e.g., from a second subject who does not have the particular disease (e.g., cancer).

As used herein the term "cancer," "cancerous tissue," or "tumor" refers to an abnormal mass of tissue in which the growth of the mass surpasses, and is not coordinated with, the growth of normal tissue, including both solid masses (*e.g.,* as in a solid tumor) or fluid masses (e.g., as in a hematological cancer). A cancer or tumor can be defined as "benign" or "malignant" depending on the following characteristics: degree of cellular differentiation including morphology and functionality, rate of growth, local invasion and metastasis. A "benign" tumor can be well differentiated, have characteristically slower growth than a malignant tumor and remain localized to the site of origin. In addition, in some cases a benign tumor does not have the capacity to infiltrate, invade or metastasize to distant sites. A "malignant" tumor can be a poorly differentiated (anaplasia), have characteristically rapid growth accompanied by progressive infiltration, invasion, and destruction of the surrounding tissue. Furthermore, a malignant tumor can have the capacity to metastasize to distant sites. Accordingly, a cancer cell is a cell found within the abnormal mass of tissue whose growth is not coordinated with the growth of normal tissue. Accordingly, a "tumor sample" refers to a biological sample obtained or derived from a tumor of a subject, as described herein.

Non-limiting examples of cancer types include ovarian cancer, cervical cancer, uveal melanoma, colorectal cancer, chromophobe renal cell carcinoma, liver cancer, endocrine tumor, oropharyngeal cancer, retinoblastoma, biliary cancer, adrenal cancer, neural cancer, neuroblastoma, basal cell carcinoma, brain cancer, breast cancer, non-clear cell renal cell carcinoma, glioblastoma, glioma, kidney cancer, gastrointestinal stromal tumor, medulloblastoma, bladder cancer, gastric cancer, bone cancer, non-small cell lung cancer, thymoma, prostate cancer, clear cell renal cell carcinoma, skin cancer, thyroid cancer, sarcoma, testicular cancer, head and neck cancer *(e.g.,* head and neck squamous cell carcinoma), meningioma, peritoneal cancer, endometrial cancer, pancreatic cancer, mesothelioma, esophageal cancer, small cell lung cancer, Her2 negative breast cancer, ovarian serous carcinoma, HR+ breast cancer, uterine serous carcinoma, uterine corpus endometrial carcinoma, gastroesophageal junction adenocarcinoma, gallbladder cancer, chordoma, and papillary renal cell carcinoma.

As used herein, the terms "cancer state" or "cancer condition" refer to a characteristic of a cancer patient's condition, *e.g.,* a diagnostic status, a type of cancer, a location of cancer, a primary origin of a cancer, a cancer stage, a cancer prognosis, and/or one or more additional characteristics of a cancer (*e.g.,* tumor characteristics such as morphology, heterogeneity, size, *etc.*). In some embodiments, one or more additional personal characteristics of the subject are used further describe the cancer state or cancer condition of the subject, *e.g.,* age, gender, weight, race, personal habits (*e.g.,* smoking, drinking, diet), other pertinent medical conditions (*e.g.,* high blood pressure, dry skin, other diseases), current medications, allergies, pertinent medical history, current side effects of cancer treatments and other medications, *etc.*

As used herein, the term "liquid biopsy" sample refers to a liquid sample obtained from a subject that includes cell-free DNA. Examples of liquid biopsy samples include, but are not limited to, blood, whole blood, plasma, serum, urine, cerebrospinal fluid, fecal material, saliva, sweat, tears, pleural fluid, pericardial fluid, or peritoneal fluid of the subject. In some embodiments, a liquid biopsy sample is a cell-free sample, *e.g.,* a cell free blood sample. In some embodiments, a liquid biopsy sample is obtained from a subject with cancer. In some embodiments, a liquid biopsy sample is collected from a subject with an unknown cancer status, *e.g.,* for use in determining a cancer status of the subject. Likewise, in some embodiments, a liquid biopsy is collected from a subject with a non-cancerous disorder, *e.g.,* a cardiovascular disease. In some embodiments, a liquid biopsy is collected from a subject with an unknown status for a non-cancerous disorder, *e.g.,* for use in determining a non-cancerous disorder status of the subject.

As used herein, the term "cell-free DNA" and "cfDNA" interchangeably refer to DNA fragments that circulate in a subject's body (*e.g.,* bloodstream) and originate from one or more healthy cells and/or from one or more cancer cells. These DNA molecules are found outside cells, in bodily fluids such as blood, whole blood, plasma, serum, urine, cerebrospinal fluid, fecal material, saliva, sweat, sweat, tears, pleural fluid, pericardial fluid, or peritoneal fluid of a subject, and are believed to be fragments of genomic DNA expelled from healthy and/or cancerous cells, *e.g.,* upon apoptosis and lysis of the cellular envelope.

As used herein, the term "locus" refers to a position (*e.g.,* a site) within a genome, *e.g.,* on a particular chromosome. In some embodiments, a locus refers to a single nucleotide position, on a particular chromosome, within a genome. In some embodiments, a locus refers to a group of nucleotide positions within a genome. In some instances, a locus is defined by a mutation *(e.g.,* substitution, insertion, deletion, inversion, or translocation) of consecutive nucleotides within a cancer genome. In some instances, a locus is defined by a gene, a sub-genic structure *(e.g.,* a regulatory element, exon, intron, or combination thereof), or a predefined span of a chromosome. Because normal mammalian cells have diploid genomes, a normal mammalian genome *(e.g.,* a human genome) will generally have two copies of every locus in the genome, or at least two copies of every locus located on the autosomal chromosomes, *e.g.,* one copy on the maternal autosomal chromosome and one copy on the paternal autosomal chromosome.

As used herein, the term "allele" refers to a particular sequence of one or more nucleotides at a chromosomal locus. In a haploid organism, the subject has one allele at every chromosomal locus. In a diploid organism, the subject has two alleles at every chromosomal locus.

As used herein, the term "base pair" or "bp" refers to a unit consisting of two nucleobases bound to each other by hydrogen bonds. Generally, the size of an organism's genome is measured in base pairs because DNA is typically double stranded. However, some viruses have single-stranded DNA or RNA genomes.

As used herein, the terms "genomic alteration," "mutation," and "variant" refer to a detectable change in the genetic material of one or more cells. A genomic alteration, mutation, or variant can refer to various type of changes in the genetic material of a cell, including changes in the primary genome sequence at single or multiple nucleotide positions, *e.g.,* a single nucleotide variant (SNV), a multi-nucleotide variant (MNV), an indel (*e.g.,* an insertion or deletion of nucleotides), a DNA rearrangement (*e.g.,* an inversion or translocation of a portion of a chromosome or chromosomes), a variation in the copy number of a locus (*e.g.,* an exon, gene, or a large span of a chromosome) (CNV), a partial or complete change in the ploidy of the cell, as well as in changes in the epigenetic information of a genome, such as altered DNA methylation patterns. In some embodiments, a mutation is a change in the genetic information of the cell relative to a particular reference genome, or one or more 'normal' alleles found in the population of the species of the subject. For instance, mutations can be found in both germline cells (e.g., non-cancerous, 'normal' cells) of a subject and in abnormal cells (e.g., pre-cancerous or cancerous cells) of the subject. As such, a mutation in a germline of the subject (e.g., which is found in substantially all 'normal cells' in the subject) is identified relative to a reference genome for the species of the subject. However, many loci of a reference genome of a species are associated with several variant alleles that are significantly represented in the population of the subject and are not associated with a diseased state, *e.g.,* such that they would not be considered "mutations." By contrast, in some embodiments, a mutation in a cancerous cell of a subject can be identified relative to either a reference genome of the subject or to the subject's own germline genome. In certain instances, identification of both types of variants can be informative. For instance, in some instances, a mutation that is present in both the cancer genome of the subject and the germline of the subject is informative for precision oncology when the mutation is a so-called 'driver mutation," which contributes to the initiation and/or development of a cancer. However, in other instances, a mutation that is present in both the cancer genome of the subject and the germline of the subject is not informative for precision oncology, *e.g.,* when the mutation is a so-called 'passenger mutation," which does not contribute to the initiation and/or development of the cancer. Likewise, in some instances, a mutation that is present in the cancer genome of the subject but not the germline of the subject is informative for precision oncology, *e.g.,* where the mutation is a driver mutation and/or the mutation facilitates a therapeutic approach, *e.g.,* by differentiating cancer cells from normal cells in a therapeutically actionable way. However, in some instances, a mutation that is present in the cancer genome but not the germline of a subject is not informative for precision oncology, *e.g.,* where the mutation is a passenger mutation and/or where the mutation fails to differentiate the cancer cell from a germline cell in a therapeutically actionable way.

As used herein, the term "reference allele" refers to the sequence of one or more nucleotides at a chromosomal locus that is either the predominant allele represented at that chromosomal locus within the population of the species (*e.g.,* the "wild-type" sequence), or an allele that is predefined within a reference genome for the species.

As used herein, the term "variant allele" refers to a sequence of one or more nucleotides at a chromosomal locus that is either not the predominant allele represented at that chromosomal locus within the population of the species (*e.g.,* not the "wild-type" sequence), or not an allele that is predefined within a reference sequence construct (*e.g.,* a reference genome or set of reference genomes) for the species. In some instances, sequence isoforms found within the population of a species that do not affect a change in a protein encoded by the genome, or that result in an amino acid substitution that does not substantially affect the function of an encoded protein, are not variant alleles.

As used herein, the term "variant allele fraction," "VAF," "allelic fraction," or "AF" refers to the number of times a variant or mutant allele was observed (e.g., a number of reads supporting a candidate variant allele) divided by the total number of times the position was sequenced (*e.g.,* a total number of reads covering a candidate locus).

As used herein, the term "germline variants" refers to genetic variants inherited from maternal and paternal DNA. Germline variants may be determined through a matched tumor-normal calling pipeline.

As used herein, the term "somatic variants" refers to variants arising as a result of dysregulated cellular processes associated with neoplastic cells, *e.g.,* a mutation. Somatic variants may be detected via subtraction from a matched normal sample.

As used herein, the term "single nucleotide variant" or "SNV" refers to a substitution of one nucleotide to a different nucleotide at a position (*e.g.,* site) of a nucleotide sequence, *e.g.,* a sequence read from an individual. A substitution from a first nucleobase X to a second nucleobase Y may be denoted as "X>Y." For example, a cytosine to thymine SNV may be denoted as "C>T."

As used herein, the term "insertions and deletions" or "indels" refers to a variant resulting from the gain or loss of DNA base pairs within an analyzed region.

As used herein, the term "copy number variation" or "CNV" refers to the process by which large structural changes in a genome associated with tumor aneuploidy and other dysregulated repair systems are detected. These processes are used to detect large scale insertions or deletions of entire genomic regions. CNV is defined as structural insertions or deletions greater than a certain base pair ("bp") in size, such as 500 bp.

As used herein, the term "gene fusion" refers to the product of large-scale chromosomal aberrations resulting in the creation of a chimeric protein. These expressed products can be non-functional, or they can be highly over or underactive. This can cause deleterious effects in cancer such as hyper-proliferative or anti-apoptotic phenotypes.

As used herein, the term "loss of heterozygosity" refers to the loss of one copy of a segment (e.g., including part or all of one or more genes) of the genome of a diploid subject (*e.g.,* a human) or loss of one copy of a sequence encoding a functional gene product in the genome of the diploid subject, in a tissue, *e.g.,* a cancerous tissue, of the subject. As used herein, when referring to a metric representing loss of heterozygosity across the entire genome of the subject, loss of heterozygosity is caused by the loss of one copy of various segments in the genome of the subject. Loss of heterozygosity across the entire genome may be estimated without sequencing the entire genome of a subject, and such methods for such estimations based on gene panel targeting-based sequencing methodologies are described in the art. Accordingly, in some embodiments, a metric representing loss of heterozygosity across the entire genome of a tissue of a subject is represented as a single value, *e.g.,* a percentage or fraction of the genome. In some cases, a tumor is composed of various sub-clonal populations, each of which may have a different degree of loss of heterozygosity across their respective genomes. Accordingly, in some embodiments, loss of heterozygosity across the entire genome of a cancerous tissue refers to an average loss of heterozygosity across a heterogeneous tumor population. As used herein, when referring to a metric for loss of heterozygosity in a particular gene, *e.g.,* a DNA repair protein such as a protein involved in the homologous DNA recombination pathway (e.g., BRCA1 or BRCA2), loss of heterozygosity refers to complete or partial loss of one copy of the gene encoding the protein in the genome of the tissue and/or a mutation in one copy of the gene that prevents translation of a full-length gene product, *e.g.,* a frameshift or truncating (creating a premature stop codon in the gene) mutation in the gene of interest. In some cases, a tumor is composed of various sub-clonal populations, each of which may have a different mutational status in a gene of interest. Accordingly, in some embodiments, loss of heterozygosity for a particular gene of interest is represented by an average value for loss of heterozygosity for the gene across all sequenced sub-clonal populations of the cancerous tissue. In other embodiments, loss of heterozygosity for a particular gene of interest is represented by a count of the number of unique incidences of loss of heterozygosity in the gene of interest across all sequenced sub-clonal populations of the cancerous tissue *(e.g.,* the number of unique frame-shift and/or truncating mutations in the gene identified in the sequencing data).

As used herein, the term "microsatellites" refers to short, repeated sequences of DNA. The smallest nucleotide repeated unit of a microsatellite is referred to as the "repeated unit" or "repeat unit." In some embodiments, the stability of a microsatellite locus is evaluated by comparing some metric of the distribution of the number of repeated units at a microsatellite locus to a reference number or distribution.

As used herein, the term "microsatellite instability" or "MSI" refers to a genetic hypermutability condition associated with various cancers that results from impaired DNA mismatch repair (MMR) in a subject. Among other phenotypes, MSI causes changes in the size of microsatellite loci, *e.g.,* a change in the number of repeated units at microsatellite loci, during DNA replication. Accordingly, the size of microsatellite repeats is varied in MSI cancers as compared to the size of the corresponding microsatellite repeats in the germline of a cancer subject. The term "Microsatellite Instability-High" or "MSI-H" refers to a state of a cancer (*e.g.,* a tumor) that has a significant MMR defect, resulting in microsatellite loci with significantly different lengths than the corresponding microsatellite loci in normal cells of the same individual. The term "Microsatellite Stable" or "MSS" refers to a state of a cancer (*e.g.,* a tumor) without significant MMR defects, such that there is no significant difference between the lengths of the microsatellite loci in cancerous cells and the lengths of the corresponding microsatellite loci in normal (*e.g.,* non-cancerous) cells in the same individual. The term "Microsatellite Equivocal" or "MSE" refers to a state of a cancer (*e.g.,* a tumor) having an intermediate microsatellite length phenotype, that cannot be clearly classified as MSI-H or MSS based on statistical cutoffs used to define those two categories.

As used herein, the term "gene product" refers to an RNA (*e.g.,* mRNA or miRNA) or protein molecule transcribed or translated from a particular genomic locus, *e.g.,* a particular gene. The genomic locus can be identified using a gene name, a chromosomal location, or any other genetic mapping metric.

As used herein, the terms "expression level," "abundance level," or simply "abundance" refers to an amount of a gene product, (an RNA species, *e.g.,* mRNA or miRNA, or protein molecule) transcribed or translated by a cell, or an average amount of a gene product transcribed or translated across multiple cells. When referring to mRNA or protein expression, the term generally refers to the amount of any RNA or protein species corresponding to a particular genomic locus, *e.g.,* a particular gene. However, in some embodiments, an expression level can refer to the amount of a particular isoform of an mRNA or protein corresponding to a particular gene that gives rise to multiple mRNA or protein isoforms. The genomic locus can be identified using a gene name, a chromosomal location, or any other genetic mapping metric.

As used herein, the term "ratio" refers to any comparison of a first metric X, or a first mathematical transformation thereof X' (e.g., measurement of a number of units of a genomic sequence in a first one or more biological samples or a first mathematical transformation thereof) to another metric Y or a second mathematical transformation thereof Y' (e.g., the number of units of a respective genomic sequence in a second one or more biological samples or a second mathematical transformation thereof) expressed as $X/Y$, $Y/X$, $\log_N(X/Y)$, $\log_N(Y/X)$, $X'/Y$, $Y/X'$, $\log_N(X'/Y)$, or $\log_N(Y/X')$, $X/Y'$, $Y'/X$, $\log_N(X/Y')$, $\log_N(Y'/X)$, $X'/Y'$, $Y'/X'$, $\log_N(X'/Y')$, or $\log_N(Y'/X')$, where N is any real number greater than 1 and where example mathematical transformations of X and Y include, but are not limited to. raising X or Y to a power Z, multiplying X or Y by a constant Q, where Z and Q are any real numbers, and/or taking an M based logarithm of X and/or Y, where M is a real number greater than 1. In one non-limiting example, X is transformed to X' prior to ratio calculation by raising X by the power of two ($X^2$) and Y is transformed to Y' prior to ratio calculation by raising Y by the power of 3.2 ($Y^{3.2}$) and the ratio of X and Y is computed as $\log_2(X'/Y')$.

As used herein, the term "relative abundance" refers to a ratio of a first amount of a compound measured in a sample, *e.g.,* a gene product (an RNA species, *e.g.,* mRNA or miRNA, or protein molecule) or nucleic acid fragments having a particular characteristic (*e.g.*, aligning to a particular locus or encompassing a particular allele), to a second amount of a compound measured in a second sample. In some embodiments, relative abundance refers to a ratio of an amount of species of a compound to a total amount of the compound in the same sample. For instance, a ratio of the amount of mRNA transcripts encoding a particular gene in a sample (*e.g.*, aligning to a particular region of the exome) to the total amount of mRNA transcripts in the sample. In other embodiments, relative abundance refers to a ratio of an amount of a compound or species of a compound in a first sample to an amount of the compound of the species of the compound in a second sample. For instance, a ratio of a normalized amount of mRNA transcripts encoding a particular gene in a first sample to a normalized amount of mRNA transcripts encoding the particular gene in a second and/or reference sample.

As used herein, the terms "sequencing," "sequence determination," and the like refer to any biochemical processes that may be used to determine the order of biological macromolecules such as nucleic acids or proteins. For example, sequencing data can include all or a portion of the nucleotide bases in a nucleic acid molecule such as an mRNA transcript or a genomic locus.

As used herein, the term "genetic sequence" refers to a recordation of a series of nucleotides present in a subject's RNA or DNA as determined by sequencing of nucleic acids from the subject.

As used herein, the term "sequence reads" or "reads" refers to nucleotide sequences produced by any nucleic acid sequencing process described herein or known in the art. Reads can be generated from one end of nucleic acid fragments ("single-end reads") or from both ends of nucleic acid fragments (*e.g.*, paired-end reads, double-end reads). The length of the sequence read is often associated with the particular sequencing technology. High-throughput methods, for example, provide sequence reads that can vary in size from tens to hundreds of base pairs (bp). In some embodiments, the sequence reads are of a mean, median or average length of about 15 bp to 900 bp long (*e.g.,* about 20 bp, about 25 bp, about 30 bp, about 35 bp, about 40 bp, about 45 bp, about 50 bp, about 55 bp, about 60 bp, about 65 bp, about 70 bp, about 75 bp, about 80 bp, about 85 bp, about 90 bp, about 95 bp, about 100 bp, about 110 bp, about 120 bp, about 130, about 140 bp, about 150 bp, about 200 bp, about 250 bp, about 300 bp, about 350 bp, about 400 bp, about 450 bp, or about 500 bp. In some embodiments, the sequence reads are of a mean, median or average length of about 1000 bp, 2000 bp, 5000 bp, 10,000 bp, or 50,000 bp or more. NANOPORE® sequencing, for example, can provide sequence reads that can vary in size from tens to hundreds to thousands of base pairs. ILLUMINA® parallel sequencing, for example, can provide sequence reads that do not vary as much, for example, most of the sequence reads can be smaller than 200 bp. A sequence read (or sequencing read) can refer to sequence information corresponding to a nucleic acid molecule (*e.g.,* a string of nucleotides). For example, a sequence read can correspond to a string of nucleotides (e.g., about 20 to about 150) from part of a nucleic acid fragment, can correspond to a string of nucleotides at one or both ends of a nucleic acid fragment, or can correspond to nucleotides of the entire nucleic acid fragment. A sequence read can be obtained in a variety of ways, e.g., using sequencing techniques or using probes, *e.g.,* in hybridization arrays or capture probes, or amplification techniques, such as the polymerase chain reaction (PCR) or linear amplification using a single primer or isothermal amplification.

As used herein, the term "read segment" refers to any form of nucleotide sequence read including the raw sequence reads obtained directly from a nucleic acid sequencing technique or from a sequence derived therefrom, e.g., an aligned sequence read, a collapsed sequence read, or a stitched sequence read.

As used herein, the term "read count" refers to the total number of nucleic acid reads generated, which may or may not be equivalent to the number of nucleic acid molecules generated, during a nucleic acid sequencing reaction.

As used herein, the term "read-depth," "sequencing depth," or "depth" can refer to a total number of unique nucleic acid fragments encompassing a particular locus or region of the genome of a subject that are sequenced in a particular sequencing reaction. Sequencing depth can be expressed as "Yx", *e.g.,* 50x, 100x, *etc.,* where "Y" refers to the number of unique nucleic acid fragments encompassing a particular locus that are sequenced in a sequencing reaction. In such a case, Y is necessarily an integer, because it represents the actual sequencing depth for a particular locus. Alternatively, read-depth, sequencing depth, or depth can refer to a measure of central tendency *(e.g.,* a mean or mode) of the number of unique nucleic acid fragments that encompass one of a plurality of loci or regions of the genome of a subject that are sequenced in a particular sequencing reaction. For example, in some embodiments, sequencing depth refers to the average depth of every locus across an arm of a chromosome, a targeted sequencing panel, an exome, or an entire genome. In such case, Y may be expressed as a fraction or a decimal, because it refers to an average coverage across a plurality of loci. When a mean depth is recited, the actual depth for any particular locus may be different than the overall recited depth. Metrics can be determined that provide a range of sequencing depths in which a defined percentage of the total number of loci fall. For instance, a range of sequencing depths within which 90% or 95%, or 99% of the loci fall. As understood by the skilled artisan, different sequencing technologies provide different sequencing depths. For instance, low-pass whole genome sequencing can refer to technologies that provide a sequencing depth of less than 5x, less than 4x, less than 3x, or less than 2x, *e.g.,* from about 0.5x to about 3x.

As used herein, the term "sequencing breadth" refers to what fraction of a particular reference exome (e.g., human reference exome), a particular reference genome (e.g., human reference genome), or part of the exome or genome has been analyzed. Sequencing breadth can be expressed as a fraction, a decimal, or a percentage, and is generally calculated as (the number of loci analyzed / the total number of loci in a reference exome or reference genome). The denominator of the fraction can be a repeat-masked genome, and thus 100% can correspond to all of the reference genome minus the masked parts. A repeat-masked exome or genome can refer to an exome or genome in which sequence repeats are masked (e.g., sequence reads align to unmasked portions of the exome or genome). In some embodiments, any part of an exome or genome can be masked and, thus, sequencing breadth can be evaluated for any desired portion of a reference exome or genome. In some embodiments, "broad sequencing" refers to sequencing/analysis of at least 0.1% of an exome or genome.

As used herein, the terms "sequence ratio" and "coverage ratio" interchangeably refer to any measurement of a number of units of a genomic sequence in a first one or more biological samples *(e.g.,* a test and/or tumor sample) compared to the number of units of the respective genomic sequence in a second one or more biological samples *(e.g.,* a reference and/or control sample). In some embodiments, a sequence ratio is a copy ratio, a $\log_2$-transformed copy ratio *(e.g.,* $\log_2$ copy ratio), a coverage ratio, a base fraction, an allele fraction *(e.g.,* a variant allele fraction), and/or a tumor ploidy. In some embodiments sequence ratio is a $\log_N$-transformed copy ratio, where N is any real number greater than 1.

As used herein, the term "sequencing probe" refers to a molecule that binds to a nucleic acid with affinity that is based on the expected nucleotide sequence of the RNA or DNA present at that locus.

As used herein, the term "targeted panel" or "targeted gene panel" refers to a combination of probes for sequencing (e.g., by next-generation sequencing) nucleic acids present in a biological sample from a subject *(e.g.,* a tumor sample, liquid biopsy sample, germline tissue sample, white blood cell sample, or tumor or tissue organoid sample), selected to map to one or more loci of interest on one or more chromosomes. An example set of loci/genes useful for precision oncology, e.g., via solid or liquid biopsy assay, that can be analyzed using a targeted panel is described in Table 1. Another example set of loci/genes useful for precision oncology, *e.g.,* via solid or liquid biopsy assay, that can be analyzed using a targeted panel is described in Table 2. In some embodiments, in addition to loci that are informative for precision oncology, a targeted panel includes one or more probes for sequencing one or more of a loci associated with a different medical condition, a loci used for internal control purposes, or a loci from a pathogenic organism (e.g., an oncogenic pathogen).

As used herein, the term, "reference exome" refers to any sequenced or otherwise characterized exome, whether partial or complete, of any tissue from any organism or pathogen that may be used to reference identified sequences from a subject. Typically, a reference exome will be derived from a subject of the same species as the subject whose sequences are being evaluated. Example reference exomes used for human subjects as well as many other organisms are provided in the on-line genome browser hosted by the National Center for Biotechnology Information ("NCBI"). An "exome" refers to the complete transcriptional profile of an organism or pathogen, expressed in nucleic acid sequences. As used herein, a reference sequence or reference exome often is an assembled or partially assembled exomic sequence from an individual or multiple individuals. In some embodiments, a reference exome is an assembled or partially assembled exomic sequence from one or more human individuals. The reference exome can be viewed as a representative example of a species' set of expressed genes. In some embodiments, a reference exome comprises sequences assigned to chromosomes.

As used herein, the term "reference genome" refers to any sequenced or otherwise characterized genome, whether partial or complete, of any organism or pathogen that may be used to reference identified sequences from a subject. Typically, a reference genome will be derived from a subject of the same species as the subject whose sequences are being evaluated. Exemplary reference genomes used for human subjects as well as many other organisms are provided in the on-line genome browser hosted by the National Center for Biotechnology Information ("NCBI") or the University of California, Santa Cruz (UCSC). A "genome" refers to the complete genetic information of an organism or pathogen, expressed in nucleic acid sequences. As used herein, a reference sequence or reference genome often is an assembled or partially assembled genomic sequence from an individual or multiple individuals. In some embodiments, a reference genome is an assembled or partially assembled genomic sequence from one or more human individuals. The reference genome can be viewed as a representative example of a species' set of genes. In some embodiments, a reference genome comprises sequences assigned to chromosomes. Exemplary human reference genomes include but are not limited to NCBI build 34 (UCSC equivalent: hg16), NCBI build 35 (UCSC equivalent: hg17), NCBI build 36.1 (UCSC equivalent: hg18), GRCh37 (UCSC equivalent: hg19), and GRCh38 (UCSC equivalent: hg38). For a haploid genome, there can be only one nucleotide at each locus. For a diploid genome, heterozygous loci can be identified; each heterozygous locus can have two alleles, where either allele can allow a match for alignment to the locus.

As used herein, the term "bioinformatics pipeline" refers to a series of processing stages used to determine characteristics of a subject's genome or exome based on sequencing data of the subject's genome or exome. A bioinformatics pipeline may be used to determine characteristics of a germline genome or exome of a subject and/or a cancer genome or exome of a subject. In some embodiments, the pipeline extracts information related to genomic alterations in the cancer genome of a subject, which is useful for guiding clinical decisions for precision oncology, from sequencing results of a biological sample, *e.g.,* a tumor sample, liquid biopsy sample, reference normal sample, *etc.,* from the subject. Certain processing stages in a bioinformatics may be 'connected,' meaning that the results of a first respective processing stage are informative and/or essential for execution of a second, downstream processing stage. For instance, in some embodiments, a bioinformatics pipeline includes a first respective processing stage for identifying genomic alterations that are unique to the cancer genome of a subject and a second respective processing stage that uses the quantity and/or identity of the identified genomic alterations to determine a metric that is informative for precision oncology, *e.g.,* a tumor mutational burden. In some embodiments, the bioinformatics pipeline includes a reporting stage that generates a report of relevant and/or actionable information identified by upstream stages of the pipeline, which may or may not further include recommendations for aiding clinical therapy decisions.

As used herein, the term "limit of detection" or "LOD" refers to the minimal quantity of a feature that can be identified with a particular level of confidence. Accordingly, level of detection can be used to describe an amount of a substance that must be present in order for a particular assay to reliably detect the substance. A level of detection can also be used to describe a level of support needed for an algorithm to reliably identify a genomic alteration based on sequencing data. For example, a minimal number of unique sequence reads to support identification of a sequence variant such as a SNV.

As used herein, the term "BAM File" or "Binary file containing Alignment Maps" refers to a file storing sequencing data aligned to a reference sequence (*e.g.,* a reference genome or exome). In some embodiments, a BAM file is a compressed binary version of a SAM (Sequence Alignment Map) file that includes, for each of a plurality of unique sequence reads, an identifier for the sequence read, information about the nucleotide sequence, information about the alignment of the sequence to a reference sequence, and optionally metrics relating to the quality of the sequence read and/or the quality of the sequence alignment. While BAM files generally relate to files having a particular format, for simplicity they are used herein to simply refer to a file, of any format, containing information about a sequence alignment, unless specifically stated otherwise.

As used herein, the term "measure of central tendency" refers to a central or representative value for a distribution of values. Non-limiting examples of measures of central tendency include an arithmetic mean, weighted mean, midrange, midhinge, trimean, geometric mean, geometric median, Winsorized mean, median, and mode of the distribution of values.

As used herein, the term "measure of dispersion" refers to the extent to which data points in a dataset, fitted to a distribution, vary or spread out from the mean (or another central measure central tendency) of the fitted distribution. Variance, standard deviation, mean absolute deviation (MAD), interquartile range (IQR), range, coefficient of variation (CV), median absolute deviation (MAD), skewness (although it measures asymmetry, it relates to the spread of the data in the distribution as well), kurtosis (though it measures the "tailedness," it still describes aspects of how data is spread in a distribution), Gini Index (measuring inequality or variation in distributions) are nonlimiting examples of measures of dispersion.

As used herein, the term "Positive Predictive Value" or "PPV" means the likelihood that a variant is properly called given that a variant has been called by an assay. PPV can be expressed as (number of true positives)/ (number of false positives + number of true positives).

As used herein, the term "assay" refers to a technique for determining a property of a substance, *e.g.,* a nucleic acid, a protein, a cell, a tissue, or an organ. An assay (*e.g., a* first assay or a second assay) can comprise a technique for determining the copy number variation of nucleic acids in a sample, the methylation status of nucleic acids in a sample, the fragment size distribution of nucleic acids in a sample, the mutational status of nucleic acids in a sample, or the fragmentation pattern of nucleic acids in a sample. Any assay known to a person having ordinary skill in the art can be used to detect any of the properties of nucleic acids mentioned herein. Properties of a nucleic acids can include a sequence, genomic identity, copy number, methylation state at one or more nucleotide positions, size of the nucleic acid, presence or absence of a mutation in the nucleic acid at one or more nucleotide positions, and pattern of fragmentation of a nucleic acid (*e.g.,* the nucleotide position(s) at which a nucleic acid fragments). An assay or method can have a particular sensitivity and/or specificity, and their relative usefulness as a diagnostic tool can be measured using ROC-AUC statistics.

As used herein, the term "classification" can refer to any number(s) or other characters(s) that are associated with a particular property of a sample. For example, in some embodiments, the term "classification" can refer to a type of cancer in a subject, a stage of cancer in a subject, a prognosis for a cancer in a subject, a tumor load, a presence of tumor metastasis in a subject, and the like. The classification can be binary (e.g., positive or negative) or have more levels of classification (e.g., a scale from 1 to 10 or 0 to 1). The terms "cutoff" and "threshold" can refer to predetermined numbers used in an operation. For example, a cutoff size can refer to a size above which fragments are excluded. A threshold value can be a value above or below which a particular classification applies. Either of these terms can be used in either of these contexts.

As used herein, the term "sensitivity" or "true positive rate" (TPR) refers to the number of true positives divided by the sum of the number of true positives and false negatives. Sensitivity can characterize the ability of an assay or method to correctly identify a proportion of the population that truly has a condition. For example, sensitivity can characterize the ability of a method to correctly identify the number of subjects within a population having cancer. In another example, sensitivity can characterize the ability of a method to correctly identify the one or more markers indicative of cancer.

As used herein, the term "specificity" or "true negative rate" (TNR) refers to the number of true negatives divided by the sum of the number of true negatives and false positives. Specificity can characterize the ability of an assay or method to correctly identify a proportion of the population that truly does not have a condition. For example, specificity can characterize the ability of a method to correctly identify the number of subjects within a population not having cancer. In another example, specificity characterizes the ability of a method to correctly identify one or more markers indicative of cancer.

As used herein, an "actionable genomic alteration" or "actionable variant" refers to a genomic alteration (e.g., a SNV, MNV, indel, rearrangement, copy number variation, or ploidy variation), or value of another cancer metric derived from nucleic acid sequencing data (*e.g.,* a tumor mutational burden, MSI status, or tumor fraction), that is known or believed to be associated with a therapeutic course of action that is more likely to produce a positive effect in a cancer patient that has the actionable variant than in a similarly situated cancer patient that does not have the actionable variant. For instance, administration of EGFR inhibitors (*e.g.,* afatinib, erlotinib, gefitinib) is more effective for treating non-small cell lung cancer in patients with an EGFR mutation in exons 19/21 than for treating non-small cell lung cancer in patients that do not have an EGFR mutations in exons 19/21. Accordingly, an EGFR mutation in exon 19/21 is an actionable variant. In some instances, an actionable variant is only associated with an improved treatment outcome in one or a group of specific cancer types. In other instances, an actionable variant is associated with an improved treatment outcome in substantially all cancer types.

As used herein, a "variant of uncertain significance" or "VUS" refers to a genomic alteration (*e.g.,* a SNV, MNV, indel, rearrangement, copy number variation, or ploidy variation), or value of another cancer metric derived from nucleic acid sequencing data (*e.g.,* a tumor mutational burden, MSI status, or tumor fraction), whose impact on disease development/progression is unknown.

As used herein, a "benign variant" or "likely benign variant" refers to a genomic alteration (*e.g.,* a SNV, MNV, indel, rearrangement, copy number variation, or ploidy variation), or value of another cancer metric derived from nucleic acid sequencing data (*e.g.,* a tumor mutational burden, MSI status, or tumor fraction), that is known or believed to not contribute to disease development/progression.

As used herein, a "pathogenic variant" or "likely pathogenic variant" refers to a genomic alteration (*e.g.,* a SNV, MNV, indel, rearrangement, copy number variation, or ploidy variation), or value of another cancer metric derived from nucleic acid sequencing data (*e.g.,* a tumor mutational burden, MSI status, or tumor fraction), that is known or believed to contribute to disease development/progression.

As used herein, an "effective amount" or "therapeutically effective amount" is an amount sufficient to affect a beneficial or desired clinical result upon treatment. An effective amount can be administered to a subject in one or more doses. In terms of treatment, an effective amount is an amount that is sufficient to palliate, ameliorate, stabilize, reverse or slow the progression of the disease, or otherwise reduce the pathological consequences of the disease. The effective amount is generally determined by the physician on a case-by-case basis and is within the skill of one in the art. Several factors are typically taken into account when determining an appropriate dosage to achieve an effective amount. These factors include age, sex and weight of the subject, the condition being treated, the severity of the condition and the form and effective concentration of the therapeutic agent being administered.

The terminology used in the present disclosure is for the purpose of describing particular embodiments only and is not intended to be limiting of the invention. As used in the description of the invention and the appended claims, the singular forms "a", "an" and "the" are intended to include the plural forms as well, unless the context clearly indicates otherwise. It will also be understood that the term "and/or" as used herein refers to and encompasses any and all possible combinations of one or more of the associated listed items. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof. Furthermore, to the extent that the terms "including," "includes," "having," "has," "with," or variants thereof are used in either the detailed description and/or the claims, such terms are intended to be inclusive in a manner similar to the term "comprising."

As used herein, the term "if" may be construed to mean "when" or "upon" or "in response to determining" or "in response to detecting," depending on the context. Similarly, the phrase "if it is determined" or "if [a stated condition or event] is detected" may be construed to mean "upon determining" or "in response to determining" or "upon detecting [the stated condition or event]" or "in response to detecting [the stated condition or event]," depending on the context.

It will also be understood that, although the terms first, second, *etc.* may be used herein to describe various elements, these elements should not be limited by these terms. These terms are only used to distinguish one element from another. For example, a first subject could be termed a second subject, and, similarly, a second subject could be termed a first subject, without departing from the scope of the present disclosure. The first subject and the second subject are both subjects, but they are not the same subject. Furthermore, the terms "subject," "user," and "patient" are used interchangeably herein.

Reference will now be made in detail to embodiments, examples of which are illustrated in the accompanying drawings. In the following detailed description, numerous specific details are set forth in order to provide a thorough understanding of the present disclosure, including example systems, methods, techniques, instruction sequences, and computing machine program products that embody illustrative implementations. However, the illustrative discussions below are not intended to be exhaustive or to limit the implementations to the precise forms disclosed. Many modifications and variations are possible in view of the above teachings. The features described herein are not limited by the illustrated ordering of acts or events, as some acts can occur in different orders and/or concurrently with other acts or events.

The implementations provided herein are chosen and described in order to best explain the principles and their practical applications, to thereby enable others skilled in the art to best utilize the various embodiments with various modifications as are suited to the particular use contemplated. In some instances, well-known methods, procedures, components, circuits, and networks have not been described in detail so as not to unnecessarily obscure aspects of the embodiments. In other instances, it will be apparent to one of ordinary skill in the art that the present disclosure may be practiced without one or more of the specific details.

It will be appreciated that, in the development of any such actual implementation, numerous implementation-specific decisions are made in order to achieve the designer's specific goals, such as compliance with use case- and business-related constraints, and that these specific goals will vary from one implementation to another and from one designer to another. Moreover, it will be appreciated that though such a design effort might be complex and time-consuming, it will nevertheless be a routine undertaking of engineering for those of ordering skill in the art having the benefit of the present disclosure.

*Example System Embodiments.*

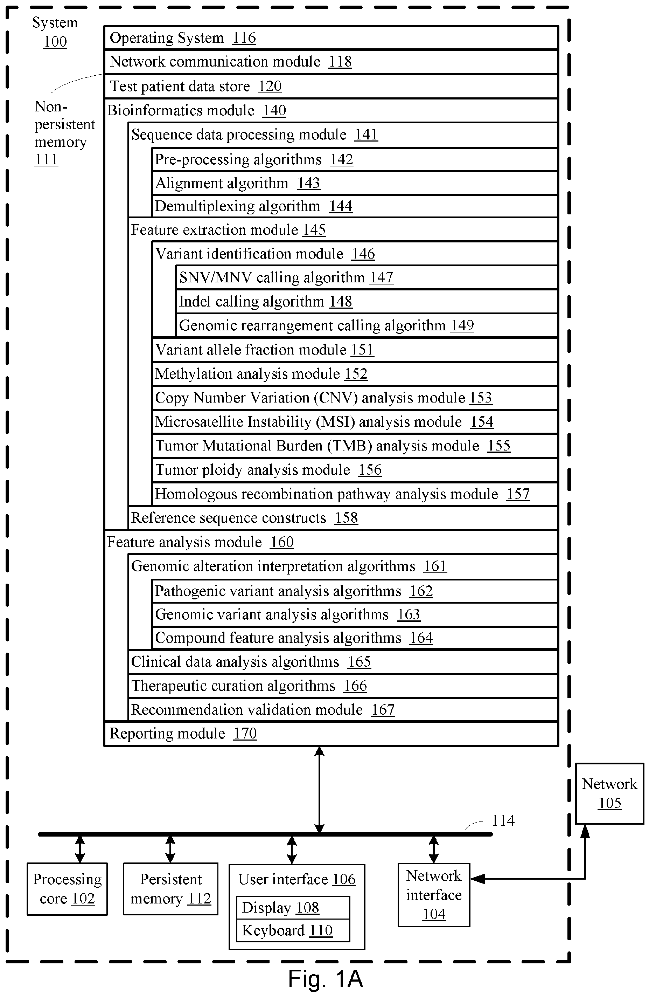

Now that an overview of some aspects of the present disclosure and some definitions used in the present disclosure have been provided, details of an exemplary system for providing clinical support for personalized cancer therapy using a liquid biopsy assay are now described in conjunction with Figures 1A-1D. Figures 1A-1D collectively illustrate the topology of an example system for providing clinical support for personalized cancer therapy using a liquid biopsy assay, in accordance with some embodiments of the present disclosure. Advantageously, the example system illustrated in Figures 1A-1D improves upon conventional methods for providing clinical support for personalized cancer therapy by determining tumor-informed circulating tumor fraction estimates using a non-bespoke liquid biopsy assay.

Figure 1A is a block diagram illustrating a system in accordance with some implementations. The device 100 in some implementations includes one or more processing units CPU(s) 102 (also referred to as processors), one or more network interfaces 104, a user interface 106, *e.g.,* including a display 108 and/or an input 110 *(e.g.,* a mouse, touchpad, keyboard, *etc.),* a non-persistent memory 111, a persistent memory 112, and one or more communication buses 114 for interconnecting these components. The one or more communication buses 114 optionally include circuitry (sometimes called a chipset) that interconnects and controls communications between system components. The non-persistent memory 111 typically includes high-speed random access memory, such as DRAM, SRAM, DDR RAM, ROM, EEPROM, flash memory, whereas the persistent memory 112 typically includes CD-ROM, digital versatile disks (DVD) or other optical storage, magnetic cassettes, magnetic tape, magnetic disk storage or other magnetic storage devices, magnetic disk storage devices, optical disk storage devices, flash memory devices, or other non-volatile solid state storage devices. The persistent memory 112 optionally includes one or more storage devices remotely located from the CPU(s) 102. The persistent memory 112, and the non-volatile memory device(s) within the non-persistent memory 112, comprise non-transitory computer readable storage medium. In some implementations, the non-persistent memory 111 or alternatively the non-transitory computer readable storage medium stores the following programs, modules and data structures, or a subset thereof, sometimes in conjunction with the persistent memory 112:

- an operating system 116, which includes procedures for handling various basic system services and for performing hardware dependent tasks;
- a network communication module (or instructions) 118 for connecting the system 100 with other devices and/or a communication network 105;
- a test patient data store 120 for storing one or more collections of features from patients (e.g., subjects);
- a bioinformatics module 140 for processing sequencing data and extracting features from sequencing data, *e.g.,* from liquid biopsy sequencing assays;
- a feature analysis module 160 for evaluating patient features, e.g., genomic alterations, compound genomic features, and clinical features; and

- a reporting module 180 for generating and transmitting reports that provide clinical support for personalized cancer therapy.

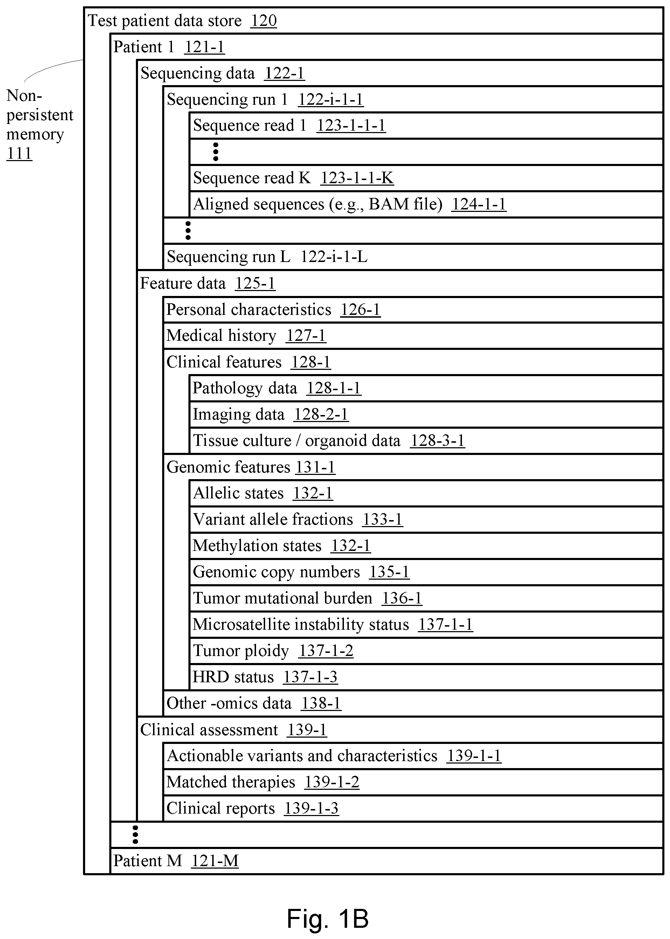

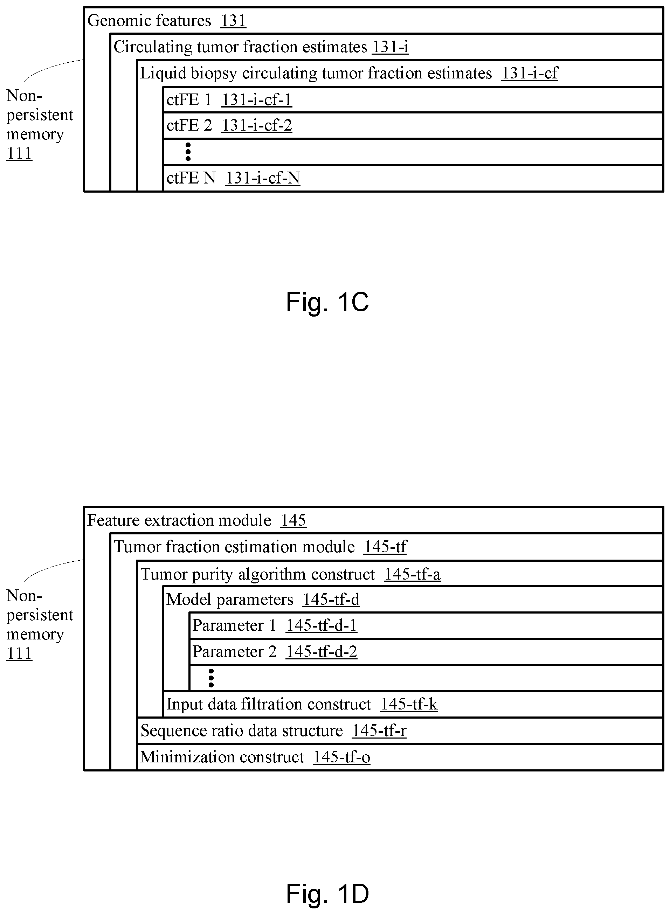

Although Figures 1A-1D depict a "system 100," the figures are intended more as a functional description of the various features that may be present in computer systems than as a structural schematic of the implementations described herein. In practice, and as recognized by those of ordinary skill in the art, items shown separately could be combined and some items could be separated. Moreover, although Figure 1 depicts certain data and modules in non-persistent memory 111, some or all of these data and modules may be in persistent memory 112. For example, in various implementations, one or more of the above identified elements are stored in one or more of the previously mentioned memory devices and correspond to a set of instructions for performing a function described above. The above identified modules, data, or programs (e.g., sets of instructions) need not be implemented as separate software programs, procedures, datasets, or modules, and thus various subsets of these modules and data may be combined or otherwise re-arranged in various implementations.

In some implementations, the non-persistent memory 111 optionally stores a subset of the modules and data structures identified above. Furthermore, in some embodiments, the memory stores additional modules and data structures not described above. In some embodiments, one or more of the above-identified elements is stored in a computer system, other than that of system 100, that is addressable by system 100 so that system 100 may retrieve all or a portion of such data when needed.

For purposes of illustration in Figure 1A, system 100 is represented as a single computer that includes all of the functionality for providing clinical support for personalized cancer therapy. However, while a single machine is illustrated, the term "system" shall also be taken to include any collection of machines that individually or jointly execute a set (or multiple sets) of instructions to perform any one or more of the methodologies discussed herein.

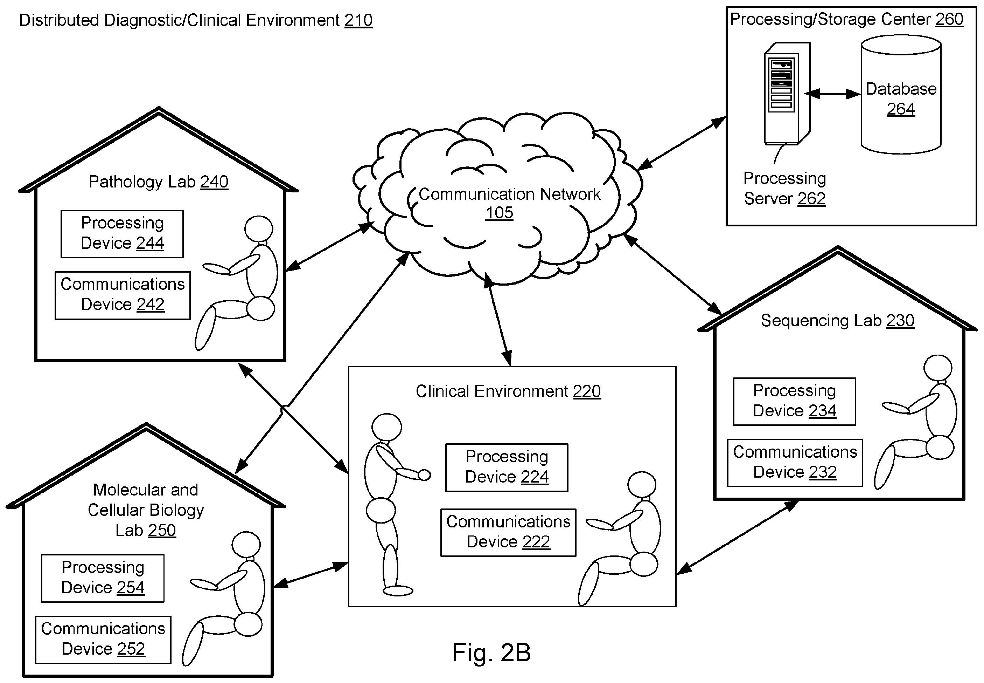

For example, in some embodiments, system 100 includes one or more computers. In some embodiments, the functionality for providing clinical support for personalized cancer therapy is spread across any number of networked computers and/or resides on each of several networked computers and/or is hosted on one or more virtual machines at a remote location accessible across the communications network 105. For example, different portions of the various modules and data stores illustrated in Figures 1A-1D can be stored and/or executed on the various instances of a processing device and/or processing server/database in the distributed diagnostic environment 210 illustrated in Figure 2B (e.g., processing devices 224, 234, 244, and 254, processing server 262, and database 264).

The system may operate in the capacity of a server or a client machine in client-server network environment, as a peer machine in a peer-to-peer (or distributed) network environment, or as a server or a client machine in a cloud computing infrastructure or environment. The system may be a personal computer (PC), a tablet PC, a set-top box (STB), a Personal Digital Assistant (PDA), a cellular telephone, a web appliance, a server, a network router, a switch or bridge, or any machine capable of executing a set of instructions (sequential or otherwise) that specify actions to be taken by that machine.

In another implementation, the system comprises a virtual machine that includes a module for executing instructions for performing any one or more of the methodologies disclosed herein. In computing, a virtual machine (VM) is an emulation of a computer system that is based on computer architectures and provides functionality of a physical computer. Some such implementations may involve specialized hardware, software, or a combination of hardware and software.

One of skill in the art will appreciate that any of a wide array of different computer topologies are used for the application and all such topologies are within the scope of the present disclosure.

*Test Patient Data Store (120).*

Referring to Figure 1B, in some embodiments, the system (e.g., system 100) includes a patient data store 120 that stores data for patients 121-1 to 121-M *(e.g.,* cancer patients or patients being tested for cancer) including one or more sequencing data 122, feature data 125, and clinical assessments 139. These data are used and/or generated by the various processes stored in the bioinformatics module 140 and feature analysis module 160 of system 100, to ultimately generate a report providing clinical support for personalized cancer therapy of a patient. While the feature scope of patient data 121 across all patients may be informationally dense, an individual patient's feature set may be sparsely populated across the entirety of the collective feature scope of all features across all patients. That is to say, the data stored for one patient may include a different set of features that the data stored for another patient. Further, while illustrated as a single data construct in Figure 1B, different sets of patient data may be stored in different databases or modules spread across one or more system memories.

In some embodiments, sequencing data 122 from one or more sequencing reactions 122-i, including a plurality of sequence reads 123-i-1 to 123-i-K, is stored in the test patient data store 120. The data store may include different sets of sequencing data from a single subject, corresponding to different samples from the patient, *e.g.,* a tumor sample, liquid biopsy sample, tumor organoid derived from a patient tumor, and/or a normal sample, and/or to samples acquired at different times, e.g., while monitoring the progression, regression, remission, and/or recurrence of a cancer in a subject. The sequence reads may be in any suitable file format, *e.g.,* BCL, FASTA, FASTQ, *etc.* In some embodiments, sequencing data 122 is accessed by a sequencing data processing module 141, which performs various pre-processing, genome alignment, and demultiplexing operations, as described in detail below with reference to bioinformatics module 140. In some embodiments, sequence data that has been aligned to a reference construct, e.g., BAM file 124, is stored in test patient data store 120.

In some embodiments, the test patient data store 120 includes feature data 125, *e.g.,* that is useful for identifying clinical support for personalized cancer therapy. In some embodiments, the feature data 125 includes personal characteristics 126 of the patient, such as patient name, date of birth, gender, ethnicity, physical address, smoking status, alcohol consumption characteristic, anthropomorphic data, *etc.*

In some embodiments, the feature data 125 includes medical history data 127 for the patient, such as cancer diagnosis information (*e.g.,* date of initial diagnosis, date of metastatic diagnosis, cancer staging, tumor characterization, tissue of origin, previous treatments and outcomes, adverse effects of therapy, therapy group history, clinical trial history, previous and current medications, surgical history, *etc.*), previous or current symptoms, previous or current therapies, previous treatment outcomes, previous disease diagnoses, diabetes status, diagnoses of depression, diagnoses of other physical or mental maladies, and family medical history. In some embodiments, the feature data 125 includes clinical features 128, such as pathology data 128-1, medical imaging data 128-2, and tissue culture and/or tissue organoid culture data 128-3.

In some embodiments, yet other clinical features, such as previous laboratory testing results, are stored in the test patient data store 120. Medical history data 127 and clinical features may be collected from various sources, including at intake directly from the patient, from an electronic medical record (EMR) or electronic health record (EHR) for the patient, or curated from other sources, such as fields from various testing records (*e.g.,* genetic sequencing reports).

In some embodiments, the feature data 125 includes genomic features 131 for the patient. Non-limiting examples of genomic features include allelic states 132 (*e.g.,* the identity of alleles at one or more loci, support for wild type or variant alleles at one or more loci, support for SNVs/MNVs at one or more loci, support for indels at one or more loci, and/or support for gene rearrangements at one or more loci), allelic fractions 133 (*e.g.,* ratios of variant to reference alleles (or vice versa), methylation states 132 (*e.g.,* a distribution of methylation patterns at one or more loci and/or support for aberrant methylation patterns at one or more loci), genomic copy numbers 135 (*e.g.,* a copy number value at one or more loci and/or support for an aberrant (increased or decreased) copy number at one or more loci), tumor mutational burden 136 (*e.g.,* a measure of the number of mutations in the cancer genome of the subject), and microsatellite instability status 137 (*e.g.,* a measure of the repeated unit length at one or more microsatellite loci and/or a classification of the MSI status for the patient's cancer). In some embodiments, one or more of the genomic features 131 are determined by a nucleic acid bioinformatics pipeline, *e.g.,* as described in detail below with reference to Figures 4A-4E. In particular, in some embodiments, the feature data 125 includes circulating tumor fraction estimates 131-i, as determined using the improved methods for determining circulating tumor fraction estimates, as described in further detail below with reference to Figures 1C, 1D, and 4E. In some embodiments, one or more of the genomic features 131 are obtained from an external testing source, *e.g.,* not connected to the bioinformatics pipeline as described below.

Referring again to Figure 1B, in some embodiments, the feature data 125 further includes data 138 from other -omics fields of study. Non-limiting examples of -omics fields of study that may yield feature data useful for providing clinical support for personalized cancer therapy include transcriptomics, epigenomics, proteomics, metabolomics, metabonomics, microbiomics, lipidomics, glycomics, cellomics, and organoidomics.

In some embodiments, yet other features may include features derived from machine learning approaches, *e.g.,* based at least in part on evaluation of any relevant molecular or clinical features, considered alone or in combination, not limited to those listed above. For instance, in some embodiments, one or more latent features learned from evaluation of cancer patient training datasets improve the diagnostic and prognostic power of the various analysis algorithms in the feature analysis module 160.

The skilled artisan will know of other types of features useful for providing clinical support for personalized cancer therapy. The listing of features above is merely representative and should not be construed to be limiting.

In some embodiments, a test patient data store 120 includes clinical assessment data 139 for patients, e.g., based on the feature data 125 collected for the subject. In some embodiments, the clinical assessment data 139 includes a catalogue of actionable variants and characteristics 139-1 (*e.g.,* genomic alterations and compound metrics based on genomic features known or believed to be targetable by one or more specific cancer therapies), matched therapies 139-2 (*e.g.,* the therapies known or believed to be particularly beneficial for treatment of subjects having actionable variants), and/or clinical reports 139-3 generated for the subject, e.g., based on identified actionable variants and characteristics 139-1 and/or matched therapies 139-2.

In some embodiments, clinical assessment data 139 is generated by analysis of feature data 125 using the various algorithms of feature analysis module 160, as described in further detail below. In some embodiments, clinical assessment data 139 is generated, modified, and/or validated by evaluation of feature data 125 by a clinician, *e.g.,* an oncologist. For instance, in some embodiments, a clinician (e.g., at clinical environment 220) uses feature analysis module 160, or accesses test patient data store 120 directly, to evaluate feature data 125 to make recommendations for personalized cancer treatment of a patient. Similarly, in some embodiments, a clinician (*e.g.,* at clinical environment 220) reviews recommendations determined using feature analysis module 160 and approves, rejects, or modifies the recommendations, *e.g.,* prior to the recommendations being sent to a medical professional treating the cancer patient.

*Bioinformatics Module (140).*

Referring again to Figure 1A, the system (*e.g.,* system 100) includes a bioinformatics module 140 that includes a feature extraction module 145 and optional ancillary data processing constructs, such as a sequence data processing module 141 and/or one or more reference sequence constructs 158 (*e.g.,* a reference genome, exome, or targeted-panel construct that includes reference sequences for a plurality of loci targeted by a sequencing panel).

In some embodiments, bioinformatics module 140 includes a sequence data processing module 141 that includes instructions for processing sequence reads, e.g., raw sequence reads 123 from one or more sequencing reactions 122, prior to analysis by the various feature extraction algorithms, as described in detail below. In some embodiments, sequence data processing module 141 includes one or more pre-processing algorithms 142 that prepare the data for analysis. In some embodiments, the pre-processing algorithms 142 include instructions for converting the file format of the sequence reads from the output of the sequencer (*e.g.,* a BCL file format) into a file format compatible with downstream analysis of the sequences (e.g., a FASTQ or FASTA file format). In some embodiments, the pre-processing algorithms 142 include instructions for evaluating the quality of the sequence reads (*e.g.,* by interrogating quality metrics like Phred score, base-calling error probabilities, Quality (Q) scores, and the like) and/or removing sequence reads that do not satisfy a threshold quality *(e.g.,* an inferred base call accuracy of at least 80%, at least 90%, at least 95%, at least 99%, at least 99.5%, at least 99.9%, or higher). In some embodiments, the pre-processing algorithms 142 include instructions for filtering the sequence reads for one or more properties, *e.g.,* removing sequences failing to satisfy a lower or upper size threshold or removing duplicate sequence reads.

In some embodiments, sequence data processing module 141 includes one or more alignment algorithms 143, for aligning pre-processed sequence reads 123 to a reference sequence construct 158, *e.g.,* a reference genome, exome, or targeted-panel construct. Many algorithms for aligning sequencing data to a reference construct are known in the art, for example, BWA, Blat, SHRiMP, LastZ, and MAQ. One example of a sequence read alignment package is the Burrows-Wheeler Alignment tool (BWA), which uses a Burrows-Wheeler Transform (BWT) to align short sequence reads against a large reference construct, allowing for mismatches and gaps. Sequence read alignment packages import raw or pre-processed sequence reads 122, *e.g.,* in BCL, FASTA, or FASTQ file formats, and output aligned sequence reads 124, *e.g.,* in SAM or BAM file formats.

In some embodiments, sequence data processing module 141 includes one or more demultiplexing algorithms 144, for dividing sequence read or sequence alignment files generated from sequencing reactions of pooled nucleic acids into separate sequence read or sequence alignment files, each of which corresponds to a different source of nucleic acids in the nucleic acid sequencing pool. For instance, because of the cost of sequencing reactions, it is common practice to pool nucleic acids from a plurality of samples into a single sequencing reaction. The nucleic acids from each sample are tagged with a sample-specific and/or molecule-specific sequence tag (*e.g.,* a UMI), which is sequenced along with the molecule. In some embodiments, demultiplexing algorithms 144 sort these sequence tags in the sequence read or sequence alignment files to demultiplex the sequencing data into separate files for each of the samples included in the sequencing reaction.

Bioinformatics module 140 includes a feature extraction module 145, which includes instructions for identifying diagnostic features, e.g., genomic features 131, from *s*equencing data 122 of biological samples from a subject, *e.g.,* one or more of a solid tumor sample, a liquid biopsy sample, or a normal tissue (*e.g.*, control) sample. For instance, in some embodiments, a feature extraction algorithm compares the identity of one or more nucleotides at a locus from the sequencing data 122 to the identity of the nucleotides at that locus in a reference sequence construct (*e.g.,* a reference genome, exome, or targeted-panel construct) to determine whether the subject has a variant at that locus. In some embodiments, a feature extraction algorithm evaluates data other than the raw sequence, to identify a genomic alteration in the subject, *e.g.,* an allelic ratio, a relative copy number, a repeat unit distribution, *etc.*

For instance, in some embodiments, feature extraction module 145 includes one or more variant identification modules that include instructions for various variant calling processes. In some embodiments, variants in the germline of the subject are identified, *e.g.,* using a germline variant identification module 146. In some embodiments, variants in the cancer genome, *e.g.,* somatic variants, are identified, *e.g.,* using a somatic variant identification module 150. While separate germline and somatic variant identification modules are illustrated in Figure 1A, in some embodiments they are integrated into a single module. In some embodiments, the variant identification module includes instructions for identifying one or more of nucleotide variants (*e.g.,* single nucleotide variants (SNV) and multi-nucleotide variants (MNV)) using one or more SNV/MNV calling algorithms (*e.g.,* algorithms 147 and/or 151), indels (*e.g.,* insertions or deletions of nucleotides) using one or more indel calling algorithms (*e.g.,* algorithms 148 and/or 152), and genomic rearrangements (*e.g.,* inversions, translocation, and fusions of nucleotide sequences) using one or more genomic rearrangement calling algorithms *(e.g.,* algorithms 149 and/or 153).

A SNV/MNV algorithm 147 may identify a substitution of a single nucleotide that occurs at a specific position in the genome. For example, at a specific base position, or locus, in the human genome, the C nucleotide may appear in most individuals, but in a minority of individuals, the position is occupied by an A. This means that there is a SNP at this specific position and the two possible nucleotide variations, C or A, are said to be alleles for this position. SNPs underlie differences in human susceptibility to a wide range of diseases (e.g. - sickle-cell anemia, β-thalassemia and cystic fibrosis result from SNPs). The severity of illness and the way the body responds to treatments are also manifestations of genetic variations. For example, a single-base mutation in the APOE (apolipoprotein E) gene is associated with a lower risk for Alzheimer's disease. A single-nucleotide variant (SNV) is a variation in a single nucleotide without any limitations of frequency and may arise in somatic cells. A somatic single-nucleotide variation (*e.g.,* caused by cancer) may also be called a single-nucleotide alteration. An MNP (Multiple-nucleotide polymorphisms) module may identify the substitution of consecutive nucleotides at a specific position in the genome.

An indel calling algorithm 148 may identify an insertion or deletion of bases in the genome of an organism classified among small genetic variations. While indels usually measure from 1 to 10 000 base pairs in length, a microindel is defined as an indel that results in a net change of 1 to 50 nucleotides. Indels can be contrasted with a SNP or point mutation. An indel inserts and/or deletes nucleotides from a sequence, while a point mutation is a form of substitution that replaces one of the nucleotides without changing the overall number in the DNA. Indels, being insertions and/or deletions, can be used as genetic markers in natural populations, especially in phylogenetic studies. Indel frequency tends to be markedly lower than that of single nucleotide polymorphisms (SNP), except near highly repetitive regions, including homopolymers and microsatellites.

A genomic rearrangement algorithm 149 may identify hybrid genes formed from two previously separate genes. It can occur as a result of translocation, interstitial deletion, or chromosomal inversion. Gene fusion can play an important role in tumorigenesis. Fusion genes can contribute to tumor formation because fusion genes can produce much more active abnormal protein than non-fusion genes. Often, fusion genes are oncogenes that cause cancer; these include BCR-ABL, TEL-AML1 (ALL with t(12 ; 21)), AML1-ETO (M2 AML with t(8 ; 21)), and TMPRSS2-ERG with an interstitial deletion on chromosome 21, often occurring in prostate cancer. In the case of TMPRSS2-ERG, by disrupting androgen receptor (AR) signaling and inhibiting AR expression by oncogenic ETS transcription factor, the fusion product regulates prostate cancer. Most fusion genes are found from hematological cancers, sarcomas, and prostate cancer. BCAM-AKT2 is a fusion gene that is specific and unique to high-grade serous ovarian cancer. Oncogenic fusion genes may lead to a gene product with a new or different function from the two fusion partners. Alternatively, a proto-oncogene is fused to a strong promoter, and thereby the oncogenic function is set to function by an upregulation caused by the strong promoter of the upstream fusion partner. The latter is common in lymphomas, where oncogenes are juxtaposed to the promoters of the immunoglobulin genes. Oncogenic fusion transcripts may also be caused by trans-splicing or read-through events. Since chromosomal translocations play such a significant role in neoplasia, a specialized database of chromosomal aberrations and gene fusions in cancer has *b*een created. This database is called Mitelman Database of Chromosome Aberrations and Gene Fusions in Cancer.

In some embodiments, feature extraction module 145 includes instructions for identifying one or more complex genomic alterations (*e.g.,* features that incorporate more than a change in the primary sequence of the genome) in the cancer genome of the subject. For instance, in some embodiments, feature extraction module 145 includes modules for identifying one or more of copy number variation (*e.g.,* copy number variation analysis module 153), microsatellite instability status (*e.g.,* microsatellite instability analysis module 154), tumor mutational burden (*e.g.,* tumor mutational burden analysis module 155), tumor ploidy (*e.g.,* tumor ploidy analysis module 156), and homologous recombination pathway deficiencies (e.g., homologous recombination pathway analysis module 157).

For example, referring to Figure 1D, in some embodiments, feature extraction module 145 comprises a tumor fraction estimation module 145-tf. In some embodiments, the tumor fraction estimation module 145-tf comprises a sequence ratio data structure 145-tf-r including a plurality of sequence ratios (*e.g.,* coverage ratios) obtained from a sequencing of a test liquid biopsy sample of a subject. In some embodiments, the sequence ratio data structure 145-tf-r includes the sequence ratios that are used as input to determine tumor fraction estimates for the test liquid biopsy sample. In some embodiments, the tumor fraction estimation module 145-tf also comprises a tumor purity algorithm construct 145-tf-a that executes, for example, a maximum likelihood estimation (*e.g.,* an expectation-maximization algorithm) to calculate an estimate of the circulating tumor fraction. The tumor purity algorithm construct 145-tf-a comprises an optional input data filtration construct 145-tf-k (*e.g.,* for removing one or more inputs passed from the sequence ratio data structure based on a minimum probe threshold or a position on a sex chromosome) and a plurality of model parameters 145-tf-d (*e.g.,* 145-tf-d-1, 145-tf-d-2,...) used for executing the algorithm. In some embodiments, model parameters include expected sequence ratios for a set of copy states at a given tumor purity; a distance (*e.g.,* an error) from a test sequence ratio to the closest expected sequence ratio at the given tumor purity; a minimum distance (e.g., a minimum error) from a test sequence ratio to the closest expected sequence ratio at the given tumor purity (e.g., an assigned test copy state selected from a minimal distance expected copy state); and/or a tumor purity score (e.g., a sum of weighted errors).

In some embodiments, referring to Figure 1C, the tumor fraction estimation module 145-tf is used to obtain one or more circulating tumor fraction estimates 131-i that are included as feature data 125 in a test patient data store 120. For example, in some embodiments, a plurality of circulating tumor fraction estimates is obtained from a test liquid biopsy sample of a subject 131-i-cf(e.g., 131-i-cf-1, 131-i-cf-2..., 131-i-cf-N). In some embodiments, the plurality of circulating tumor fraction estimates is obtained from a single patient at different collection times.

*Feature Analysis Module (160).*

Referring again to Figure 1A, the system (e.g., system 100) includes a feature analysis module 160 that includes one or more genomic alteration interpretation algorithms 161, one or more optional clinical data analysis algorithms 165, an optional therapeutic curation algorithm 165, and an optional recommendation validation module 167. In some embodiments, feature analysis module 160 identifies actionable variants and characteristics 139-1 and corresponding matched therapies 139-2 and/or clinical trials using one or more analysis algorithms (e.g., algorithms 162, 163, 164, and 165) to evaluate feature data 125. The identified actionable variants and characteristics 139-1 and corresponding matched therapies 139-2, which are optionally stored in test patient data store 120, are then curated by feature analysis module 160 to generate a clinical report 139-3, which is optionally validated by a user, e.g., a clinician, before being transmitted to a medical professional, e.g., an oncologist, treating the patient.

In some embodiments, the genomic alteration interpretation algorithms 161 include instructions for evaluating the effect that one or more genomic features 131 of the subject, e.g., as identified by feature extraction module 145, have on the characteristics of the patient's cancer and/or whether one or more targeted cancer therapies may improve the clinical outcome for the patient. For example, in some embodiments, one or more genomic variant analysis algorithms 163 evaluate various genomic features 131 by querying a database, e.g., a look-up-table ("LUT") of actionable genomic alterations, targeted therapies associated with the actionable genomic alterations, and any other conditions that should be met before administering the targeted therapy to a subject having the actionable genomic alteration. For instance, evidence suggests that depatuxizumab mafodotin (an anti-EGFR mAb conjugated to monomethyl auristatin F) has improved efficacy for the treatment of recurrent glioblastomas having EGFR focal amplifications. van den Bent et al., 2017, Cancer Chemother Pharmacol., 80(6):1209-17. Accordingly, the actionable genomic alteration LUT would have an entry for the focal amplification of the EGFR gene indicating that depatuxizumab mafodotin is a targeted therapy for glioblastomas (e.g., recurrent glioblastomas) having a focal gene amplification. In some instances, the LUT may also include counter indications for the associated targeted therapy, e.g., adverse drug interactions or personal characteristics that are counter-indicated for administration of the particular targeted therapy.

In some embodiments, a genomic alteration interpretation algorithm 161 determines whether a particular genomic feature 131 should be reported to a medical professional treating the cancer patient. In some embodiments, genomic features 131 (e.g., genomic alterations and compound features) are reported when there is clinical evidence that the feature significantly impacts the biology of the cancer, impacts the prognosis for the cancer, and/or impacts pharmacogenomics, e.g., by indicating or counter-indicating particular therapeutic approaches. For instance, a genomic alteration interpretation algorithm 161 may classify a particular CNV feature 135 as "Reportable," e.g., meaning that the CNV has been identified as influencing the character of the cancer, the overall disease state, and/or pharmacogenomics, as "Not Reportable," e.g., meaning that the CNV has not been identified as influencing the character of the cancer, the overall disease state, and/or pharmacogenomics, as "No Evidence," e.g., meaning that no evidence exists supporting that the CNV is "Reportable" or "Not Reportable," or as "Conflicting Evidence," e.g., meaning that evidence exists supporting both that the CNV is "Reportable" and that the CNV is "Not Reportable."

In some embodiments, the genomic alteration interpretation algorithms 161 include one or more pathogenic variant analysis algorithms 162, which evaluate various genomic features to identify the presence of an oncogenic pathogen associated with the patient's cancer and/or targeted therapies associated with an oncogenic pathogen infection in the cancer. For instance, RNA expression patterns of some cancers are associated with the presence of an oncogenic pathogen that is helping to drive the cancer. See, for example, U.S. Patent No. 11,043,304, the content of which is hereby incorporated by reference, in its entirety, for all purposes. In some instances, the recommended therapy for the cancer is different when the cancer is associated with the oncogenic pathogen infection than when it is not. Accordingly, in some embodiments, e.g., where feature data 125 includes RNA abundance data for the cancer of the patient, one or more pathogenic variant analysis algorithms 162 evaluate the RNA abundance data for the patient's cancer to determine whether a signature exists in the data that indicates the presence of the oncogenic pathogen in the cancer. Similarly, in some embodiments, bioinformatics module 140 includes an algorithm that searches for the presence of pathogenic nucleic acid sequences in sequencing data 122. See, for example, U.S. Patent Application No. 17/800,492 entitled "Systems and Methods for Detecting Viral DNA from Sequencing," filed August 17, 2022, the content of which is hereby incorporated by reference, in its entirety, for all purposes. Accordingly, in some embodiments, one or more pathogenic variant analysis algorithms 162 evaluates whether the presence of an oncogenic pathogen in a subject is associated with an actionable therapy for the infection. In some embodiments, system 100 queries a database, e.g., a look-up-table ("LUT"), of actionable oncogenic pathogen infections, targeted therapies associated with the actionable infections, and any other conditions that should be met before administering the targeted therapy to a subject that is infected with the oncogenic pathogen. In some instances, the LUT may also include counter indications for the associated targeted therapy, *e.g.,* adverse drug interactions or personal characteristics that are counter-indicated for administration of the particular targeted therapy.

In some embodiments, the genomic alteration interpretation algorithms 161 include one or more multi-feature analysis algorithms 164 that evaluate a plurality of features to classify a cancer with respect to the effects of one or more targeted therapies. For instance, in some embodiments, feature analysis module 160 includes one or more classifiers trained against feature data, one or more clinical therapies, and their associated clinical outcomes for a plurality of training subjects to classify cancers based on their predicted clinical outcomes following one or more therapies.

In some embodiments, the classifier is implemented as an artificial intelligence engine and may include gradient boosting models, random forest models, neural networks (NN), regression models, Naive Bayes models, and/or machine learning algorithms (MLA). An MLA or a NN may be trained from a training data set that includes one or more features 125, including personal characteristics 126, medical history 127, clinical features 128, genomic features 131, and/or other -omic features 138. MLAs include supervised algorithms (such as algorithms where the features/classifications in the data set are annotated) using linear regression, logistic regression, decision trees, classification and regression trees, naive Bayes, nearest neighbor clustering; unsupervised algorithms (such as algorithms where no features/classification in the data set are annotated) using Apriori, means clustering, principal component analysis, random forest, adaptive boosting; and semi-supervised algorithms (such as algorithms where an incomplete number of features/classifications in the data set are annotated) using generative approach (such as a mixture of Gaussian distributions, mixture of multinomial distributions, hidden Markov models), low density separation, graph-based approaches (such as mincut, harmonic function, manifold regularization), heuristic approaches, or support vector machines.

NNs include conditional random fields, convolutional neural networks, attention based neural networks, deep learning, long short term memory networks, or other neural models where the training data set includes a plurality of tumor samples, RNA expression data for each sample, and pathology reports covering imaging data for each sample.

While MLA and neural networks identify distinct approaches to machine learning, the terms may be used interchangeably herein. Thus, a mention of MLA may include a corresponding NN or a mention of NN may include a corresponding MLA unless explicitly stated otherwise. Training may include providing optimized datasets, labeling these traits as they occur in patient records, and training the MLA to predict or classify based on new inputs. Artificial NNs are efficient computing models which have shown their strengths in solving hard problems in artificial intelligence. They have also been shown to be universal approximators, that is, they can represent a wide variety of functions when given appropriate parameters.

In some embodiments, system 100 includes a classifier training module that includes instructions for training one or more untrained or partially trained classifiers based on feature data from a training dataset. In some embodiments, system 100 also includes a database of training data for use in training the one or more classifiers. In other embodiments, the classifier training module accesses a remote storage device hosting training data. In some embodiments, the training data includes a set of training features, including but not limited to, various types of the feature data 125 illustrated in Figure 1B. In some embodiments, the classifier training module uses patient data 121, *e.g.,* when test patient data store 120 also stores a record of treatments administered to the patient and patient outcomes following therapy.

In some embodiments, feature analysis module 160 includes one or more clinical data analysis algorithms 165, which evaluate clinical features 128 of a cancer to identify targeted therapies which may benefit the subject. For example, in some embodiments, e.g., where feature data 125 includes pathology data 128-1, one or more clinical data analysis algorithms 165 evaluate the data to determine whether an actionable therapy is indicated based on the histopathology of a tumor biopsy from the subject, e.g., which is indicative of a particular cancer type and/or stage of cancer. In some embodiments, system 100 queries a database, *e.g.,* a look-up-table ("LUT"), of actionable clinical features (*e.g.,* pathology features), targeted therapies associated with the actionable features, and any other conditions that should be met before administering the targeted therapy to a subject associated with the actionable clinical features 128 (*e.g.*, pathology features 128-1). In some embodiments, system 100 evaluates the clinical features 128 (*e.g.*, pathology features 128-1) directly to determine whether the patient's cancer is sensitive to a particular therapeutic agent. Further details on example methods, systems, and algorithms for classifying cancer and identifying targeted therapies based on clinical data, such as pathology data 128-1, imaging data 138-2, and/or tissue culture/organoid data 128-3 are discussed, for example, in U.S. Patent Nos. 10,957,041; 10,957,445, 11,244,763; 11,848,107; and 11,145,416, the contents of which are hereby incorporated by reference, in their entireties, for all purposes.

In some embodiments, feature analysis module 160 includes a clinical trials module that evaluates test patient data 121 to determine whether the patient is eligible for inclusion in a clinical trial for a cancer therapy, *e.g.,* a clinical trial that is currently recruiting patients, a clinical trial that has not yet begun recruiting patients, and/or an ongoing clinical trial that may recruit additional patients in the future. In some embodiments, a clinical trial module evaluates test patient data 121 to determine whether the results of a clinical trial are relevant for the patient, *e.g.,* the results of an ongoing clinical trial and/or the results of a completed clinical trial. For instance, in some embodiments, system 100 queries a database, *e.g.,* a look-up-table ("LUT") of clinical trials, *e.g.,* active and/or completed clinical trials, and compares patient data 121 with inclusion criteria for the clinical trials, stored in the database, to identify clinical trials with inclusion criteria that closely match and/or exactly match the patient's data 121. In some embodiments, a record of matching clinical trials, *e.g.,* those clinical trials that the patient may be eligible for and/or that may inform personalized treatment decisions for the patient, are stored in clinical assessment database 139.

In some embodiments, feature analysis module 160 includes a therapeutic curation algorithm 166 that assembles actionable variants and characteristics 139-1, matched therapies 139-2, and/or relevant clinical trials identified for the patient, as described above. In some embodiments, a therapeutic curation algorithm 166 evaluates certain criteria related to which actionable variants and characteristics 139-1, matched therapies 139-2, and/or relevant clinical trials should be reported and/or whether certain matched therapies, considered alone or in combination, may be counter-indicated for the patient, *e.g.*, based on personal characteristics 126 of the patient and/or known drug-drug interactions. In some embodiments, the therapeutic curation algorithm then generates one or more clinical reports 139-3 for the patient. In some embodiments, the therapeutic curation algorithm generates a first clinical report 139-3-1 that is to be reported to a medical professional treating the patient and a second clinical report 139-3-2 that will not be communicated to the medical professional, but may be used to improve various algorithms within the system.

In some embodiments, feature analysis module 160 includes a recommendation validation module 167 that includes an interface allowing a clinician to review, modify, and approve a clinical report 139-3 prior to the report being sent to a medical professional, e.g., an oncologist, treating the patient.

In some embodiments, each of the one or more feature collections, sequencing modules, bioinformatics modules (including, e.g., alteration module(s), structural variant calling and data processing modules), classification modules and outcome modules are communicatively coupled to a data bus to transfer data between each module for processing and/or storage. In some alternative embodiments, each of the feature collection, alteration module(s), structural variant and feature store are communicatively coupled to each other for independent communication without sharing the data bus.

Further details on systems and exemplary embodiments of modules and feature collections are discussed in U.S. Patent No. 11,830,587 which is hereby incorporated herein by reference in its entirety.

*Example Methods.*

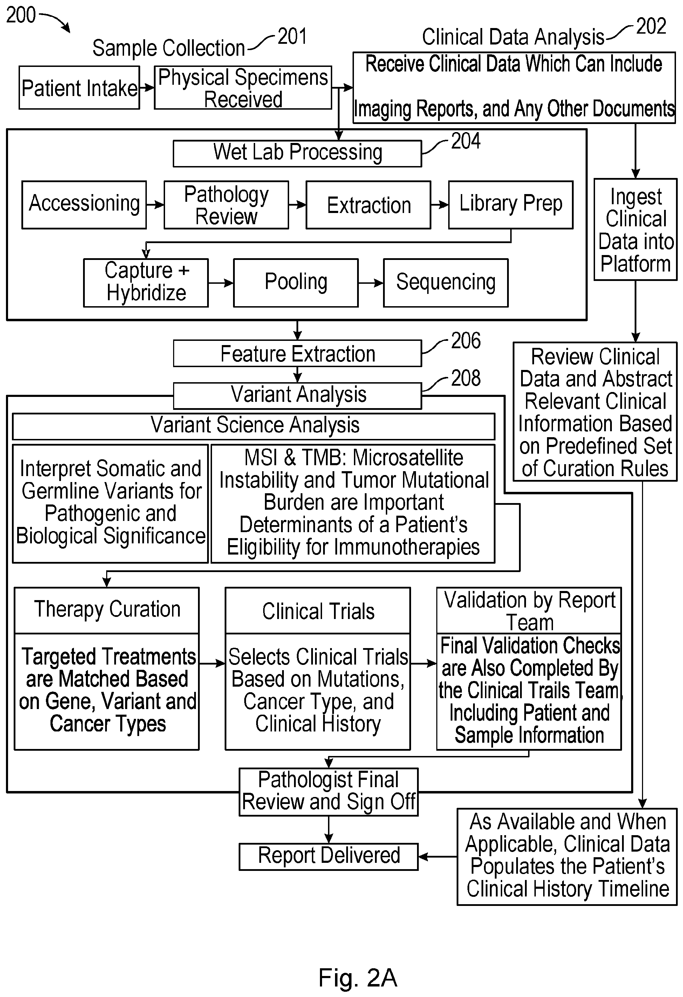

Now that details of a system 100 for providing clinical support for personalized cancer therapy, e.g., with improved circulating tumor fraction estimates, have been disclosed, details regarding processes and features of the system, in accordance with various embodiments of the present disclosure, are disclosed below. Specifically, example processes are described below with reference to Figures 2A, 3, 4A-4E, 5A-5F, and 6A-6G. In some embodiments, such processes and features of the system are carried out by modules 118, 120, 140, 160, and/or 170, as illustrated in Figure 1A. Referring to these methods, the systems described herein (e.g., system 100) include instructions for determining accurate circulating tumor fraction estimates that are improved compared to conventional methods for obtaining circulating tumor fraction estimates.

*Figure 2B: Distributed Diagnostic and Clinical Environment.*

In some aspects, the methods described herein for providing clinical support for personalized cancer therapy are performed across a distributed diagnostic/clinical environment, *e.g.*, as illustrated in Figure 2B. However, in some embodiments, the improved methods described herein for providing clinical support for personalized cancer therapy, (*e.g.*, by determining accurate circulating tumor fraction estimates), are performed at a single location, *e.g.,* at a single computing system or environment, although ancillary procedures supporting the methods described herein, and/or procedures that make further use of the results of the methods described herein, may be performed across a distributed diagnostic/clinical environment.

Figure 2B illustrates an example of a distributed diagnostic/clinical environment 210. In some embodiments, the distributed diagnostic/clinical environment is connected via communication network 105. In some embodiments, one or more biological samples, *e.g.*, one or more liquid biopsy samples, solid tumor biopsy, normal tissue samples, and/or control samples, are collected from a subject in clinical environment 220, *e.g.*, a doctor's office, hospital, or medical clinic, or at a home health care environment (not depicted). Advantageously, while solid tumor samples should be collected within a clinical setting, liquid biopsy samples can be acquired in a less invasive fashion and are more easily collected outside of a traditional clinical setting. In some embodiments, one or more biological samples, or portions thereof, are processed within the clinical environment 220 where collection occurred, using a processing device 224, *e.g.,* a nucleic acid sequencer for obtaining sequencing data, a microscope for obtaining pathology data, a mass spectrometer for obtaining proteomic data, *etc*. In some embodiments, one or more biological samples, or portions thereof are sent to one or more external environments, e.g., sequencing lab 230, pathology lab 240, and/or molecular biology lab 250, each of which includes a processing device 234, 244, and 254, respectively, to generate biological data 121 for the subject. Each environment includes a communications device 222, 232, 242, and 252, respectively, for communicating biological data 121 about the subject to a processing server 262 and/or database 264, which may be located in yet another environment, e.g., processing/storage center 260. Thus, in some embodiments, different portions of the systems and methods described herein are fulfilled by different processing devices located in different physical environments.

Accordingly, in some embodiments, a method for providing clinical support for personalized cancer therapy, *e.g.*, with improved circulating tumor fraction estimates, is performed across one or more environments, as illustrated in Figure 2B. For instance, in some such embodiments, a liquid biopsy sample is collected at clinical environment 220 or in a home healthcare environment. The sample, or a portion thereof, is sent to sequencing lab 230 where raw sequence reads 123 of nucleic acids in the sample are generated by sequencer 234. The raw sequencing data 123 is communicated, *e.g.*, from communications device 232, to database 264 at processing/storage center 260, where processing server 262 extracts features from the sequence reads by executing one or more of the processes in bioinformatics module 140, thereby generating genomic features 131 for the sample. Processing server 262 may then analyze the identified features by executing one or more of the processes in feature analysis module 160, thereby generating clinical assessment 139, including a clinical report 139-3. A clinician may access clinical report 139-3, *e.g.,* at processing/storage center 260 or through communications network 105, via recommendation validation module 167. After final approval, clinical report 139-3 is transmitted to a medical professional, *e.g.*, an oncologist, at clinical environment 220, who uses the report to support clinical decision making for personalized treatment of the patient's cancer.

*Figure 2A: Example Workflow for Precision Oncology.*

Figure 2A is a flowchart of an example workflow 200 for collecting and analyzing data in order to generate a clinical report 139 to support clinical decision making in precision oncology. Advantageously, the methods described herein improve this process, for example, by improving various stages within feature extraction 206, including determining circulating tumor fraction estimates.

Briefly, the workflow begins with patient intake and sample collection 201, where one or more liquid biopsy samples, one or more tumor biopsy, and one or more normal and/or control tissue samples are collected from the patient (*e.g.*, at a clinical environment 220 or home healthcare environment, as illustrated in Figure 2B). In some embodiments, personal data 126 corresponding to the patient and a record of the one or more biological samples obtained (*e.g.*, patient identifiers, patient clinical data, sample type, sample identifiers, cancer conditions, *etc.*) are entered into a data analysis platform, *e.g.,* test patient data store 120. Accordingly, in some embodiments, the methods disclosed herein include obtaining one or more biological samples from one or more subjects, e.g., cancer patients. In some embodiments, the subject is a human, *e.g.,* a human cancer patient.

Sequence reads are then generated (312) from the sequencing library or pool of sequencing libraries. Sequencing data may be acquired by any methodology known in the art. For example, next generation sequencing (NGS) techniques such as sequencing-by-synthesis technology (Illumina), pyrosequencing (454 Life Sciences), ion semiconductor technology (Ion Torrent sequencing), single-molecule real-time sequencing (Pacific Biosciences), sequencing by ligation (SOLiD sequencing), NANOPORE® sequencing (Oxford Nanopore Technologies), or paired-end sequencing. In some embodiments, massively parallel sequencing is performed using sequencing-by-synthesis with reversible dye terminators. In some embodiments, sequencing is performed using next generation sequencing technologies, such as short-read technologies. In other embodiments, long-read sequencing or another sequencing method known in the art is used.

*Homologous Recombination Status (HRD):*

In some embodiments, analysis of aligned sequence reads, *e.g.,* in SAM or BAM format, includes analysis of whether the cancer is homologous recombination deficient (HRD status 137-3), using a homologous recombination pathway analysis module 157.

Homologous recombination (HR) is a normal, highly conserved DNA repair process that enables the exchange of genetic information between identical or closely related DNA molecules. It is most widely used by cells to accurately repair harmful breaks (*e.g.* damage) that occur on both strands of DNA. DNA damage may occur from exogenous (external) sources like UV light, radiation, or chemical damage; or from endogenous (internal) sources like errors in DNA replication or other cellular processes that create DNA damage. Double strand breaks are a type of DNA damage. Using poly (ADP-ribose) polymerase (PARP) inhibitors in patients with HRD compromises two pathways of DNA repair, resulting in cell death (apoptosis). The efficacy of PARP inhibitors is improved not only in ovarian cancers displaying germline or somatic BRCA mutations, but also in cancers in which HRD is caused by other underlying etiologies.

In some embodiments, HRD status can be determined by inputting features correlated with HRD status into a classifier trained to distinguish between cancers with homologous recombination pathway deficiencies and cancers without homologous recombination pathway deficiencies. For example, in some embodiments, the features include one or more of (i) a heterozygosity status for a first plurality of DNA damage repair genes in the genome of the cancerous tissue of the subject, (ii) a measure of the loss of heterozygosity across the genome of the cancerous tissue of the subject, (iii) a measure of variant alleles detected in a second plurality of DNA damage repair genes in the genome of the cancerous tissue of the subject, and (iv) a measure of variant alleles detected in the second plurality of DNA damage repair genes in the genome of the non-cancerous tissue of the subject. In some embodiments, all four of the features described above are used as features in an HRD classifier. More details about HRD classifiers using these and other features are described in U.S. Patent Application Publication No. 2020/0255909, the content of which is hereby incorporated by reference, in its entirety, for all purposes.

*Concurrent Testing*

Unless stated otherwise, as used herein, the term "concurrent" as it relates to assays refers to a period of time between zero and ninety days. In some embodiments, concurrent tests using different biological samples from the same subject (e.g., two or more of a liquid biopsy sample, cancerous tissue-such as a solid tumor sample or blood sample for a blood-based cancer-and a non-cancerous sample) are performed within a period of time (e.g., the biological samples are collected within the period of time) of from 0 days to 90 days. In some embodiments, concurrent tests using different biological samples from the same subject (e.g., two or more of a liquid biopsy sample, cancerous tissue-such as a solid tumor sample or blood sample for a blood-based cancer-and a non-cancerous sample) are performed within a period of time (e.g., the biological samples are collected within the period of time) of from 0 days to 60 days. In some embodiments, concurrent tests using different biological samples from the same subject (e.g., two or more of a liquid biopsy sample, cancerous tissue-such as a solid tumor sample or blood sample for a blood-based cancer-and a non-cancerous sample) are performed within a period of time (e.g., the biological samples are collected within the period of time) of from 0 days to 30 days. In some embodiments, concurrent tests using different biological samples from the same subject (e.g., two or more of a liquid biopsy sample, cancerous tissue-such as a solid tumor sample or blood sample for a blood-based cancer-and a non-cancerous sample) are performed within a period of time (e.g., the biological samples are collected within the period of time) of from 0 days to 21 days. In some embodiments, concurrent tests using different biological samples from the same subject (e.g., two or more of a liquid biopsy sample, cancerous tissue-such as a solid tumor sample or blood sample for a blood-based cancer-and a non-cancerous sample) are performed within a period of time (e.g., the biological samples are collected within the period of time) of from 0 days to 14 days. In some embodiments, concurrent tests using different biological samples from the same subject (e.g., two or more of a liquid biopsy sample, cancerous tissue-such as a solid tumor sample or blood sample for a blood-based cancer-and a non-cancerous sample) are performed within a period of time (e.g., the biological samples are collected within the period of time) of from 0 days to 7 days. In some embodiments, concurrent tests using different biological samples from the same subject (e.g., two or more of a liquid biopsy sample, cancerous tissue-such as a solid tumor sample or blood sample for a blood-based cancer-and a non-cancerous sample) are performed within a period of time (e.g., the biological samples are collected within the period of time) of from 0 days to 3 days.

In some embodiments, a liquid biopsy assay is used concurrently with a solid tumor assay to return more comprehensive information about a patient's variants. For example, a blood specimen and a solid tumor specimen may be sent to a laboratory for evaluation. The solid tumor specimen may be analyzed using a bioinformatics pipeline to produce a solid tumor result. A solid tumor assay is described, for instance, in U.S. Patent No. 11/705,226, the content of which is hereby incorporated by reference, in its entirety, for all purposes. The cancer type of the solid tumor may include, for example, non small-cell lung cancer, colorectal cancer, or breast cancer. Alterations identified in the tumor/matched normal result may include, for example, EGFR+ for non-small cell lung cancer; HER2+ for breast cancer; or KRAS G12C for several cancers.

In some embodiments, a blood specimen may be divided into a first portion and a second portion. The first portion of the blood specimen and the solid tumor specimen may be analyzed using a bioinformatics pipeline to produce a tumor/matched normal result. The second portion of the blood specimen may be analyzed using a bioinformatics pipeline to produce a liquid biopsy result. For example, the blood specimen may be analyzed using at least an improvement in somatic variant identification, e.g., as described herein in the section entitled "Variant Identification." For example, the blood specimen may be analyzed using an improvement in focal copy number identification, e.g., as described herein in the section entitled "Copy Number Variation." For example, the blood specimen may be analyzed using an improvement in circulating tumor fraction determination, e.g., using the methods disclosed herein.

In some embodiments, therapies are identified for further consideration in response to receiving the tumor or tumor/matched normal result along with the liquid biopsy result. In one example, when the results overall indicate that the patient has HER2+ breast cancer, the cancer treatment NERATINIB is identified along with the test results for further consideration by the ordering clinician.

In some embodiments, the solid tumor or tumor/matched normal assay is ordered concurrently; their results are delivered concurrently; and they are analyzed concurrently.

*Systems and Methods for Improved Circulating Tumor Fraction Estimates*

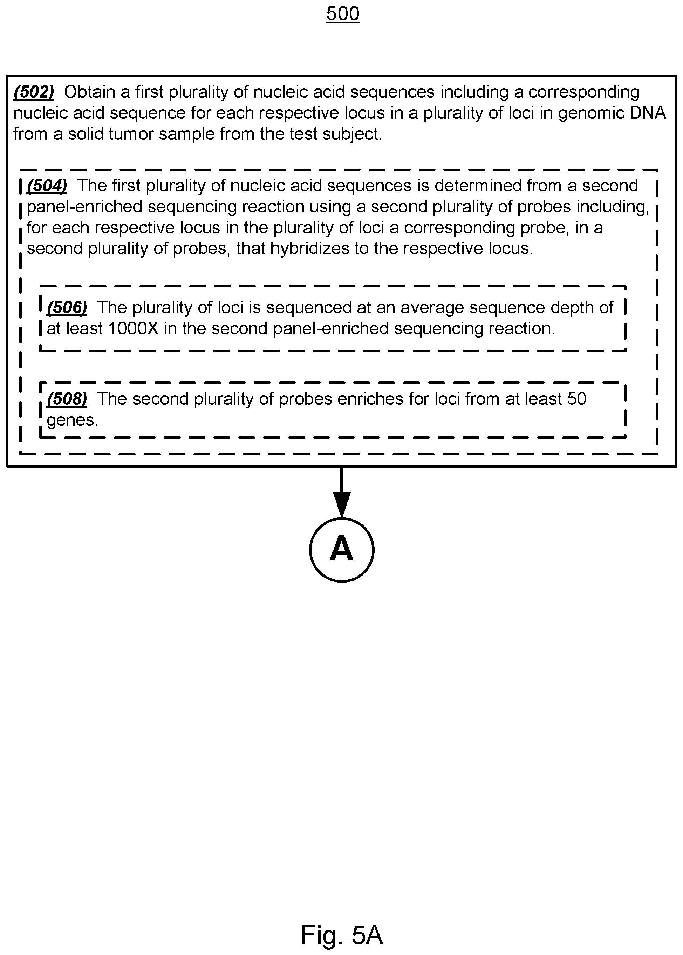

An overview of methods for providing clinical support for personalized cancer therapy is described above with reference to Figures 2-4 above. Below, systems and methods for improving circulating tumor fraction estimates, e.g., within the context of the methods and systems described above, are described with reference to Figures 5A-5D.

Many of the embodiments described below, in conjunction with Figures 5A-5D, relate to analyses performed using sequencing data for genomic data from solid tumor samples and for cfDNA obtained from a liquid biopsy sample of a subject, e.g., a cancer patient. Generally, these embodiments are independent and, thus, not reliant upon any particular DNA sequencing methods. However, in some embodiments, the methods described below include generating the sequencing data.

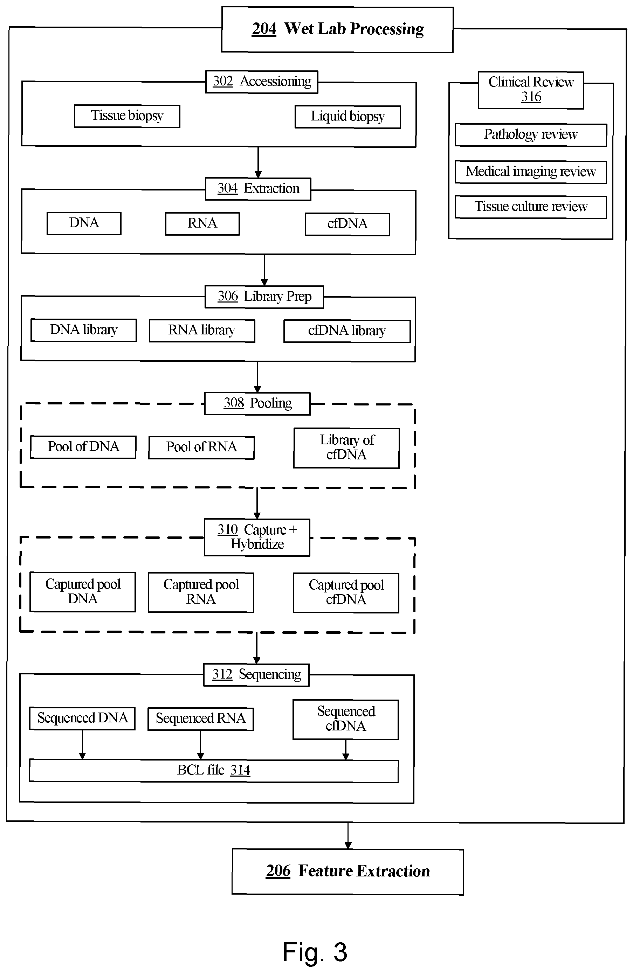

As described herein, in some embodiments, the methods described herein (*e.g.,* method 500 as illustrated in Figures 5A-5D) include one or more data collection steps, in addition to data analysis and downstream steps. For example, as described herein, *e.g.*, with reference to Figures 2 and 3, in some embodiments, the methods include collection of a solid tumor biopsy, a liquid biopsy sample and, optionally, one or more matching biological samples from the subject (*e.g., a* matched cancerous sample from the subject). Likewise, as described herein, *e.g.*, with reference to Figures 2 and 3, in some embodiments, the methods include extraction of genomic DNA (gDNA) from the solid tumor biopsy and, optionally, matched non-cancerous sample. Similarly, as described herein, e.g., with reference to Figures 2 and 3, in some embodiments, the methods include isolation of cfDNA fragments from the liquid biopsy sample (cfDNA). Similarly, as described herein, *e.g.*, with reference to Figures 2 and 3, in some embodiments, the methods include nucleic acid sequencing of the gDNA extracted from a solid tumor biopsy, the cfDNA from the liquid biopsy sample and/or, optionally, one or more matching biological samples from the subject (*e.g., a* matched cancerous and/or matched non-cancerous sample from the subject).

However, in other embodiments, the methods described herein begin with obtaining nucleic acid sequencing results, *e.g.*, raw or collapsed sequence reads of gDNA from a solid tumor biopsy, cfDNA from a liquid biopsy sample (cfDNA) and, optionally, one or more matching biological samples from the subject (*e.g., a* matched cancerous and/or matched non-cancerous sample from the subject), from which the genomic features needed for estimating circulating tumor fraction (*e.g.*, variant allele identification and/or variant allele fraction) can be determined. For example, in some embodiments, sequencing data 122 for a patient 121 is accessed and/or downloaded over network 105 by system 100.

In some embodiments, the method further comprises isolating the plurality of cell-free nucleic acids from the liquid biopsy sample of the test subject prior to the sequencing. In some embodiments, the sequencing is multiplexed sequencing. In some embodiments, the sequencing is short-read sequencing or long-read sequencing.

In some embodiments, the pathology data 128-1 collected during clinical evaluation includes visual features identified by a pathologist's inspection of a specimen (*e.g.,* a solid tumor biopsy), *e.g.,* of stained H&E or IHC slides. In some embodiments, the sample is a solid tissue biopsy sample. In some embodiments, the tissue biopsy sample is a formalin-fixed tissue (FFT), *e.g.,* a formalin-fixed paraffin-embedded (FFPE) tissue. In some embodiments, the tissue biopsy sample is an FFPE or FFT block. In some embodiments, the tissue biopsy sample is a fresh-frozen tissue biopsy. The tissue biopsy sample can be prepared in thin sections (*e.g.*, by cutting and/or affixing to a slide), to facilitate pathology review (*e.g.*, by staining with immunohistochemistry stain for IHC review and/or with hematoxylin and eosin stain for H&E pathology review). For instance, analysis of slides for H&E staining or IHC staining may reveal features such as tumor infiltration, programmed death-ligand 1 (PD-L1) status, human leukocyte antigen (HLA) status, or other immunological features.

Similarly, in some embodiments, the methods described herein begin with obtaining the genomic features needed for filtering of clonal hematopoiesis variants from a sequencing of a liquid biopsy sample and, optionally, one or more matching biological samples from the subject (*e.g.,* a matched cancerous and/or matched non-cancerous sample from the subject). For example, in some embodiments, (i) one or more fragment length metrics, (ii) a variant allele fraction for the candidate somatic variant and a ctFE for the liquid biopsy sample or one or more features determined from the variant allele fraction for the candidate somatic variant and the ctFE for the liquid biopsy sample, and (iii) one or more metrics of clonal hematopoiesis prevalence for the first nucleotide position, is accessed and/or downloaded over network 105 by system 100.

Similarly, in some embodiments, the methods described herein begin with obtaining the genomic features needed for estimating circulating tumor fraction (*e.g.*, variant allele identification and/or variant allele fraction) for sequencing data for gDNA from a solid tumor biopsy, cfDNA from a liquid biopsy sample (cfDNA) and, optionally, one or more matching biological samples from the subject (*e.g.,* a matched cancerous and/or matched non-cancerous sample from the subject). For example, in some embodiments, variant allele counts and/or variant allele fractions for sequencing data 122 of patient 121 is accessed and/or downloaded over network 105 by system 100.

Figures 5A-5D collectively provide a flow chart of processes and features for determining an estimate of a circulating tumor fraction for a test subject, in accordance with some embodiments of the present disclosure.

The present disclosure provides a method 500 for estimating a circulating tumor fraction for a test subject from panel-enriched sequencing data for a plurality of sequences.

*Block 502.* Referring to block 502, in some embodiments, the method includes obtaining a first plurality of nucleic acid sequences including a corresponding nucleic acid sequence for each respective locus in a plurality of loci in genomic DNA from a solid tumor sample from the test subject.

In some embodiments, the solid tumor sample is a particular type of cancer. Nonlimiting examples of cancer types include lung cancer, breast cancer, ovarian cancer, cervical cancer, uveal melanoma, colorectal cancer, chromophobe renal cell carcinoma, liver cancer, endocrine tumor, oropharyngeal cancer, retinoblastoma, biliary cancer, adrenal cancer, neural cancer, neuroblastoma, basal cell carcinoma, brain cancer, non-clear cell renal cell carcinoma, glioblastoma, glioma, kidney cancer, gastrointestinal stromal tumor, medulloblastoma, bladder cancer, gastric cancer, bone cancer, thymoma, prostate cancer, clear cell renal cell carcinoma, skin cancer, thyroid cancer, sarcoma, testicular cancer, head and neck cancer (*e.g.,* head and neck squamous cell carcinoma), meningioma, peritoneal cancer, endometrial cancer, pancreatic cancer, mesothelioma, esophageal cancer, small cell lung cancer, Her2 negative breast cancer, ovarian serous carcinoma, HR+ breast cancer, uterine serous carcinoma, uterine corpus endometrial carcinoma, gastroesophageal junction adenocarcinoma, gallbladder cancer, chordoma, and papillary renal cell carcinoma.

In some embodiments, the cancer is a particular stage of a particular type of cancer. In some such embodiments, the stage of the particular type of cancer is a stage of cancer the subject was diagnosed with prior to treatment. Cancer is typically staged to determine the extent of its spread and to guide treatment decisions. The stage of cancer refers to the extent to which it has grown and spread from its original location. Each cancer has its own criteria for determine stage but generally relies on a determination of the size of the primary tumor (T) and whether it has invaded nearby tissues, evaluate of lymph node involvement (N) to find indications of whether cancer has spread to nearby lymph nodes, and assessment of distant metastasis (M), which indicates whether the cancer has spread to distant organs or tissues. Metastasis means that cancer has spread from the primary site to other parts of the body. In some embodiments the staging system used is the cancer TNM system, which combines the T, N, and M information to assign a stage. In some embodiments the stages are denoted using Roman numerals (I, II, III, IV) and may have subcategories (e.g., stage IIA, stage IIB) to provide more precise information. In a brief overview of these stages , in stage 0, the cancer is *in situ,* meaning it is confined to the layer of cells where it began and has not invaded nearby tissues, in stage I: the cancer is localized and small in size, in stage II, the cancer may be larger and/or have spread to nearby lymph nodes, but it is still relatively localized, in stage III, the cancer has typically spread further into nearby tissues and may involve more lymph nodes, in stage IV, the cancer has spread to distant organs or tissues, indicating metastasis. This is often the most advanced stage. The specific criteria for each stage can vary depending on the type of cancer. See, for example, details of TNM staging for breast cancer in Part et al., 2011, "Clinical relevance of TNM staging system according to breast cancer subtypes," Annals of Oncology 22(7), pp. 1554-1560, which is hereby incorporated by reference. Additionally, some cancers have their own staging systems tailored to their characteristics. *See* the Internet at the URL cancer.gov/about-cancer/diagnosis-staging/staging.

In some embodiments, the first plurality of nucleic acid sequences is obtained from a first sequencing reaction that is performed at a read depth of at least 1X. In some embodiments, the first sequencing reaction is a panel-enriched sequencing reaction is performed at a read depth of at least 2X, at least 3X, at least 4X, at least 5X, at least 10X, at least 25X, at least 50X, at least 100X, at least 250X, 400X, 500X, 600X or greater. In some embodiments, the first sequencing reaction is a panel based or a whole genome sequencing reaction that is performed at a read depth of no more than 1000X, no more than 500X, no more than 100X, no more than 50X, or less. In some embodiments, the first sequencing reaction is performed at a read depth of from 1X to 500X, from 1X to 100X, or from 1X to 50X. In some embodiments, the first sequencing reaction is performed at a read depth of from 2.5X to 500X, from 2.5X to 100X, or from 2.5X to 50X. In some embodiments, the first sequencing reaction is performed at a read depth of from 5X to 500X, from 5X to 100X, or from 5X to 50X. In some embodiments, the first sequencing reaction is performed at a read depth of from 10X to 500X, from 10X to 100X, or from 10X to 50X.

In some embodiments, the first plurality of sequence reads is from a panel-enriched sequencing reaction that includes a first subset of sequence reads corresponding to cfDNA fragments targeted by one or more probes in a targeted enrichment panel. In some embodiments, each respective cell-free DNA fragment in the first plurality of cell-free DNA fragments corresponds to a respective probe sequence in a plurality of probe sequences used to enrich cell-free DNA fragments in the liquid biopsy sample in the panel-enriched sequencing reaction. In some embodiments, the plurality of probe sequences map to no more than 150 genes in the human genome.

In some embodiments, the first plurality of nucleic acid sequences is obtained from a plurality of sequence reads that are obtained by a whole genome or whole exome sequencing methodology. In some such embodiments, whole exome capture is performed with an automated system, using a liquid handling robot. Whole genome sequencing, and to some extent whole exome sequencing, is typically performed at lower sequencing depth than smaller target-panel sequencing reactions, because many more loci are being sequenced. For example, in some embodiments, whole genome or whole exome sequencing is performed to an average sequencing depth of at least 3x, at least 5x, at least 10x, at least 15x, at least 20x, or greater. In some embodiments, low-pass whole genome sequencing (LPWGS) techniques are used for whole genome or whole exome sequencing. LPWGS is typically performed to an average sequencing depth of about 0.25x to about 5x, more typically to an average sequencing depth of about 0.5x to about 3x.

Because of the differences in the sequencing methodologies, data obtained from targeted-panel sequencing is better suited for certain analyses than data obtained from whole genome/whole exome sequencing, and vice versa. For instance, because of the higher sequencing depth achieved by targeted-panel sequencing, the resulting sequence data is better suited for the identification of variant alleles present at low allelic fractions in the sample, *e.g.,* less than 20%. By contrast, data generated from whole genome/whole exome sequencing is better suited for the estimation of genome-wide metrics, such as tumor mutational burden, because the entire genome is better represented in the sequencing data. Accordingly, in some embodiments, a nucleic acid sample, *e.g.,* a cfDNA, gDNA, or mRNA sample, is evaluated using both targeted-panel sequencing and whole genome/whole exome sequencing (*e.g.*, LPWGS).

In some embodiments, the raw sequence reads resulting from the sequencing reaction are output from the sequencer in a native file format, *e.g.,* a BCL file. In some embodiments, the native file is passed directly to a bioinformatics pipeline (*e.g.*, variant analysis 206), components of which are described in detail below. In other embodiments, pre-processing is performed prior to passing the sequences to the bioinformatics platform. For instance, in some embodiments, the format of the sequence read file is converted from the native file format (*e.g.,* BCL) to a file format compatible with one or more algorithms used in the bioinformatics pipeline (*e.g.*, FASTQ or FASTA). In some embodiments, the raw sequence reads are filtered to remove sequences that do not meet one or more quality thresholds. In some embodiments, raw sequence reads generated from the same unique nucleic acid molecule in the sequencing read are collapsed into a single sequence read representing the molecule, *e.g.*, using UMIs as described above. In some embodiments, one or more of these pre-processing activities is performed within the bioinformatics pipeline itself.

In one example, a sequencer may generate a BCL file. A BCL file may include raw image data of a plurality of patient specimens which are sequenced. BCL image data is an image of the flow cell across each cycle during sequencing. A cycle may be implemented by illuminating a patient specimen with a specific wavelength of electromagnetic radiation, generating a plurality of images which may be processed into base calls via BCL to FASTQ processing algorithms which identify which base pairs are present at each cycle. The resulting FASTQ file includes the entirety of reads for each patient specimen paired with a quality metric, *e.g.,* in a range from 0 to 64 where a 64 is the best quality and a 0 is the worst quality.

FASTQ format is a text-based format for storing both a biological sequence, such as a nucleotide sequence, and its corresponding quality scores. These FASTQ files are analyzed to determine what genetic variants or copy number changes are present in the sample. Each FASTQ file contains reads that may be paired-end or single reads, and may be short-reads or long-reads, where each read represents one detected sequence of nucleotides in a nucleic acid molecule that was isolated from the patient sample or a copy of the nucleic acid molecule, detected by the sequencer. Each read in the FASTQ file is also associated with a quality rating. The quality rating may reflect the likelihood that an error occurred during the sequencing procedure that affected the associated read. In some embodiments, the results of paired-end sequencing of each isolated nucleic acid sample are contained in a split pair of FASTQ files, for efficiency. Thus, in some embodiments, forward (Read 1) and reverse (Read 2) sequences of each isolated nucleic acid sample are stored separately but in the same order and under the same identifier.

In various embodiments, the bioinformatics pipeline may filter FASTQ data from the corresponding sequence data file for each respective biological sample. Such filtering may include correcting or masking sequencer errors and removing (trimming) low quality sequences or bases, adapter sequences, contaminations, chimeric reads, overrepresented sequences, biases caused by library preparation, amplification, or capture, and other errors.

While workflow 200 illustrates obtaining a biological sample, extracting nucleic acids from the biological sample, and sequencing the isolated nucleic acids, in some embodiments, sequencing data used in the improved systems and methods described herein (*e.g.*, which include improved methods for determining accurate circulating tumor fraction estimates) is obtained by receiving previously generated sequence reads, in electronic form.

In some embodiments, sequencing of the plurality nucleic acids in the biopsy sample of the subject is performed at a central laboratory or sequencing facility. In some such embodiments, the method comprises accessing one or more sequencing datasets and/or one or more auxiliary files, in electronic form, through a cloud-based interface. For example, a dataset can be obtained by performing a bioinformatics pipeline using tumor BAM files, normal BAM files, a human reference genome file, a target region BED file, a list of mappable regions of the genome, and/or a blacklist of recurrent problematic areas of the genome.

In some embodiments, the obtaining the dataset comprises accessing the dataset, in electronic form, through a cloud-based interface. For example, a dataset can comprise one or more outputs from a bioinformatics pipeline (*e.g.*, CNVkit outputs ".cns" and/or ".cnr").

Additional methods and embodiments for sequencing nucleic acids, including aligning and preprocessing sequence reads, are described in further detail herein. Additional methods and embodiments for performing the presently disclosed methods at a distributed diagnostic and clinical environment are described in detail above (see, Example Methods: Figure 2B: Distributed Diagnostic and Clinical Environment). Other embodiments and/or any combinations, substitutions, additions or deletions thereof are possible, as will be apparent to one skilled in the art.

In accordance with block 502 and with further reference to Figure 2B, nucleic acid sequencing of one or more samples collected from the subject is performed, *e.g.,* at sequencing lab 230, during wet lab processing 204, in some embodiments. An example workflow for nucleic acid sequencing is illustrated in Figure 3. In some embodiments, the one or more biological samples obtained at the sequencing lab 230 are accessioned (302), to track the sample and data through the sequencing process.

Next, nucleic acids, *e.g.,* RNA and/or DNA are extracted (304) from the one or more biological samples. Methods for isolating nucleic acids from biological samples are known in the art, and are dependent upon the type of nucleic acid being isolated (*e.g.*, cfDNA, DNA, and/or RNA) and the type of sample from which the nucleic acids are being isolated (*e.g.*, liquid biopsy samples, white blood cell buffy coat preparations, formalin-fixed paraffin-embedded (FFPE) solid tissue samples, and fresh frozen solid tissue samples). The selection of any particular nucleic acid isolation technique for use in conjunction with the embodiments described herein is well within the skill of the person having ordinary skill in the art, who will consider the sample type, the state of the sample, the type of nucleic acid being sequenced and the sequencing technology being used.

For instance, many techniques for DNA isolation, *e.g.*, genomic DNA isolation, from a tissue sample are known in the art, such as organic extraction, silica adsorption, and anion exchange chromatography. Likewise, many techniques for RNA isolation, *e.g.*, mRNA isolation, from a tissue sample are known in the art. For example, acid guanidinium thiocyanate-phenol-chloroform extraction (see, for example, Chomczynski and Sacchi, 2006, Nat Protoc, 1(2):581-85, which is hereby incorporated by reference herein), and silica bead/glass fiber adsorption (see, for example, Poeckh et al., 2008, Anal Biochem., 373(2):253-62, which is hereby incorporated by reference herein). The selection of any particular DNA or RNA isolation technique for use in conjunction with the embodiments described herein is well within the skill of the person having ordinary skill in the art, who will consider the tissue type, the state of the tissue, *e.g.*, fresh, frozen, formalin-fixed, paraffin-embedded (FFPE), and the type of nucleic acid analysis that is to be performed.

With reference to block 508 below, in embodiments where the biological sample is a liquid biopsy sample, *e.g.,* a blood or blood plasma sample, and cfDNA is isolated from blood samples using commercially available reagents, including proteinase K, to generate a liquid solution of cfDNA.

In some embodiments, isolated DNA molecules are mechanically sheared to an average length using an ultrasonicator (for example, a Covaris ultrasonicator). In some embodiments, isolated nucleic acid molecules are analyzed to determine their fragment size, *e.g.,* through gel electrophoresis techniques and/or the use of a device such as a LabChip GX Touch. The skilled artisan will know of an appropriate range of fragment sizes, based on the sequencing technique being employed, as different sequencing techniques have differing fragment size requirements for robust sequencing. In some embodiments, quality control testing is performed on the extracted nucleic acids (*e.g.,* DNA and/or RNA), *e.g.,* to assess the nucleic acid concentration and/or fragment size. For example, sizing of DNA fragments provides valuable information used for downstream processing, such as determining whether DNA fragments require additional shearing prior to sequencing.

Wet lab processing 204 then includes preparing a nucleic acid library from the isolated nucleic acids (*e.g.*, cfDNA, DNA, and/or RNA). For example, in some embodiments, DNA libraries (*e.g.*, gDNA and/or cfDNA libraries) are prepared from isolated DNA from the one or more biological samples. In some embodiments, the DNA libraries are prepared using a commercial library preparation kit, *e.g.,* the KAPA Hyper Prep Kit, a New England Biolabs (NEB) kit, or a similar kit.

In some embodiments, during library preparation, adapters (*e.g.,* UDI adapters, such as Roche SeqCap dual end adapters, or UMI adapters such as full length or stubby Y adapters) are ligated onto the nucleic acid molecules. In some embodiments, the adapters include unique molecular identifiers (UMIs), which are short nucleic acid sequences (e.g., 3-10 base pairs) that are added to ends of DNA fragments during adapter ligation. In some embodiments, UMIs are degenerate base pairs that serve as a unique tag that can be used to identify sequence reads originating from a specific DNA fragment. In some embodiments, *e.g.,* when multiplex sequencing will be used to sequence DNA from a plurality of samples (*e.g.,* from the same or different subjects) in a single sequencing reaction, a patient-specific index is also added to the nucleic acid molecules. In some embodiments, the patient specific index is a short nucleic acid sequence (*e.g.,* 3-20 nucleotides) that are added to ends of DNA fragments during library construction, that serve as a unique tag that can be used to identify sequence reads originating from a specific patient sample. Examples of identifier sequences are described in Kivioja et al., 2011, Nat. Methods 9(1):72-74 and Islam et al., 2014, Nat. Methods 11(2):163-66, the contents of which are hereby incorporated by reference, in their entireties, for all purposes.

In some embodiments, an adapter includes a PCR primer landing site, designed for efficient binding of a PCR or second-strand synthesis primer used during the sequencing reaction. In some embodiments, an adapter includes an anchor binding site, to facilitate binding of the DNA molecule to anchor oligonucleotide molecules on a sequencer flow cell, serving as a seed for the sequencing process by providing a starting point for the sequencing reaction. During PCR amplification following adapter ligation, the UMIs, patient indexes, and binding sites are replicated along with the attached DNA fragment. This provides a way to identify sequence reads that came from the same original fragment in downstream analysis.

In some embodiments, DNA libraries are amplified and purified using commercial reagents, (*e.g.*, Axygen MAG PCR clean up beads). In some such embodiments, the concentration and/or quantity of the DNA molecules are then quantified using a fluorescent dye and a fluorescence microplate reader, standard spectrofluorometer, or filter fluorometer. In some embodiments, library amplification is performed on a device (*e.g.,* an Illumina C-Bot2) and the resulting flow cell containing amplified target-captured DNA libraries is sequenced on a next generation sequencer (*e.g.*, an Illumina HiSeq 4000 or an Illumina NovaSeq 6000) to a unique on-target depth selected by the user. In some embodiments, DNA library preparation is performed with an automated system, using a liquid handling robot (*e.g.,* a SciClone NGSx).

In some embodiments, where feature data 125 includes methylation states 132 for one or more genomic locations, nucleic acids isolated from the biological sample (*e.g.*, cfDNA) are treated to convert unmethylated cytosines to uracils, *e.g.*, prior to generating the sequencing library. Accordingly, when the nucleic acids are sequenced, all cytosines called in the sequencing reaction were necessarily methylated, since the unmethylated cytosines were converted to uracils and accordingly would have been called as thymidines, rather than cytosines, in the sequencing reaction. Commercial kits are available for bisulfite-mediated conversion of methylated cytosines to uracils. Commercial kits are also available for enzymatic conversion of methylated cytosines to uracils.

In some embodiments, wet lab processing 204 includes pooling (308) DNA molecules from a plurality of libraries, corresponding to different samples from the same and/or different patients, to form a sequencing pool of DNA libraries. When the pool of DNA libraries is sequenced, the resulting sequence reads correspond to nucleic acids isolated from multiple samples. The sequence reads can be separated into different sequence read data structures (e.g., files), corresponding to the various samples represented in the sequencing read based on the unique identifiers present in the added nucleic acid fragments. In this fashion, a single sequencing reaction can generate sequence reads from multiple samples. Advantageously, this allows for the processing of more samples per sequencing reaction.

In some embodiments, wet lab processing 204 includes enriching (310) a sequencing library, or pool of sequencing libraries, for target nucleic acids, *e.g.*, nucleic acids encompassing loci that are informative for precision oncology and/or used as internal controls for the sequencing or bioinformatics processes. In some embodiments, enrichment is achieved by hybridizing target nucleic acids in the sequencing library to probes that hybridize to the target sequences, and then isolating the captured nucleic acids away from off-target nucleic acids that are not bound by the capture probes. In some embodiments, some off-target nucleic acids will remain in the final sequencing pool.

In some embodiments, the first plurality of sequence reads that is obtained from the above described sequencing includes at least 10,000 sequence reads, at least 50,000 sequence reads, at least 100,000 sequence reads, at least 500,000 sequence reads, at least 1 million sequence reads, at least 5 million sequence reads, at least 10 million sequence reads, or more. In some embodiments, the first plurality of sequence reads includes no more than 1 billion sequence reads, no more than 500 million sequence reads, no more than 100 million sequence reads, no more than 50 million sequence reads, no more than 10 million sequence reads, no more than 5 million sequence reads, no more than 1 million sequence reads, or less. In some embodiments, the first plurality of sequence reads is from 10,000 sequence reads to 1 billion sequence reads, from 10,000 sequence reads to 500 million sequence reads, from 10,000 sequence reads to 100 million sequence reads, from 10,000 sequence reads to 50 million sequence reads, from 10,000 sequence reads to 10 million sequence reads, from 10,000 sequence reads to 5 million sequence reads, or from 10,000 sequence reads to 1 million sequence reads. In some embodiments, the first plurality of sequence reads is from 100,000 sequence reads to 1 billion sequence reads, from 100,000 sequence reads to 500 million sequence reads, from 100,000 sequence reads to 100 million sequence reads, from 100,000 sequence reads to 50 million sequence reads, from 100,000 sequence reads to 10 million sequence reads, from 100,000 sequence reads to 5 million sequence reads, or from 100,000 sequence reads to 1 million sequence reads. In some embodiments, the first plurality of sequence reads is from 500,000 sequence reads to 1 billion sequence reads, from 500,000 sequence reads to 500 million sequence reads, from 500,000 sequence reads to 100 million sequence reads, from 500,000 sequence reads to 50 million sequence reads, from 500,000 sequence reads to 10 million sequence reads, from 500,000 sequence reads to 5 million sequence reads, or from 500,000 sequence reads to 1 million sequence reads. In some embodiments, the first plurality of sequence reads is from 1 million sequence reads to 1 billion sequence reads, from 1 million sequence reads to 500 million sequence reads, from 1 million sequence reads to 100 million sequence reads, from 1 million sequence reads to 50 million sequence reads, from 1 million sequence reads to 10 million sequence reads, or from 1 million sequence reads to 5 million sequence reads.

In some embodiments, the genomic DNA from the solid tumor sample of the subject comprises a first plurality of nucleic acid fragments. In some embodiments the first plurality of nucleic acid fragments includes at least 1000 DNA fragments, at least 5000 DNA fragments, at least 10,000 DNA fragments, at least 50,000 DNA fragments, at least 100,000 DNA fragments, at least 500,000 DNA fragments, at least 1 million DNA fragments, at least 5 million DNA fragments, or more. In some embodiments, the first plurality of nucleic acid fragments includes no more than 100 million DNA fragments, no more than 50 million DNA fragments, no more than 10 million DNA fragments, no more than 5 million DNA fragments, no more than 1 million DNA fragments, no more than 500,000 DNA fragments, or no more than 100,000 DNA fragments. In some embodiments, the first plurality of DNA fragments is from 1000 DNA fragments to 500 million DNA fragments, from 1000 DNA fragments to 100 million DNA fragments, from 1000 DNA fragments to 50 million DNA fragments, from 1000 DNA fragments to 10 million DNA fragments, from 1000 DNA fragments to 5 million DNA fragments, from 1000 DNA fragments to 1 million DNA fragments, from 1000 DNA fragments to 500,000 DNA fragments, from 1000 DNA fragments to 250,000 DNA fragments, or from 1000 DNA fragments to 100,000 DNA fragments. In some embodiments, the first plurality of DNA fragments is from 5000 DNA fragments to 500 million DNA fragments, from 5000 DNA fragments to 100 million DNA fragments, from 5000 DNA fragments to 50 million DNA fragments, from 5000 DNA fragments to 10 million DNA fragments, from 5000 DNA fragments to 5 million DNA fragments, from 5000 DNA fragments to 1 million DNA fragments, from 5000 DNA fragments to 500,000 DNA fragments, from 5000 DNA fragments to 250,000 DNA fragments, or from 5000 DNA fragments to 100,000 DNA fragments. In some embodiments, the first plurality of DNA fragments is from 10,000 DNA fragments to 500 million DNA fragments, from 10,000 DNA fragments to 100 million DNA fragments, from 10,000 DNA fragments to 50 million DNA fragments, from 10,000 DNA fragments to 10 million DNA fragments, from 10,000 DNA fragments to 5 million DNA fragments, from 10,000 DNA fragments to 1 million DNA fragments, from 10,000 DNA fragments to 500,000 DNA fragments, from 10,000 DNA fragments to 250,000 DNA fragments, or from 10,000 DNA fragments to 100,000 DNA fragments. In some embodiments, the first plurality of DNA fragments is from 25,000 DNA fragments to 500 million DNA fragments, from 25,000 DNA fragments to 100 million DNA fragments, from 25,000 DNA fragments to 50 million DNA fragments, from 25,000 DNA fragments to 10 million DNA fragments, from 25,000 DNA fragments to 5 million DNA fragments, from 25,000 DNA fragments to 1 million DNA fragments, from 25,000 DNA fragments to 500,000 DNA fragments, from 25,000 DNA fragments to 250,000 DNA fragments, or from 25,000 DNA fragments to 100,000 DNA fragments.

In some embodiments, the sequencing data is processed (*e.g.*, using sequence data processing module 141) to prepare it for genomic feature identification 385. For instance, in some embodiments as described above, the sequencing data is present in a native file format provided by the sequencer. Accordingly, in some embodiments, the system (*e.g.*, system 100) applies a pre-processing algorithm 142 to convert the file format (318) to one that is recognized by one or more upstream processing algorithms. For example, BCL file outputs from a sequencer can be converted to a FASTQ file format using the bcl2fastq or bcl2fastq2 conversion software (Illumina®). FASTQ format is a text-based format for storing both a biological sequence, such as nucleotide sequence, and its corresponding quality scores. These FASTQ files are analyzed to determine what genetic variants, copy number changes, *etc.,* are present in the sample.

In some embodiments, other preprocessing functions are performed, *e.g.*, filtering sequence reads 122 based on a desired quality, *e.g.*, size and/or quality of the base calling. In some embodiments, quality control checks are performed to ensure the data is sufficient for variant calling. For instance, entire reads, individual nucleotides, or multiple nucleotides that are likely to have errors may be discarded based on the quality rating associated with the read in the FASTQ file, the known error rate of the sequencer, and/or a comparison between each nucleotide in the read and one or more nucleotides in other reads that has been aligned to the same location in the reference genome. Filtering may be done in part or in its entirety by various software tools, for example, a software tool such as Skewer. See, Jiang et al., 2014, BMC Bioinformatics 15(182):1-12. FASTQ files may be analyzed for rapid assessment of quality control and reads, for example, by a sequencing data QC software such as AfterQC, Kraken, RNA-SeQC, FastQC, or another similar software program. For paired end reads, reads may be merged.

For efficiency, in some embodiments, the results of paired-end sequencing of each isolate are contained in a split pair of FASTQ files. Forward (Read 1) and reverse (Read 2) sequences of each tumor and normal isolate are stored separately but in the same order and under the same identifier. See, for example, Figure 4C. In various embodiments, the bioinformatics pipeline may filter FASTQ data from each isolate. Such filtering may include correcting or masking sequencer errors and removing (trimming) low quality sequences or bases, adapter sequences, contaminations, chimeric reads, overrepresented sequences, biases caused by library preparation, amplification, or capture, and other errors. See, for example, Figure 4D.

Similarly, in some embodiments, sequencing (312) is performed on a pool of nucleic acid sequencing libraries prepared from different biological samples, e.g., from the same or different patients. Accordingly, in some embodiments, the system demultiplexes (320) the data (*e.g.,* using demultiplexing algorithm 144) to separate sequence reads into separate files for each sequencing library included in the sequencing pool, *e.g.,* based on UMI or patient identifier sequences added to the nucleic acid fragments during sequencing library preparation, as described above. In some embodiments, the demultiplexing algorithm is part of the same software package as one or more pre-processing algorithms 142. For instance, commercial conversion software includes instructions for both converting the native file format output from the sequencer and demultiplexing sequence reads 122 output from the reaction.

The sequence reads are then aligned (322), *e.g.,* using an alignment algorithm 143, to a reference sequence construct 158, *e.g.,* a reference genome, reference exome, or other reference construct prepared for a particular sequencing reaction. For example, in some embodiments, individual sequence reads 123, in electronic form (*e.g.,* in FASTQ files), are aligned against a reference sequence construct for the species of the subject (*e.g.,* a reference human genome) by identifying a sequence in a region of the reference sequence construct that best matches the sequence of nucleotides in the sequence read. In some embodiments, the sequence reads are aligned to a reference exome or reference genome using known methods in the art to determine alignment position information. The alignment position information may indicate a beginning position and an end position of a region in the reference genome that corresponds to a beginning nucleotide base and end nucleotide base of a given sequence read. Alignment position information may also include sequence read length, which can be determined from the beginning position and end position. A region in the reference genome may be associated with a gene or a segment of a gene. Any of a variety of alignment tools can be used for this task.

For instance, local sequence alignment algorithms compare subsequences of different lengths in the query sequence (*e.g.*, sequence read) to subsequences in the subject sequence (*e.g.*, reference construct) to create the best alignment for each portion of the query sequence. In contrast, global sequence alignment algorithms align the entirety of the sequences, *e.g.*, end to end. Examples of local sequence alignment algorithms include the Smith-Waterman algorithm.

In some embodiments, the read mapping process starts by building an index of either the reference genome or the reads, which is then used to retrieve the set of positions in the reference sequence where the reads are more likely to align. Once this subset of possible mapping locations has been identified, alignment is performed in these candidate regions with slower and more sensitive algorithms. See, for example, Hatem et al., 2013, "Benchmarking short sequence mapping tools," BMC Bioinformatics 14: p. 184; and Flicek and Birney, 2009, "Sense from sequence reads: methods for alignment and assembly," Nat Methods 6(Suppl. 11), S6-S12, each of which is hereby incorporated by reference. In some embodiments, the mapping tools methodology makes use of a hash table or a Burrows-Wheeler transform (BWT). See, for example, Li and Homer, 2010, "A survey of sequence alignment algorithms for next-generation sequencing," Brief Bioinformatics 11, pp. 473-483, which is hereby incorporated by reference.

Other software programs designed to align reads include, for example, Novoalign (Novocraft, Inc.), Bowtie, Burrows Wheeler Aligner (BWA), and/or programs that use a Smith-Waterman algorithm. Candidate reference genomes include, for example, HG19, GRCh38, hg38, GRCh37, and/or other reference genomes developed by the Genome Reference Consortium. In some embodiments, the alignment generates a SAM file, which stores the locations of the start and end of each read according to coordinates in the reference genome and the coverage (number of reads) for each nucleotide in the reference genome.

For example, in some embodiments, each read of a FASTQ file is aligned to a location in the human genome having a sequence that best matches the sequence of nucleotides in the read. There are many software programs designed to align reads, for example, Novoalign (Novocraft, Inc.), Bowtie, Burrows Wheeler Aligner (BWA), programs that use a Smith-Waterman algorithm, *etc.* Alignment may be directed using a reference genome (for example, HG19, GRCh38, HG38, GRCh37, other reference genomes developed by the Genome Reference Consortium, *etc.)* by comparing the nucleotide sequences in each read with portions of the nucleotide sequence in the reference genome to determine the portion of the reference genome sequence that is most likely to correspond to the sequence in the read. In some embodiments, one or more SAM files are generated for the alignment, which store the locations of the start and end of each read according to coordinates in the reference genome and the coverage (number of reads) for each nucleotide in the reference genome. The SAM files may be converted to BAM files. In some embodiments, the BAM files are sorted, and duplicate reads are marked for deletion, resulting in de-duplicated BAM files.

In some embodiments, adapter-trimmed FASTQ files are aligned to the 19th edition of the human reference genome build (HG19). Following alignment, reads are grouped by alignment position and UMI family and collapsed into consensus sequences. Bases with insufficient quality or significant disagreement among family members (for example, when it is uncertain whether the base is an adenine, cytosine, guanine, *etc.*) may be replaced by N's to represent a wildcard nucleotide type. PHRED scores are then scaled based on initial base calling estimates combined across all family members. Following single-strand consensus generation, duplex consensus sequences are generated by comparing the forward and reverse oriented PCR products with mirrored UMI sequences. In various embodiments, a consensus can be generated across read pairs. Otherwise, single-strand consensus calls will be used. Following consensus calling, filtering is performed to remove low-quality consensus fragments. The consensus fragments are then re-aligned to the human reference genome using BWA. A BAM output file is generated after the re-alignment, then sorted by alignment position, and indexed.

In some embodiments, the sequencing data is normalized, e.g., to account for pull-down, amplification, and/or sequencing bias (*e.g.,* mappability, GC bias *etc.*).

In some embodiments, SAM files generated after alignment are converted to BAM files 124. Thus, after preprocessing sequencing data generated for a pooled sequencing reaction, BAM files are generated for each of the sequencing libraries present in the master sequencing pools. In some embodiments, BAM files are sorted, and duplicate reads are marked for deletion, resulting in de-duplicated BAM files. For example, tools like SamBAMBA mark and filter duplicate alignments in the sorted BAM files.

Alignment files prepared as described above (*e.g.*, BAM files 124) are then passed to a feature extraction module 145, where the sequences are analyzed (324) to identify genomic alterations (*e.g.*, SNVs/MNVs, indels, genomic rearrangements, copy number variations, *etc.*) and/or determine various characteristics of the patient's cancer (*e.g.*, MSI status, TMB, tumor ploidy, HRD status, tumor fraction, tumor purity, methylation patterns, *etc.*). Many software packages for identifying genomic alterations are known in the art. Generally, these software packages identify variants in sorted SAM or BAM files 124, relative to one or more reference sequence constructs 158. The software packages then output a file *e.g.*, a raw VCF (variant call format), listing the variants (*e.g.*, genomic features 131) called and identifying their location relevant to the reference sequence construct (*e.g.*, where the sequence of the sample nucleic acids differ from the corresponding sequence in the reference construct). In some embodiments, system 100 digests the contents of the native output file to populate feature data 125 in test patient data store 120. In other embodiments, the native output file serves as the record of these genomic features 131 in test patient data store 120.

Generally, the systems described herein can employ any combination of available variant calling software packages and internally developed variant identification algorithms. In some embodiments, the output of a particular algorithm of a variant calling software is further evaluated, *e.g.*, to improve variant identification. Accordingly, in some embodiments, system 100 employs an available variant calling software package to perform some of all of the functionality of one or more of the algorithms shown in feature extraction module 145.

In various aspects, the detected genetic variants and genetic features are analyzed as a form of quality control. For example, a pattern of detected genetic variants or features may indicate an issue related to the sample, sequencing procedure, and/or bioinformatics pipeline (*e.g.*, example, contamination of the sample, mislabeling of the sample, a change in reagents, a change in the sequencing procedure and/or bioinformatics pipeline, *etc.*).

Figure 4E illustrates an example workflow for genomic feature identification (324). This particular workflow is only an example of one possible collection and arrangement of algorithms for feature extraction from sequencing data 124. Generally, any combination of the modules and algorithms of feature extraction module 145, *e.g.*, illustrated in Figure 1A, can be used for a bioinformatics pipeline. For instance, in some embodiments, an architecture useful for the methods and systems described herein includes at least one of the modules or variant calling algorithms shown in feature extraction module 145. In some embodiments, an architecture includes at least 2, 3, 4, 5, 6, 7, 8, 9, 10, or more of the modules or variant calling algorithms shown in feature extraction module 145. Further, in some embodiments, feature extraction modules and/or algorithms not illustrated in Figure 1A find use in the methods and systems described herein.

In some embodiments, the obtaining, accessioning, storing, preparing, processing and/or analyzing the biopsy sample of block 502 from the test subject comprises any of the methods and/or embodiments described above in the present disclosure. In some embodiments, the sequencing reaction comprises any of the methods and/or embodiments described above in the present disclosure.

In some embodiments, all, or nearly all, of the aligned sequence reads are evaluated to identify candidate sequence variants (*e.g.*, candidate somatic sequence variants and/or candidate germline sequence variants). In other embodiments, such as discussed in block 504 below, a subset of the aligned sequence reads is evaluated to identify candidate sequence variants. For example, in one embodiment, targeted-panel sequencing reaction is used to generate sequencing data 122 and only sequence reads corresponding to the target panel (on-target reads) are evaluated to identify candidate sequence variants. In some embodiments, targeted-panel sequencing reaction is used to generate sequencing data 122 and a subset of sequence reads corresponding to a subset of the target panel are evaluated to identify candidate sequence variants. In some embodiments, a subset of the sequence reads corresponding to a subset of genes, regardless of whether the sequencing reaction is a targeted-panel sequencing reaction, a whole exome sequencing reaction, or a whole genome sequencing reaction, are evaluated to identify candidate sequence variants. In some embodiments, a subset of sequence reads corresponding to a defined set of regions within the genome, *e.g.*, one or more genes, one or more introns, one or more exons, one or more subregion of an intron and/or exon associated with cancer etiology, *etc.*, are evaluated to identify candidate sequence variants.

Alternatively, in some embodiments, regardless of what subset of aligned sequence reads are evaluated to identify candidate sequence variants, only a subset of candidate sequence variants is further validated. For example, in some embodiments, only candidate sequence variants corresponding to the target panel (on-target reads) are validated. Similarly, in some embodiments, only candidate sequence variants corresponding to a subset of the target panel are validated. Likewise, in some embodiments, only candidate sequence variants corresponding to a subset of genes, regardless of whether the sequencing reaction is a targeted-panel sequencing reaction, a whole exome sequencing reaction, or a whole genome sequencing reaction, are validated. Similarly, in some embodiments, only candidate variants corresponding to a defined set of regions within the genome, *e.g.*, one or more genes, one or more introns, one or more exons, one or more subregion of an intron and/or exon associated with cancer etiology, *etc.*, are validated.

In some embodiments, the enrichment is performed prior to pooling multiple nucleic acid sequencing libraries. However, in other embodiments, the enrichment is performed after pooling nucleic acid sequencing libraries, which has the advantage of reducing the number of enrichment assays that have to be performed.

In some embodiments, the enrichment is performed prior to generating a nucleic acid sequencing library. This has the advantage that fewer reagents are needed to perform both the enrichment (because there are fewer target sequences at this point, prior to library amplification) and the library production (because there are fewer nucleic acid molecules to tag and amplify after the enrichment). However, this raises the possibility of pull-down bias and/or that small variations in the enrichment protocol will result in less consistent results.

In some embodiments, nucleic acid libraries are pooled (two or more DNA libraries may be mixed to create a pool) and treated with reagents to reduce off-target capture. Pools may be dried in a vacufuge and resuspended. DNA libraries or pools may be hybridized to a probe set (for example, a probe set specific to a panel that includes loci from at least 100, 600, 1,000, 10,000, *etc.* of the 19,000 known human genes) and amplified with commercially available reagents. For example, in some embodiments, a pool is incubated in an incubator, PCR machine, water bath, or other temperature-modulating device to allow probes to hybridize. Pools may then be mixed with Streptavidin-coated beads or another means for capturing hybridized DNA-probe molecules, such as DNA molecules representing exons of the human genome and/or genes selected for a genetic panel.

Pools may be amplified and purified more than once using commercially available reagents. The pools or DNA libraries may be analyzed to determine the concentration or quantity of DNA molecules, for example by using a fluorescent dye and a fluorescence microplate reader, standard spectrofluorometer, or filter fluorometer. In one example, the DNA library preparation and/or capture is performed with an automated system, using a liquid handling robot.

In some embodiments, *e.g.,* where a whole genome sequencing method is used, nucleic acid sequencing libraries are not target-enriched prior to sequencing, in order to obtain sequencing data on substantially all of the competent nucleic acids in the sequencing library. Similarly, in some embodiments, *e.g.,* where a whole genome sequencing method is used, nucleic acid sequencing libraries are not mixed, because of bandwidth limitations related to obtaining significant sequencing depth across an entire genome. However, in other embodiments, *e.g.,* where a low pass whole genome sequencing (LPWGS) methodology is used, nucleic acid sequencing libraries can still be pooled, because very low average sequencing coverage is achieved across a respective genome, *e.g.,* between about 0.5x and about 5x.

In some embodiments, a plurality of nucleic acid probes (*e.g.,* a probe set) is used to enrich one or more target sequences in a nucleic acid sample (*e.g.,* an isolated nucleic acid sample or a nucleic acid sequencing library), *e.g.,* where one or more target sequences is informative for precision oncology. For instance, in some embodiments, one or more of the target sequences encompasses a locus that is associated with an actionable allele. That is, variations of the target sequence are associated with targeted therapeutic approaches. In some embodiments, one or more of the target sequences and/or a property of one or more of the target sequences is used in a classifier trained to distinguish two or more cancer states.

*Block 504.* Referring to block 504, in some embodiments, the first plurality of nucleic acid sequences is determined from a second panel-enriched sequencing reaction using a second plurality of probes including, for each respective locus in the plurality of loci a corresponding probe, in a second plurality of probes, that hybridizes to the respective locus. In other embodiments, the first plurality of nucleic acid sequences is obtained by whole genome sequencing of genomic DNA from the solid tumor sample.

Advantageously, enriching for target sequences prior to sequencing nucleic acids significantly reduces the costs and time associated with sequencing, facilitates multiplex sequencing by allowing multiple samples to be mixed together for a single sequencing reaction, and significantly reduces the computation burden of aligning the resulting sequence reads, as a result of significantly reducing the total amount of nucleic acids analyzed from each sample.

*Block 506.* Referring to block 506, in some embodiments, the plurality of loci is sequenced at an average sequence depth of at least 50X, 75X, 100X, 125X, 500X, or 1000X in the second panel-enriched sequencing reaction. In some embodiments, the second panel-enriched sequencing reaction is performed at a read depth of at least 100X. In some embodiments, the panel-enriched sequencing reaction is performed at a read depth of at least 100X, at least 500X, at least 1000X, at least 5000X, at least 10,000X, at least 50,000X, or greater. In some embodiments, the panel-enriched sequencing reaction is performed at a read depth of no more than 100,000X, no more than 50,000X, no more than 10,000X, no more than 5000X, or less. In some embodiments, the panel-enriched sequencing reaction is performed at a read depth of from 100X to 50,000X, from 100X to 10,000X, from 100X to 5000X, from 100X to 1000X, or from 100X to 500X. In some embodiments, the panel-enriched sequencing reaction is performed at a read depth of from 500X to 50,000X, from 500X to 10,000X, from 500X to 5000X, or from 500X to 1000X. In some embodiments, the panel-enriched sequencing reaction is performed at a read depth of from 1000X to 50,000X, from 1000X to 10,000X, or from 1000X to 5000X. In some embodiments, the sequencing depth threshold is a minimum depth selected by a user or practitioner.

*Block 508.* Referring to block 508, in some embodiments, the second plurality of probes enriches for loci from at least 50 genes. In some embodiments, the panel-enriched sequencing reaction uses a sequencing panel that enriches for at least 25 genes, at least 50 genes, at least 100 genes, at least 250 genes, at least 500 genes, at least 1000 genes, at least 2500 genes, at least 5000 genes, or more. In some embodiments, the panel-enriched sequencing reaction uses a sequencing panel that enriches for no more than 40,000 genes, no more than 20,000 genes, no more than 10,000 genes, no more than 5000 genes, no more than 2500 genes, no more than 1000 genes, or less. In some embodiments, the panel-enriched sequencing reaction uses a sequencing panel that enriches for from 25 genes to 10,000 genes, from 25 genes to 5000 genes, from 25 genes to 2500 genes, from 25 genes to 1000 genes, from 25 genes to 500 genes, or from 25 genes to 250 genes. In some embodiments, the panel-enriched sequencing reaction uses a sequencing panel that enriches for from 50 genes to 10,000 genes, from 50 genes to 5000 genes, from 50 genes to 2500 genes, from 50 genes to 1000 genes, from 50 genes to 500 genes, or from 50 genes to 250 genes. In some embodiments, the panel-enriched sequencing reaction uses a sequencing panel that enriches for from 100 genes to 10,000 genes, from 100 genes to 5000 genes, from 100 genes to 2500 genes, from 100 genes to 1000 genes, from 100 genes to 500 genes, or from 100 genes to 250 genes.

In some embodiments, the plurality of probe sequences used to enrich cell-free DNA fragments in the liquid biopsy sample in the first panel-enriched sequencing reaction collectively map to at least 25 different genes in a human reference genome. In some embodiments, the panel-enriched sequencing reaction uses a sequencing panel that enriches for at least 25 human genes, at least 50 human genes, at least 100 human genes, at least 250 human genes, at least 500 human genes, at least 1000 human genes, at least 2500 human genes, at least 5000 human genes, or more. In some embodiments, the panel-enriched sequencing reaction uses a sequencing panel that enriches for no more than 40,000 human genes, no more than 20,000 human genes, no more than 10,000 human genes, no more than 5000 human genes, no more than 2500 human genes, no more than 1000 human genes, or less. In some embodiments, the panel-enriched sequencing reaction uses a sequencing panel that enriches for from 25 human genes to 10,000 human genes, from 25 human genes to 5000 human genes, from 25 human genes to 2500 human genes, from 25 human genes to 1000 human genes, from 25 human genes to 500 human genes, or from 25 human genes to 250 human genes. In some embodiments, the panel-enriched sequencing reaction uses a sequencing panel that enriches for from 50 human genes to 10,000 human genes, from 50 human genes to 5000 human genes, from 50 human genes to 2500 human genes, from 50 human genes to 1000 human genes, from 50 human genes to 500 human genes, or from 50 human genes to 250 human genes. In some embodiments, the panel-enriched sequencing reaction uses a sequencing panel that enriches for from 100 human genes to 10,000 human genes, from 100 human genes to 5000 human genes, from 100 human genes to 2500 human genes, from 100 human genes to 1000 human genes, from 100 human genes to 500 human genes, or from 100 human genes to 250 human genes.

Accordingly, in some embodiments, the second panel-enriched sequencing reaction is performed at a read depth of at least 100X. In some embodiments, the second panel-enriched sequencing reaction is performed at a read depth of at least 100X, at least 500X, at least 1000X, at least 5000X, at least 10,000X, at least 50,000X, or greater. In some embodiments, the second panel-enriched sequencing reaction is performed at a read depth of no more than 100,000X, no more than 50,000X, no more than 10,000X, no more than 5000X, or less. In some embodiments, the second panel-enriched sequencing reaction is performed at a read depth of from 100X to 50,000X, from 100X to 10,000X, from 100X to 5000X, from 100X to 1000X, or from 100X to 500X. In some embodiments, the second panel-enriched sequencing reaction is performed at a read depth of from 500X to 50,000X, from 500X to 10,000X, from 500X to 5000X, or from 500X to 1000X. In some embodiments, the second panel-enriched sequencing reaction is performed at a read depth of from 1000X to 50,000X, from 1000X to 10,000X, or from 1000X to 5000X.

In some embodiments, the second panel-enriched sequencing reaction uses a sequencing panel that enriches (includes probes for) for between 50 genes and 150 genes. In some embodiments, the plurality of probe sequences used to enrich cell-free DNA fragments in the second panel-enriched sequencing reaction collectively map to from 25 different genes to 150 different genes in a human reference genome. In some embodiments, the second panel-enriched sequencing reaction uses a sequencing panel that enriches for between 50 genes and 150 genes, between 100 genes and 200 genes, between 150 genes and 300 genes, or between 250 genes and 500 genes. In some embodiments, the second panel-enriched sequencing reaction uses a sequencing panel that enriches for between 50 genes and 1000 genes, between 60 genes and 800 genes, between 70 genes and 700 genes, between 80 genes and 600 genes, or between 90 genes and 500 genes. In some embodiments, each of the genes that are enriched for in the sequencing panel are human.

In some embodiments, a second plurality of probe sequences used to enrich nucleic fragments in the sample in the second panel-enriched sequencing reaction collectively map to at least 25 different genes in a human reference genome. In some embodiments, the second panel-enriched sequencing reaction uses a sequencing panel that enriches for at least 25 human genes, at least 50 human genes, at least 100 human genes, at least 250 human genes, at least 500 human genes, at least 1000 human genes, at least 2500 human genes, at least 5000 human genes, or more. In some embodiments, the second panel-enriched sequencing reaction uses a sequencing panel that enriches for no more than 1,000 human genes, no more than 500 human genes, no more than 250 human genes, no more than 200 human genes, no more than 175 human genes, no more than 100 human genes, or less. In some embodiments, second the panel-enriched sequencing reaction uses a sequencing panel that enriches for from 25 human genes to 1,000 human genes, from 25 human genes to 500 human genes, from 10 human genes to 250 human genes, from 10 human genes to 200 human genes, from 5 human genes to 150 human genes, or from 5 human genes to 100 human genes. In some embodiments, the second panel-enriched sequencing reaction uses a sequencing panel that enriches for from 25 human genes to 400 human genes, from 30 human genes to 500 human genes, from 50 human genes to 300 human genes, from 5 human genes to 95 human genes, from 15 human genes to 130 human genes, or from 15 human genes to 165 human genes. In some embodiments, the second panel-enriched sequencing reaction uses a sequencing panel that enriches for from 25 human genes to 600 human genes, from 40 human genes to 80 human genes, from 35 human genes to 95 human genes, from 45 human genes to 80 human genes, from 20 human genes to 80 human genes, or from 20 human genes to 120 human genes.

In some embodiments, the second panel-enriched sequencing reaction uses a sequencing panel that enriches for at least 10, 20, 30, 40, or 50 genes listed in Table 1. In some embodiments, the second panel-enriched sequencing reaction uses a sequencing panel that enriches for at least 10 genes of which at least 10, 20, 30, 40, or 50 are genes listed in Table 1. In some embodiments, the second panel-enriched sequencing reaction uses a sequencing panel that enriches for at most 100 genes of which at least 10, 20, 30, 40, or 50 genes listed in Table 1. In some embodiments, the sequencing panel only enriches for genes in Table 1 whereas in other embodiments the sequencing panel enriches for some genes that are in Table 1 and some genes that are not in Table 1. In some embodiments, the second panel-enriched sequencing reaction uses a sequencing panel that enriches between 25 different genes and 150 different genes listed in Table 1. In some embodiments, the second panel-enriched sequencing reaction uses a sequencing panel that enriches for between 50 genes and 150 genes, between 100 genes and 200 genes, or between 150 genes and 300 genes listed in Table 1.

In some embodiments, the second panel-enriched sequencing reaction uses a sequencing panel that enriches for at least 10, 20, 30, 40, or 50 genes listed in Table 2. In some embodiments, the second panel-enriched sequencing reaction uses a sequencing panel that enriches for at least 10 genes of which at least 10, 20, 30, 40, or 50 are genes listed in Table 2. In some embodiments, the second panel-enriched sequencing reaction uses a sequencing panel that enriches for at most 100 genes of which at least 10, 20, 30, 40, or 50 are genes listed in Table 2. In some embodiments, the sequencing panel only enriches for genes in Table 2 whereas in other embodiments the sequencing panel enriches for some genes that are in Table 2 and some genes that are not in Table 2. In some embodiments, the second panel-enriched sequencing reaction uses a sequencing panel that enriches between 25 different genes and 150 different genes listed in Table 2. In some embodiments, the second panel-enriched sequencing reaction uses a sequencing panel that enriches for between 50 genes and 150 genes, between 100 genes and 200 genes, or between 150 genes and 300 genes listed in Table 2.

In some embodiments, the probe set includes probes that target no more than 50 genes, 100 genes, 150 genes or 200 genes. In some such embodiments, the probe set includes probes targeting one or more of the genes listed in Table 1. In some such embodiments, the probe set includes probes targeting at least 5 of the genes listed in Table 1. In some such embodiments, the probe set includes probes targeting at least 10 of the genes listed in Table 1. In some such embodiments, the probe set includes probes targeting at least 25 of the genes listed in Table 1. In some such embodiments, the probe set includes probes targeting at least 50 of the genes listed in Table 1. In some such embodiments, the probe set includes probes targeting at least 75 of the genes listed in Table 1. In some such embodiments, the probe set includes probes targeting at least 100 of the genes listed in Table 1. In some such embodiments, the probe set includes probes targeting all of the genes listed in Table 1.

In some embodiments, the probe set includes probes that target no more than 50 genes, 100 genes, 150 genes or 200 genes. In some such embodiments, the probe set includes probes targeting one or more of the genes listed in Table 2. In some such embodiments, the probe set includes probes targeting at least 5 of the genes listed in Table 2. In some such embodiments, the probe set includes probes targeting at least 10 of the genes listed in Table 2. In some such embodiments, the probe set includes probes targeting at least 25 of the genes listed in Table 2. In some such embodiments, the probe set includes probes targeting at least 50 of the genes listed in Table 2. In some such embodiments, the probe set includes probes targeting at least 75 of the genes listed in Table 2. In some such embodiments, the probe set includes probes targeting at least 100 of the genes listed in Table 2. In some such embodiments, the probe set includes probes targeting all of the genes listed in Table 2.

**Table 1.** An example panel of 105 genes.

| ALK | B2M | ERRFI1 | IDH2 | MSH6 | PIK3R1 | SPOP |
|------|-------|--------|---------|--------|---------|--------|
| FGFR2 | BAP1 | ESR1 | JAK1 | MTOR | PMS2 | STK11 |
| FGFR3 | BRCA1 | EZH2 | JAK2 | MYCN | PTCH1 | TERT |
| NTRK1 | BRCA2 | FBXW7 | JAK3 | NF1 | PTEN | TP53 |
| RET | BTK | FGFR1 | KDR | NF2 | PTPN11 | TSC1 |
| ROS1 | CCND1 | FGFR4 | KEAP1 | NFE2L2 | RAD51C | TSC2 |
| BRAF | CCND2 | FLT3 | KIT | NOTCH1 | RAF1 | UGT1A1 |
| AKT1 | CCND3 | FOXL2 | KRAS | NPM1 | RB1 | VHL |
| AKT2 | CDH1 | GATA3 | MAP2K 1 | NRAS | RHEB | CCNE1 |
| APC | CDK4 | GNA11 | MAP2K2 | PALB2 | RHOA | CD274 |

(continued)

| AR | CDK6 | GNAQ | MAPK1 | PBRM1 | RIT1 | EGFR |
|---|---|---|---|---|---|---|
| ARAF | CDKN2A | GNAS | MLH1 | PDCD 1LG2 | RNF43 | ERBB2 |
| ARID1A | CTNNB1 | HNF1A | MPL | PDGFRA | SDHA | MET |
| ATM | DDR2 | HRAS | MSH2 | PDGFRB | SMAD4 | MYC |
| ATR | DPYD | IDH1 | MSH3 | PIK3CA | SMO | KMT2A |

**Table 2.** An example panel of 523 genes.

| BCC3 | CIC | FGFR4 | KLF4 | PBRM1 | SIRPA |
|---|---|---|---|---|---|
| ABL1 | CKS1B | FH | KLHL6 | PDCD1 | SLC34A2 |
| ABL2 | CREBBP | FHIT | KLLN | PDCD 1LG2 | SLC9A3R1 |
| ABRAXAS1 | CRKL | FLCN | KMT2A | PDGFRA | SLFN11 |
| ACVR1 | CSF1R | FLT1 | KMT2C | PDGFRB | SLIT2 |
| ACVR1B | CSF3R | FLT3 | KMT2D | PDK1 | SMAD2 |
| AJUBA | CTC1 | FLT4 | KRAS | PHGDH | SMAD3 |
| AKT1 | CTCF | FOLH1 | LATS1 | PHLPP 1 | SMAD4 |
| AKT2 | CTLA4 | FOXA1 | LCK | PHLPP2 | SMARCA2 |
| AKT3 | CTNNA1 | FOXL2 | LMO1 | PIAS4 | SMARCA4 |
| ALK | CTNNB1 | FOXO1 | LRP1B | PIK3C2B | SMARCB1 |
| ALOX12B | CUL3 | FOXO3 | LTK | PIK3C2G | SMC1A |
| AMER1 | CUL4A | FOXP1 | LYN | PIK3CA | SMC3 |
| APC | CUX1 | FRS2 | LZTR1 | PIK3CB | SMO |
| APLNR | CXCR4 | FUBP1 | MAF | PIK3CD | SNCAIP |
| AR | CYLD | GABRA6 | MALT1 | PIK3CG | SOCS1 |
| ARAF | CYP17A1 | GALNT12 | MAP2K 1 | PIK3R1 | SOS1 |
| ARFRP1 | CYSLTR2 | GATA1 | MAP2K2 | PIK3R2 | SOX2 |
| ARID1A | DAXX | GATA3 | MAP2K4 | PIM1 | SOX9 |
| ARID1B | DDB2 | GATA4 | MAP3K1 | PLCG1 | SPEN |
| ARID2 | DDR1 | GATA6 | MAP3K13 | PLCG2 | SPOP |
| ASNS | DDR2 | GID4 | MAP3K21 | PMS1 | SRC |
| ASXL1 | DDX3X | GLI2 | MAP3K7 | PMS2 | SRSF2 |
| ATM | DDX41 | GNA11 | MAPK1 | POLA1 | STAG2 |
| ATR | DEPTOR | GNA13 | MAPK3 | POLD1 | STAT3 |
| ATRX | DICER1 | GNAQ | MAX | POLE | STAT5B |
| AURKA | DIS3 | GNAS | MC1R | POLQ | STAT6 |
| AURKB | DNMT1 | GPC3 | MCL1 | POT1 | STK11 |
| AURKC | DNMT3A | GPS2 | MDM2 | PPARG | SUFU |
| AXINI1 | DOT1L | GREM1 | MDM4 | PPM1D | SUZ12 |
| AXIN2 | DPYD | GRIN2A | MED12 | PPP2R1A | SYK |
| AXL | EBF1 | GRM3 | MEF2B | PPP2R2A | TBX3 |
| B2M | EED | GSK3B | MEN1 | PPP6C | TCF7L2 |

(continued)

| | | | | | |
|---|---|---|---|---|---|
| BAP1 | EEF2 | GSTP1 | MERTK | PRDM1 | TEK |
| BARD1 | EGFR | H3F3A | MET | PREX2 | TERC |
| BAX | EGLN1 | HAVCR2 | MITF | PRKACA | TERT |
| BCL2 | EIF1AX | HDAC1 | MKNK1 | PRKAR1A | TET2 |
| BCL2L1 | ELF3 | HDAC2 | MLH1 | PRKCI | TFEB |
| BCL2L 11 | EMSY | HGF | MLH3 | PRKN | TGFB1 |
| BCL2L2 | EP300 | HIF1A | MPL | PTCH1 | TGFBR1 |
| BCL6 | EPCAM | HIST1H3B | MRE11 | PTEN | TGFBR2 |
| BCLAF1 | EPHA2 | HLA-B | MS4A1 | PTK2 | TIGIT |
| BCOR | EPHA3 | HNF1A | MSH2 | PTPN11 | TIPARP |
| BCORL1 | EPHB1 | HNF1B | MSH3 | PTPN13 | TMEM127 |
| BCR | EPHB4 | HOXB13 | MSH6 | PTPRD | TMPRSS2 |
| BIRC3 | ERBB2 | HRAS | MST1R | PTPRO | TNFAIP3 |
| BLM | ERBB3 | HSD3B1 | MTAP | PTPRT | TNFRSF14 |
| BMPR1A | ERBB4 | HSP90AA1 | MTHFR | QKI | TNFRSF17 |
| BRAF | ERCC2 | HSPH1 | MTOR | RAC1 | TOP1 |
| BRCA1 | ERCC3 | ID3 | MUC16 | RAD21 | TOP2A |
| BRCA2 | ERCC4 | IDH1 | MUTYH | RAD50 | TP53 |
| BRD4 | ERCC6 | IDH2 | MYB | RAD51 | TP53BP1 |
| BRIP1 | ERG | IFNA21 | MYC | RAD51B | TP63 |
| BTG1 | ERRFI1 | IFNAR1 | MYCL | RAD51C | TRAF3 |
| BTG2 | ESR1 | IFNAR2 | MYCN | RAD51D | TRAF7 |
| BTK | ETNK1 | IFNG | MYD88 | RAD52 | TSC1 |
| CALR | ETV1 | IFNGR1 | NBN | RAD54L | TSC2 |
| CARD11 | ETV4 | IFNGR2 | NCOA2 | RAF1 | TSHR |
| CARM1 | ETV5 | IFNW1 | NCOR1 | RARA | TYMS |
| CASP8 | ETV6 | IGF1 | NF1 | RASA1 | TYRO3 |
| CBFB | EWSR1 | IGF1R | NF2 | RB1 | U2AF1 |
| CBL | EZH2 | IKBKE | NFE2L2 | RBM10 | UGT1A1 |
| CCND1 | EZR | IKZF1 | NFKBIA | RECQL4 | VEGFA |
| CCND2 | FAM46C | IL10RA | NKX2-1 | REL | VHL |
| CCND3 | FANCA | IL32 | NOTCH1 | RET | VSIR |
| CCNE1 | FANCC | IL6R | NOTCH2 | RHEB | WEE1 |
| CD22 | FANCD2 | IL7R | NOTCH3 | RHOA | WNK1 |
| CD274 | FANCE | IMPDH1 | NOTCH4 | RICTOR | WRN |
| CD70 | FANCG | ING1 | NPM1 | RIT1 | WT1 |
| CD74 | FANCI | INPP4B | NQO1 | RNF43 | XBP1 |
| CD79A | FANCL | INSR | NRAS | ROS1 | XPA |
| CD79B | FANCM | IRF1 | NRG1 | RPS6KB1 | XPC |
| CDC73 | FAS | IRF2 | NSD1 | RPTOR | XPO1 |

(continued)

| CDH1 | FAT1 | IRF4 | NSD2 | RRM1 | XRCC1 |
|------|------|------|------|------|-------|
| CDK12 | FBXW7 | IRS2 | NSD3 | RSF1 | XRCC2 |
| CDK4 | FCGR2A | JAK1 | NT5C2 | RSPO2 | YEATS4 |
| CDK6 | FCGR3A | JAK2 | NTRK1 | RUNX1 | ZFHX3 |
| CDK8 | FGF10 | JAK3 | NTRK2 | RXRA | ZMYM3 |
| CDK9 | FGF12 | JUN | NTRK3 | SDC4 | ZNF217 |
| CDKN1A | FGF14 | KAT6A | NUTM1 | SDHA | ZNF703 |
| CDKN1B | FGF19 | KDM5A | P2RY8 | SDHAF2 | ZNF750 |
| CDKN2A | FGF23 | KDM5C | PAK1 | SDHB | ZNRF3 |
| CDKN2B | FGF3 | KDM5D | PALB2 | SDHC | ZRSR2 |
| CDKN2C | FGF4 | KDM6A | PALLD | SDHD | |
| CEBPA | FGF6 | KDR | PARP1 | SETBP1 | |
| CHD4 | FGFR1 | KEAP1 | PARP2 | SETD2 | |
| CHEK1 | FGFR2 | KEL | PARP3 | SF3B1 | |
| CHEK2 | FGFR3 | KIT | PAX5 | SGK1 | |

In some embodiments, the second panel-enriched sequencing reaction uses a sequencing panel that enriches for at least 10, 20, 30, 40, or 50 genes listed in List 1. In some embodiments, the second panel-enriched sequencing reaction uses a sequencing panel that enriches between 25 different genes and 50 different genes listed in List 1. In some embodiments, the second panel-enriched sequencing reaction uses a sequencing panel that enriches for between 5 genes and all the genes listed in List 1, between 10 genes and all the genes listed in List 1, or between 20 genes and all the genes listed in List 1.

In some embodiments, the probe set includes probes that target no more than 50 genes, 100 genes, 150 genes or 200 genes. In some such embodiments, the probe set consists of or comprises probes targeting one or more of the genes in List 1. In some such embodiments, the probe set consists of or comprises probes targeting at least 5 of the genes listed in List 1. In some such embodiments, the probe set consists of or comprises probes targeting at least 10 of the genes in List 1. In some such embodiments, the probe set consists of or comprises probes targeting at least 25 of the genes in List 1. In some such embodiments, the probe set consists of or comprises probes targeting at least 50 of the genes listed in List 1. In some such embodiments, the probe set consists of or comprises probes targeting all of the genes in List 1.

List 1: AKT1 (14q32.33), ALK (2p23.2-23.1), APC (5q22.2), AR (Xq12), ARAF (Xp11.3), ARID1A (1p36.11), ATM (11q22.3), BRAF (7q34), BRCA1 (17q21.31), BRCA2 (13q13.1), CCND1 (11q13.3), CCND2 (12p13.32), CCNE1 (19q12), CDH1 (16q22.1), CDK4 (12q14.1), CDK6 (7q21.2), CDKN2A (9p21.3), CTNNB1 (3p22.1), DDR2 (1q23.3), EGFR (7p11.2), ERBB2 (17q12), ESR1 (6q25.1-25.2), EZH2 (7q36.1), FBXW7 (4q31.3), FGFR1 (8p11.23), FGFR2 (10q26.13), FGFR3 (4p16.3), GATA3 (10p14), GNA11 (19p13.3), GNAQ (9q21.2), GNAS (20q13.32), HNF1A (12q24.31), HRAS (11p15.5), IDH1 (2q34), IDH2 (15q26.1), JAK2 (9p24.1), JAK3 (19p13.11), KIT (4q12), KRAS (12p12.1), MAP2K1 (15q22.31), MAP2K2 (19p13.3), MAPK1 (22q11.22), MAPK3 (16p11.2), MET (7q31.2), MLH1 (3p22.2), MPL (1p34.2), MTOR (1p36.22), MYC (8q24.21), NF1 (17q11.2), NFE2L2 (2q31.2), NOTCH1 (9q34.3), NPM1 (5q35.1), NRAS (1p13.2), NTRK1 (1q23.1), NTRK3 (15q25.3), PDGFRA (4q12), PIK3CA (3q26.32), PTEN (10q23.31), PTPN11 (12q24.13), RAF1 (3p25.2), RB1 (13q14.2), RET (10q11.21), RHEB (7q36.1), RHOA (3p21.31), RIT1 (1q22), ROS1 (6q22.1), SMAD4 (18q21.2), SMO (7q32.1), STK11 (19p13.3), TERT (5p15.33), TP53 (17p13.1), TSC1 (9q34.13), and VHL (3p25.3).

In some embodiments, the second panel-enriched sequencing reaction uses a sequencing panel that enriches for at least 10, 20, 30, 40, or 50 genes listed in List 2. In some embodiments, the second panel-enriched sequencing reaction uses a sequencing panel that enriches between 25 different genes and 50 different genes listed in List 2. In some embodiments, the second panel-enriched sequencing reaction uses a sequencing panel that enriches for between 5 genes and all the genes listed in List 2, between 10 genes and all the genes listed in List 2, or between 20 genes and all the genes listed in List 2.

In some embodiments, the probe set includes probes that target no more than 50 genes, 100 genes, 150 genes or 200 genes. In some such embodiments, the probe set consists of or comprises probes targeting one or more of the genes in List 2. In some such embodiments, the probe set consists of or comprises probes targeting at least 5 of the genes listed in List 2. In some such embodiments, the probe set consists of or comprises probes targeting at least 10 of the genes in List 2. In some such embodiments, the probe set consists of or comprises probes targeting at least 25 of the genes in List 2. In some such embodiments, the probe set consists of or comprises probes targeting at least 50 of the genes listed in List 2. In some such embodiments, the probe set consists of or comprises probes targeting all of the genes in List 2.

List 2: ABL1, ACVR1B, AKT1, AKT2, AKT3, ALK, ALOX12B, AMER1 (FAM123B), APC, AR, ARAF, ARFRP1, ARID1A, ASXL1, ATM, ATR, ATRX, AURKA, AURKB, AXIN1, AXL, BAP1, BARD1, BCL2, BCL2L1, BCL2L2, BCL6, BCOR, BCORL1, BRAF, BRCA1, BRCA2, BRD4, BRIP1, BTG1, BTG2, BTK, C11orf30 (EMSY), C17orf39 (GID4), CALR, CARD11, CASP8, CBFB, CBL, CCND1, CCND2, CCND3, CCNE1, CD22, CD274 (PD-L1), CD70, CD79A, CD79B, CDC73, CDH1, CDK12, CDK4, CDK6, CDK8, CDKN1A, CDKN1B, CDKN2A, CDKN2B, CDKN2C, CEBPA, CHEK1, CHEK2, CIC, CREBBP, CRKL, CSF1R, CSF3R, CTCF, CTNNA1, CTNNB1, CUL3, CUL4A, CXCR4, CYP17A1, DAXX, DDR1, DDR2, DIS3, DNMT3A, DOT1L, EED, EGFR, EP300, EPHA3, EPHB1, EPHB4, ERBB2, ERBB3, ERBB4, ERCC4, ERG, ERRFI1, ESR1, EZH2, FAM46C, FANCA, FANCC, FANCG, FANCL, FAS, FBXW7, FGF10, FGF12, FGF14, FGF19, FGF23, FGF3, FGF4, FGF6, FGFR1, FGFR2, FGFR3, FGFR4, FH, FLCN, FLT1, FLT3, FOXL2, FUBP1, GABRA6, GATA3, GATA4, GATA6, GNA11, GNA13, GNAQ, GNAS, GRM3, GSK3B, H3F3A, HDAC1, HGF, HNF1A, HRAS, HSD3B1, ID3, IDH1, IDH2, IGF1R, IKBKE, IKZF1, INPP4B, IRF2, IRF4, IRS2, JAK1, JAK2, JAK3, JUN, KDM5A, KDM5C, KDM6A, KDR, KEAP1, KEL, KIT, KLHL6, KMT2A, KMT2D (MLL2), KRAS, LTK, LYN, MAF, MAP2K1 (MEK1), MAP2K2 (MEK2), MAP2K4, MAP3K1, MAP3K13, MAPK1, MCL1, MDM2, MDM4, MED12, MEF2B, MEN1, MERTK, MET, MITF, MKNK1, MLH1, MPL, MRE11A, MSH2, MSH3, MSH6, MST1R, MTAP, MTOR, MUTYH, MYC, MYCL (MYCL1), MYCN, MYD88, NBN, NF1, NF2, NFE2L2, NFKBIA, NKX2-1, NOTCH1, NOTCH2, NOTCH3, NPM1, NRAS, NSD3 (WHSC1L1), NT5C2, NTRK1, NTRK2, NTRK3, P2RY8, PALB2, PARK2, PARP1, PARP2, PARP3, PAX5, PBRM1, PDCD1 (PD-1), PDCD1LG2 (PD-L2), PDGFRA, PDGFRB, PDK1, PIK3C2B, PIK3C2G, PIK3CA, PIK3CB, PIK3R1, PIM1, PMS2, POLD1, POLE, PPARG, PPP2R1A, PPP2R2A, PRDM1, PRKAR1A, PRKCI, PTCH1, PTEN, PTPN11, PTPRO, QKI, RAC1, RAD21, RAD51, RAD51B, RAD51C, RAD51D, RAD52, RAD54L, RAF1, RARA, RB1, RBM10, REL, RET, RICTOR, RNF43, ROS1, RPTOR, SDHA, SDHB, SDHC, SDHD, SETD2, SF3B1, SGK1, SMAD2, SMAD4, SMARCA4, SMARCB1, SMO, SNCAIP, SOCS1, SOX2, SOX9, SPEN, SPOP, SRC, STAG2, STAT3, STK11, SUFU, SYK, TBX3, TEK, TERC, TERT, TET2, ncRNA, TGFBR2, TIPARP, TNFAIP3, TNFRSF14, TP53, TSC1, TSC2, TYRO3, U2AF1, VEGFA, VHL, WHSC1, WT1, XPO1, XRCC2, ZNF217, and ZNF703.

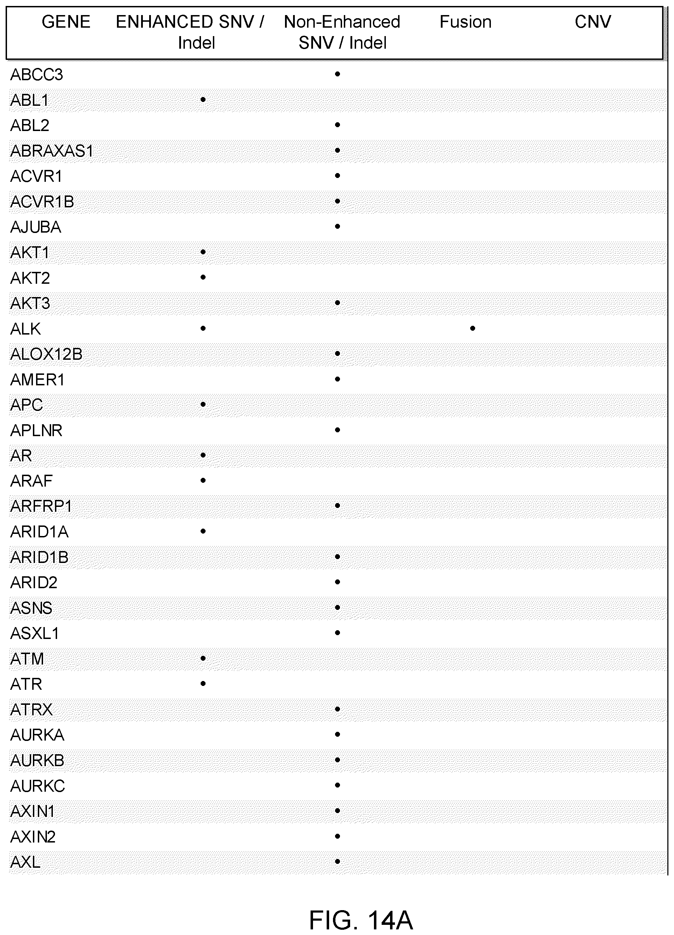

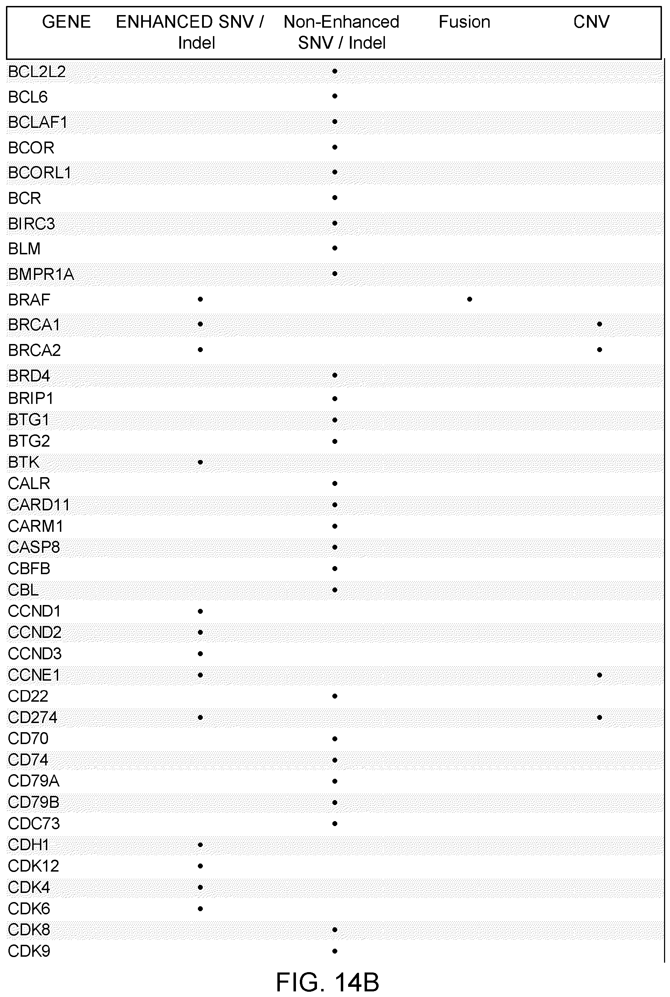

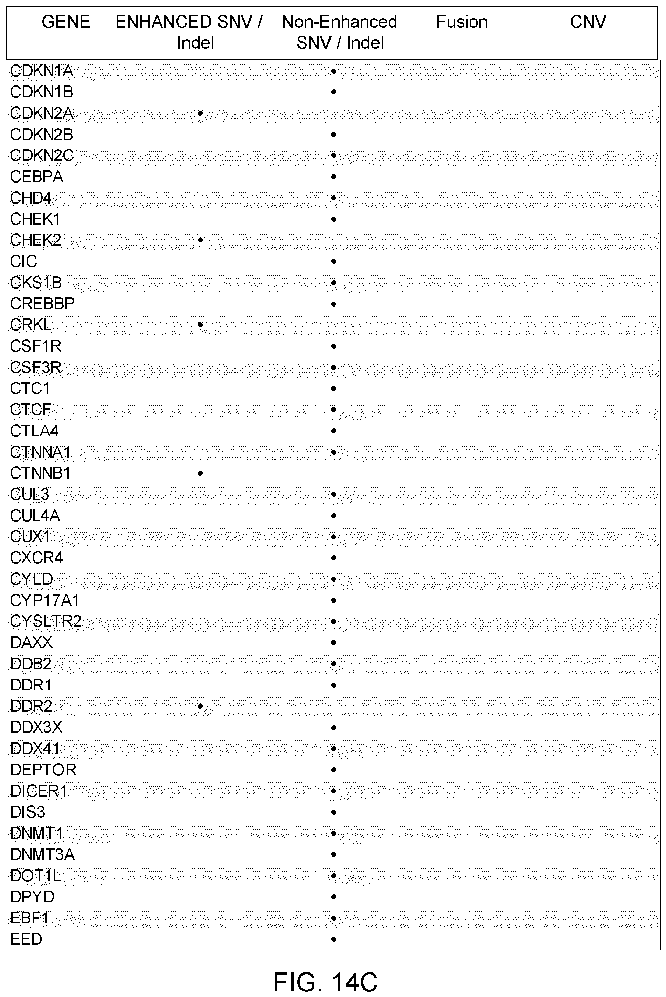

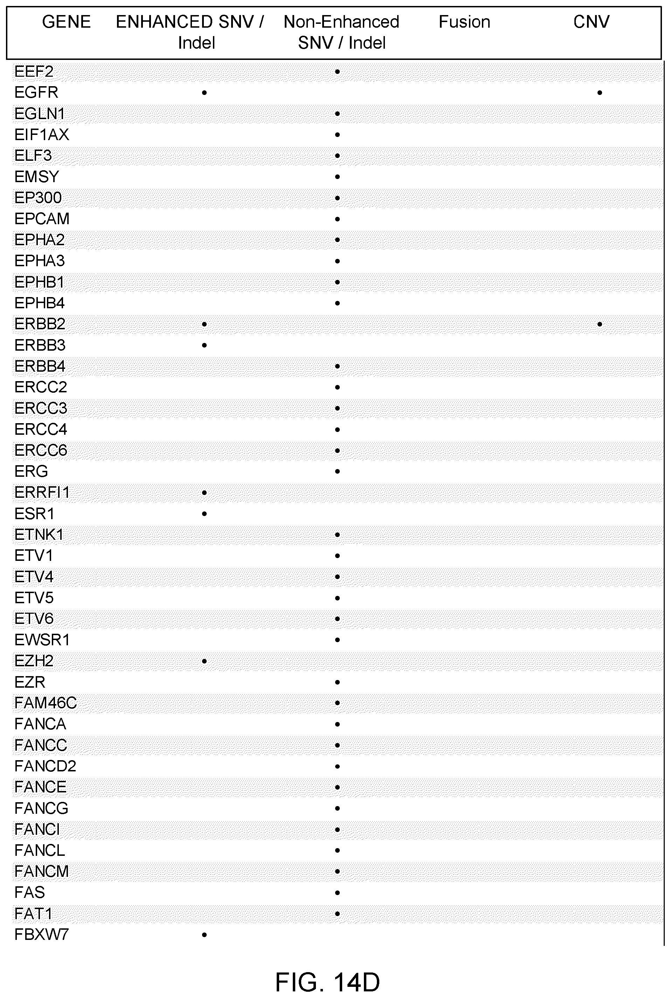

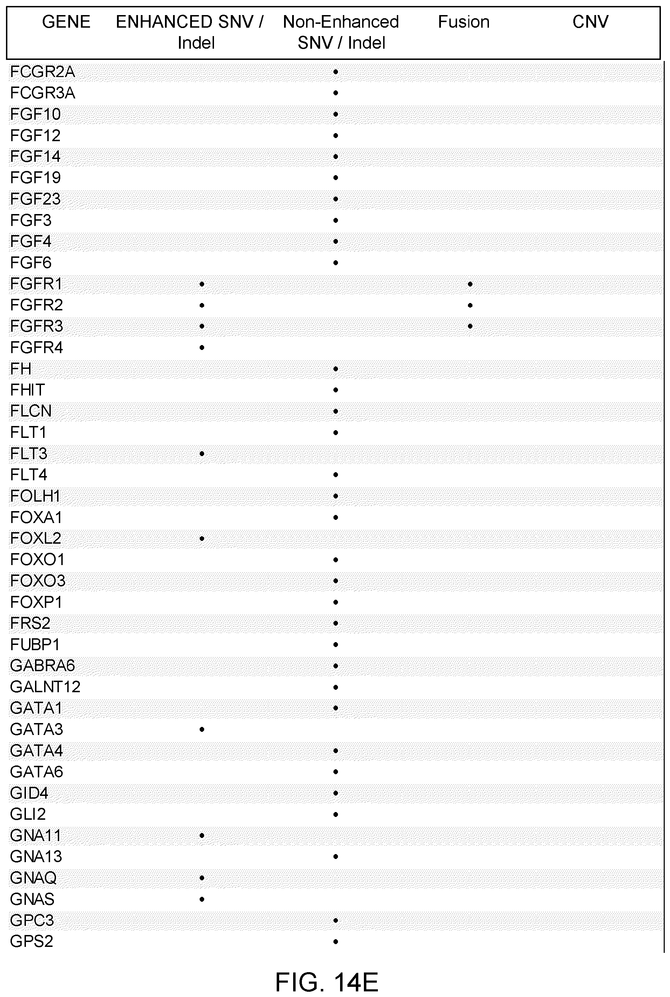

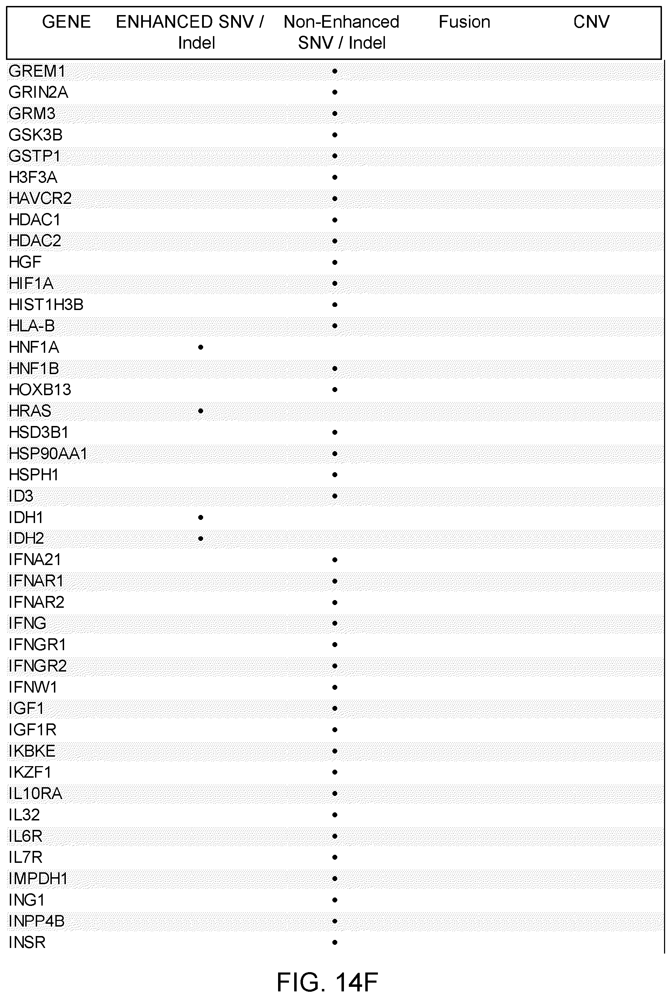

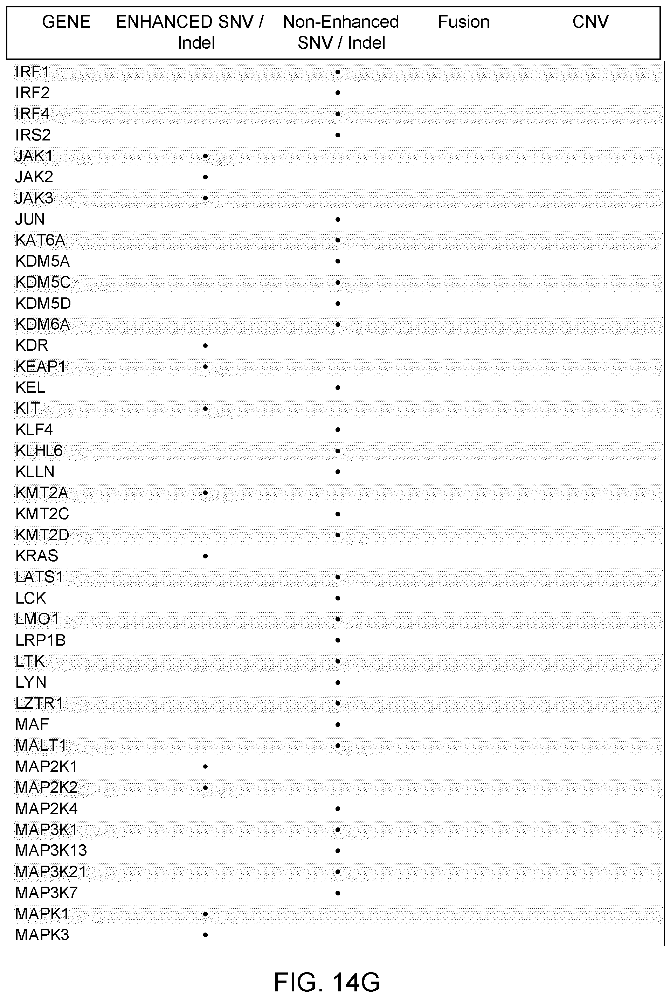

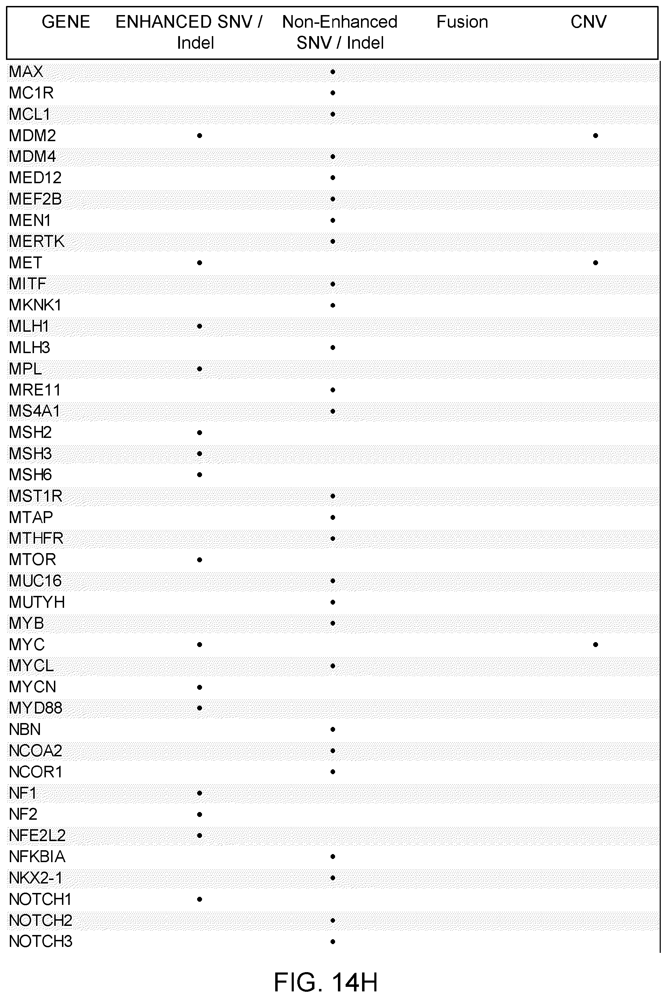

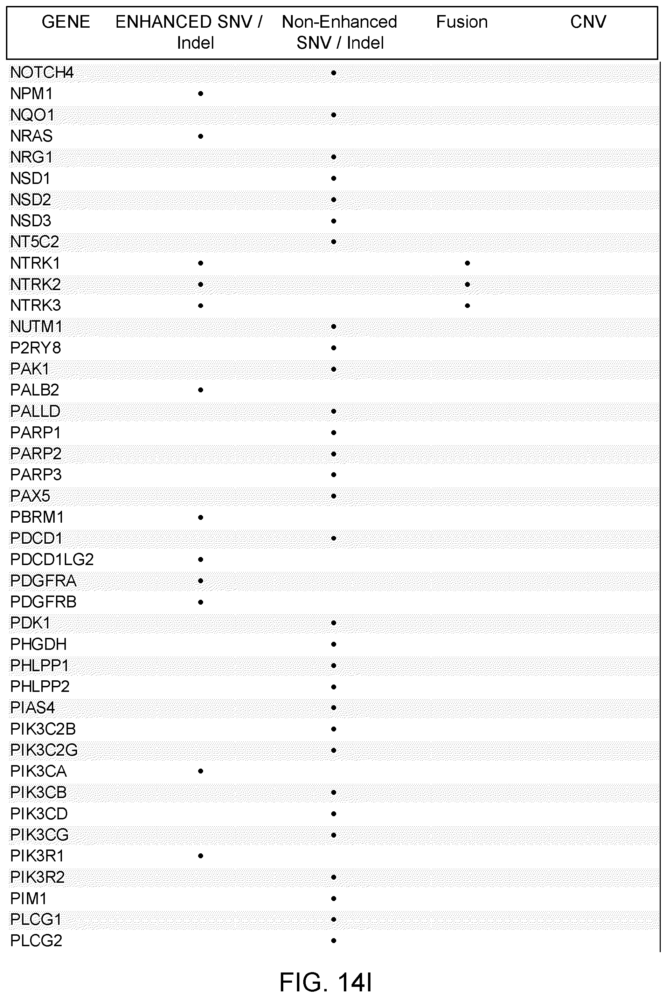

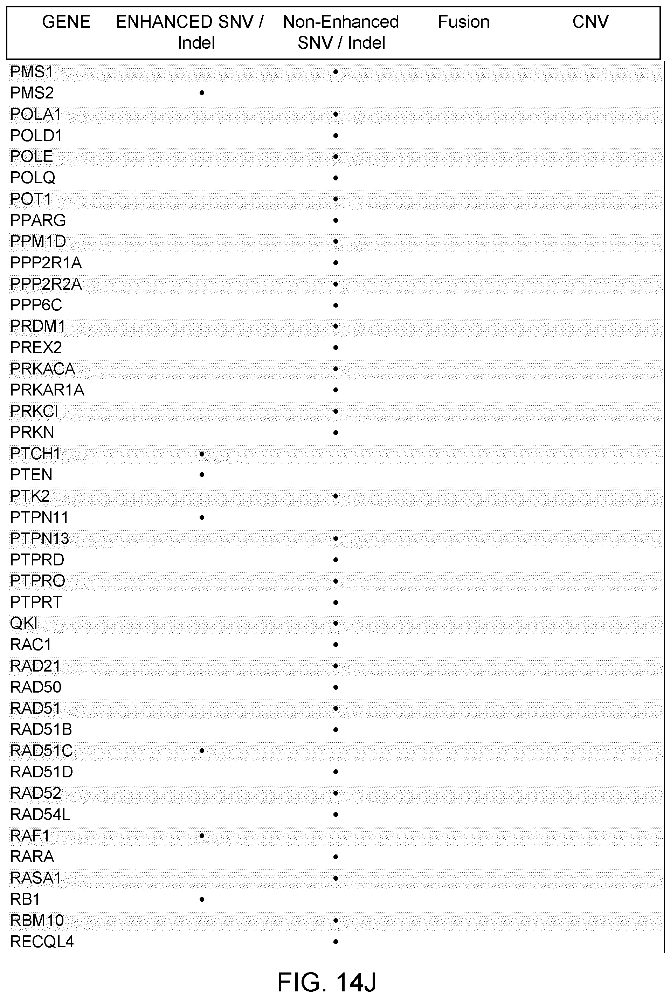

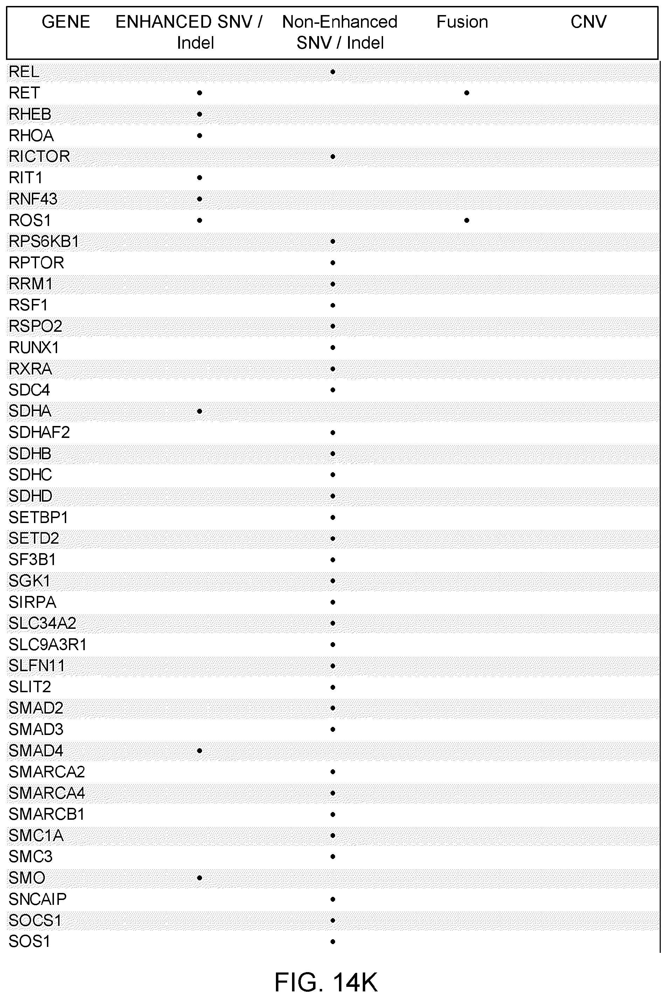

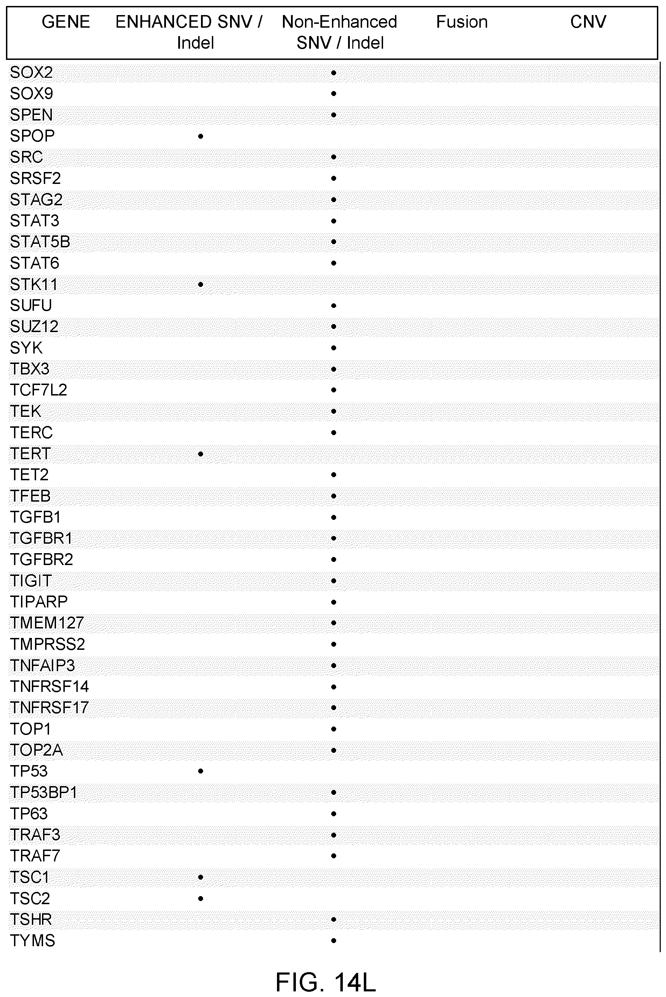

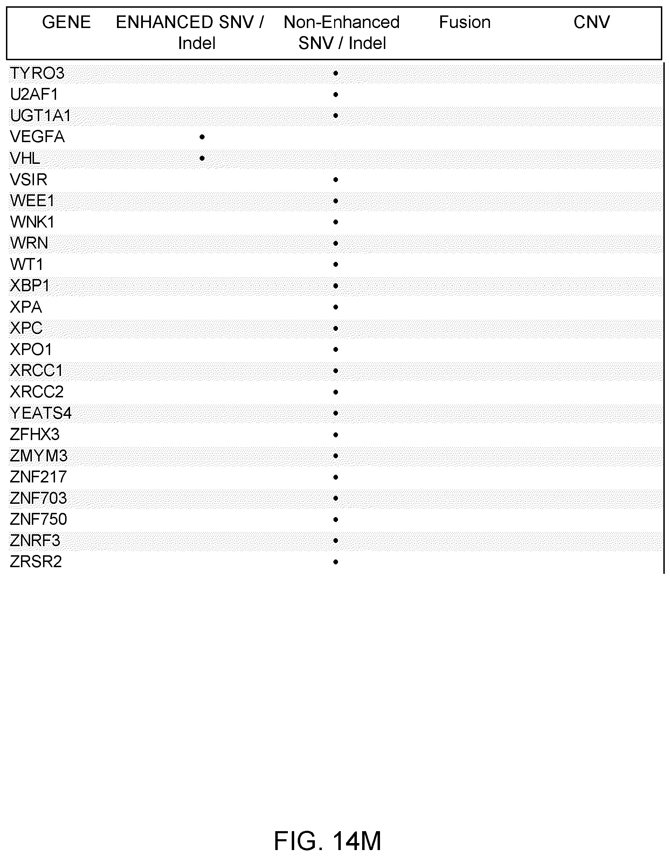

In some embodiments, the second panel-enriched sequencing reaction uses a sequencing panel that enriches for at least 10, 20, 30, 40, 50 or all the genes listed in Figure 14 (any combination of Figures 14A, 14B, 14C, 14D, 14E, 14F, 14G, 14H, 14I, 14j, 14K, 14L, and 14M, collectively "Figure 14"). In some embodiments, the second panel-enriched sequencing reaction uses a sequencing panel that enriches between 25 different genes and 50 different genes listed in Figure 14. In some embodiments, the second panel-enriched sequencing reaction uses a sequencing panel that enriches for between 5 genes and all the genes listed in Figure 14, between 10 genes and all the genes listed in Figure 14, or between 20 genes and all the genes listed in Figure 14. While Figure 14 and List 2 provide the same genes, Figure 14 indicates, in preferred embodiments, the type of variant that is such genes.

In some embodiments, the probe set includes probes that target no more than 50 genes, 100 genes, 150 genes or 200 genes. In some such embodiments the probe set consists of or comprises probes targeting one or more of the genes in Figure 14. In some such embodiments, the probe set consists of or comprises probes targeting at least 5 of the genes listed in Figure 14. In some such embodiments, the probe set consists of or comprises probes targeting at least 10 of the genes in Figure 14. In some such embodiments, the probe set consists of or comprises probes targeting at least 25 of the genes in Figure 14. In some such embodiments, the probe set consists of or comprises probes targeting at least 50 of the genes listed in Figure 14. In some such embodiments, the probe set consists of or comprises probes targeting all of the genes in Figure 14.

In some embodiments the second panel-enriched sequencing reaction uses a sequencing panel that enriches for at least 10, 20, 30, 40, or 50 genes listed in any of Figures 14A, 14B, 14C, 14D, 14E, 14F, 14G, 14H, 14I, 14j, 14K, 14L, and 14M.

In some embodiments the second panel-enriched sequencing reaction uses a sequencing panel that enriches for at least 20, 30, 40, 50, 60, 70, 80, 90, 100, 110, 120, 130, 140, 150, 160, 170, 180, 190, or 200 genes listed in any of Figures 14A, 14B, 14C, 14D, 14E, 14F, 14G, 14H, 14I, 14j, 14K, 14L, and 14M.

In some embodiments the second panel-enriched sequencing reaction uses a sequencing panel that enriches for at most 20, 30, 40, 50, 60, 70, 80, 90, 100, 110, 120, 130, 140, 150, 160, 170, 180, 190, or 200 genes listed in any of Figures 14A, 14B, 14C, 14D, 14E, 14F, 14G, 14H, 14I, 14j, 14K, 14L, and 14M.

In some embodiments, the second panel-enriched sequencing reaction uses a sequencing panel that enriches for between 25 different genes and 150 different genes listed in any of Figures 14A, 14B, 14C, 14D, 14E, 14F, 14G, 14H, 14I, 14j, 14K, 14L, and 14M. In some embodiments, the second panel-enriched sequencing reaction uses a sequencing panel that enriches for between 50 genes and 150 genes, between 100 genes and 200 genes, or between 150 genes and 300 genes listed in any of Figures 14A, 14B, 14C, 14D, 14E, 14F, 14G, 14H, 14I, 14j, 14K, 14L, and 14M.

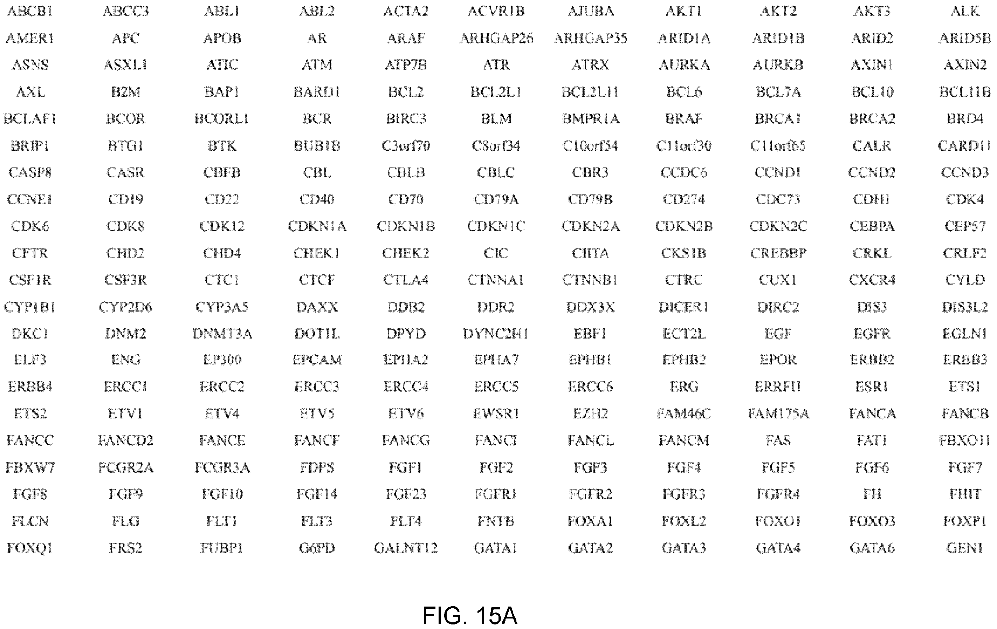

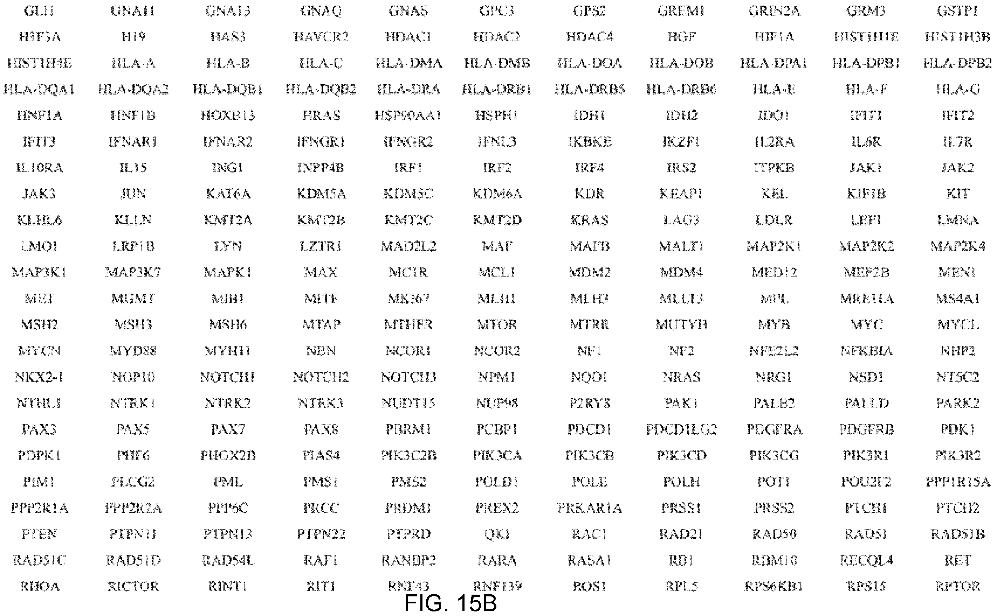

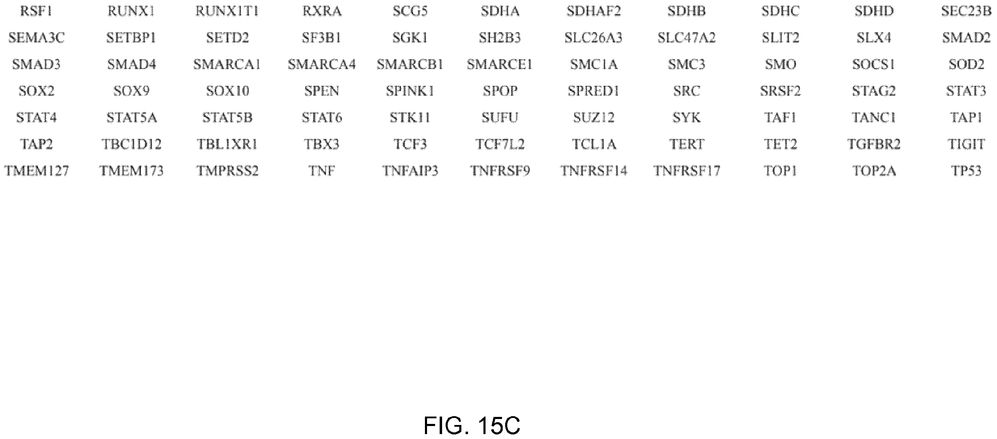

In some embodiments, the second panel-enriched sequencing reaction uses a sequencing panel that enriches for at least 10, 20, 30, 40, 50 or all the genes listed in Figure 15 (any combination of Figures 15A, 15B, and 15C, collectively "Figure 15"). In some embodiments, the second panel-enriched sequencing reaction uses a sequencing panel that enriches between 25 different genes and 50 different genes listed in Figure 15. In some embodiments, the second panel-enriched sequencing reaction uses a sequencing panel that enriches for between 5 genes and all the genes listed in Figure 14, between 10 genes and all the genes listed in Figure 15, or between 20 genes and all the genes listed in Figure 15.

In some such embodiments the probe set consists of or comprises probes targeting one or more of the genes in Figure 15. In some such embodiments, the probe set consists of or comprises probes targeting at least 5 of the genes listed in Figure 15. In some such embodiments, the probe set consists of or comprises probes targeting at least 10 of the genes in Figure 15. In some such embodiments, the probe set consists of or comprises probes targeting at least 25 of the genes in Figure 15. In some such embodiments, the probe set consists of or comprises probes targeting at least 50 of the genes listed in Figure 15. In some such embodiments, the probe set consists of or comprises probes targeting all of the genes in Figure 15.

In some embodiments the second panel-enriched sequencing reaction uses a sequencing panel that enriches for at least 10, 20, 30, 40, or 50 genes listed in any of Figures 145, 15B, and 15C.

In some embodiments the second panel-enriched sequencing reaction uses a sequencing panel that enriches for at least 20, 30, 40, 50, 60, 70, 80, 90, 100, 110, 120, 130, 140, 150, 160, 170, 180, 190, or 200 genes listed in any of Figures 15A, 15B, and 15C.

In some embodiments the second panel-enriched sequencing reaction uses a sequencing panel that enriches for at most 20, 30, 40, 50, 60, 70, 80, 90, 100, 110, 120, 130, 140, 150, 160, 170, 180, 190, or 200 genes listed in any of Figures 15A, 15B, and 15C.

In some embodiments, the second panel-enriched sequencing reaction uses a sequencing panel that enriches for between 25 different genes and 150 different genes listed in any of Figures 15A, 15B, and 15C,. In some embodiments, the second panel-enriched sequencing reaction uses a sequencing panel that enriches for between 50 genes and 150 genes, between 100 genes and 200 genes, or between 150 genes and 300 genes listed in any of Figures 15A, 15B, and 15C.

Generally, probes for enrichment of nucleic acids (e.g., cfDNA obtained from a liquid biopsy sample) include DNA, RNA, or a modified nucleic acid structure with a base sequence that is complementary to a locus of interest. For instance, a probe designed to hybridize to a locus in a cfDNA molecule can contain a sequence that is complementary to either strand, because the cfDNA molecules are double stranded. In some embodiments, each probe in the plurality of probes includes a nucleic acid sequence that is identical or complementary to at least 10, at least 11, at least 12, at least 13, at least 14, or at least 15 consecutive bases of a locus of interest. In some embodiments, each probe in the plurality of probes includes a nucleic acid sequence that is identical or complementary to at least 20, 25, 30, 40, 50, 75, 100, 150, 200, or more consecutive bases of a locus of interest.

Targeted panels provide several benefits for nucleic acid sequencing. For example, in some embodiments, algorithms for discriminating between, *e.g.,* a first and second cancer condition can be trained on smaller, more informative data sets *(e.g.,* fewer genes), which leads to more computationally efficient training of classifiers that discriminate between the first and second cancer states. Such improvements in computational efficiency, owing to the reduced size of the discriminating gene set, can advantageously either be used to speed up classifier training or be used to improve the performance of such classifiers (e.g., through more extensive training of the classifier).

In some embodiments, the gene panel is a whole-exome panel that analyzes the exomes of a biological sample. In some embodiments, the gene panel is a whole-genome panel that analyzes the genome of a specimen.

In some embodiments, the probes include additional nucleic acid sequences that do not share any homology to the loci of interest. For example, in some embodiments, the probes also include nucleic acid sequences containing an identifier sequence, *e.g.,* a unique molecular identifier (UMI), *e.g.,* that is unique to a particular sample or subject. Examples of identifier sequences are described in Kivioja et al., 2011, Nat. Methods 9(1), pp. 72-74 and Islam et al., 2014, Nat. Methods 11(2), pp. 163-66, which are incorporated by reference herein. Similarly, in some embodiments, the probes also include primer nucleic acid sequences useful for amplifying the nucleic acid molecule of interest, *e.g.*, using PCR. In some embodiments, the probes also include a capture sequence designed to hybridize to an anti-capture sequence for recovering the nucleic acid molecule of interest from the sample.

Likewise, in some embodiments, the probes each include a non-nucleic acid affinity moiety covalently attached to nucleic acid molecule that is complementary to the locus of interest, for recovering the nucleic acid molecule of interest. Non-limited examples of non-nucleic acid affinity moieties include biotin, digoxigenin, and dinitrophenol. In some embodiments, the probe is attached to a solid-state surface or particle, *e.g.,* a dipstick or magnetic bead, for recovering the nucleic acid of interest. In some embodiments, the methods described herein include amplifying the nucleic acids that bound to the probe set prior to further analysis, *e.g.,* sequencing. Methods for amplifying nucleic acids, *e.g.,* by PCR, are well known in the art.

Next-generation sequencing produces millions of short reads (*e.g.*, sequence reads) for each biological sample. Accordingly, in some embodiments, the plurality of sequence reads obtained by next-generation sequencing of cfDNA molecules are DNA sequence reads. In some embodiments, the sequence reads have an average length of at least fifty nucleotides. In other embodiments, the sequence reads have an average length of at least 50, 60, 70, 80, 90, 100, 150, 200, 250, 300, or more nucleotides.

In some embodiments, sequencing is performed after enriching for nucleic acids (*e.g.,* cfDNA, gDNA, and/or RNA) encompassing a plurality of predetermined target sequences, *e.g.,* human genes and/or non-coding sequences associated with cancer. Advantageously, sequencing a nucleic acid sample that has been enriched for target nucleic acids, rather than all nucleic acids isolated from a biological sample, significantly reduces the average time and cost of the sequencing reaction. Accordingly, in some preferred embodiments, the methods described herein include obtaining a plurality of sequence reads of nucleic acids that have been hybridized to a probe set for hybrid-capture enrichment (*e.g.,* of one or more genes listed in Table 1 or of one or more genes listed in Table 2, one or more genes listed in List 1, one or more genes listed in List 2, or one or more genes listed in Figure 14).

In some embodiments, the second panel-targeting sequencing is performed to an average on-target depth of at least 50X, at least 100X, at least 125X at least 150X, at least 500X, at least 750X, at least 1000X, at least 2500X, at least 10,000X, or greater depth. In some embodiments, samples are further assessed for uniformity above a sequencing depth threshold (*e.g.,* 95% of all targeted base pairs at 300x sequencing depth). In some embodiments, the sequencing depth threshold is a minimum depth selected by a user or practitioner.

*Block 509.* Referring to block 509, in some embodiments, a second plurality of nucleic acid sequences is obtained that includes a corresponding nucleic acid sequence for each cell-free DNA fragment in a plurality of cell-free DNA fragments obtained from a liquid biopsy sample from a first panel-enriched sequencing assay using a first plurality of probes including, for each respective locus in the plurality of loci, a corresponding probe that hybridizes the respective locus.

In some embodiments, the second plurality of nucleic acids includes at least 10,000 sequences, at least 50,000 sequences, at least 100,000 sequences, at least 500,000 sequences, at least 1 million sequences, at least 5 million sequences, at least 10 million sequences, or more. In some embodiments, the second plurality of sequences includes no more than 1 billion sequences, no more than 500 million sequences, no more than 100 million sequences, no more than 50 million sequences, no more than 10 million sequences, no more than 5 million sequences, no more than 1 million sequences, or less. In some embodiments, the second plurality of sequences is from 10,000 sequences to 1 billion sequences, from 10,000 sequences to 500 million sequences, from 10,000 sequences to 100 million sequences, from 10,000 sequences to 50 million sequences, from 10,000 sequences to 10 million sequences, from 10,000 sequences to 5 million sequences, or from 10,000 sequences to 1 million sequences. In some embodiments, the second plurality of sequences is from 100,000 sequences to 1 billion sequences, from 100,000 sequences to 500 million sequences, from 100,000 sequences to 100 million sequences, from 100,000 sequences to 50 million sequences, from 100,000 sequences to 10 million sequences, from 100,000 sequences to 5 million sequences, or from 100,000 sequences to 1 million sequences. In some embodiments, the second plurality of sequences is from 500,000 sequences to 1 billion sequences, from 500,000 sequences to 500 million sequences, from 500,000 sequences to 100 million sequences, from 500,000 sequences to 50 million sequences, from 500,000 sequences to 10 million sequences, from 500,000 sequences to 5 million sequences, or from 500,000 sequences to 1 million sequences. In some embodiments, the second plurality of sequences is from 1 million sequences to 1 billion sequences, from 1 million sequences to 500 million sequences, from 1 million sequences to 100 million sequences, from 1 million sequences to 50 million sequences, from 1 million sequences to 10 million sequences, or from 1 million sequences to 5 million sequences.

In some embodiments, the first plurality of probe used to enrich cell-free DNA fragments in the liquid biopsy sample in the first panel-enriched sequencing reaction collectively map to at least 25 different genes in human reference genome. In some embodiments, the first plurality of probes collectively maps to at least 50, at least 100, at least 250, at least 500, or at least 1000 different genes in the human reference genome. In some embodiments, the first plurality of probes collectively maps to at least 10 of the genes listed in Table 1. In some embodiments, the first plurality of probes collectively map to at least 20, 25, 30, 40, 50, 60, 75, 100, or all 105 of the genes listed in Table 1, Table 2, List 1, List 2, and/or Figure 14.

For example, in some embodiments, a targeted enrichment panel of block 509 (first plurality of probes) comprises any of the embodiments of the second plurality of probes described in block 504 for the second panel-enriched sequencing reaction.

In some embodiments, the targeted enrichment panel of block 509 includes probes targeting one or more gene loci, *e.g.,* exon or intron loci. In some embodiments, the targeted enrichment panel of block 509 includes probes targeting one or more loci not encoding a protein, *e.g.,* regulatory loci, miRNA loci, and other non-coding loci, *e.g.,* that have been found to be associated with cancer. In some embodiments, the plurality of loci targeted in block 509 includes at least 25, 50, 100, 150, 200, 250, 300, 350, 400, 500, 750, 1000, 2500, 5000, or more human genomic loci.

In some embodiments, the targeted enrichment panel of block 509 includes probes targeting one or more of the genes listed in Table 1, Table 2, List 1, List 2, Figure 14 and/or Figure 15. In some embodiments, the targeted enrichment panel of block 509 includes probes targeting at least 5 of the genes listed in Table 1, Table 2, List 1, List 2, Figure 14 and/or

EP 4 629 247 A1

Figure 15. In some embodiments, the targeted enrichment panel of block 509 includes probes targeting at least 10 of the genes listed in Table 1, Table 2, List 1, List 2, Figure 14 and/or Figure 15. In some embodiments, the targeted enrichment panel of block 509 includes probes targeting at least 25 of the genes listed in Table 1, Table 2, List 1, List 2, Figure 14 and/or Figure 15. In some embodiments, the targeted enrichment panel of block 509 includes probes targeting at least 50 of the genes listed in Table 1, Table 2, List 1, List 2, Figure 14 and/or Figure 15. In some embodiments, the targeted enrichment panel of block 509 includes probes targeting at least 75 of the genes listed in Table 1, Table 2, List 1, List 2, Figure 14 and/or Figure 15. In some embodiments, the targeted enrichment panel of block 509 includes probes targeting at least 100 of the genes listed in Table 1, Table 2, List 1, List 2, Figure 14 and/or Figure 15. In some embodiments, the targeted enrichment panel of block 509 includes probes targeting all of the genes listed in Table 1, Table 2, List 1, List 2, Figure 14 and/or Figure 15.

In some embodiments, the obtaining, accessioning, storing, preparing, processing and/or analyzing the liquid biopsy sample from the test subject comprises any of the methods and/or embodiments described above in the present disclosure. In some embodiments, the sequencing reaction comprises any of the methods and/or embodiments described above in the present disclosure.

*Block 510.* Referring to block 510, in some embodiments, the first plurality of probes and the second plurality of probes are different. That is, in some embodiments, a different probe set is used to enrich genomic DNA sequences from the solid tumor sample than the probe set used to enrich cell free DNA from the liquid biopsy sample.

In fact, in some embodiments, the only requirement for the relationship between probe sets used to enrich DNA from the solid tumor sample is that there is some overlap in the genomic regions being pulled down, such that the variable allele frequency for one or more somatic mutations identified from the solid tumor sample can be used to estimate the circulating tumor fraction of the liquid biopsy sample.

In some embodiments, the only requirement for the relationship between probe sets used to enrich DNA from the solid tumor sample is that there is some overlap in the genes that are enriched by the probe sets used in block 509 and the probe sets used in used in block 504. In some embodiments the first plurality of nucleic acid sequences is obtained using whole genome sequencing and thus there is overlap between the first plurality of nucleic acid sequences of block 502 and the second plurality of nucleic sequences of block 509.

In some embodiments the first plurality of nucleic acid sequences and the second plurality of nucleic sequences of block 509 each collectively map to 5 or more, 10 or more, 20 or more, 30 or more, 40 or more, 50 or more, 60 or more, 70 or more, 80 or more of the same genes.

In some embodiments the first plurality of nucleic acid sequences and the second plurality of nucleic sequences of block 509 each collectively map to between 5 and 500, between 10 and 1000, between 20 and 500, between 30 and 2000, or between 40 and 400 of the same genes.

*Block 512.* Referring to block 512, in some embodiments, the plurality of loci is sequenced at an average sequence depth of at least 500X in the first panel-enriched sequencing reaction. In some embodiments, the first panel-enriched sequencing reaction is performed at a read depth of at least 1000X. In some embodiments, the panel-enriched sequencing reaction is performed at a read depth of at least 100X, at least 500X, at least 1000X, at least 5000X, at least 10,000X, at least 50,000X, or greater. In some embodiments, the panel-enriched sequencing reaction is performed at a read depth of no more than 100,000X, no more than 50,000X, no more than 10,000X, no more than 5000X, or less. In some embodiments, the panel-enriched sequencing reaction is performed at a read depth of from 100X to 50,000X, from 100X to 10,000X, from 100X to 5000X, from 100X to 1000X, or from 100X to 500X. In some embodiments, the panel-enriched sequencing reaction is performed at a read depth of from 500X to 50,000X, from 500X to 10,000X, from 500X to 5000X, or from 500X to 1000X. In some embodiments, the panel-enriched sequencing reaction is performed at a read depth of from 1000X to 50,000X, from 1000X to 10,000X, or from 1000X to 5000X.

*Block 514.* Referring to block 514, in some embodiments, the first plurality of probes enriches for loci from at least 50 genes. In some embodiments, the panel-enriched sequencing reaction uses a sequencing panel that enriches for at least 25 genes, at least 50 genes, at least 100 genes, at least 250 genes, at least 500 genes, at least 1000 genes, at least 2500 genes, at least 5000 genes, or more. In some embodiments, the panel-enriched sequencing reaction uses a sequencing panel that enriches for no more than 40,000 genes, no more than 20,000 genes, no more than 10,000 genes, no more than 5000 genes, no more than 2500 genes, no more than 1000 genes, or less. In some embodiments, the panel-enriched sequencing reaction uses a sequencing panel that enriches for from 25 genes to 10,000 genes, from 25 genes to 5000 genes, from 25 genes to 2500 genes, from 25 genes to 1000 genes, from 25 genes to 500 genes, or from 25 genes to 250 genes. In some embodiments, the panel-enriched sequencing reaction uses a sequencing panel that enriches for from 50 genes to 10,000 genes, from 50 genes to 5000 genes, from 50 genes to 2500 genes, from 50 genes to 1000 genes, from 50 genes to 500 genes, or from 50 genes to 250 genes. In some embodiments, the panel-enriched sequencing reaction uses a sequencing panel that enriches for from 100 genes to 10,000 genes, from 100 genes to 5000 genes, from 100 genes to 2500 genes, from 100 genes to 1000 genes, from 100 genes to 500 genes, or from 100 genes to 250 genes.

In some embodiments, the plurality of probe sequences used to enrich genomic DNA sequences from the solid tumor samples in the second panel-enriched sequencing reaction collectively map to at least 25 different genes in a human reference genome. In some embodiments, the panel-enriched sequencing reaction uses a sequencing panel that enriches for at least 25 human genes, at least 50 human genes, at least 100 human genes, at least 250 human genes, at least 500 human genes, at least 1000 human genes, at least 2500 human genes, at least 5000 human genes, or more. In some embodiments, the panel-enriched sequencing reaction uses a sequencing panel that enriches for no more than 40,000 human genes, no more than 20,000 human genes, no more than 10,000 human genes, no more than 5000 human genes, no more than 2500 human genes, no more than 1000 human genes, or less. In some embodiments, the panel-enriched sequencing reaction uses a sequencing panel that enriches for from 25 human genes to 10,000 human genes, from 25 human genes to 5000 human genes, from 25 human genes to 2500 human genes, from 25 human genes to 1000 human genes, from 25 human genes to 500 human genes, or from 25 human genes to 250 human genes. In some embodiments, the panel-enriched sequencing reaction uses a sequencing panel that enriches for from 50 human genes to 10,000 human genes, from 50 human genes to 5000 human genes, from 50 human genes to 2500 human genes, from 50 human genes to 1000 human genes, from 50 human genes to 500 human genes, or from 50 human genes to 250 human genes. In some embodiments, the panel-enriched sequencing reaction uses a sequencing panel that enriches for from 100 human genes to 10,000 human genes, from 100 human genes to 5000 human genes, from 100 human genes to 2500 human genes, from 100 human genes to 1000 human genes, from 100 human genes to 500 human genes, or from 100 human genes to 250 human genes.

*Block 516.* Referring to block 516, in some embodiments, the identity of the first plurality of probes is non-bespoke for the test subject. Advantageously, the methods and systems described herein facilitate tumor-informed estimation of tumor fraction without requiring bespoke sequencing of the liquid biopsy sample. In this fashion, sequencing data from any number of commercial panel-enriched sequencing assays for evaluating solid tumor biopsies can be used to inform evaluation of any number of commercial panel-enriched sequencing assays for liquid biopsy samples.

*Block 518.* Referring to block 518, in some embodiments, the solid tumor sample is collected prior to collecting the liquid biopsy sample. In some embodiments, the solid tumor sample is collected at least a day before the liquid biopsy sample is collected. In some embodiments, the solid tumor sample is collected at least a week, at least a month, at least 2 months, at least 3 months, at least 4 months, at least 5 months, at least 6 months, at least 9 months, at least 12 months, at least 18 months, at least 2 years, or more before the liquid biopsy sample is collected. In some embodiments, the solid tumor sample is collected no more than 5 years before the liquid biopsy sample. In some embodiments, the solid tumor sample is collected no more than 4 years, no more than 3 years, no more than 2 years, no more than 18 months, no more than 12 months, no more than 9 months, no more than 6 months, no more than 5 months, no more than 4 months, no more than 3 months, no more than 2 months, no more than 1 month, no more than 3 weeks, no more than 2 weeks, no more than 1 week, or less before collection of the liquid biopsy sample. In some embodiments, the solid tumor sample is collected between 1 day and 5 years before the liquid biopsy sample is collected. In some embodiments, the solid tumor sample is collected between 1 day and 2 years, between 1 day and 1 year, between 1 day and 6 months, between 1 day and 3 months, between 1 day and 1 month, or between 1 day and 1 week before the liquid biopsy sample is collected. In some embodiments, the solid tumor biopsy and the liquid biopsy sample are collected on the same day. In some embodiments, the liquid biopsy sample is collected before the solid tumor biopsy is collected.

*Block 520.* Referring to block 520, in some embodiments, the solid tumor sample and the liquid biopsy sample are collected within 6 months of each other. In some embodiments, the solid tumor sample and the liquid biopsy sample are collected within 1 day, 1 week, 2 weeks, 1 month, 3 months, 6 months, 9 months, 12 months, 18 months, 2 years, 3 years, 4 years, or 5 years, of each other.

*Blocks 522-524.* Referring to block 522, in some embodiments, the liquid biopsy sample is blood. Referring to block 524, in some embodiments, the liquid biopsy sample includes blood, whole blood, peripheral blood, plasma, serum, or lymph of the subject. In some embodiments, one or more of the biological samples obtained from the patient are a biological liquid sample, also referred to as a liquid biopsy sample. In some embodiments, one or more of the biological samples obtained from the patient are selected from blood, plasma, serum, urine, vaginal fluid, fluid from a hydrocele (*e.g.,* of the testis), vaginal flushing fluids, pleural fluid, ascitic fluid, cerebrospinal fluid, saliva, sweat, tears, sputum, bronchoalveolar lavage fluid, discharge fluid from the nipple, aspiration fluid from different parts of the body (*e.g.*, thyroid, breast), etc. In some embodiments, the liquid biopsy sample includes blood and/or saliva. In some embodiments, the liquid biopsy sample is peripheral blood. In some embodiments, one or more blood samples are collected from a subject in commercial blood collection containers. In some embodiments, saliva samples are collected from patients in commercial saliva collection containers.

In some embodiments, the volume of the first liquid biopsy sample is less than 30 mL. In some embodiments, the volume of the liquid biopsy sample is from 1 mL to 50 mL, from 2 mL to 40 mL, from 3 mL to 35 mL, or from 5 mL to 31 mL. For example, in some embodiments, the liquid biopsy sample has a volume of about 1 mL, about 2 mL, about 3 mL, about 4 mL, about 5 mL, about 6 mL, about 7 mL, about 8 mL, about 9 mL, about 10 mL, about 11 mL, about 12 mL, about 13 mL, about 14 mL, about 15 mL, about 16 mL, about 17 mL, about 18 mL, about 19 mL, about 20 mL, or greater.

As described herein, cfDNA is a particularly useful source of biological data for various implementations of the methods and systems described herein, because it is readily obtained from various body fluids. Advantageously, use of bodily fluids facilitates serial monitoring because of the ease of collection, as these fluids are collectable by non-invasive or minimally invasive methodologies. This is in contrast to methods that rely upon solid tissue samples, such as biopsies, which often requires invasive surgical procedures. Further, because bodily fluids, such as blood, circulate throughout the body, the cfDNA population represents a sampling of many different tissue types from many different locations.

In some embodiments, a liquid biopsy sample is separated into two different samples. For example, in some embodiments, a blood sample is separated into a blood plasma sample, containing cfDNA, and a buffy coat preparation, containing white blood cells.

In some embodiments, a plurality of liquid biopsy samples is obtained from a respective subject at intervals over a period of time (*e.g.*, using serial testing). For example, in some such embodiments, the time between obtaining liquid biopsy samples from a respective subject is at least 1 day, at least 2 days, at least 1 week, at least 2 weeks, at least 1 month, at least 2 months, at least 3 months, at least 4 months, at least 6 months, or at least 1 year.

Liquid biopsy samples include cell free nucleic acids, including cell-free DNA (cfDNA). As described above, cfDNA isolated from cancer patients includes DNA originating from cancerous cells, also referred to as circulating tumor DNA (ctDNA), cfDNA originating from germline (*e.g.*, healthy or non-cancerous) cells, and cfDNA originating from hematopoietic cells (*e.g.,* white blood cells). The relative proportions of cancerous and non-cancerous cfDNA present in a liquid biopsy sample varies depending on the characteristics (*e.g.,* the type, stage, lineage, genomic profile, *etc.*) of the patient's cancer.

In some embodiments cell-free DNA is isolated from the liquid biological sample using commercially available reagents, including digestion with proteinase K. In some embodiments, the selective binding properties of a silica membrane are used to extract cell-free DNA from the first liquid biological sample using circulating nucleic acid kits. In some such embodiments, the liquid biological sample is lysed in an optimized buffer and adjusted to binding conditions. Then, the liquid biological sample is loaded directly onto a spin column. In this step, cell-free DNA is bound to the silica membrane, and contaminants are removed in wash steps. Finally, pure cell-free DNA is eluted in small volumes of a low-salt buffer for downstream applications. *See,* Hai et al., 2022, "Whole-genome circulating tumor DNA methylation landscape reveals sensitive biomarkers of breast cancer," MedComm (2020) Sep 3(3): e134, which is hereby incorporated by reference.

In some embodiments, adapters such as unique dual index (UDI) adapters are ligated onto the cell-free DNA fragments. In some embodiments, adapters with unique molecular indices (UMI), which are short nucleic acid sequences (*e.g.,* 4-10 base pairs) , are ligated onto the cell-free DNA fragments. In some embodiments, the UDI adapters include UMIs. In some embodiments, UMIs are degenerate base pairs that serve as a unique tag that can be used to identify sequence reads originating from a specific DNA fragment. In some embodiments, *e.g.,* when multiplex sequencing will be used to sequence cell-free DNA fragments from a plurality of samples (*e.g.*, from the same or different subjects) in a single sequencing reaction, a patient-specific index is also added to the nucleic acid molecules. In some embodiments, the sample specific index is a short nucleic acid sequence (*e.g.,* 3-20 nucleotides) that are added to ends of cell-free DNA fragments during library construction, that serve as a unique tag that can be used to identify sequence reads originating from a specific patient sample.

In some embodiments, an adapter includes a PCR primer landing site, designed for efficient binding of a PCR or second-strand synthesis primer used during the sequencing reaction. In some embodiments, an adapter includes an anchor binding site, to facilitate binding of the cell-free DNA fragment to anchor oligonucleotide molecules on a sequencer flow cell, serving as a seed for the sequencing process by providing a starting point for the sequencing reaction. During PCR amplification following adapter ligation, the UMIs, patient indexes, and binding sites are replicated along with the attached cell-free DNA fragment. This provides a way to identify sequence reads that came from the same original cell-free DNA fragment in downstream analysis.

In some embodiments the sequence reads in the first plurality of sequence reads are trimmed to remove sequencing adapters, amplification primers, and low-quality bases in read ends. This can be done, for example, using trim_galore (version 0.4.2) or cutadept. *See,* respectively, the Internet at URL bioinformatics.babraham.ac.uk/projects/trim_galore and Martin, 2011, "Cutadapt removes adapter sequences from high-throughput sequencing reads," EMBnet.journal, [S.l.] 17(1), pp. 10-12.

In some embodiments, the cell-free DNA fragments are amplified and purified using commercial reagents. In some such embodiments, the concentration and/or quantity of the cell-free DNA fragments are quantified using a fluorescent dye and a fluorescence microplate reader, standard spectrofluorometer, or filter fluorometer. In some embodiments, library amplification is performed on a device (*e.g.,* an Illumina C-Bot2) and the resulting flow cell containing amplified cell-free DNA fragments is sequenced.

In some embodiments sequencing is performed on a next generation sequencer (*e.g.,* an Illumina HiSeq 4000, Illumina NovaSeq 6000, Oxford Nanopore, Biomodal) to a unique on-target depth selected by the user. In some embodiments, sequencing is performed using sequencing-by-synthesis technology (Illumina), pyrosequencing (454 Life Sciences), ion semiconductor technology (Ion Torrent sequencing), single-molecule real-time sequencing (Pacific Biosciences), or sequencing by ligation (SOLiD sequencing).

Referring to Figure 2, in some embodiments the biological samples collected from the patient are, optionally, sent to various analytical environments (*e.g.*, sequencing lab 230, pathology lab 240, and/or molecular biology lab 250) for processing (*e.g.*, data collection) and/or analysis (*e.g.*, feature extraction). Wet lab processing 204 may include cataloguing samples (*e.g.,* accessioning), examining clinical features of one or more samples (*e.g.,* pathology review), and nucleic acid sequence analysis (*e.g.*, extraction, library prep, capture + hybridize, pooling, and sequencing). In some embodiments, the workflow includes clinical analysis of one or more biological samples collected from the subject, *e.g.*, at a pathology lab 240 and/or a molecular and cellular biology lab 250, to generate clinical features such as pathology features 128-3, imaging data 128-3, and/or tissue culture / organoid data 128-3.

In some embodiments, the pathology data 128-1 collected during clinical evaluation includes visual features identified by a pathologist's inspection of a specimen (*e.g.,* a solid tumor biopsy), *e.g.,* of stained H&E or IHC slides. In some embodiments, the sample is a solid tissue biopsy sample. In some embodiments, the tissue biopsy sample is a formalin-fixed tissue (FFT), *e.g.,* a formalin-fixed paraffin-embedded (FFPE) tissue. In some embodiments, the tissue biopsy sample is an FFPE or FFT block. In some embodiments, the tissue biopsy sample is a fresh-frozen tissue biopsy. The tissue biopsy sample can be prepared in thin sections (*e.g.*, by cutting and/or affixing to a slide), to facilitate pathology review (*e.g.*, by staining with immunohistochemistry stain for IHC review and/or with hematoxylin and eosin stain for H&E pathology review). For instance, analysis of slides for H&E staining or IHC staining may reveal features such as tumor infiltration, programmed death-ligand 1 (PD-L1) status, human leukocyte antigen (HLA) status, or other immunological features.

In some embodiments, a liquid sample (*e.g.*, blood) collected from the patient (*e.g.,* in EDTA-containing collection tubes) is prepared on a slide (*e.g.,* by smearing) for pathology review. In some embodiments, macrodissected FFPE tissue sections, which may be mounted on a histopathology slide, from solid tissue samples (*e.g.*, tumor or normal tissue) are analyzed by pathologists. In some embodiments, tumor samples are evaluated to determine, *e.g.,* the tumor purity of the sample, the percent tumor cellularity as a ratio of tumor to normal nuclei, *etc.* For each section, background tissue may be excluded or removed such that the section meets a tumor purity threshold, *e.g.,* where at least 20% of the nuclei in the section are tumor nuclei, or where at least 25%, 30%, 40%, 50%, 60%, 70%, 80%, 90%, or more of the nuclei in the section are tumor nuclei.

In some embodiments, pathology data 128-1 is extracted, in addition to or instead of visual inspection, using computational approaches to digital pathology, *e.g.,* providing morphometric features extracted from digital images of stained tissue samples. In some embodiments, pathology data 128-1 includes features determined using machine learning algorithms to evaluate pathology data collected as described above.

Further details on methods, systems, and algorithms for using pathology data to classify cancer and identify targeted therapies are discussed, for example, in U.S. Patent No. 10,957,041, 11,244,763, 11,848,107, and 11,145,416, the contents of which are each hereby incorporated by reference, in their entireties, for all purposes.

In some embodiments, imaging data 128-2 collected during clinical evaluation includes features identified by review of in-vitro and/or in-vivo imaging results (*e.g.,* of a tumor site), for example a size of a tumor, tumor size differentials over time (such as during treatment or during other periods of change). In some embodiments, imaging data 128-2 includes features determined using machine learning algorithms to evaluate imaging data collected as described above.

Further details on methods, systems, and algorithms for using medical imaging to classify cancer and identify targeted therapies are discussed, for example, in U.S. Patent No. 10,957,041, 11,244,763, 11,848,107, and 11,145,416, the contents of which are each hereby incorporated by reference, in their entireties, for all purposes.

In some embodiments, tissue culture / organoid data 128-3 collected during clinical evaluation includes features identified by evaluation of cultured tissue from the subject. For instance, in some embodiments, tissue samples obtained from the patients (*e.g.*, tumor tissue, normal tissue, or both) are cultured (*e.g.,* in liquid culture, solid-phase culture, and/or organoid culture) and various features, such as cell morphology, growth characteristics, genomic alterations, and/or drug sensitivity, are evaluated. In some embodiments, tissue culture / organoid data 128-3 includes features determined using machine learning algorithms to evaluate tissue culture / organoid data collected as described above. Examples of tissue organoid (*e.g.*, personal tumor organoid) culturing and feature extractions thereof are described in PCT publication No. WO2021/081253 and U.S. Patent No. 11,629,385, the contents of which are each hereby incorporated by reference, in their entireties, for all purposes.

In some embodiments, the method further comprises obtaining the liquid biopsy sample from a sample repository or database (*e.g.*, BioIVT, TSC Biosample Repository, BioLINCC, *etc.*). In some embodiments, the liquid biopsy sample is obtained from the subject at least 1 hour, at least 2 hours, at least 12 hours, at least 1 day, at least 2 days, at least 1 week, at least 1 month, or at least 1 year prior to processing and/or sequencing the liquid biopsy sample. In some such embodiments, the liquid biopsy sample is fresh, frozen, dried, and/or fixed. In some embodiments, the liquid biopsy sample is processed and/or sequenced at least 1 day, at least 2 days, at least 1 week, at least 1 month, or at least 1 year prior to obtaining the first dataset. For example, in some embodiments, the sequencing data for the liquid biopsy sample are obtained from a data repository (e.g., GenBank, NCBI Assembly, DNA DataBank of Japan, European Nucleotide Archive, European Variation Archive, *etc.*).

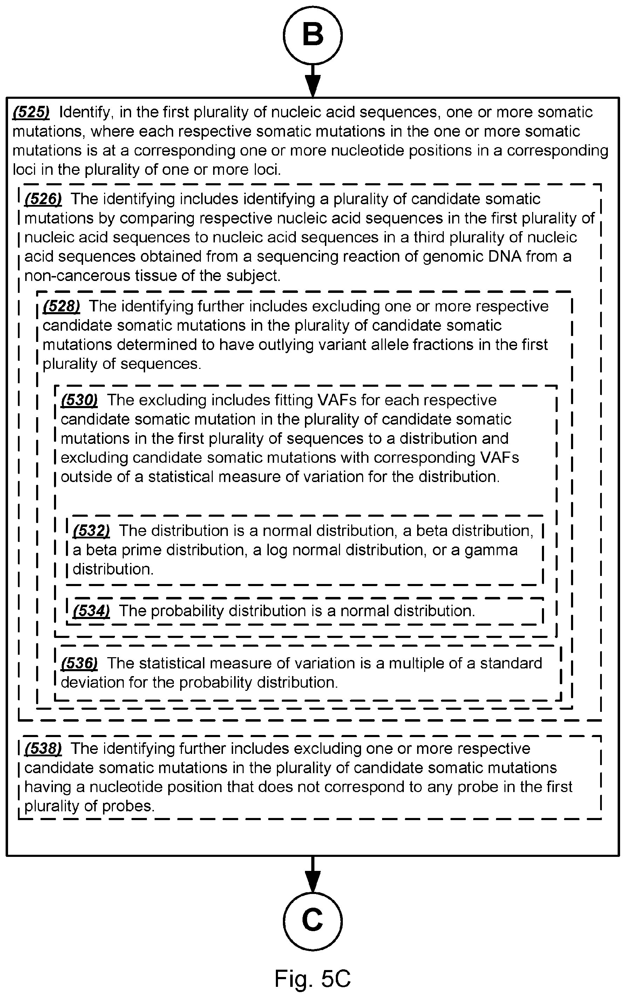

*Block 525.* Referring to block 525 of Figure 5C, method 500 also includes identifying, in the first plurality of nucleic acid sequences, one or more somatic mutations, where each respective somatic variant in the one or more somatic variants is at a corresponding one or more nucleotide positions in a corresponding loci in the plurality of one or more loci.

Example 1 provides an illustration of block 525.

Also, referring to Figure 2A, in some embodiments nucleic acid sequencing data 122 generated from the one or more patient samples (e.g., the first plurality of nucleic acid sequences comprising a corresponding nucleic acid sequence for each respective locus in a plurality of loci in genomic DNA from a solid tumor sample from the test subject) is evaluated (*e.g.,* via variant analysis 206) in a bioinformatics pipeline, *e.g.,* using bioinformatics module 140 of system 100, to identify genomic alterations in the cancer genome of the patient. An example overview for a bioinformatics pipeline is described herein with respect to Figures 4A-4E. Advantageously, in some embodiments, the present disclosure improves bioinformatics pipelines, like pipeline 206, by improving circulating tumor fraction estimates. In some embodiments in which panel-based sequencing was used to acquire the sequencing data 122, the analysis is limited to the plurality of loci that the panel-based sequencing encompasses.

Figure 4A illustrates an example bioinformatics pipeline 206 (*e.g.*, as used for feature extraction in the workflows illustrated in Figures 2A and 3) for providing clinical support for precision oncology. As shown in Figure 4A, sequencing data 122 obtained from the wet lab processing 204 (*e.g.,* sequence reads 314; the first plurality of nucleic acid sequences comprising a corresponding nucleic acid sequence for each respective locus in a plurality of loci in genomic DNA from a solid tumor sample from the test subject) is input into the pipeline.

In various embodiments, the bioinformatics pipeline includes a circulating tumor DNA (ctDNA) pipeline for analyzing liquid biopsy samples. In some embodiments, the pipeline detects SNVs, INDELs, copy number amplifications/deletions and genomic rearrangements (for example, fusions). In some embodiments, the pipeline employs unique molecular index (UMI)-based consensus base calling as a method of error suppression as well as a Bayesian tri-nucleotide context-based position level error suppression. In various embodiments, the pipeline is able to detect variants having a 0.1%, 0.15%, 0.2%, 0.25%, 0.3%, 0.4%, or 0.5% variant allele fraction.

As illustrated in Example 1, in some embodiments in accordance with block 525, 3, 4, 5, 6, 7, 8, 9, 10, 11, 12, 13, 14, 15, 16, 17, 18, 19, 20, 21, 22, 23, 24, 25, 26, 27, 28, 29, or 30 or more somatic mutations are identified using the first plurality of nucleic acid sequences comprising a corresponding nucleic acid sequence for each respective locus in a plurality of loci in genomic DNA from a solid tumor sample from the test subject. In some embodiments at least 30, 40, 50, 60, 70, 80, 90, 100, 110, 120, 130, 140, 150, 160, 170, 180, 190, 200, 210, 220, 230, 240, 250, 260, 270, 280, 290, or 300 more somatic mutations are identified using the first plurality of nucleic acid sequences comprising a corresponding nucleic acid sequence for each respective locus in a plurality of loci in genomic DNA from a solid tumor sample from the test subject.

In some embodiments in accordance with block 525, less than 30, 40, 50, 60, 70, 80, 90, 100, 110, 120, 130, 140, 150, 160, 170, 180, 190, 200, 210, 220, 230, 240, 250, 260, 270, 280, 290, or 300 somatic mutations are identified using the first plurality of nucleic acid sequences comprising a corresponding nucleic acid sequence for each respective locus in a plurality of loci in genomic DNA from a solid tumor sample from the test subject.

In some embodiments in accordance with block 525, between 3 and 30, 3 and 40, 3 and 50, 3 and 60, 3 and 70, 3 and 80, 3 and 90, 3 and 100, 3 and 110, 3 and 120, 3 and 130, 3 and 140, 3 and 150, 3 and 160, 3 and 170, 3 and 180, 3 and 190, 3 and 200, 3 and 210, 3 and 220, 3 and 230, 3 and 240, 3 and 250, 3 and 260, 3 and 270, 3 and 280, 3 and 290, or 3 and 300 somatic mutations are identified using the first plurality of nucleic acid sequences comprising a corresponding nucleic acid sequence for each respective locus in a plurality of loci in genomic DNA from a solid tumor sample from the test subject.

In some embodiments, each respective somatic mutation identified in accordance with block 525 has a variant allele frequency for the respective somatic mutation of at least 0.1 percent. In some embodiments, each respective somatic mutation identified in accordance with block 525 has a variant allele frequency for the respective somatic mutation of at least 1 percent. In some embodiments, each respective somatic mutation identified in accordance with block 525 has a variant allele frequency for the respective somatic mutation of at least 2 percent. In some embodiments, each respective somatic mutation identified in accordance with block 525 has a variant allele frequency for the respective somatic mutation of at least 5 percent.

In some embodiments, each respective somatic mutation identified in accordance with block 525 has a variant allele frequency for the respective somatic mutation that is greater than 0.1 percent but is less than 60 percent. In some embodiments, each respective somatic mutation identified in accordance with block 525 has a variant allele frequency for the respective somatic mutation that is greater than 0.1 percent but less than 50 percent. In some embodiments, each respective somatic mutation identified in accordance with block 525 has a variant allele frequency for the respective somatic mutation that is greater than 0.1 percent but less than 40 percent. In some embodiments, each respective somatic mutation identified in accordance with block 525 has a variant allele frequency for the respective somatic mutation that is greater than 0.1 percent but less than 30 percent.

In some embodiments, each respective somatic mutation identified in accordance with block 525 has a variant allele frequency for the respective somatic mutation that is greater than 5 percent but is less than 60 percent. In some embodiments, each respective somatic mutation identified in accordance with block 525 has a variant allele frequency for the respective somatic mutation that is greater than 5 percent but is less than 50 percent. In some embodiments, each respective somatic mutation identified in accordance with block 525 has a variant allele frequency for the respective somatic mutation that is greater than 5 percent but is less than 40 percent. In some embodiments, each respective somatic mutation identified in accordance with block 525 has a variant allele frequency for the respective somatic mutation that is greater than 5 percent but is less than 30 percent.

*Block 526.* Referring to block 526, in some embodiments, the identifying of block 525 includes identifying the plurality of candidate somatic mutations by comparing respective nucleic acid sequences in the first plurality of nucleic acid sequences to nucleic acid sequences in a third plurality of nucleic acid sequences obtained from a sequencing reaction of genomic DNA from a non-cancerous tissue of the subject. That is, in some embodiments, the process for identifying somatic mutations includes excluding mutations that are present in the germline of the subject, even if they are pathogenic or likely pathogenic in nature.

In some such embodiments, a dedicated normal sample is collected from the patient, for co-processing with a liquid biopsy sample. Generally, the normal sample is of a non-cancerous tissue, and can be collected using any tissue collection means described herein. In some embodiments, buccal cells collected from the inside of a patient's cheeks are used as a normal sample. Buccal cells can be collected by placing an absorbent material, *e.g.*, a swab, in the subject's mouth and rubbing it against their cheek, *e.g.,* for at least 15 second or for at least 30 seconds. The swab is then removed from the patient's mouth and inserted into a tube, such that the tip of the tube is submerged into a liquid that serves to extract the buccal cells off of the absorbent material. An example of buccal cell recovery and collection devices is provided in U.S. Patent No. 9,138,205, the content of which is hereby incorporated by reference, in its entirety, for all purposes. In some embodiments, the buccal swab DNA is used as a source of normal DNA in circulating heme malignancies.

*Block 528.* Referring to block 528, in some embodiments, the identifying of block 525 and/or 526 further includes excluding one or more respective somatic mutations, in the somatic mutations identified using the first plurality of sequence reads, that are determined to have outlying variant allele fractions in the first plurality of sequences.

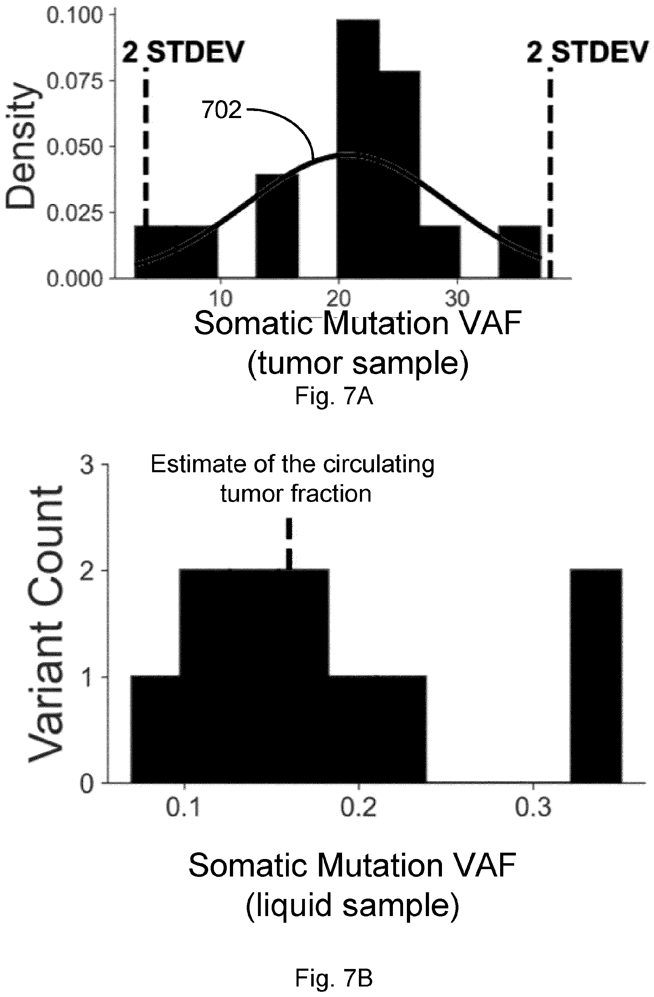

For instance, in some embodiments, somatic mutations with very high or very low VAFs, relative to the VAFs for the majority of identified somatic mutations, are excluded from the analysis, as they are likely not representative of the true circulating tumor fraction. For example, somatic variants having VAFs that are considerably lower than the VAFs for the majority of somatic mutations may arise from a minor sub-clonal cell population in a tumor. Figure 7A illustrates. As further described in block 530 below and in Example 1, the identified somatic variants are fitted to a distribution (a normal distribution in the case of Figure 7A) based on their respective VAF values in the first plurality of sequence reads. Then, those variants whose VAF values fall more than two standard deviations from the mean of this normal distribution are excluded from the somatic mutations that are analyzed using the liquid biopsy data below in order to calculate circulating tumor fraction.

*Block 530.* Referring to block 530, in some embodiments, the excluding includes fitting VAFs for each respective somatic mutation in the somatic mutations obtained using in the first plurality of sequences (after removing germ line mutations) to a distribution and excluding candidate somatic mutations with corresponding VAFs outside of a measure of dispersion for the distribution.

In some embodiments, the quality of this distribution fitting is evaluated using a distribution quality methodology such as a Kolmogorov-Smirnov goodness-of-fit test, a chi-square test, an Anderson-Darling test, a Shapiro-Wilk test, a Lilliefors test, a Cramér-von Mises test, a Jarque-Bera test, a Kuiper V test, or a Watson U test. See Jantschi and Bolboaca, 2017, "Performance of Shannon's Entropy Statistic in Assessment of Distribution Data," and the references cited therein, which is hereby incorporated by reference, for disclosure on the Anderson-Darling, Kolmogorov-Smirnov, Cramér-von Mises, Kuiper V, and Watson U goodness-of-fit tests. See also Huber-Carol *et al.,* (ed.), March 8, 2002, "Goodness-of-Fit Test and Model Validity (Statistics for Industry and Technology), Birkhauser, ISBN-10 0817642099, which is hereby incorporated by reference.

In some embodiments the quality of the fitting must have a p-value greater than a threshold amount in order to use the distribution to remove outliers from the mutations that are considered in the analysis of the somatic mutations from the liquid biopsy in block 539. In some embodiments, the threshold amount is 0.05 as was the case in Example 1. In other words, when the distribution quality methodology evaluates that the goodness of fit to the distribution exceeds $p = 0.05$, the distribution can be used to identify outliers to the distribution (e.g., more than two standard deviations from the mean of the distribution) to eliminate from the somatic mutations that are used in block 539. When the distribution quality methodology evaluates that the goodness of fit to the distribution is less than $p = 0.05$, the distribution is not used to identify outliers to the distribution on the basis that VAFs of the somatic mutations from the solid tumor sample from the test subject do not map well to a distribution. In some embodiments, the threshold amount is 0.10, meaning that the distribution quality methodology must find that the VAFs of the mutations from the solid tumor sample from the test subject fit a distribution with a p-value of at least 0.10. In some embodiments, the threshold value is 0.15, 0.20, 0.25, 0.50 or greater.

*Blocks 532-534.* Referring to block 532, in some embodiments, the VAF values of the somatic mutations from the genomic DNA from the solid tumor sample from the test subject are fitted to a distribution such as a normal distribution, a beta distribution, a beta prime distribution, a log normal distribution, or a gamma distribution. Referring to block 534, in some embodiments, the VAF values of the somatic mutations from the genomic DNA from the solid tumor sample from the test subject are fitted to a normal distribution.

In some embodiments, the type of distribution is predetermined. For instance, in Example 1, it was determined that the normal distribution fits most of the subjects analyzed in that example the best. Accordingly, in some embodiments the normal distribution is always used. In some embodiments, several different types of distributions are evaluated and the distribution that has the best goodness of fit is selected. In some embodiments, any of the distribution types disclosed herein is used.

In some embodiments the VAF values of the somatic mutations is fitted to the distribution using maximum likelihood estimation, in which the parameters of the distribution that maximize the likelihood of the observed data (the VAF values of the somatic mutations) under the assumed distribution (e.g., the normal distributions).

In some embodiments the VAF values of the somatic mutations is fitted to the distribution using a method of moments algorithm, in which the sample moments (e.g., mean, variance) are matched to theoretical moments in the chosen distribution.

In some embodiments the VAF values of the somatic mutations is fitted to the distribution using Bayesian inference. Bayesian inference provides a probabilistic framework for estimating the parameters of a distribution by treating the VAF values of the somatic mutations as random variables. Instead of finding a single point estimate for the parameters (like in MLE), Bayesian methods generate a distribution of possible parameter values (the posterior distribution) given the observed data and prior knowledge (prior distribution).

In some embodiments the VAF values of the somatic mutations is fitted to the distribution using a least squares method, in which the sum of the squared differences between the observed VAF values and the values predicted by the chosen distribution are minimized. *See,* for example, Levie, 2000, "Curve Fitting with Least Squares. Critical Reviews in Analytical Chemistry," 30(1), 59-74, which is hereby incorporated by reference.

In some embodiments the VAF values of the somatic mutations is fitted to the distribution using a quantile matching algorithm (e.g., by matching the quantiles of the observed VAF values with the quantiles of the theoretical distribution, in which the sum of the squared differences between the observed VAF values and the values predicted by the theoretical distribution (e.g., normal distribution) are minimized. *See,* Sgouropoulos et al., 2015, "Matching a Distribution by Matching Quantiles Estimation," Journal of the American Statistical Association, 110(510), pp. 742-759, which is hereby incorporated by reference.

In some embodiments the VAF values of the somatic mutations is fitted to the distribution using an expectation-maximization (EM) algorithm. The EM algorithm is an iterative method for finding maximum likelihood estimates in the presence of latent (unobserved) variables. The EM algorithm alternates between two steps: expectation (E-step) in which the expected value of the latent variables given the current parameter estimates is estimated, and. maximization (M-step) in which the likelihood function with respect to the parameters based on the estimated latent variables is maximized. *See* Koch, 2013, "Robust estimation by expectation maximization algorithm," Journal of Geodesy 87, pp. 107-116, which is hereby incorporated by reference.

In some embodiments the VAF values of the somatic mutations is fitted to the distribution using a nonparametric method such as kernel density estimation. Non-parametric methods do not assume a specific form for the distribution of the VAF values of the somatic mutations. One example is Kernel Density Estimation (KDE), which is used to estimate the probability density function (PDF) of the VAF values of the somatic mutations directly from the observed VAF values, without assuming an underlying parametric distribution. *See* Chen et al., 2017, "A tutorial on kernel density estimation and recent advances," Biostatistics & Epidemiology, 1(1), 161-187, which is hereby incorporated by reference.

*Block 536.* Referring to block 536, in some embodiments, the measure of dispersion is a multiple of a standard deviation for the probability distribution. In some embodiments, the threshold for excluding a somatic variant is a VAF that is more than a threshold number of standard deviations from a measure of central tendency (e.g., a mean or median) for the set of VAFs for each respective candidate somatic mutation in the plurality of candidate somatic mutations in the first plurality of sequences (in the solid tumor sequencing reaction). In some embodiments, the threshold number of standard deviations is at least 2 standard deviations. In some embodiments, the threshold number of standard deviations is at least 0.5, at least 0.75, at least 1, at least 1.25, at least 1.5, at least 1.75, at least 2, at least 2.5, at least 3, at least 4, or more standard deviations from the measure of central tendency (e.g., a mean or median) for the set of VAFs for each respective candidate somatic mutation in the plurality of candidate somatic mutations in the first plurality of sequences. In some embodiments, the threshold number of standard deviations is no more than 4 standard deviations from the measure of central tendency (e.g., a mean or median) for the set of VAFs for each respective candidate somatic mutation in the plurality of candidate somatic mutations in the first plurality of sequences. In some embodiments, the threshold number of standard deviations is no more than 5, no more than 4, no more than 3, no more than 2.5, no more than 2, no more than 1.75, no more than 1.5, no more than 1.25, no more than 1, or fewer standard deviations from the measure of central tendency (e.g., a mean or median) for the set of VAFs for each from the measure of central tendency for the set of VAFs for each respective candidate somatic mutation in the plurality of candidate somatic mutations in the first plurality of sequences.

In some embodiments, the measure of dispersion is a multiple of a standard deviation about a measure of central tendency (e.g., mean, median, average, etc.) of the distribution.

In some embodiments, the measure of dispersion is a multiple of a mean absolute deviation (MAD) about a measure of central tendency of the distribution.

In some embodiments, the measure of dispersion is an interquartile range (IQR), range, a coefficient of variation (CV) range, a skewness range, a kurtosis range, or a Gini Index within the distribution (*e.g.*, centered on a measure of central tendency of the distribution).

*Block 538.* Referring to block 538, in some embodiments, the identifying of block 524 further includes excluding one or more respective candidate somatic mutations in the plurality of candidate somatic mutations having a nucleotide position that does not correspond to any probe in the first plurality of probes. That is, in embodiments where different panel-enriched sequencing reactions are used to sequence the solid tumor biopsy genomic DNA and liquid biopsy cfDNA, somatic variants identified from the solid tumor biopsy that do not fall within a region targeted by the enrichment panel for the liquid biopsy sequencing reaction are excluded from the analysis because the loci are not enriched in the liquid biopsy sequencing reaction.

*Block 539.* Referring to block 539, in some embodiments, a determination of the corresponding variant allele frequency (VAF) in the liquid biopsy sample is made for each respective somatic mutation in the one or more somatic mutations, where the corresponding VAF is determined from the frequency of the respective somatic variant in the second plurality of nucleic acid sequences at the corresponding one or more nucleotide positions for the respective somatic variant in the second plurality of nucleic acid sequences, thereby determining a set of VAFs for the one or more somatic mutations in the liquid biopsy sample. In some embodiments, the one or more somatic mutations is at least one somatic mutation.

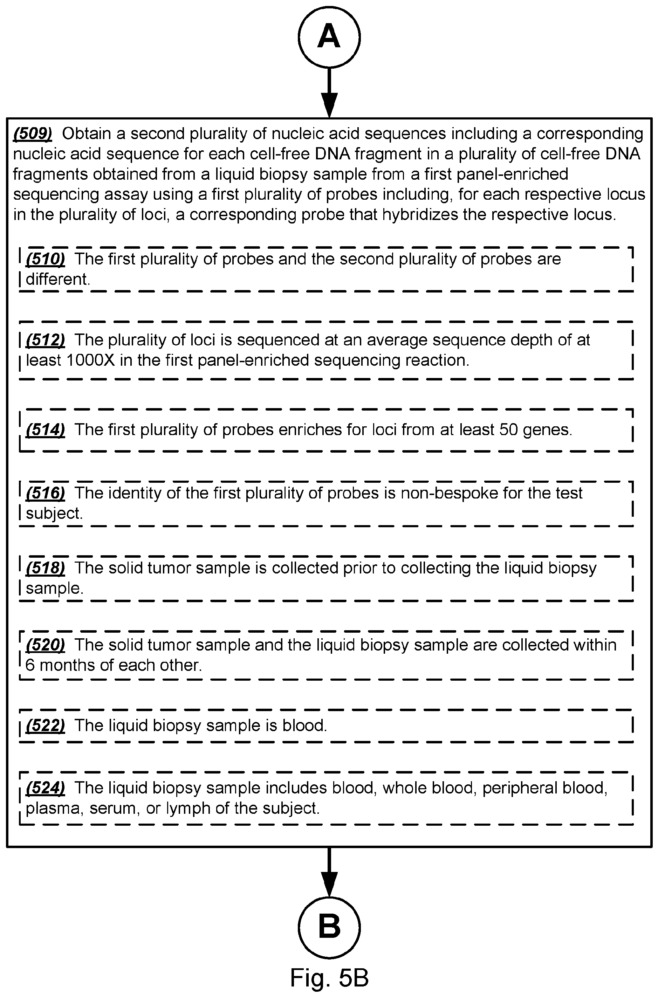

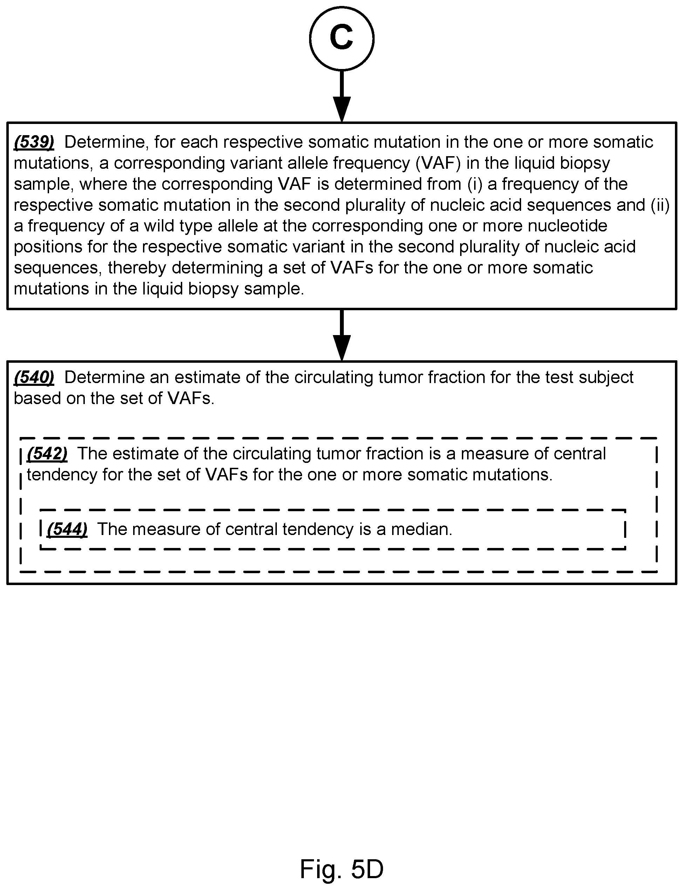

In some embodiments, a set of VAFs is formed that comprises the respective VAF of each respective somatic mutation in the one or more somatic mutations (identified in accordance with the previous blocks of Figure 5 described above) having a measurable variant allele frequency (VAF) in the liquid biopsy sample, as determined from a frequency of the respective somatic mutation in the second plurality of nucleic acid sequences. In some embodiments the solid assay is used to identified the set of somatic mutations in accordance with block 525 and block 539 serves to determine the variant allele frequency of each of these somatic mutations in the corresponding liquid biopsy using the second plurality of nucleic acid sequences. As such, in some embodiments there is no minimum VAF for each respective somatic mutation identified in accordance with block 539 for the set of VAFs and using the second plurality of sequence reads. In some alternative embodiments, each respective somatic mutation identified in accordance with block 539 for the set of VAFs and using the second plurality of sequence reads has a variant allele frequency for the respective somatic mutation of at least 0.1 percent. In some embodiments, each respective somatic mutation identified in accordance with block 539 for the set of VAFs and using the second plurality of sequence reads has a variant allele frequency for the respective somatic mutation of at least 5 percent.

In some embodiments, the one or more somatic mutations is at least 2, at least 3, at least 4, at least 5, at least 6, at least 7, at least 8, at least 9, at least 10, at least 11, at least 12, at least 13, at least 14, at least 15, at least 20, at least 25, or more somatic mutations. In some embodiments, the one or more somatic mutations is no more than 5000 somatic variants, no more than 2500 somatic variants, no more than 1000 somatic variants, no more than 500 somatic variants, no more than 250 somatic variants, or were somatic variants. In some embodiments, the one or more somatic variants is between 1 and 5000 somatic variants. In some embodiments, the one or more somatic variants is between 1 and 2500, between 1 and 1000, between 1 and 500, between 1 and 250, between 2 and 5000, between 2 and 2500, between 2 and 1000, between 2 and 500, between 2 and 250, between 5 and 5000, between 5 and 2500, between 5 and 1000, between 5 and 500, or between 5 and 250 somatic mutations.

*Block 540.* Referring to block 540, in some embodiments, an estimate of the circulating tumor fraction for the test subject is determined based on the set of VAFs. Advantageously, this set is tumor informed in that each of the somatic mutations contributing to the set was also found in the tumor sample. Advantageously, this set is also filtered by the distribution of the VAFs of the somatic mutations in the liquid biopsy to exclude outliers. In some embodiments, after such filtering, the one or more somatic mutations contributing to the set of FAFs is 2, 3, 4, 5, 6, 7, 8, 9, 10, 11, 12, 13, 14, 15, 16, 17, 18, 19, 20, 21, 22, 23, 24, 25, 26, 27, 28, 29, or 30 or more somatic mutations. In some embodiments, after such filtering, the one or more somatic mutations contributing to the set of VAFs is a plurality of somatic mutations.

*Block 542-544.* Referring to block 542, in some embodiments, the estimate of the circulating tumor fraction is a measure of central tendency for the set of VAFs.

For example, in some embodiments, the ctFE is an arithmetic mean, weighted mean, midrange, midhinge, trimean, geometric mean, geometric median, Winsorized mean, median, or a mode of VAFs for the plurality of somatic mutations. Referring to block 544, in some embodiments, the measure of central tendency is a median.

In some embodiments, the one or more somatic mutations is a single somatic mutation and the ctFE is the VAF for the single somatic mutation.

*Mean VAF method.* In some embodiments the estimate of the circulating tumor fraction is determined using a mean VAF method. In some embodiments, the mean VAF method computes:

$$\text{Mean VAF} = \frac{\sum_{i=1}^{n} \text{VAF}_i}{n}$$

where, n is the number of mutations and $\text{VAF}_i$ is the VAF for the $i^{th}$ mutation. Then, in some embodiments, to compute the estimate of the circulating tumor fraction, the mean VAF is divided by the tumor allele frequence ($f_{tumor}$). For example, consider the case in which there are five somatic mutations, M1, M2, M3, M4, and M5 in the set of FAVs with respective VAF values of 0.1, 0.15, 0.12, 0.18, and 02.0. The mean VAF would be calculated as:

$$\text{Mean VAF} = \frac{0.1 + 0.15 + 0.12 + 0.18 + 0.2}{5} = \frac{0.75}{5} = 0.15$$

Then, assuming that the somatic mutations are heterozygous in the tumor, $f_{tumor} = 0.5$ (the allele frequency in the tumor), the estimate for CFT would be 0.15 / 0.5 = 0.3, so the estimated CTF would be 30 percent.

*VAF-based CFT estimation.* In some embodiments the estimate of the circulating tumor fraction is determined using the VAF-based method. The VAF-based method for estimating the circulating tumor fraction (CTF) utilizes the VAF (the proportion of sequencing reads that carry a mutation in a given genomic region) of each of the somatic mutations contributing to set of VAFs. The method assumes that the VAF for a somatic mutation observed in the cfDNA liquid biopsy sample is a function of both the tumor DNA fraction (CTF) and the mutation allele frequency in the tumor. In accordance with VAF-based CFT estimation, the formula that relates the VAF of a mutation in cfDNA to the CTF is:

$$\text{VAF} = \frac{2 \cdot \text{CTF} \cdot f_{tumor}}{(1 - \text{CTF}) + 2 \cdot \text{CTF} \cdot f_{tumor}}$$

where, VAF is the variant allele fraction of a given mutation in cfDNA, CTF is the circulating tumor fraction (the fraction of the cfDNA that is derived from the tumor), $f_{tumor}$ is typically 0.5, and for homozygous mutations, it is 1.0, and the factor of 2 in the numerator accounts for the fact that the heterozygous mutations, you can have both mutant and normal alleles contributing to the VAF. To estimate for CTF, the formula can be rearranged to solve for CTF:

$$\text{CTF} = \frac{\text{VAF} \cdot (1 - f_{tumor})}{2 \cdot \text{VAF} \cdot f_{tumor} + (1 - f_{tumor})}$$

Thus, for a heterozygous somatic mutation with VAF = 0.1, $f_{tumor} = 0.5$ (since the mutation is heterozygous in the tumor). Using the formula to estimate CTF:

$$\text{CTF} = \frac{0.1 \cdot (1 - 0.5)}{2 \cdot 0.1 \cdot 0.5 + (1 - 0.5)}$$

$$\text{CTF} = \frac{0.1 \cdot (1 - 0.5)}{2 \cdot 0.1 \cdot 0.5 + (1 - 0.5)}$$

In the case where there is more than one candidate somatic mutation contributing to the set of VAFs, an estimate of the CTF for each somatic mutation contributing to the set of VAFs can be computed individually, and then an average of all the estimates can be taken to realize the final estimate of the CTF across all the somatic mutations.

In some embodiments, the obtained circulating tumor fraction estimate is used for further downstream analysis and biomarker detection (*e.g.*, calculation of variant allele fractions, variant calling, and/or identification of other metrics). In some embodiments, the obtained circulating tumor fraction estimate is used as a metric for disease detection, diagnosis, and/or treatment. In some embodiments, the obtained circulating tumor fraction estimate is included in a clinical report made available to the patient or a clinician. In some embodiments, the obtained circulating tumor fraction estimate is used to select appropriate therapies and/or clinical trials for assessment of treatment response.

Accordingly, in some embodiments, the method also includes generating a report for the test subject (*e.g.,* for use by a physician) including the circulating tumor fraction for the test subject. In some embodiments, the report further includes matched therapies (*e.g.,* treatments and/or clinical trials) for the test subject based on the reported circulating tumor fraction for the test subject.

In some embodiments, the methods described herein include generating a clinical report 139-3 (*e.g.,* a patient report), providing clinical support for personalized cancer therapy, and/or using the information curated from sequencing of a liquid biopsy sample, as described above. In some embodiments, the report is provided to a patient, physician, medical personnel, or researcher in a digital copy (for example, a JSON object, a pdf file, or an image on a website or portal), a hard copy (for example, printed on paper or another tangible medium). A report object, such as a JSON object, can be used for further processing and/or display. For example, information from the report object can be used to prepare a clinical laboratory report for return to an ordering physician. In some embodiments, the report is presented as text, as audio (for example, recorded or streaming), as images, or in another format and/or any combination thereof.

In some embodiments, the report includes information related to the specific characteristics of the patient's cancer, *e.g.,* detected genetic variants, epigenetic abnormalities, associated oncogenic pathogenic infections, and/or pathology abnormalities. In some embodiments, other characteristics of a patient's sample and/or clinical records are also included in the report. For example, in some embodiments, the clinical report includes information on clinical variants, *e.g.,* one or more of copy number variants (*e.g.,* for actionable genes CCNE1, CD274(PD-L1), EGFR, ERBB2(HER2), MET, MYC, BRCA1, and/or BRCA2), fusions, translocations, and/or rearrangements (*e.g.,* in actionable genes ALK, ROS1, RET, NTRK1, FGFR2, FGFR3, NTRK2 and/or NTRK3), pathogenic single nucleotide polymorphisms, insertion-deletions (*e.g.,* somatic/tumor and/or germline/normal), therapy biomarkers, microsatellite instability status, and/or tumor mutational burden.

In some embodiments, the results are used to design cell-based studies of the patient's biology, *e.g.,* tumor organoid experiments. For example, an organoid may be genetically engineered to have the same characteristics as the specimen and may be observed after exposure to a therapy to determine whether the therapy can reduce the growth rate of the organoid, and thus may be likely to reduce the growth rate of cancer in the patient associated with the specimen. Similarly, in some embodiments, the results are used to direct studies on tumor organoids derived directly from the patient. An example of such experimentation is described in U.S. Provisional Patent Application No. 62/944,292, filed December 5, 2019, the content of which is hereby incorporated by reference, in its entirety, for all purposes.

As illustrated in Figure 2A, in some embodiments, a clinical report is checked for final validation, review, and sign-off by a medical practitioner (*e.g.,* a pathologist). The clinical report is then sent for action (*e.g.,* for precision oncology applications).

*Longitudinal Reporting.* In various embodiments, the report includes and/or compares the results of multiple liquid biopsy tests and/or solid tumor tests (for example, multiple tests associated with the same patient). In some embodiments, the results of multiple liquid biopsy tests and/or solid tumor tests is displayed on a portal in a variety of user selectable or customizable configurations. In some embodiments the tests are performed at different times, and the samples on which the tests are performed are collected at different times. In some embodiments the tests are performed at the same time, and the samples on which the tests are performed are collected at the same time.

*Download result.* In some embodiments, clinical and/or molecular data associated with a patient (for example, information that would be included in the report), is aggregated and made available via the portal. In some embodiments any portion of the report data is available for download (for example, as a .CSV, a PDF file or a WORD File) by the physician and/or patient. In various embodiments, the data includes data related to genetic variants, RNA expression levels, immunotherapy markers (including MSI and TMB), RNA fusions, *etc.* In one embodiment, if a physician or medical facility orders multiple tests (all tests may be associated with the same patient or tests may be associated with multiple patients), and results associated with more than one test are aggregated into a single file for downloading.

*Longitudinal Testing.* In some embodiments, one or more liquid biopsy assays described herein are used to analyze specimens from a patient taken over the course of the patient's treatment. For example, a blood specimen may be obtained periodically and/or upon indication of response to therapy, disease relapse, and/or disease progression. In some embodiments, the one or more liquid biopsy assays may be used on a liquid specimen collected from the patient each month, every two months, every three months, every four months, every five months, every 6-12 months, and so forth. In such embodiments, each successive analysis is in accordance with block 509 and the same first plurality of nucleic acid sequences is used to filter which mutations are used in accordance with the methods of any of blocks 524-538 described above. In some embodiments, the longitudinal use of liquid biopsy assays is used to track clonal evolution to identify resistance mutations. In some embodiments, the longitudinal use of liquid biopsy assays is used to track evolution of mutations, such as EGFR or APC mutations.

In some embodiments, longitudinal use of liquid biopsy assays is used to detect emerging therapy resistance mechanisms. In some embodiments, longitudinal use of liquid biopsy assays is used to detect AR gene alterations. In some embodiments, longitudinal use of liquid biopsy assays is used to detect WNT pathway alterations in mCRPC associated with resistance to enzalutimide and abiraterone. In some embodiments, longitudinal use of liquid biopsy assays is used to detect ER mutations, such as ER mutations associated with resistance to endocrine therapy in breast cancer. In some embodiments, longitudinal use of liquid biopsy assays is used to detect EGFR mutations responsible for anti-EGFR therapy resistance (*e.g.*, T790M) in NSCLC. In some embodiments, longitudinal use of liquid biopsy assays may is to detect KRAS, NRAS, MET, ERBB2, FLT3, or EGFR mutations associated with primary or acquired resistance to EGFR inhibitors in colorectal cancer. In some embodiments, longitudinal use of liquid biopsy assays is used to assess gene alterations from tumor cells shed by primary tumor and metastatic sites.

In some embodiments the one or more blood specimens is collected from the patient in a home-based environment. In one example, the blood specimens are collected by a mobile phlebotomist. In another example, a first blood specimen, a second blood specimen, and a third blood specimen are collected from a patient during the course of treatment.

*Variant Characterization.* In some embodiments, a predicted functional effect and/or clinical interpretation for one or more identified variants is curated by using information from variant databases. In some embodiments, a weighted-heuristic model is used to characterize each variant.

In some embodiments, identified clinical variants are labeled as "potentially actionable", "biologically relevant", "variants of unknown significance (VUSs)", or "benign". Potentially actionable alterations are protein-altering variants with an associated therapy based on evidence from the medical literature. Biologically relevant alterations are protein-altering variants that may have functional significance or have been observed in the medical literature but are not associated with a specific therapy. Variants of unknown significance (VUSs) are protein-altering variants exhibiting an unclear effect on function and/or without sufficient evidence to determine their pathogenicity. In some embodiments, benign variants are not reported. In some embodiments, variants are identified through aligning the patient's DNA sequence to the human genome reference sequence version hg19 (GRCh37). In some embodiments, actionable and biologically relevant somatic variants are provided in a clinical summary during report generation.

For instance, in some embodiments, variant classification and reporting is performed, where detected variants are investigated following criteria from known evolutionary models, functional data, clinical data, literature, and other research endeavors, including tumor organoid experiments. In some embodiments, variants are prioritized and classified based on known gene-disease relationships, hotspot regions within genes, internal and external somatic databases, primary literature, and other features of somatic drivers. Variants can be added to a patient (or sample, for example, organoid sample) report based on recommendations from the AMP/ASCO/CAP guidelines. Additional guidelines may be followed. Briefly, pathogenic variants with therapeutic, diagnostic, or prognostic significance may be prioritized in the report. Non-actionable pathogenic variants may be included as biologically relevant, followed by variants of uncertain significance. Translocations may be reported based on features of known gene fusions, relevant breakpoints, and biological relevance. Evidence may be curated from public and private databases or research and presented as 1) consensus guidelines 2) clinical research, or 3) case studies, with a link to the supporting literature. Germline alterations may be reported as secondary findings in a subset of genes for consenting patients. These may include genes recommended by the ACMG and additional genes associated with cancer predisposition or drug resistance.

It should be understood that the examples given above are illustrative and do not limit the uses of the systems and methods described herein in combination with a digital and laboratory health care platform.

The results of the bioinformatics pipeline may be provided for report generation. Report generation may comprise variant science analysis, including the interpretation of variants (including somatic and germline variants as applicable) for pathogenic and biological significance. The variant science analysis may also estimate microsatellite instability (MSI) or tumor mutational burden. Targeted treatments may be identified based on gene, variant, and cancer type, for further consideration and review by the ordering physician. In some aspects, clinical trials may be identified for which the patient may be eligible, based on mutations, cancer type, and/or clinical history. Subsequent validation may occur, after which the report may be finalized for sign-out and delivery. In some embodiments, a first or second report may include additional data provided through a clinical dataflow 202, such as patient progress notes, pathology reports, imaging reports, and other relevant documents. Such clinical data is ingested, reviewed, and abstracted based on a predefined set of curation rules. The clinical data is then populated into the patient's clinical history timeline for report generation.

Further details on clinical report generation are disclosed in US Patent Application No. 16/789,363 (PCT/US20/180002), filed February 12, 2020, which is hereby incorporated herein by reference in its entirety.

In some embodiments, a clinical report 139-3 includes information about clinical trials for which the patient is eligible, therapies that are specific to the patient's cancer, and/or possible therapeutic adverse effects associated with the specific characteristics of the patient's cancer, *e.g.,* the patient's genetic variations, epigenetic abnormalities, associated oncogenic pathogenic infections, and/or pathology abnormalities, or other characteristics of the patient's sample and/or clinical records. For example, in some embodiments, the clinical report includes such patient information and analysis metrics, including cancer type and/or diagnosis, variant allele fraction, patient demographic and/or institution, matched therapies (*e.g.*, FDA approved and/or investigational), matched clinical trials, variants of unknown significance (VUS), genes with low coverage, panel information, specimen information, details on reported variants, patient clinical history, status and/or availability of previous test results, and/or version of bioinformatics pipeline.

In some embodiments, the results included in the report, and/or any additional results (for example, from the bioinformatics pipeline), are used to query a database of clinical data, for example, to determine whether there is a trend showing that a particular therapy was effective or ineffective in treating (*e.g.*, slowing or halting cancer progression), and/or adverse effects of such treatments in other patients having the same or similar characteristics.

In some embodiments, the results are used to design cell-based studies of the patient's biology, *e.g.*, tumor organoid experiments. For example, an organoid may be genetically engineered to have the same characteristics as the specimen and may be observed after exposure to a therapy to determine whether the therapy can reduce the growth rate of the organoid, and thus may be likely to reduce the growth rate of cancer in the patient associated with the specimen. Similarly, in some embodiments, the results are used to direct studies on tumor organoids derived directly from the patient. An example of such experimentation is described in U.S. Patent No. 11,415,571, the content of which is hereby incorporated by reference, in its entirety, for all purposes.

As illustrated in Figure 2A, in some embodiments, a clinical report is checked for final validation, review, and sign-off by a medical practitioner (*e.g.*, a pathologist). The clinical report is then sent for action (*e.g.*, for precision oncology applications).

*Stand-alone Device Integration.* Hardware devices incorporating one or more embodiments as described herein may be implemented. In one example, a hardware device may record progress notes or other documents, automatically converting recorded audio into features and storing them in a structured format with respect to a patient. In another example, a hardware device may broadcast a response containing one or more analytical results, patient features, or reports as described in any of the embodiments above. For more information see, for example, PCT Publication No. WO 2021/168146, the disclosure of which is incorporated herein by reference, in its entirety, for all purposes.

*Specific Embodiments of the Disclosure.* In some aspects, the systems and methods disclosed herein may be used to support clinical decisions for personalized treatment of cancer. For example, in some embodiments, the methods described herein identify actionable genomic variants and/or genomic states with associated recommended cancer therapies. In some embodiments, the recommended treatment is dependent upon whether or not the subject has a particular actionable variant and/or genomic status. Recommended treatment modalities can be therapeutic drugs and/or assignment to one or more clinical trials. Generally, current treatment guidelines for various cancers are maintained by various organizations, including the National Cancer Institute and Merck & Co., in the Merck Manual.

In some embodiments, the methods described herein further includes assigning therapy and/or administering therapy to the subject based on the identification of an actionable genomic variant and/or genomic state, *e.g.*, based on whether or not the subject's cancer will be responsive to a particular personalized cancer therapy regimen. For example, in some embodiments, when the subject's cancer is classified as having a first actionable variant and/or genomic state, the subject is assigned or administered a first personalized cancer therapy that is associated with the first actionable variant and/or genomic state, and when the subject's cancer is classified as having a second actionable variant and/or genomic state, the subject is assigned or administered a second personalized cancer therapy that is associated with the second actionable variant. Assignment or administration of a therapy or a clinical trial to a subject is thus tailored for treatment of the actionable variants and/or genomic states of the cancer patient.

*Examples.*

*Example 1 - Development of a tissue-informed, comprehensive genomic profiling (CGP) and non-bespoke blood-based profiling for quantifying circulating tumor DNA (ctDNA).*

This example describes a novel combination of tissue-informed, comprehensive genomic profiling (CGP) and non-bespoke blood-based profiling for quantifying circulating tumor DNA (ctDNA) in a liquid biopsy assay.

In particular, this example describes a method of determining an estimate of a circulating tumor fraction for a test subject that was performed at a computer system having one or more processors, and memory storing one or more programs for execution by the one or more processors.

A first plurality of nucleic acid sequences comprising a corresponding nucleic acid sequence for each respective locus in a plurality of loci in genomic DNA from a solid tumor sample from each of a plurality of test subjects was obtained in accordance with block 502. In particular, the first plurality of nucleic acid sequences, for each of the test subjects, was determined from a second panel-enriched sequencing reaction using a second plurality of probes including, for each respective locus in the plurality of loci a corresponding probe, in a second plurality of probes, that hybridizes to the respective locus, in accordance with block 504.

A second plurality of nucleic acid sequences including a corresponding nucleic acid sequence for each cell-free DNA fragment in a plurality of cell-free DNA fragments obtained from a liquid biopsy sample from a first panel-enriched sequencing assay using a first plurality of probes including, for each respective locus in the plurality of loci, a corresponding probe that hybridizes the respective locus, was obtained from each subject in the plurality of test subjects in accordance with block 509.

In this example, the plurality of test subjects was 153 patients with advanced pan-cancer.

The first panel-enriched sequencing assay (for the liquid biopsy samples) targeted either the 105 human genes of Table 1 or the 523 human genes of Table 2.

The second panel-enriched sequencing assay (for the solid tumor samples) targeted 648 genes collectively listed in Figures 15A, 15B, 15C and published as Table 1 in Beaubier et al., 2019, "Clinical validation of the tempus xT next-generation targeted oncology sequencing assay," Oncotarget 10(24), pp. 2384-2396, which is hereby incorporated by reference. For the second panel-enriched sequencing assay both the solid tumor and matched buffy coat were analyzed.

In this example, the liquid biopsy samples and solid tumor samples from each individual were collected within 90 days of each other.

Data from each sequencing reaction was processed through the bioinformatics pipeline described herein.

Then, one or more somatic mutations, where each respective somatic mutation in the one or more somatic mutations is at a corresponding one or more nucleotide positions in a corresponding loci in the plurality of one or more loci, were identified.

This was done in this example by identifying a plurality of candidate somatic mutations through comparison of respective nucleic acid sequences in the first plurality of nucleic acid sequences (from the solid tumor sample) of each respective subject to nucleic acid sequences in a third plurality of nucleic acid sequences obtained from a sequencing reaction of genomic DNA from a non-cancerous tissue of the subject in accordance with block 526. In this example the non-cancerous tissue of the subject was buffy coat collected contemporaneously with the solid tumor sample. As noted in block 526, this comparison was done to remove from the plurality of candidate somatic mutations those mutations that were germline.

Next, for each test subject, the remaining candidate somatic mutations after filtering out germline mutations were evaluated. From the analysis above, the variant base fraction of each of the remaining candidate somatic mutations was determined from the proportion of sequence reads in the first plurality of nucleic acid sequences mapping to the genomic locus of the candidate somatic mutation versus all sequence reads in the first plurality of nucleic acid sequences mapping to the genomic locus of the candidate somatic mutation. For example, if 30 percent of the first plurality of nucleic acid sequences mapping to the genomic locus of the candidate somatic mutation have the allele of a particular somatic mutation while the remaining 70 percent of the first plurality of nucleic acid sequences mapping to the genomic locus of the particular candidate somatic mutation have the wild type allele, the variant base fraction for the particular variant base fraction is 30 percent. In this the first plurality of nucleic acid sequences have been de-duplicated, meaning that each sequence read represents a unique nucleic acid molecule in the tumor tissue.

For each respective subject, the variant base fraction values of each of the candidate somatic mutations was evaluated to see if it followed a normal distribution using a Kolmogorov-Smirnov goodness-of-fit test. In other words, the Kolmogorov-Smirnov (KS) test was used to assess how well the base fractions of the candidate somatic mutations for each subject fit a normal distribution. A high p-value (greater than 0.05 in this example) for a given subject was construed to mean that the base fractions for that subject do not significantly differ from a normal distribution, allowing the use of the normal model. If the p-value was low for a given subject, the base fractions for that subject were not considered as fitting a normal distribution were excluded from further analysis in this example. In this example, the Kolmogorov-Smirnov (KS) test was also used to assess how well the $\log_2$ transformed base fractions of the candidate somatic mutations for each subject fit a normal distribution. However, it was found that the $\log_2$ transform may transform subclonal or artifactual variants that could be excluded as outliers. Thus, the untransformed base fractions for each subject were used for the KS goodness-of-fit test.

In further detail, different distributions were then fit against the distribution of VAF for the somatic mutations detected in each solid tumor sample using the KS goodness-of-fit test. These distributions included normal, beta, beta prime, log normal, and gamma distributions. Both $\log_2$ transformed VAFs (used to alleviate left skew towards the variant limit of detection for the panel-enriched sequencing assay) and untransformed VAFs were fit against each distribution.

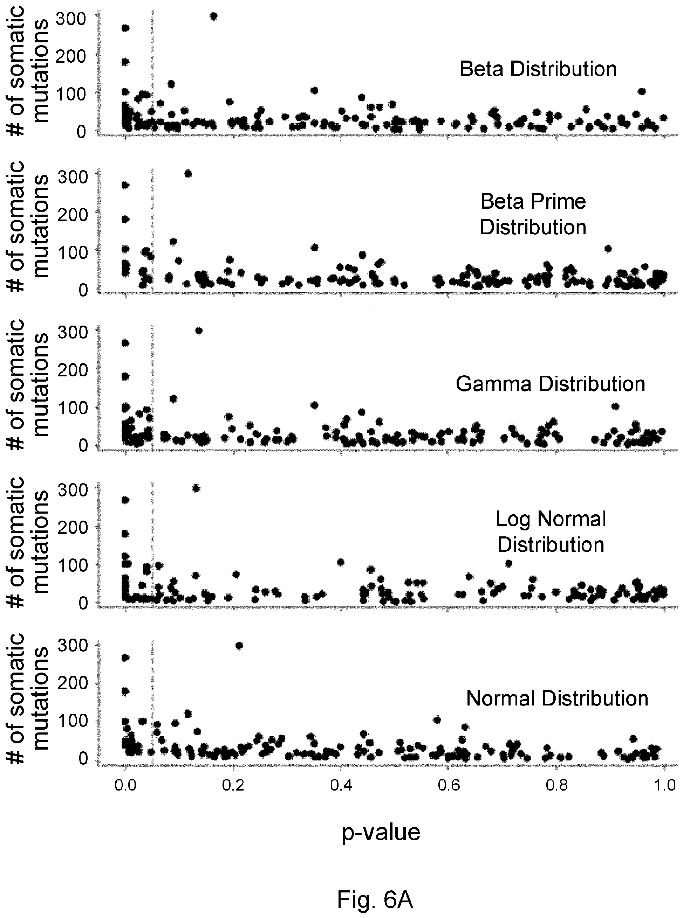

Samples and distributions where the KS p-value was < 0.05 were found to fail the hypothesis that the distribution adequately fit the solid tumor VAFs with 95% confidence. The distribution of KS values across the 153 samples for each of the distributions fit to the untransformed data are shown in Figure 6A, where each point represents a different subject in the set of 148 subjects, the x-axis represents the KS p-value and the y-axis represents the number of somatic mutations remain for a subject after filtering out possible germline mutations using the matched buffy coat samples.

The number of samples with KS p-values < 0.05, indicating that the goodness-of-fit test failed, were Beta: 41, BetaPrime: 20, Gamma: 43, lognorm: 54, and normal: 28. The normal: 28 shows that, without transforming the data, only 28 of the 158 solid tumor samples had variants that did not appear to be normally distributed when evaluated by the KS test. Figure 6A further shows that the number of somatic mutations for each subject, after removing germline mutations ranges from the single digits (less than 10) to on the order of 300 in this example.

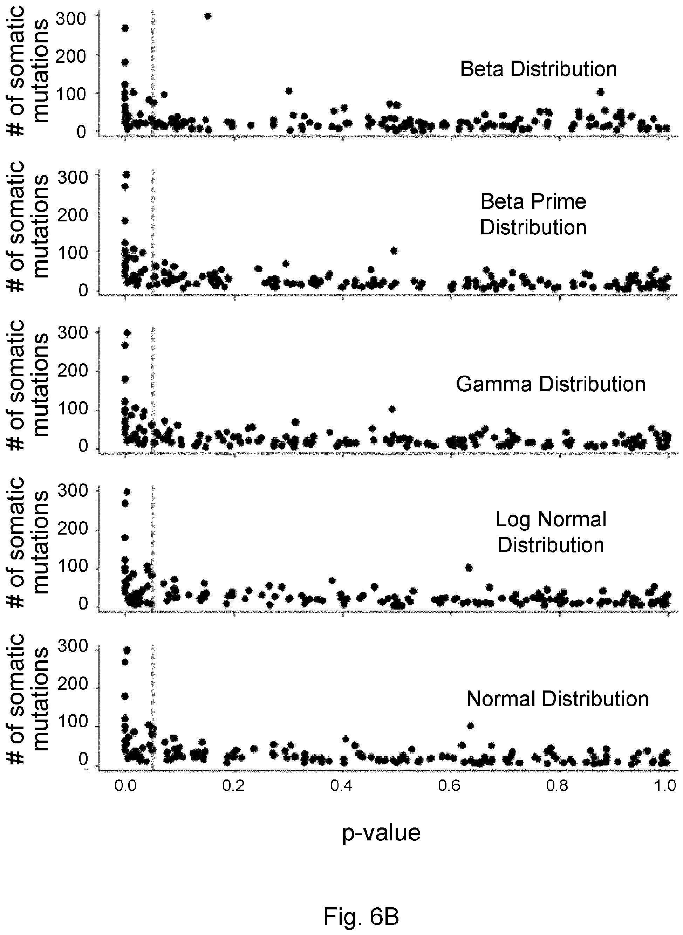

The distribution of KS values across the 153 samples for the log2 transformed VAFs of the sets of somatic mutations are shown in Figure 6B, where each point represents a different subject. The number of samples with KS p-values < 0.05 for this log2 transformed data were Beta: 41, BetaPrime: 36, Gamma: 39, lognorm: 43, and norm: 36.

As shown in Figures 6A and 6B, a normal distribution fitted to untransformed VAFs (Figure 6A, bottom chart) was more successful. Accordingly, this fit was selected for further analysis.

An example of a normal distribution 702 fit to solid tumor VAFs of the somatic mutations for a particular subject drawn from the 158 subjects is illustrated in Figure 7A. Figure 7A is a bar graph on which the x-axis is variant allele frequency, ranging from 0 to some maximal value, whereas the y-axis is the number of somatic mutations the subject has for any given variant allele frequency represented on the x-axis. For instance, if the subject had five somatic mutations each with a VAF of 10, they would be represented in Figure 7A as a bar on the x-axis, at 10, with the height of the bar being 5 divided by the total number somatic mutations identified in the cancer sample for the subject (density). A normal distribution is applied to this plot and those mutations that fall more than two standard deviations from the mean of the distribution are marked as outliers.

Identified somatic variants with VAFs falling outside of 2 standard deviations of the mean of the normal distribution were then excluded as outliers in accordance with block 536 of Figure 5C.

The VAF of the somatic mutations within two standard deviations of the mean of the normal distribution were then determined using the second plurality of nucleic acid sequences comprising a corresponding nucleic acid sequence for each cell-free DNA fragment in the plurality of cell-free DNA fragments obtained from the liquid biopsy sample of the subject in accordance with block 539 of Figure 5C. The VAF of these mutations identified from the solid tumor sequencing data, and which were also identified in the liquid biopsy panel-enriched sequencing assay, were then used to evaluate the ctDNA fraction in accordance with block 540. Specifically, for each subject, the median value of the VAFs for the identified candidate mutations, in the liquid biopsy panel-enriched sequencing assay, was determined to be the ctFE. An example of a distribution of VAFs for identified candidate mutations in a liquid biopsy assay, with the median value indicated (and thus representing the ctDNA fraction for the subject), is illustrated in Figure 7B.

*Example 2 - Validation of a tissue-informed, comprehensive genomic profiling (CGP) and non-bespoke blood-based profiling for quantifying circulating tumor DNA (ctDNA).*

This example describes validation of the tumor-informed ctDNA estimation assay described in Example 1.

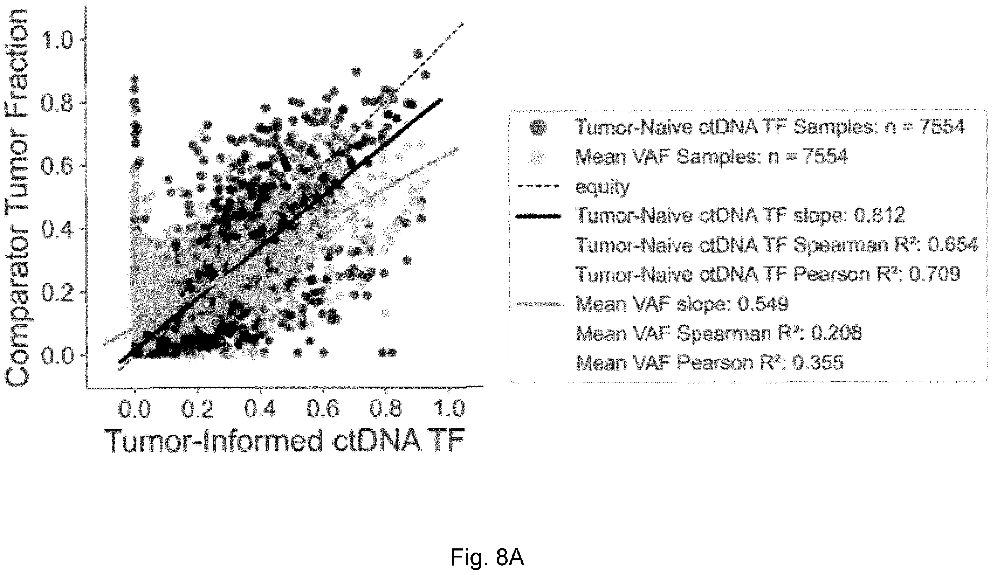

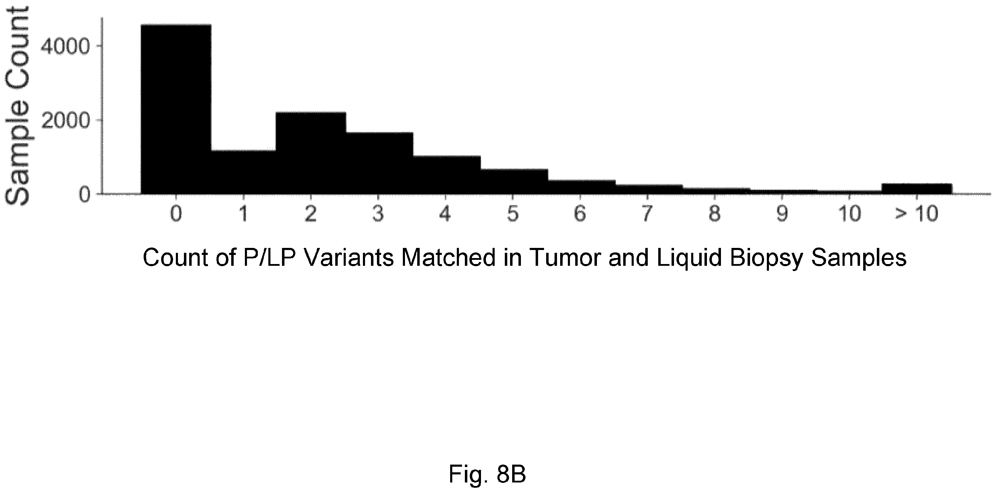

As a first evaluation, the assay described in Example 1 was performed on matching solid tumor and liquid biopsy samples from 7554 cancer subjects with at least one somatic mutation identified in both a solid tumor panel-enriched sequencing assay (648 genes) and a liquid biopsy panel-enriched sequencing assay (105 genes). The resulting ctFE was then compared to a tumor naive ctDNA estimate. By way of comparison, the mean VAF for all identified somatic mutations in the liquid biopsy was also compared to the conventional naive ctDNA estimate. As shown in Figure 8A, the tumor-informed model described in Example 1 better matched the conventional estimate for ctDNA fraction than did the mean VAF. The number of candidate mutations matching in solid tumor and liquid biopsy assays across 12,080 matching pairs is illustrated in Figure 8B. Note that samples lacking any matching somatic mutations were excluded from the evaluation.

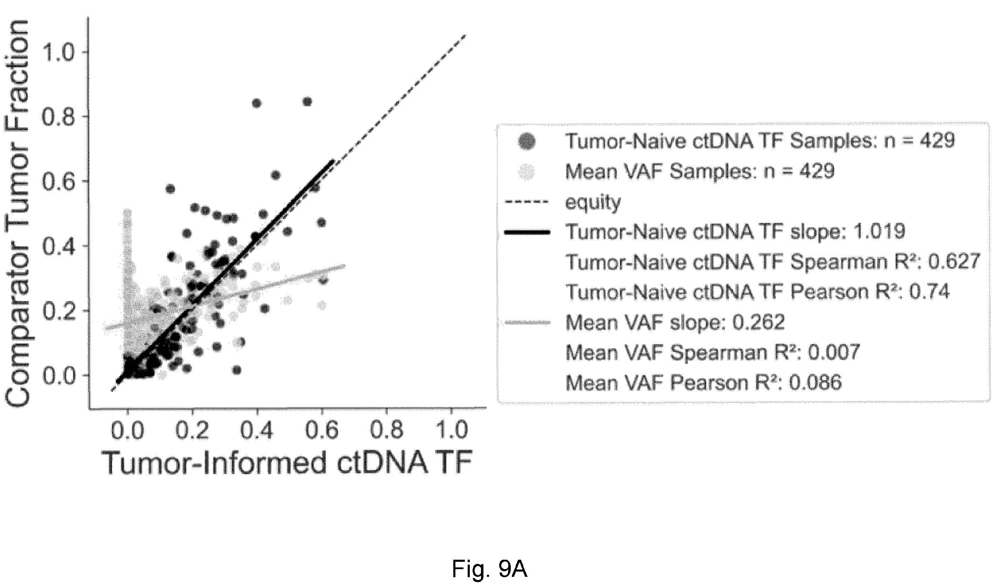

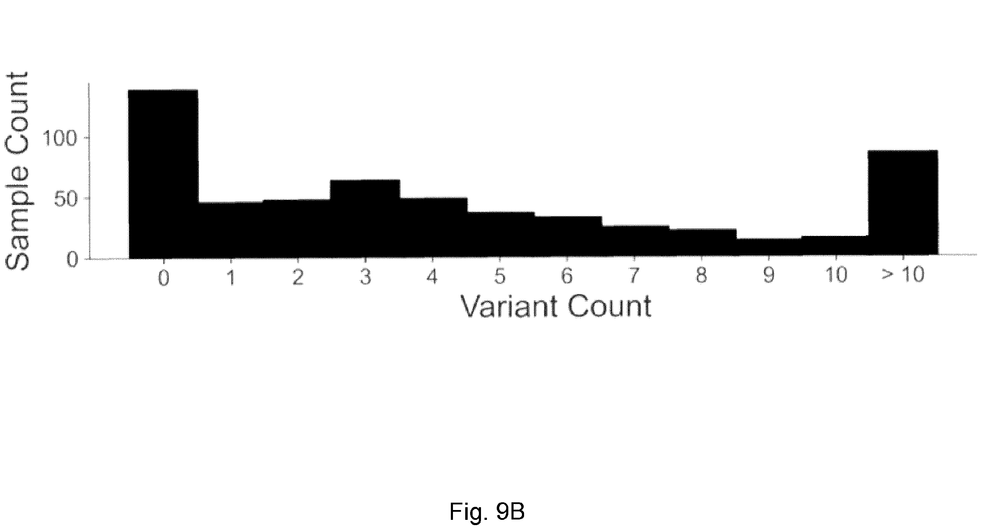

As a second evaluation, the assay described in Example 1 was performed on matching solid tumor and liquid biopsy samples from 429 cancer subjects with at least one somatic mutation identified in both a solid tumor panel-enriched sequencing assay (648 genes) and a liquid biopsy panel-enriched sequencing assay (523 genes). The resulting ctFE was then compared to a conventional tumor naïve ctDNA estimate. By way of comparison, the mean VAF for all identified somatic mutations in the liquid biopsy was also compared to the conventional naive ctDNA estimate. As shown in Figure 9A, the tumor-informed model described in Example 1 better matched the conventional estimate for ctDNA fraction than did the mean VAF. The number of somatic mutations matching in solid tumor and liquid biopsy assays across 567 matching pairs is illustrated in Figure 9B. Note that samples lacking any matching somatic mutations were excluded from the evaluation.

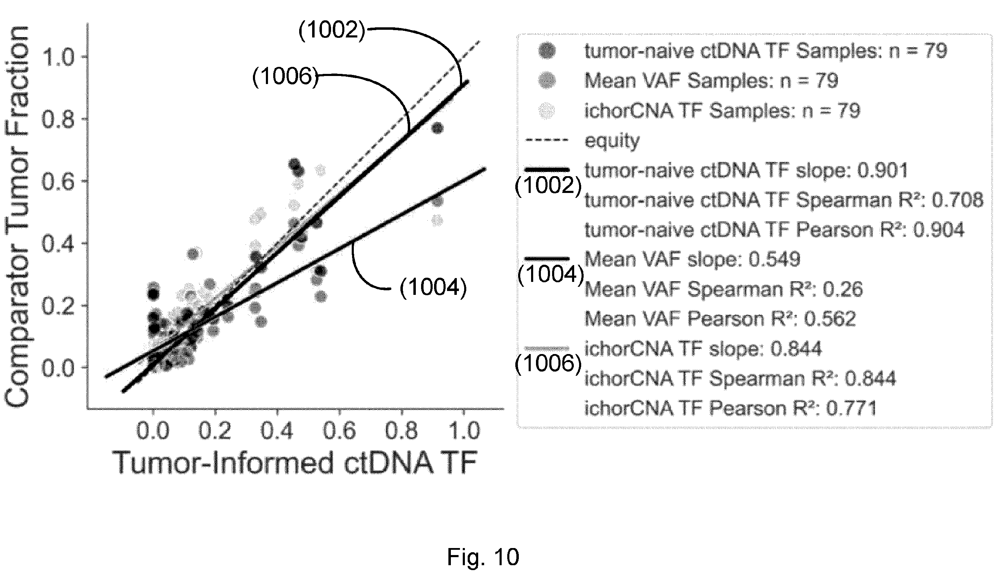

Next, the assay described in Example 1 was performed on matching solid tumor, liquid biopsy, and LPWGS samples from 79 cancer subjects with at least one somatic mutation identified in both a solid tumor panel-enriched sequencing assay (648 genes) and a liquid biopsy panel-enriched sequencing assay (105 or 523 genes). The resulting ctFE was compared to ctFE determined using ichorCNA and mean VAF for all identified somatic mutations in the liquid biopsy. As shown in Figure 10, the tumor-informed model described in Example 1 better matched the ichorCNA estimate for ctDNA fraction than did the mean VAF.

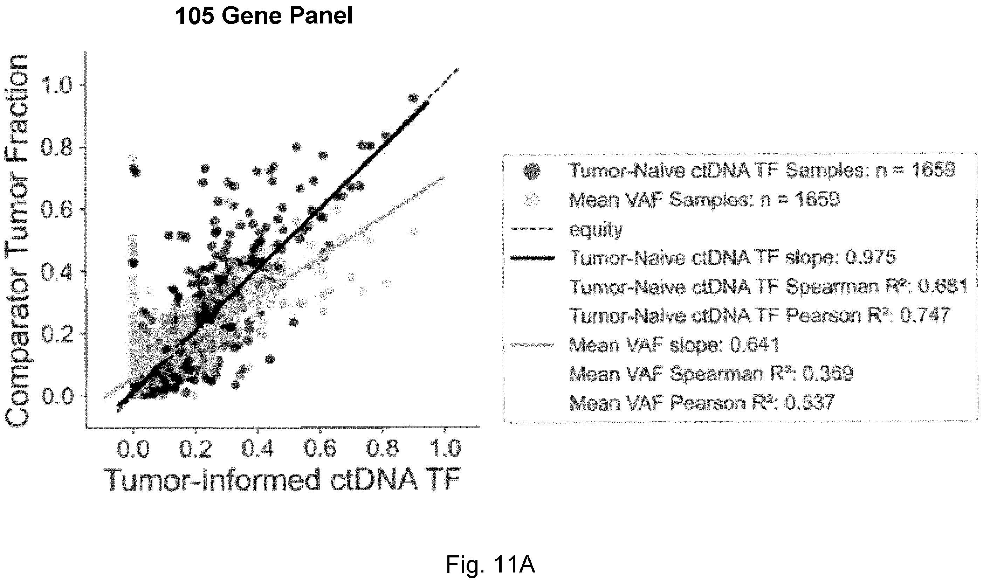

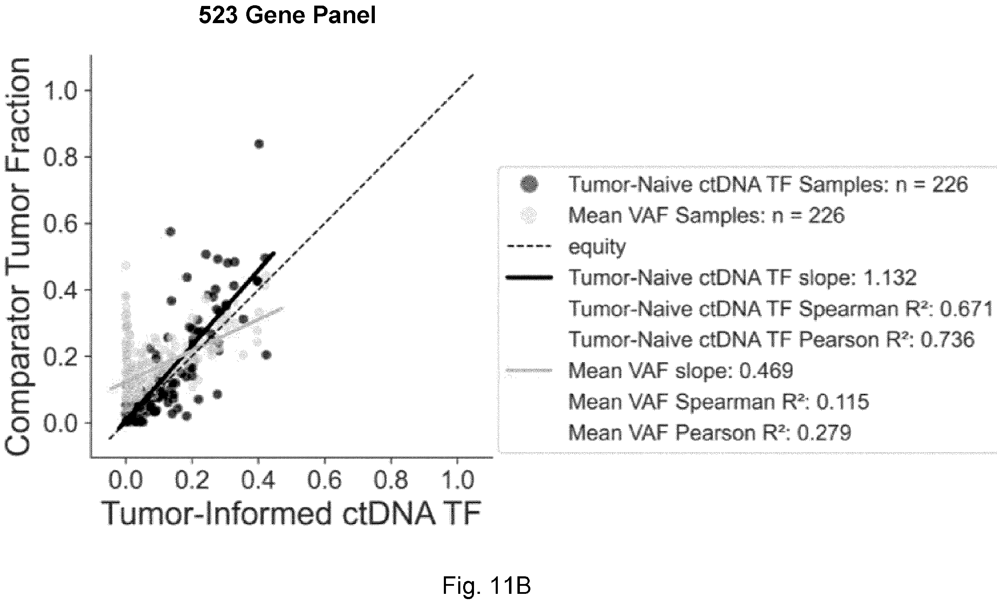

Next, the samples used in the first and second evaluations were further limited to those samples with at least five somatic mutations identified in both the solid tumor panel-enriched sequencing assay (648 genes) and the liquid biopsy panel-enriched sequencing assay (105 or 523 genes). As shown in Figures 11A (105 gene liquid biopsy panel-enriched sequencing assay) and 11B (523 gene liquid biopsy panel-enriched sequencing assay), requiring a greater number of common somatic mutations further improved the concordance of the tumor-informed and tumor-naïve models.

*Example 3 - Tumor-informed ctDNA TF achieves low LOB and LOD using presumed healthy subjects and titered controls.*

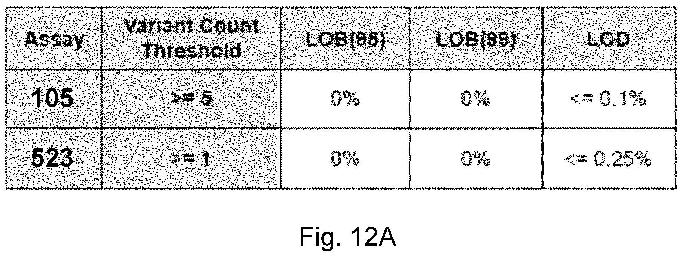

Level of blank (LOB) and level of detection (LOD) were determined for the tumor-informed ctDNA estimation model described in Example 1, using either the 105-gene liquid biopsy panel-enriched sequencing assay (applying a variant count threshold of at least 1 variant) or 523-gene liquid biopsy panel-enriched sequencing assay (applying a variant count threshold of at least 5 variants). Figure 12A shows performance metrics for the tumor-informed estimate performed on presumed healthy subjects and titered controls. The LOB(95) and LOB(99) were 0%. LOD hit-rate was 100% at the lowest titer evaluated in each assay. As shown in Figure 12A, tumor-informed ctDNA TF achieves low LOB and LOD using presumed healthy subjects and titered controls.

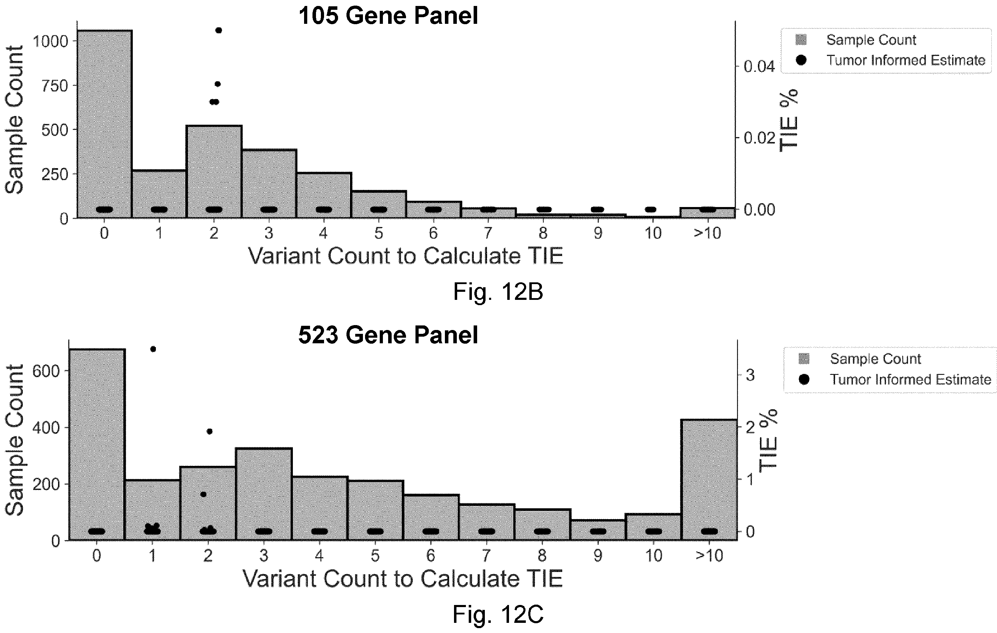

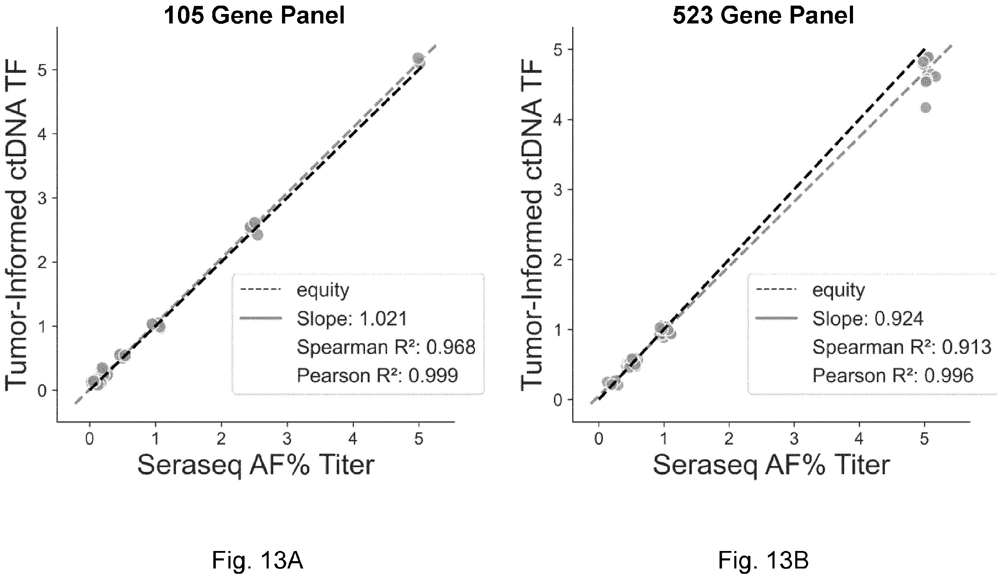

Figures 12B and 12C report 100x bootstrapped LOB calculated using the tumor informed ctFE model described in Example 1 from presumed healthy subjects yields a somatic mutation count distribution similar to that observed in tumor specimens. Figures 13A-13B reports LOD calculated from titered Seraseq ctDNA reference material processed using the 105 and 523 gene liquid biopsy panel-enriched sequencing assays. As shown in the Figure, the ctFEs determined using the tumor informed model have low inter-titer variability and a strong linear relationship.

*Additional Embodiments.* Another aspect of the present disclosure provides a computer system comprising one or more processors and a non-transitory computer-readable medium including computer-executable instructions that, when executed by the one or more processors, cause the processors to perform any of the methods and/or embodiments disclosed herein.

Yet another aspect of the present disclosure provides a non-transitory computer-readable storage medium having stored thereon program code instructions that, when executed by a processor, cause the processor to perform any of the methods and/or embodiments disclosed herein.

Although inventions have been particularly shown and described with reference to a preferred embodiment and various alternate embodiments, it will be understood by persons skilled in the relevant art that various changes in form and details can be made therein without departing from the spirit and scope of the invention.

*Additional Considerations.* The foregoing description of the embodiments has been presented for the purpose of illustration; it is not intended to be exhaustive or to limit the patent rights to the precise forms disclosed. Persons skilled in the relevant art will appreciate that many modifications and variations are possible in light of the above disclosure.

Any feature mentioned in one claim category, e.g., method, can be claimed in another claim category, e.g., computer program product, system, storage medium, as well. The dependencies or references back in the attached claims are chosen for formal reasons only. However, any subject matter resulting from a deliberate reference back to any previous claims (in particular multiple dependencies) can be claimed as well, so that any combination of claims and the features thereof is disclosed and can be claimed regardless of the dependencies chosen in the attached claims. The subject matter, in some embodiments, includes not only the combinations of features as set out in the disclosed embodiments but also any other combination of features from different embodiments. Various features mentioned in the different embodiments can be combined with explicit mentioning of such combination or arrangement in an example embodiment or without any explicit mentioning. Furthermore, any of the embodiments and features described or depicted herein, in some embodiments, are claimed in a separate claim and/or in any combination with any embodiment or feature described or depicted herein or with any of the features.

Some portions of this description describe the embodiments in terms of algorithms and symbolic representations of operations on information. These operations and algorithmic descriptions, while described functionally, computationally, or logically, are understood to be implemented by computer programs or equivalent electrical circuits, microcode, or the like. Furthermore, it has also proven convenient at times, to refer to these arrangements of operations as engines, without loss of generality. The described operations and their associated engines are, in some embodiments, embodied in software, firmware, hardware, or any combinations thereof.

Any of the steps, operations, or processes described herein, in some embodiments, are performed or implemented with one or more hardware or software engines, alone or in combination with other devices. In one embodiment, a software engine is implemented with a computer program product comprising a computer-readable medium containing computer program code, which can be executed by a computer processor for performing any or all of the steps, operations, or processes described. The term "steps" does not mandate or imply a particular order. For example, while this disclosure describes, in some embodiments, a process that includes multiple steps sequentially with arrows present in a flowchart, the steps in the process do not need to be performed by the specific order claimed or described in the disclosure. In some implementations, some steps are performed before others even though the other steps are claimed or described first in this disclosure. Likewise, any use of (i), (ii), (iii), *etc.,* or (a), (b), (c), *etc.* in the specification or in the claims, unless specified, is used to better enumerate items or steps and also does not mandate a particular order.

*Equivalents and Incorporation by Reference.* All references cited herein are incorporated by reference to the same extent as if each individual publication, database entry (*e.g.*, Genbank sequences or GeneID entries), patent application, or patent, was specifically and individually indicated to be incorporated by reference in its entirety, for all purposes. This statement of incorporation by reference is intended by Applicants, pursuant to 37 C.F.R. §1.57(b)(1), to relate to each and every individual publication, database entry (e.g., Genbank sequences or GeneID entries), patent application, or patent, each of which is clearly identified in compliance with 37 C.F.R. §1.57(b)(2), even if such citation is not immediately adjacent to a dedicated statement of incorporation by reference. The inclusion of dedicated statements of incorporation by reference, if any, within the specification does not in any way weaken this general statement of incorporation by reference. Citation of the references herein is not intended as an admission that the reference is pertinent prior art, nor does it constitute any admission as to the contents or date of these publications or documents.

$\log_2$ transformed copy ratios, $\log_2$ copy ratios, $\log_2$-transformed depths, $\log_2$-transformed read depths, $\log_2$ depths, corrected $\log_2$ depths, $\log_2$ ratios, $\log_2$ read depths, and $\log_2$ depth correction values have been discussed herein by way of example. In each instance where such a term is used, it will be appreciated that log base 2 is presented by way of example only and that the present disclosure is not so limited. Indeed, logarithms to any base N may be used, (*e.g.*, where N is a positive number greater than 1 for instance), and thus the present disclosure fully supports $\log_N$ transformed copy ratios, $\log_N$ copy ratios, $\log_N$-transformed depths, $\log_N$-transformed read depths, $\log_N$ depths, corrected $\log_N$ depths, $\log_N$ ratios, $\log_N$ read depths, and $\log_N$ depth correction values as respective substitutes for $\log_2$ transformed copy ratios, $\log_2$ copy ratios, $\log_2$-transformed depths, $\log_2$-transformed read depths, $\log_2$ depths, corrected $\log_2$ depths, $\log_2$ ratios, $\log_2$ read depths, and $\log_2$ depth correction values.

The present invention can be implemented as a computer program product that comprises a computer program mechanism embedded in a non-transitory computer readable storage medium. These program modules can be stored on a CD-ROM, DVD, magnetic disk storage product, USB key, or any other non-transitory computer readable data or program storage product.

Many modifications and variations of this invention can be made without departing from its spirit and scope, as will be apparent to those skilled in the art. The specific embodiments described herein are offered by way of example only. The embodiments were chosen and described in order to best explain the principles of the invention and its practical applications, to thereby enable others skilled in the art to best utilize the invention and various embodiments with various modifications as are suited to the particular use contemplated. The invention is to be limited only by the terms of the appended claims, along with the full scope of equivalents to which such claims are entitled.

Disclosed items:

1. A method of determining an estimate of a circulating tumor fraction for a test subject comprising:
at a computer system having one or more processors, and memory storing one or more programs for execution by the one or more processors:

A) obtaining a first plurality of nucleic acid sequences comprising a corresponding nucleic acid sequence for each respective locus in a plurality of loci in genomic DNA from a solid tumor sample from the test subject;
B) obtaining a second plurality of nucleic acid sequences comprising a corresponding nucleic acid sequence for each cell-free DNA fragment in a plurality of cell-free DNA fragments obtained from a liquid biopsy sample from a first panel-enriched sequencing assay using a first plurality of probes comprising, for each respective locus in the plurality of loci, a corresponding probe that hybridizes the respective locus;
C) identifying, in the first plurality of nucleic acid sequences, one or more somatic mutations, wherein each respective somatic mutation in the one or more somatic mutation is at a corresponding one or more nucleotide positions in a corresponding loci in the plurality of one or more loci;
D) forming a set of VAFs that comprises the respective VAF of each respective somatic mutation in the one or more somatic mutations, as determined from a frequency of the respective somatic mutation in the second plurality of nucleic acid sequences; and
E) determining an estimate of the circulating tumor fraction for the test subject based on the set of VAFs.

2. The method of item 1, wherein the first plurality of nucleic acid sequences is determined from a second panel-enriched sequencing reaction using a second plurality of probes comprising, for each respective locus in the plurality of loci a corresponding probe, in a second plurality of probes, that hybridizes to the respective locus.

3. The method of item 2, wherein the plurality of loci is sequenced at an average sequence depth of at least 100X in the second panel-enriched sequencing reaction.

4. The method of item 2 or 3, wherein the second plurality of probes enriches for loci from at least 50 genes.

5. The method of item 2 or 3, wherein the second plurality of probes enriches for loci from at least 50 genes in Table 1, Table 2, List 1, List 2, Figure 14, or Figure 15.

6. The method of any one of items 1-5, wherein the first plurality of probes and the second plurality of probes are different.

7. The method of any one of items 1-6, wherein the plurality of loci is sequenced at an average sequence depth of at least 500X in the first panel-enriched sequencing reaction.

8. The method of any one of items 1-8, wherein the first plurality of probes enriches for loci from at least 50 genes.

9. The method of any one of items 1-9, wherein the identity of the first plurality of probes is non-bespoke for the test subject.

10. The method of any one of items 1-10, wherein the solid tumor sample is collected prior to collecting the liquid biopsy sample.

11. The method of any one of items 1-10, wherein the solid tumor sample and the liquid biopsy sample are collected within 6 months of each other.

12. The method of any one of items 1-12, wherein the liquid biopsy sample is blood.

13. The method of any one of items 1-12, wherein the liquid biopsy sample comprises blood, whole blood, peripheral blood, plasma, serum, or lymph of the subject.

14. The method of any one of items 1-13, wherein the identifying C) comprises identifying a plurality of candidate somatic mutations by comparing respective nucleic acid sequences in the first plurality of nucleic acid sequences to nucleic acid sequences in a third plurality of nucleic acid sequences obtained from a sequencing reaction of genomic DNA from a non-cancerous tissue of the subject.

15. The method of item 14, wherein the identifying C) further comprises excluding one or more respective candidate somatic mutations in the plurality of candidate somatic mutations determined to have outlying variant allele fractions in the first plurality of sequences.

16. The method of item 15, wherein the excluding comprises fitting VAFs for each respective candidate somatic mutation in the plurality of candidate somatic mutations in the first plurality of sequences to a distribution and excluding candidate somatic mutations with corresponding VAFs outside of a measure of dispersion for the distribution.

17. The method of item 16, wherein the distribution is a normal distribution, a beta distribution, a beta prime distribution, a log normal distribution, or a gamma distribution.

18. The method of item 16, wherein the fitting comprises application of maximum likelihood estimation, method of moments, Bayesian inference, least squares, quantile matching, or expectation-maximization to the VAFs of each respective candidate somatic mutation in the plurality of candidate somatic mutations.

19. The method of item 16 or 17, wherein the distribution is a normal distribution.

20. The method of any one of items 16-19, wherein the measure of dispersion is a multiple of a standard deviation about a measure of central tendency of the distribution.

21. The method of any one of items 16-19, wherein the measure of dispersion is a multiple of a mean absolute deviation (MAD) about a measure of central tendency of the distribution.

22. The method of any one of items 16-19, wherein the measure of dispersion is an interquartile range (IQR), range, a coefficient of variation (CV) range, a skewness range, a kurtosis range, or a Gini Index within the distribution.

23. The method of item 15, wherein the excluding comprises determining a distribution for the VAFs for each respective candidate somatic mutation in the plurality of candidate somatic mutations in the first plurality of sequences using a nonparametric method and excluding candidate somatic mutations with corresponding VAFs outside of a measure of dispersion for the distribution.

24. The method of item 23, wherein the nonparametric method is Kernel Density Estimation.

25. The method of any one of items 14-24, wherein the identifying C) further comprises excluding one or more respective candidate somatic mutations in the plurality of candidate somatic mutations having a nucleotide position that does not correspond to any probe in the first plurality of probes.

26. The method of any one of items 1-25, wherein the estimate of the circulating tumor fraction is a measure of central tendency for the set of VAFs.

27. The method of item 26, wherein the measure of central tendency is a median.

28. The method of any one of items 1-25, wherein the estimate of the circulating tumor fraction is determined from the set of VAFs using a mean VAF method.

29. The method of any one of items 1-25, wherein the estimate of the circulating tumor fraction is determined from the set of VAFs using VAF-based CFT estimation.

30. The method of any one of items 1-29, wherein the method further comprises: F) reporting the estimate of the circulating tumor fraction for the test subject.

31. The method of item 30, wherein the reporting F) further comprises, responsive to determining that the estimate of the circulating tumor fraction for the test subject satisfies a therapeutic threshold, reporting a matched therapeutic recommendation for the test subject.

32. The method of any one of items 1-31, further comprising administering a cancer agent to the test subject when the estimate of the circulating tumor fraction for the test subject satisfies a therapeutic threshold.

33. The method of any one of items 1-32, further comprising altering a cancer agent therapy regimen applied to the test subject when the estimate of the circulating tumor fraction for the test subject satisfies a therapeutic threshold.

34. The method of item 30, wherein the reporting F) further comprises, responsive to determining that the estimate of the circulating tumor fraction for the test subject satisfies a therapeutic threshold, reporting a matched clinical trial recommendation for the test subject.

35. The method of any one of items 1-34, further comprising, enrolling the test subject in a clinical trial when the estimate of the circulating tumor fraction for the test subject satisfies a clinical trial threshold.

36. The method of any one of items 1-35, wherein the one or more somatic mutations comprises two or more somatic mutations.

37. The method of any one of items 1-35, wherein the one or more somatic mutations comprises five or more somatic mutations.

38. The method of any one of items 1-35, wherein the one or more somatic mutations comprises ten or more somatic mutations.

39. The method of any one of items 1-35, wherein the one or more somatic mutations comprises twenty or more somatic mutations.

40. The method of any one of items 1-39, wherein the estimate of a circulating tumor fraction for a test subject has a limit-of-detection of less than 0.1%.

41. A computer system comprising:

one or more processors; and
a non-transitory computer-readable medium including computer-executable instructions that, when executed by the one or more processors, cause the processors to perform a method according to any one of items 1-40.

42. A non-transitory computer-readable storage medium having stored thereon program code instructions that, when executed by a processor, cause the processor to perform the method according to any one of items 1-40.

43. A method of determining an estimate of a circulating tumor fraction for a test subject comprising:
at a computer system having one or more processors, and memory storing one or more programs for execution by the one or more processors:

> A) obtaining a second plurality of nucleic acid sequences comprising a corresponding nucleic acid sequence for each cell-free DNA fragment in a plurality of cell-free DNA fragments obtained from a liquid biopsy sample from a first panel-enriched sequencing assay using a first plurality of probes comprising, for each respective locus in a plurality of loci, a corresponding probe that hybridizes the respective locus;
> B) identifying, in the first plurality of nucleic acid sequences, a plurality of somatic mutations, wherein each respective somatic mutation in the plurality of somatic mutations is at a corresponding one or more nucleotide positions in a corresponding loci in a plurality of loci;
> C) removing, from the plurality of somatic mutations, each respective somatic mutation that has been flagged as an outlier of a distribution of variant allele fractions determined for somatic mutations using a solid tumor sample from the test subject;
> D) removing, from the plurality of somatic mutations, each respective somatic mutation that has not been identified as having a variant allele fraction using the solid tumor sample;
> E) forming a set of VAFs that comprises the respective VAF of each respective somatic mutation in the plurality of somatic mutations after the removing C) and removing D); and
> F) determining an estimate of the circulating tumor fraction for the test subject based on the set of VAFs.

44. The method of item 43, the method further comprising:

> obtaining a first plurality of nucleic acid sequences comprising a corresponding nucleic acid sequence for each respective locus in a plurality of loci in genomic DNA from a solid tumor sample from the test subject;
> identifying, in the first plurality of nucleic acid sequences, one or more somatic mutations, wherein each respective somatic mutation in the one or more somatic mutation is at a corresponding one or more nucleotide positions in a corresponding loci in the plurality of one or more loci and is identified as having a variant allele fraction based on the first plurality of nucleic acid sequences.

45. The method of item 44, wherein the first plurality of nucleic acid sequences is determined from a second panel-enriched sequencing reaction using a second plurality of probes comprising, for each respective locus in the plurality of loci a corresponding probe, in a second plurality of probes, that hybridizes to the respective locus.

46. The method of item 45, wherein the plurality of loci is sequenced at an average sequence depth of at least 100X in the second panel-enriched sequencing reaction.

47. The method of item 45 or 46, wherein the second plurality of probes enriches for loci from at least 50 genes.

48. The method of item 45 or 46, wherein the second plurality of probes enriches for loci from at least 50 genes in Table 1, Table 2, List 1, List 2, Figure 14, or Figure 15.

49. The method of any one of items 45-48, wherein the first plurality of probes and the second plurality of probes are different.

50. The method of any one of items 43-49, wherein the plurality of loci is sequenced at an average sequence depth of at least 500X in the first panel-enriched sequencing reaction.

51. The method of any one of items 43-50, wherein the first plurality of probes enriches for loci from at least 50 genes.

52. The method of any one of items 43-51, wherein the identity of the first plurality of probes is non-bespoke for the test subject.

53. The method of any one of items 43-52, wherein the solid tumor sample is collected prior to collecting the liquid biopsy sample.

54. The method of any one of items 43-53, wherein the solid tumor sample and the liquid biopsy sample are collected within 6 months of each other.

55. The method of any one of items 43-54, wherein the liquid biopsy sample is blood.

56. The method of any one of items 43-54, wherein the liquid biopsy sample comprises blood, whole blood, peripheral blood, plasma, serum, or lymph of the subject.

57. The method of any one of items 43-56, wherein the distribution is a normal distribution, a beta distribution, a beta prime distribution, a log normal distribution, or a gamma distribution.

58. The method of any one of items 43-56, wherein the distribution is a normal distribution.

59. The method of any one of items 43-58, wherein a somatic mutation is flagged as an outlier of the distribution based on a measure of dispersion.

60. The method of item 59, wherein the measure of dispersion is a multiple of a standard deviation about a measure of central tendency of the distribution.

61. The method of item 59, wherein the measure of dispersion is a multiple of a mean absolute deviation (MAD) about a measure of central tendency of the distribution.

62. The method of item 59, wherein the measure of dispersion is an interquartile range (IQR), range, a coefficient of variation (CV) range, a skewness range, a kurtosis range, or a Gini Index within the distribution.

63. The method of any one of items 43-62, wherein the method further comprises excluding one or more respective somatic mutations in the plurality of somatic mutations having a nucleotide position that does not correspond to any probe in the first plurality of probes.

64. The method of any one of items 43-63, wherein the estimate of the circulating tumor fraction is a measure of central tendency for the set of VAFs.

65. The method of item 64, wherein the measure of central tendency is a median.

66. The method of any one of items 43-63, wherein the estimate of the circulating tumor fraction is determined from the set of VAFs using a mean VAF method.

67. The method of any one of items 43-63, wherein the estimate of the circulating tumor fraction is determined from the set of VAFs using VAF-based CFT estimation.

68. The method of any one of items 43-67, wherein the method further comprises: G) reporting the estimate of the circulating tumor fraction for the test subject.

69. The method of item 68, wherein the reporting G) further comprises, responsive to determining that the estimate of the circulating tumor fraction for the test subject satisfies a therapeutic threshold, reporting a matched therapeutic recommendation for the test subject.

70. The method of any one of items 43-69, further comprising administering a cancer agent to the test subject when the estimate of the circulating tumor fraction for the test subject satisfies a therapeutic threshold.

71. The method of any one of items 43-70, further comprising altering a cancer agent therapy regimen applied to the test subject when the estimate of the circulating tumor fraction for the test subject satisfies a therapeutic threshold.

72. The method of item 68, wherein the reporting g) further comprises, responsive to determining that the estimate of the circulating tumor fraction for the test subject satisfies a therapeutic threshold, reporting a matched clinical trial recommendation for the test subject.

73. The method of any one of items 43-72, further comprising, enrolling the test subject in a clinical trial when the estimate of the circulating tumor fraction for the test subject satisfies a clinical trial threshold.

74. The method of any one of items 43-73, wherein the plurality of somatic mutations, prior to the removing C) and removing D), comprises five or more somatic mutations.

75. The method of any one of items 43-73, wherein the plurality of somatic mutations, prior to the removing C) and removing D), comprises ten or more somatic mutations.

76. The method of any one of items 43-73, wherein the one or more somatic mutations, prior to the removing C) and removing D), comprises twenty or more somatic mutations.

77. The method of any one of items 43-76, wherein the estimate of a circulating tumor fraction for a test subject has a limit-of-detection of less than 0.1%.

78. A computer system comprising:

one or more processors; and
a non-transitory computer-readable medium including computer-executable instructions that, when executed by the one or more processors, cause the processors to perform a method according to any one of items 43-77.

79. A non-transitory computer-readable storage medium having stored thereon program code instructions that, when executed by a processor, cause the processor to perform the method according to any one of items 43-77.

**Claims**

1. A method of determining an estimate of a circulating tumor fraction for a test subject comprising:
at a computer system having one or more processors, and memory storing one or more programs for execution by the one or more processors:

A) identifying, in a first plurality of nucleic acid sequences, a plurality of somatic mutations, wherein each respective somatic mutation in the plurality of somatic mutations is at a corresponding one or more nucleotide positions in a corresponding loci in a plurality of loci;
B) obtaining a second plurality of nucleic acid sequences comprising a corresponding nucleic acid sequence for each cell-free DNA fragment in a plurality of cell-free DNA fragments obtained from a liquid biopsy sample from a first panel-enriched sequencing assay using a first plurality of probes comprising, for each respective locus in a plurality of loci, a corresponding probe that hybridizes the respective locus;
C) removing, from the plurality of somatic mutations, each respective somatic mutation that has been flagged as an outlier of a distribution of variant allele fractions determined for somatic mutations using a solid tumor sample from the test subject;
D) removing, from the plurality of somatic mutations, each respective somatic mutation that has not been identified as having a variant allele fraction using the solid tumor sample;
E) forming a set of VAFs, using the second plurality of nucleic acid sequences, that comprises the respective VAF of each respective somatic mutation in the plurality of somatic mutations after the removing C) and removing D); and
F) determining an estimate of the circulating tumor fraction for the test subject based on the set of VAFs.

2. The method of claim 1, the method further comprising:

obtaining the first plurality of nucleic acid sequences, wherein the first plurality of nucleic acid sequences comprises a corresponding nucleic acid sequence for each respective locus in the plurality of loci in genomic DNA from the solid tumor sample from the test subject;
identifying, in the first plurality of nucleic acid sequences, one or more somatic mutations, wherein each respective somatic mutation in the one or more somatic mutation is at a corresponding one or more nucleotide positions in a corresponding loci in the plurality of one or more loci and is identified as having a variant allele fraction based on the first plurality of nucleic acid sequences;
wherein the first plurality of nucleic acid sequences is determined from a second panel-enriched sequencing reaction using a second plurality of probes comprising, for each respective locus in the plurality of loci, a corresponding probe, in a second plurality of probes, that hybridizes to the respective locus.

3. The method of claim 2, wherein the plurality of loci is sequenced at an average sequence depth of at least 100X in the second panel-enriched sequencing reaction, or wherein the second plurality of probes enriches for loci from at least 50 genes.

4. The method of claim 2 or 3, wherein the second plurality of probes enriches for loci from at least 50 genes in Table 1, Table 2, List 1, List 2, Figure 14, or Figure 15.

5. The method of any one of claims 2-4, wherein the first plurality of probes and the second plurality of probes are different.

6. The method of any one of claims 1-5, wherein the plurality of loci is sequenced at an average sequence depth of at least 500X in the first panel-enriched sequencing reaction or wherein the first plurality of probes enriches for loci from at least 50 genes.

7. The method of any one of claims 1-6, wherein the identity of the first plurality of probes is non-bespoke for the test subject.

8. The method of any one of claims 1-7, wherein the solid tumor sample and the liquid biopsy sample have been collected within 6 months of each other.

9. The method of any one of claims 1-8, wherein the liquid biopsy sample comprises blood, whole blood, peripheral blood, plasma, serum, or lymph of the subject.

10. The method of any one of claims 1-9, wherein the distribution is a normal distribution, a beta distribution, a beta prime distribution, a log normal distribution, or a gamma distribution.

11. The method of any one of claims 1-10, wherein a somatic mutation is flagged as an outlier of the distribution based on a measure of dispersion, wherein the measure of dispersion is a multiple of a standard deviation about a measure of central tendency of the distribution, a multiple of a mean absolute deviation (MAD) about a measure of central tendency of the distribution, an interquartile range (IQR), range, a coefficient of variation (CV) range, a skewness range, a kurtosis range, or a Gini Index within the distribution.

12. The method of any one of claims 1-11, wherein the method further comprises excluding one or more respective somatic mutations in the plurality of somatic mutations having a nucleotide position that does not correspond to any probe in the first plurality of probes.

13. The method of any one of claims 1-12, wherein the estimate of the circulating tumor fraction is a measure of central tendency for the set of VAFs.

14. The method of any one of claims 1-13, wherein the estimate of the circulating tumor fraction is determined from the set of VAFs using a mean VAF method or a VAF-based CFT estimation.

15. A computer system comprising:

   one or more processors; and
   a non-transitory computer-readable medium including computer-executable instructions that, when executed by the one or more processors, cause the processors to perform a method according to any one of claims 1-14.

| System 100 | | |
|---|---|---|
| | Operating System 116 | |
| Non-persistent memory 111 | Network communication module 118 | |
| | Test patient data store 120 | |
| | Bioinformatics module 140 | |
| | Sequence data processing module 141 | |
| | Pre-processing algorithms 142 | |
| | Alignment algorithm 143 | |
| | Demultiplexing algorithm 144 | |
| | Feature extraction module 145 | |
| | Variant identification module 146 | |
| | SNV/MNV calling algorithm 147 | |
| | Indel calling algorithm 148 | |
| | Genomic rearrangement calling algorithm 149 | |
| | Variant allele fraction module 151 | |
| | Methylation analysis module 152 | |
| | Copy Number Variation (CNV) analysis module 153 | |
| | Microsatellite Instability (MSI) analysis module 154 | |
| | Tumor Mutational Burden (TMB) analysis module 155 | |
| | Tumor ploidy analysis module 156 | |
| | Homologous recombination pathway analysis module 157 | |
| | Reference sequence constructs 158 | |
| | Feature analysis module 160 | |
| | Genomic alteration interpretation algorithms 161 | |
| | Pathogenic variant analysis algorithms 162 | |
| | Genomic variant analysis algorithms 163 | |
| | Compound feature analysis algorithms 164 | |
| | Clinical data analysis algorithms 165 | |
| | Therapeutic curation algorithms 166 | |
| | Recommendation validation module 167 | |
| | Reporting module 170 | |

Network 105

114

Processing core 102

Persistent memory 112

User interface 106
Display 108
Keyboard 110

Network interface 104

## Fig. 1A

| Test patient data store 120 | | | | |
|---|---|---|---|---|
| | Patient 1 121-1 | | | |
| | | Sequencing data 122-1 | | |
| | | | Sequencing run 1 122-i-1-1 | |
| | | | | Sequence read 1 123-1-1-1 |
| | | | | ⋮ |
| | | | | Sequence read K 123-1-1-K |
| | | | | Aligned sequences (e.g., BAM file) 124-1-1 |
| | | | ⋮ | |
| | | | Sequencing run L 122-i-1-L | |
| | | Feature data 125-1 | | |
| | | | Personal characteristics 126-1 | |
| | | | Medical history 127-1 | |
| | | | Clinical features 128-1 | |
| | | | | Pathology data 128-1-1 |
| | | | | Imaging data 128-2-1 |
| | | | | Tissue culture / organoid data 128-3-1 |
| | | | Genomic features 131-1 | |
| | | | | Allelic states 132-1 |
| | | | | Variant allele fractions 133-1 |
| | | | | Methylation states 132-1 |
| | | | | Genomic copy numbers 135-1 |
| | | | | Tumor mutational burden 136-1 |
| | | | | Microsatellite instability status 137-1-1 |
| | | | | Tumor ploidy 137-1-2 |
| | | | | HRD status 137-1-3 |
| | | | Other -omics data 138-1 | |
| | | Clinical assessment 139-1 | | |
| | | | Actionable variants and characteristics 139-1-1 | |
| | | | Matched therapies 139-1-2 | |
| | | | Clinical reports 139-1-3 | |
| | ⋮ | | | |
| | Patient M 121-M | | | |

Non-persistent memory 111

## Fig. 1B

| Genomic features 131 | | | | |
|---|---|---|---|---|
| | Circulating tumor fraction estimates 131-i | | | |
| | | Liquid biopsy circulating tumor fraction estimates 131-i-cf | | |
| | | | ctFE 1 131-i-cf-1 | |
| | | | ctFE 2 131-i-cf-2 | |
| | | | ⋮ | |
| | | | ctFE N 131-i-cf-N | |

Non-persistent memory 111

## Fig. 1C

| Feature extraction module 145 | | | | |
|---|---|---|---|---|
| | Tumor fraction estimation module 145-tf | | | |
| | | Tumor purity algorithm construct 145-tf-a | | |
| | | | Model parameters 145-tf-d | |
| | | | | Parameter 1 145-tf-d-1 |
| | | | | Parameter 2 145-tf-d-2 |
| | | | | ⋮ |
| | | Input data filtration construct 145-tf-k | | |
| | Sequence ratio data structure 145-tf-r | | | |
| | Minimization construct 145-tf-o | | | |

Non-persistent memory 111

## Fig. 1D

Fig. 2A

Fig. 2B

**204  Wet Lab Processing**

**302  Accessioning**

Tissue biopsy

Liquid biopsy

**Clinical Review 316**

Pathology review

Medical imaging review

Tissue culture review

**304  Extraction**

DNA

RNA

cfDNA

**306  Library Prep**

DNA library

RNA library

cfDNA library

**308  Pooling**

Pool of DNA

Pool of RNA

Library of cfDNA

**310  Capture + Hybridize**

Captured pool DNA

Captured pool RNA

Captured pool cfDNA

**312  Sequencing**

Sequenced DNA

Sequenced RNA

Sequenced cfDNA

BCL file 314

**206  Feature Extraction**

Fig. 3

**206** Feature Extraction

Sequence reads (314) → **318** File format conversion → **320** Demultiplex → **322** Alignment

S1: Tumor BAM (124-1-i-t) → **324** Genomic Feature Identification → Genomic features (131-1-i-t)

S1: Liquid BAM (124-1-i-cf) → Genomic features (131-1-i-cf)

S1: Germline BAM (124-1-i-g) → Genomic features (131-1-i-g)

S2: Tumor BAM (124-2-j-t) → Genomic features (131-2-i-t)

Variant Analysis **208**

Fig. 4A

normal isolate

tumor isolate

Fragmentation
Amplification
Sequencing
Base calling
Demultiplexing / adapter trimming

normal FASTQ

tumor FASTQ

Joint
variant calling

Tumor-only
variant calling

Fig. 4B

```
order.tar.gz
└── order/
    ├── order_N_1.fastq.gz
    ├── order_N_2.fastq.gz
    ├── order_T_1.fastq.gz
    └── order_T_2.fastq.gz
```

Sequence identifier
Sequence base calls
Base call quality scores
(phred)

Fig. 4C

Raw FASTQ files → FASTQ Quality Control (trimming, filtering, correcting for artifacts, etc.) → Processesed FASTQ files

Fig. 4D

Fig. 4E

EP 4 629 247 A1

<u>500</u>

**(502)** Obtain a first plurality of nucleic acid sequences including a corresponding nucleic acid sequence for each respective locus in a plurality of loci in genomic DNA from a solid tumor sample from the test subject.

**(504)** The first plurality of nucleic acid sequences is determined from a second panel-enriched sequencing reaction using a second plurality of probes including, for each respective locus in the plurality of loci a corresponding probe, in a second plurality of probes, that hybridizes to the respective locus.

**(506)** The plurality of loci is sequenced at an average sequence depth of at least 1000X in the second panel-enriched sequencing reaction.

**(508)** The second plurality of probes enriches for loci from at least 50 genes.

(A)

Fig. 5A

(A)

**(509)** Obtain a second plurality of nucleic acid sequences including a corresponding nucleic acid sequence for each cell-free DNA fragment in a plurality of cell-free DNA fragments obtained from a liquid biopsy sample from a first panel-enriched sequencing assay using a first plurality of probes including, for each respective locus in the plurality of loci, a corresponding probe that hybridizes the respective locus.

**(510)** The first plurality of probes and the second plurality of probes are different.

**(512)** The plurality of loci is sequenced at an average sequence depth of at least 1000X in the first panel-enriched sequencing reaction.

**(514)** The first plurality of probes enriches for loci from at least 50 genes.

**(516)** The identity of the first plurality of probes is non-bespoke for the test subject.

**(518)** The solid tumor sample is collected prior to collecting the liquid biopsy sample.

**(520)** The solid tumor sample and the liquid biopsy sample are collected within 6 months of each other.

**(522)** The liquid biopsy sample is blood.

**(524)** The liquid biopsy sample includes blood, whole blood, peripheral blood, plasma, serum, or lymph of the subject.

(B)

Fig. 5B

**B**

**(525)** Identify, in the first plurality of nucleic acid sequences, one or more somatic mutations, where each respective somatic mutations in the one or more somatic mutations is at a corresponding one or more nucleotide positions in a corresponding loci in the plurality of one or more loci.

**(526)** The identifying includes identifying a plurality of candidate somatic mutations by comparing respective nucleic acid sequences in the first plurality of nucleic acid sequences to nucleic acid sequences in a third plurality of nucleic acid sequences obtained from a sequencing reaction of genomic DNA from a non-cancerous tissue of the subject.

**(528)** The identifying further includes excluding one or more respective candidate somatic mutations in the plurality of candidate somatic mutations determined to have outlying variant allele fractions in the first plurality of sequences.

**(530)** The excluding includes fitting VAFs for each respective candidate somatic mutation in the plurality of candidate somatic mutations in the first plurality of sequences to a distribution and excluding candidate somatic mutations with corresponding VAFs outside of a statistical measure of variation for the distribution.

**(532)** The distribution is a normal distribution, a beta distribution, a beta prime distribution, a log normal distribution, or a gamma distribution.

**(534)** The probability distribution is a normal distribution.

**(536)** The statistical measure of variation is a multiple of a standard deviation for the probability distribution.

**(538)** The identifying further includes excluding one or more respective candidate somatic mutations in the plurality of candidate somatic mutations having a nucleotide position that does not correspond to any probe in the first plurality of probes.

**C**

Fig. 5C

C

**(539)** Determine, for each respective somatic mutation in the one or more somatic mutations, a corresponding variant allele frequency (VAF) in the liquid biopsy sample, where the corresponding VAF is determined from (i) a frequency of the respective somatic mutation in the second plurality of nucleic acid sequences and (ii) a frequency of a wild type allele at the corresponding one or more nucleotide positions for the respective somatic variant in the second plurality of nucleic acid sequences, thereby determining a set of VAFs for the one or more somatic mutations in the liquid biopsy sample.

**(540)** Determine an estimate of the circulating tumor fraction for the test subject based on the set of VAFs.

**(542)** The estimate of the circulating tumor fraction is a measure of central tendency for the set of VAFs for the one or more somatic mutations.

**(544)** The measure of central tendency is a median.

Fig. 5D

Fig. 6A

Fig. 6B

Fig. 7A

Fig. 7B

Fig. 8A

Fig. 8B

Fig. 9A

EP 4 629 247 A1

Fig. 9B

EP 4 629 247 A1

Fig. 10

Fig. 11A

**523 Gene Panel**

Fig. 11B

| Assay | Variant Count Threshold | LOB(95) | LOB(99) | LOD |
|-------|------------------------|---------|---------|-----------|
| 105 | >= 5 | 0% | 0% | <= 0.1% |
| 523 | >= 1 | 0% | 0% | <= 0.25% |

Fig. 12A

**105 Gene Panel**

Fig. 12B

**523 Gene Panel**

Fig. 12C

**Fig. 13A**

**Fig. 13B**

EP 4 629 247 A1

| GENE | ENHANCED SNV / Indel | Non-Enhanced SNV / Indel | Fusion | CNV |
|---|---|---|---|---|
| ABCC3 | | • | | |
| ABL1 | • | | | |
| ABL2 | | • | | |
| ABRAXAS1 | | • | | |
| ACVR1 | | • | | |
| ACVR1B | | • | | |
| AJUBA | | • | | |
| AKT1 | • | | | |
| AKT2 | • | | | |
| AKT3 | | • | | |
| ALK | • | | • | |
| ALOX12B | | • | | |
| AMER1 | | • | | |
| APC | • | | | |
| APLNR | | • | | |
| AR | • | | | |
| ARAF | • | | | |
| ARFRP1 | | • | | |
| ARID1A | • | | | |
| ARID1B | | • | | |
| ARID2 | | • | | |
| ASNS | | • | | |
| ASXL1 | | • | | |
| ATM | • | | | |
| ATR | • | | | |
| ATRX | | • | | |
| AURKA | | • | | |
| AURKB | | • | | |
| AURKC | | • | | |
| AXIN1 | | • | | |
| AXIN2 | | • | | |
| AXL | | • | | |

FIG. 14A

| GENE | ENHANCED SNV / Indel | Non-Enhanced SNV / Indel | Fusion | CNV |
|---|:---:|:---:|:---:|:---:|
| BCL2L2 | | • | | |
| BCL6 | | • | | |
| BCLAF1 | | • | | |
| BCOR | | • | | |
| BCORL1 | | • | | |
| BCR | | • | | |
| BIRC3 | | • | | |
| BLM | | • | | |
| BMPR1A | | • | | |
| BRAF | • | | • | |
| BRCA1 | • | | | • |
| BRCA2 | • | | | • |
| BRD4 | | • | | |
| BRIP1 | | • | | |
| BTG1 | | • | | |
| BTG2 | | • | | |
| BTK | • | | | |
| CALR | | • | | |
| CARD11 | | • | | |
| CARM1 | | • | | |
| CASP8 | | • | | |
| CBFB | | • | | |
| CBL | | • | | |
| CCND1 | • | | | |
| CCND2 | • | | | |
| CCND3 | • | | | |
| CCNE1 | • | | | • |
| CD22 | | • | | |
| CD274 | • | | | • |
| CD70 | | • | | |
| CD74 | | • | | |
| CD79A | | • | | |
| CD79B | | • | | |
| CDC73 | | • | | |
| CDH1 | • | | | |
| CDK12 | • | | | |
| CDK4 | • | | | |
| CDK6 | • | | | |
| CDK8 | | • | | |
| CDK9 | | • | | |

FIG. 14B

| GENE | ENHANCED SNV / Indel | Non-Enhanced SNV / Indel | Fusion | CNV |
|---|---|---|---|---|
| CDKN1A | | • | | |
| CDKN1B | | • | | |
| CDKN2A | • | | | |
| CDKN2B | | • | | |
| CDKN2C | | • | | |
| CEBPA | | • | | |
| CHD4 | | • | | |
| CHEK1 | | • | | |
| CHEK2 | • | | | |
| CIC | | • | | |
| CKS1B | | • | | |
| CREBBP | | • | | |
| CRKL | • | | | |
| CSF1R | | • | | |
| CSF3R | | • | | |
| CTC1 | | • | | |
| CTCF | | • | | |
| CTLA4 | | • | | |
| CTNNA1 | | • | | |
| CTNNB1 | • | | | |
| CUL3 | | • | | |
| CUL4A | | • | | |
| CUX1 | | • | | |
| CXCR4 | | • | | |
| CYLD | | • | | |
| CYP17A1 | | • | | |
| CYSLTR2 | | • | | |
| DAXX | | • | | |
| DDB2 | | • | | |
| DDR1 | | • | | |
| DDR2 | • | | | |
| DDX3X | | • | | |
| DDX41 | | • | | |
| DEPTOR | | • | | |
| DICER1 | | • | | |
| DIS3 | | • | | |
| DNMT1 | | • | | |
| DNMT3A | | • | | |
| DOT1L | | • | | |
| DPYD | | • | | |
| EBF1 | | • | | |
| EED | | • | | |

FIG. 14C

| GENE | ENHANCED SNV / Indel | Non-Enhanced SNV / Indel | Fusion | CNV |
|---|---|---|---|---|
| EEF2 | | • | | |
| EGFR | • | | | • |
| EGLN1 | | • | | |
| EIF1AX | | • | | |
| ELF3 | | • | | |
| EMSY | | • | | |
| EP300 | | • | | |
| EPCAM | | • | | |
| EPHA2 | | • | | |
| EPHA3 | | • | | |
| EPHB1 | | • | | |
| EPHB4 | | • | | |
| ERBB2 | • | | | • |
| ERBB3 | • | | | |
| ERBB4 | | • | | |
| ERCC2 | | • | | |
| ERCC3 | | • | | |
| ERCC4 | | • | | |
| ERCC6 | | • | | |
| ERG | | • | | |
| ERRFI1 | • | | | |
| ESR1 | • | | | |
| ETNK1 | | • | | |
| ETV1 | | • | | |
| ETV4 | | • | | |
| ETV5 | | • | | |
| ETV6 | | • | | |
| EWSR1 | | • | | |
| EZH2 | • | | | |
| EZR | | • | | |
| FAM46C | | • | | |
| FANCA | | • | | |
| FANCC | | • | | |
| FANCD2 | | • | | |
| FANCE | | • | | |
| FANCG | | • | | |
| FANCI | | • | | |
| FANCL | | • | | |
| FANCM | | • | | |
| FAS | | • | | |
| FAT1 | | • | | |
| FBXW7 | • | | | |

FIG. 14D

| GENE | ENHANCED SNV / Indel | Non-Enhanced SNV / Indel | Fusion | CNV |
|---|---|---|---|---|
| FCGR2A | | • | | |
| FCGR3A | | • | | |
| FGF10 | | • | | |
| FGF12 | | • | | |
| FGF14 | | • | | |
| FGF19 | | • | | |
| FGF23 | | • | | |
| FGF3 | | • | | |
| FGF4 | | • | | |
| FGF6 | | • | | |
| FGFR1 | • | | | • |
| FGFR2 | • | | | • |
| FGFR3 | • | | | • |
| FGFR4 | • | | | |
| FH | | • | | |
| FHIT | | • | | |
| FLCN | | • | | |
| FLT1 | | • | | |
| FLT3 | • | | | |
| FLT4 | | • | | |
| FOLH1 | | • | | |
| FOXA1 | | • | | |
| FOXL2 | • | | | |
| FOXO1 | | • | | |
| FOXO3 | | • | | |
| FOXP1 | | • | | |
| FRS2 | | • | | |
| FUBP1 | | • | | |
| GABRA6 | | • | | |
| GALNT12 | | • | | |
| GATA1 | | • | | |
| GATA3 | • | | | |
| GATA4 | | • | | |
| GATA6 | | • | | |
| GID4 | | • | | |
| GLI2 | | • | | |
| GNA11 | • | | | |
| GNA13 | | • | | |
| GNAQ | • | | | |
| GNAS | • | | | |
| GPC3 | | • | | |
| GPS2 | | • | | |

FIG. 14E

| GENE | ENHANCED SNV / Indel | Non-Enhanced SNV / Indel | Fusion | CNV |
|---|---|---|---|---|
| GREM1 | | • | | |
| GRIN2A | | • | | |
| GRM3 | | • | | |
| GSK3B | | • | | |
| GSTP1 | | • | | |
| H3F3A | | • | | |
| HAVCR2 | | • | | |
| HDAC1 | | • | | |
| HDAC2 | | • | | |
| HGF | | • | | |
| HIF1A | | • | | |
| HIST1H3B | | • | | |
| HLA-B | | • | | |
| HNF1A | • | | | |
| HNF1B | | • | | |
| HOXB13 | | • | | |
| HRAS | • | | | |
| HSD3B1 | | • | | |
| HSP90AA1 | | • | | |
| HSPH1 | | • | | |
| ID3 | | • | | |
| IDH1 | • | | | |
| IDH2 | • | | | |
| IFNA21 | | • | | |
| IFNAR1 | | • | | |
| IFNAR2 | | • | | |
| IFNG | | • | | |
| IFNGR1 | | • | | |
| IFNGR2 | | • | | |
| IFNW1 | | • | | |
| IGF1 | | • | | |
| IGF1R | | • | | |
| IKBKE | | • | | |
| IKZF1 | | • | | |
| IL10RA | | • | | |
| IL32 | | • | | |
| IL6R | | • | | |
| IL7R | | • | | |
| IMPDH1 | | • | | |
| ING1 | | • | | |
| INPP4B | | • | | |
| INSR | | • | | |

## FIG. 14F

| GENE | ENHANCED SNV / Indel | Non-Enhanced SNV / Indel | Fusion | CNV |
|------|---------------------|-------------------------|--------|-----|
| IRF1 | | • | | |
| IRF2 | | • | | |
| IRF4 | | • | | |
| IRS2 | | • | | |
| JAK1 | • | | | |
| JAK2 | • | | | |
| JAK3 | • | | | |
| JUN | | • | | |
| KAT6A | | • | | |
| KDM5A | | • | | |
| KDM5C | | • | | |
| KDM5D | | • | | |
| KDM6A | | • | | |
| KDR | • | | | |
| KEAP1 | • | | | |
| KEL | | • | | |
| KIT | • | | | |
| KLF4 | | • | | |
| KLHL6 | | • | | |
| KLLN | | • | | |
| KMT2A | • | | | |
| KMT2C | | • | | |
| KMT2D | | • | | |
| KRAS | • | | | |
| LATS1 | | • | | |
| LCK | | • | | |
| LMO1 | | • | | |
| LRP1B | | • | | |
| LTK | | • | | |
| LYN | | • | | |
| LZTR1 | | • | | |
| MAF | | • | | |
| MALT1 | | • | | |
| MAP2K1 | • | | | |
| MAP2K2 | • | | | |
| MAP2K4 | | • | | |
| MAP3K1 | | • | | |
| MAP3K13 | | • | | |
| MAP3K21 | | • | | |
| MAP3K7 | | • | | |
| MAPK1 | • | | | |
| MAPK3 | • | | | |

FIG. 14G

| GENE | ENHANCED SNV / Indel | Non-Enhanced SNV / Indel | Fusion | CNV |
|---|---|---|---|---|
| MAX | | • | | |
| MC1R | | • | | |
| MCL1 | | • | | |
| MDM2 | • | | | • |
| MDM4 | | • | | |
| MED12 | | • | | |
| MEF2B | | • | | |
| MEN1 | | • | | |
| MERTK | | • | | |
| MET | • | | | • |
| MITF | | • | | |
| MKNK1 | | • | | |
| MLH1 | • | | | |
| MLH3 | | • | | |
| MPL | • | | | |
| MRE11 | | • | | |
| MS4A1 | | • | | |
| MSH2 | • | | | |
| MSH3 | • | | | |
| MSH6 | • | | | |
| MST1R | | • | | |
| MTAP | | • | | |
| MTHFR | | • | | |
| MTOR | • | | | |
| MUC16 | | • | | |
| MUTYH | | • | | |
| MYB | | • | | |
| MYC | • | | | • |
| MYCL | | • | | |
| MYCN | • | | | |
| MYD88 | • | | | |
| NBN | | • | | |
| NCOA2 | | • | | |
| NCOR1 | | • | | |
| NF1 | • | | | |
| NF2 | • | | | |
| NFE2L2 | • | | | |
| NFKBIA | | • | | |
| NKX2-1 | | • | | |
| NOTCH1 | • | | | |
| NOTCH2 | | • | | |
| NOTCH3 | | • | | |

FIG. 14H

| GENE | ENHANCED SNV / Indel | Non-Enhanced SNV / Indel | Fusion | CNV |
|---|---|---|---|---|
| NOTCH4 | | • | | |
| NPM1 | • | | | |
| NQO1 | | • | | |
| NRAS | • | | | |
| NRG1 | | • | | |
| NSD1 | | • | | |
| NSD2 | | • | | |
| NSD3 | | • | | |
| NT5C2 | | • | | |
| NTRK1 | • | | | • |
| NTRK2 | • | | | • |
| NTRK3 | • | | | • |
| NUTM1 | | • | | |
| P2RY8 | | • | | |
| PAK1 | | • | | |
| PALB2 | • | | | |
| PALLD | | • | | |
| PARP1 | | • | | |
| PARP2 | | • | | |
| PARP3 | | • | | |
| PAX5 | | • | | |
| PBRM1 | • | | | |
| PDCD1 | | • | | |
| PDCD1LG2 | • | | | |
| PDGFRA | • | | | |
| PDGFRB | • | | | |
| PDK1 | | • | | |
| PHGDH | | • | | |
| PHLPP1 | | • | | |
| PHLPP2 | | • | | |
| PIAS4 | | • | | |
| PIK3C2B | | • | | |
| PIK3C2G | | • | | |
| PIK3CA | • | | | |
| PIK3CB | | • | | |
| PIK3CD | | • | | |
| PIK3CG | | • | | |
| PIK3R1 | • | | | |
| PIK3R2 | | • | | |
| PIM1 | | • | | |
| PLCG1 | | • | | |
| PLCG2 | | • | | |

FIG. 14I

| GENE | ENHANCED SNV / Indel | Non-Enhanced SNV / Indel | Fusion | CNV |
|---|---|---|---|---|
| PMS1 | | • | | |
| PMS2 | • | | | |
| POLA1 | | • | | |
| POLD1 | | • | | |
| POLE | | • | | |
| POLQ | | • | | |
| POT1 | | • | | |
| PPARG | | • | | |
| PPM1D | | • | | |
| PPP2R1A | | • | | |
| PPP2R2A | | • | | |
| PPP6C | | • | | |
| PRDM1 | | • | | |
| PREX2 | | • | | |
| PRKACA | | • | | |
| PRKAR1A | | • | | |
| PRKCI | | • | | |
| PRKN | | • | | |
| PTCH1 | • | | | |
| PTEN | • | | | |
| PTK2 | | • | | |
| PTPN11 | • | | | |
| PTPN13 | | • | | |
| PTPRD | | • | | |
| PTPRO | | • | | |
| PTPRT | | • | | |
| QKI | | • | | |
| RAC1 | | • | | |
| RAD21 | | • | | |
| RAD50 | | • | | |
| RAD51 | | • | | |
| RAD51B | | • | | |
| RAD51C | • | | | |
| RAD51D | | • | | |
| RAD52 | | • | | |
| RAD54L | | • | | |
| RAF1 | • | | | |
| RARA | | • | | |
| RASA1 | | • | | |
| RB1 | • | | | |
| RBM10 | | • | | |
| RECQL4 | | • | | |

FIG. 14J

| GENE | ENHANCED SNV / Indel | Non-Enhanced SNV / Indel | Fusion | CNV |
|---|---|---|---|---|
| REL | | • | | |
| RET | • | | • | |
| RHEB | • | | | |
| RHOA | • | | | |
| RICTOR | | • | | |
| RIT1 | • | | | |
| RNF43 | • | | | |
| ROS1 | • | | • | |
| RPS6KB1 | | • | | |
| RPTOR | | • | | |
| RRM1 | | • | | |
| RSF1 | | • | | |
| RSPO2 | | • | | |
| RUNX1 | | • | | |
| RXRA | | • | | |
| SDC4 | | • | | |
| SDHA | • | | | |
| SDHAF2 | | • | | |
| SDHB | | • | | |
| SDHC | | • | | |
| SDHD | | • | | |
| SETBP1 | | • | | |
| SETD2 | | • | | |
| SF3B1 | | • | | |
| SGK1 | | • | | |
| SIRPA | | • | | |
| SLC34A2 | | • | | |
| SLC9A3R1 | | • | | |
| SLFN11 | | • | | |
| SLIT2 | | • | | |
| SMAD2 | | • | | |
| SMAD3 | | • | | |
| SMAD4 | • | | | |
| SMARCA2 | | • | | |
| SMARCA4 | | • | | |
| SMARCB1 | | • | | |
| SMC1A | | • | | |
| SMC3 | | • | | |
| SMO | • | | | |
| SNCAIP | | • | | |
| SOCS1 | | • | | |
| SOS1 | | • | | |

FIG. 14K

| GENE | ENHANCED SNV / Indel | Non-Enhanced SNV / Indel | Fusion | CNV |
|------|------|------|------|------|
| SOX2 | | • | | |
| SOX9 | | • | | |
| SPEN | | • | | |
| SPOP | • | | | |
| SRC | | • | | |
| SRSF2 | | • | | |
| STAG2 | | • | | |
| STAT3 | | • | | |
| STAT5B | | • | | |
| STAT6 | | • | | |
| STK11 | • | | | |
| SUFU | | • | | |
| SUZ12 | | • | | |
| SYK | | • | | |
| TBX3 | | • | | |
| TCF7L2 | | • | | |
| TEK | | • | | |
| TERC | | • | | |
| TERT | • | | | |
| TET2 | | • | | |
| TFEB | | • | | |
| TGFB1 | | • | | |
| TGFBR1 | | • | | |
| TGFBR2 | | • | | |
| TIGIT | | • | | |
| TIPARP | | • | | |
| TMEM127 | | • | | |
| TMPRSS2 | | • | | |
| TNFAIP3 | | • | | |
| TNFRSF14 | | • | | |
| TNFRSF17 | | • | | |
| TOP1 | | • | | |
| TOP2A | | • | | |
| TP53 | • | | | |
| TP53BP1 | | • | | |
| TP63 | | • | | |
| TRAF3 | | • | | |
| TRAF7 | | • | | |
| TSC1 | • | | | |
| TSC2 | • | | | |
| TSHR | | • | | |
| TYMS | | • | | |

FIG. 14L

| GENE | ENHANCED SNV / Indel | Non-Enhanced SNV / Indel | Fusion | CNV |
|---|---|---|---|---|
| TYRO3 | | • | | |
| U2AF1 | | • | | |
| UGT1A1 | | • | | |
| VEGFA | • | | | |
| VHL | • | | | |
| VSIR | | • | | |
| WEE1 | | • | | |
| WNK1 | | • | | |
| WRN | | • | | |
| WT1 | | • | | |
| XBP1 | | • | | |
| XPA | | • | | |
| XPC | | • | | |
| XPO1 | | • | | |
| XRCC1 | | • | | |
| XRCC2 | | • | | |
| YEATS4 | | • | | |
| ZFHX3 | | • | | |
| ZMYM3 | | • | | |
| ZNF217 | | • | | |
| ZNF703 | | • | | |
| ZNF750 | | • | | |
| ZNRF3 | | • | | |
| ZRSR2 | | • | | |

FIG. 14M

| | | | | | | | | | | |
|---|---|---|---|---|---|---|---|---|---|---|
| ABCB1 | ABCC3 | ABL1 | ABL2 | ACTA2 | ACVR1B | AJUBA | AKT1 | AKT2 | AKT3 | ALK |
| AMER1 | APC | APOB | AR | ARAF | ARHGAP26 | ARHGAP35 | ARID1A | ARID1B | ARID2 | ARID5B |
| ASNS | ASXL1 | ATIC | ATM | ATP7B | ATR | ATRX | AURKA | AURKB | AXIN1 | AXIN2 |
| AXL | B2M | BAP1 | BARD1 | BCL2 | BCL2L1 | BCL2L11 | BCL6 | BCL7A | BCL10 | BCL11B |
| BCLAF1 | BCOR | BCORL1 | BCR | BIRC3 | BLM | BMPR1A | BRAF | BRCA1 | BRCA2 | BRD4 |
| BRIP1 | BTG1 | BTK | BUB1B | C3orf70 | C8orf34 | C10orf54 | C11orf30 | C11orf65 | CALR | CARD11 |
| CASP8 | CASR | CBFB | CBL | CBLB | CBLC | CBR3 | CCDC6 | CCND1 | CCND2 | CCND3 |
| CCNE1 | CD19 | CD22 | CD40 | CD70 | CD79A | CD79B | CD274 | CDC73 | CDH1 | CDK4 |
| CDK6 | CDK8 | CDK12 | CDKN1A | CDKN1B | CDKN1C | CDKN2A | CDKN2B | CDKN2C | CEBPA | CEP57 |
| CFTR | CHD2 | CHD4 | CHEK1 | CHEK2 | CIC | CIITA | CKS1B | CREBBP | CRKL | CRLF2 |
| CSF1R | CSF3R | CTC1 | CTCF | CTLA4 | CTNNA1 | CTNNB1 | CTRC | CUX1 | CXCR4 | CYLD |
| CYP1B1 | CYP2D6 | CYP3A5 | DAXX | DDB2 | DDR2 | DDX3X | DICER1 | DIRC2 | DIS3 | DIS3L2 |
| DKC1 | DNM2 | DNMT3A | DOT1L | DPYD | DYNC2H1 | EBF1 | ECT2L | EGF | EGFR | EGLN1 |
| ELF3 | ENG | EP300 | EPCAM | EPHA2 | EPHA7 | EPHB1 | EPHB2 | EPOR | ERBB2 | ERBB3 |
| ERBB4 | ERCC1 | ERCC2 | ERCC3 | ERCC4 | ERCC5 | ERCC6 | ERG | ERRFI1 | ESR1 | ETS1 |
| ETS2 | ETV1 | ETV4 | ETV5 | ETV6 | EWSR1 | EZH2 | FAM46C | FAM175A | FANCA | FANCB |
| FANCC | FANCD2 | FANCE | FANCF | FANCG | FANCI | FANCL | FANCM | FAS | FAT1 | FBXO11 |
| FBXW7 | FCGR2A | FCGR3A | FDPS | FGF1 | FGF2 | FGF3 | FGF4 | FGF5 | FGF6 | FGF7 |
| FGF8 | FGF9 | FGF10 | FGF14 | FGF23 | FGFR1 | FGFR2 | FGFR3 | FGFR4 | FH | FHIT |
| FLCN | FLG | FLT1 | FLT3 | FLT4 | FNTB | FOXA1 | FOXL2 | FOXO1 | FOXO3 | FOXP1 |
| FOXQ1 | FRS2 | FUBP1 | G6PD | GALNT12 | GATA1 | GATA2 | GATA3 | GATA4 | GATA6 | GEN1 |

FIG. 15A

| | | | | | | | | | |
|---|---|---|---|---|---|---|---|---|---|
| GLI1 | GNA11 | GNA13 | GNAQ | GNAS | GPC3 | GPS2 | GREM1 | GRIN2A | GRM3 | GSTP1 |
| H3F3A | H19 | HAS3 | HAVCR2 | HDAC1 | HDAC2 | HDAC4 | HGF | HIF1A | HIST1H1E | HIST1H3B |
| HIST1H4E | HLA-A | HLA-B | HLA-C | HLA-DMA | HLA-DMB | HLA-DOA | HLA-DOB | HLA-DPA1 | HLA-DPB1 | HLA-DPB2 |
| HLA-DQA1 | HLA-DQA2 | HLA-DQB1 | HLA-DQB2 | HLA-DRA | HLA-DRB1 | HLA-DRB5 | HLA-DRB6 | HLA-E | HLA-F | HLA-G |
| HNF1A | HNF1B | HOXB13 | HRAS | HSP90AA1 | HSPH1 | IDH1 | IDH2 | IDO1 | IFIT1 | IFIT2 |
| IFIT3 | IFNAR1 | IFNAR2 | IFNGR1 | IFNGR2 | IFNL3 | IKBKE | IKZF1 | IL2RA | IL6R | IL7R |
| IL10RA | IL15 | ING1 | INPP4B | IRF1 | IRF2 | IRF4 | IRS2 | ITPKB | JAK1 | JAK2 |
| JAK3 | JUN | KAT6A | KDM5A | KDM5C | KDM6A | KDR | KEAP1 | KEL | KIF1B | KIT |
| KLHL6 | KLLN | KMT2A | KMT2B | KMT2C | KMT2D | KRAS | LAG3 | LDLR | LEF1 | LMNA |
| LMO1 | LRP1B | LYN | LZTR1 | MAD2L2 | MAF | MAFB | MALT1 | MAP2K1 | MAP2K2 | MAP2K4 |
| MAP3K1 | MAP3K7 | MAPK1 | MAX | MC1R | MCL1 | MDM2 | MDM4 | MED12 | MEF2B | MEN1 |
| MET | MGMT | MIB1 | MITF | MKI67 | MLH1 | MLH3 | MLLT3 | MPL | MRE11A | MS4A1 |
| MSH2 | MSH3 | MSH6 | MTAP | MTHFR | MTOR | MTRR | MUTYH | MYB | MYC | MYCL |
| MYCN | MYD88 | MYH11 | NBN | NCOR1 | NCOR2 | NF1 | NF2 | NFE2L2 | NFKBIA | NHP2 |
| NKX2-1 | NOP10 | NOTCH1 | NOTCH2 | NOTCH3 | NPM1 | NQO1 | NRAS | NRG1 | NSD1 | NT5C2 |
| NTHL1 | NTRK1 | NTRK2 | NTRK3 | NUDT15 | NUP98 | P2RY8 | PAK1 | PALB2 | PALLD | PARK2 |
| PAX3 | PAX5 | PAX7 | PAX8 | PBRM1 | PCBP1 | PDCD1 | PDCD1LG2 | PDGFRA | PDGFRB | PDK1 |
| PDPK1 | PHF6 | PHOX2B | PIAS4 | PIK3C2B | PIK3CA | PIK3CB | PIK3CD | PIK3CG | PIK3R1 | PIK3R2 |
| PIM1 | PLCG2 | PML | PMS1 | PMS2 | POLD1 | POLE | POLH | POT1 | POU2F2 | PPP1R15A |
| PPP2R1A | PPP2R2A | PPP6C | PRCC | PRDM1 | PREX2 | PRKAR1A | PRSS1 | PRSS2 | PTCH1 | PTCH2 |
| PTEN | PTPN11 | PTPN13 | PTPN22 | PTPRD | QKI | RAC1 | RAD21 | RAD50 | RAD51 | RAD51B |
| RAD51C | RAD51D | RAD54L | RAF1 | RANBP2 | RARA | RASA1 | RB1 | RBM10 | RECQL4 | RET |
| RHOA | RICTOR | RINT1 | RIT1 | RNF43 | RNF139 | ROS1 | RPL5 | RPS6KB1 | RPS15 | RPTOR |

<p style="text-align:center">FIG. 15B</p>

EP 4 629 247 A1

| RSF1 | RUNX1 | RUNX1T1 | RXRA | SCG5 | SDHA | SDHAF2 | SDHB | SDHC | SDHD | SEC23B |
|------|-------|---------|------|------|------|--------|------|------|------|--------|
| SEMA3C | SETBP1 | SETD2 | SF3B1 | SGK1 | SH2B3 | SLC26A3 | SLC47A2 | SLIT2 | SLX4 | SMAD2 |
| SMAD3 | SMAD4 | SMARCA1 | SMARCA4 | SMARCB1 | SMARCE1 | SMC1A | SMC3 | SMO | SOCS1 | SOD2 |
| SOX2 | SOX9 | SOX10 | SPEN | SPINK1 | SPOP | SPRED1 | SRC | SRSF2 | STAG2 | STAT3 |
| STAT4 | STAT5A | STAT5B | STAT6 | STK11 | SUFU | SUZ12 | SYK | TAF1 | TANC1 | TAP1 |
| TAP2 | TBC1D12 | TBL1XR1 | TBX3 | TCF3 | TCF7L2 | TCL1A | TERT | TET2 | TGFBR2 | TIGIT |
| TMEM127 | TMEM173 | TMPRSS2 | TNF | TNFAIP3 | TNFRSF9 | TNFRSF14 | TNFRSF17 | TOP1 | TOP2A | TP53 |

FIG. 15C

Europäisches
Patentamt

European
Patent Office

Office européen
des brevets

# EUROPEAN SEARCH REPORT

**Application Number**

EP 25 16 8546

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DRIESSEN TERRI ET AL: "Poster 3498: A novel combination of tissue-informed, comprehensive genomic profiling (CGP) and non-bespoke blood-based profiling for quantifying circulating tumor DNA (ctDNA)", CANCER RESEARCH, [Online] vol. 84, no. 6_Supplement, 22 March 2024 (2024-03-22), pages 3498-3498, XP093305864, US ISSN: 1538-7445, DOI: 10.1158/1538-7445.AM2024-3498 Retrieved from the Internet: URL:https://www.tempus.com/wp-content/uploads/2024/03/3498_Driessen_AACR_2024.pdf> [retrieved on 2025-08-18] * the whole document * | 1-15 | INV. G16B20/20 |
| X | & DRIESSEN TERRI ET AL: "Abstract 3498: A novel combination of tissue-informed, comprehensive genomic profiling (CGP) and non-bespoke blood-based profiling for quantifying circulating tumor DNA (ctDNA) ¦ Cancer Research ¦ American Association for Cancer Research", CANCER RESEARCH, vol. 84, no. 6_Supplement, 22 March 2024 (2024-03-22), pages 3498-3498, XP093305859, US ISSN: 1538-7445, DOI: 10.1158/1538-7445.AM2024-3498 Retrieved from the Internet: URL:https://aacrjournals.org/cancerres/article/84/6_Supplement/3498/741151/Abstract-3498-A-novel-combination-of-tissue> [retrieved on 2025-08-18] * the whole document * | 1-15 | |

**TECHNICAL FIELDS SEARCHED (IPC)**

G16B

The present search report has been drawn up for all claims

| Place of search | Date of completion of the search | Examiner |
|---|---|---|
| Munich | 18 August 2025 | Rákossy, Z |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63574758
- US 11043304 B
- US 80049222
- US 10957041 B
- US 10957445 B
- US 11244763 B
- US 11848107 B
- US 11145416 B
- US 11830587 B
- US 20200255909

- US 705226
- WO 2021081253 A
- US 11629385 B
- US 9138205 B
- US 62944292
- US 789363
- US 20180002 W
- US 11415571 B
- WO 2021168146 A


**Non-patent literature cited in the description**

- **RADOVICH M. et al.** *Oncotarget*, 2016, vol. 7 (35), 56491-500
- **TSIMBERIDOU et al.** *ASCO*, 2018
- **WHEELER et al.** *Cancer Res.*, 2016, vol. 76, 3690-701
- **SCHWAEDERLE et al.** *J Clin Oncol.*, 2015, vol. 33 (32), 3817-25
- **SCHWAEDERLE et al.** *JAMA Oncol.*, 2016, vol. 2 (11), 1452-59
- **WHELER et al.** *Cancer Res.*, 2016, vol. 76 (13), 3690-701
- **COYNE et al.** *Curr. Probl. Cancer*, 2017, vol. 41 (3), 182-93
- **MARKMAN.** *Oncology*, vol. 31 (3), 158, 168
- **FERNANDES et al.** *Clinics*, vol. 72 (10), 588-94
- **ROSS et al.** *JAMA Oncol.*, 2015, vol. 1 (1), 40-49
- **ROSS et al.** *Arch. Pathol. Lab Med.*, 2015, vol. 139, 642-49
- **HIRSHFIELD KM et al.** *Oncologist*, 2016, vol. 21 (11), 1315-25
- **GROISBERG et al.** *Oncotarget*, 2017, vol. 8, 39254-67
- **ILIE ; HOFMAN.** *Transl. Lung Cancer Res.*, 2016, vol. 5 (4), 420-23
- **ILIE ; HOFMAN.** *Transl Lung Cancer Res.*, 2016, vol. 5 (4), 420-23
- **CHAN et al.** *Ann. Clin. Biochem.*, 2003, vol. 40, 122-30
- **STROUN et al.** *Oncology*, 1989, vol. 46 (5), 318-22
- **GOESSL et al.** *Cancer Res.*, 2000, vol. 60 (21), 5941-45

- **FRENEL et al.** *Clin. Cancer Res.*, 2015, vol. 21 (20), 4586-96
- **COLLEONI et al.** Annual Hazard Rates of Recurrence for Breast Cancer During 24 Years of Follow-Up: Results From the International Breast Cancer Study Group Trials I to V. *J Clin Oncol*, 2016 (34), 927
- **YATES et al.** Genomic Evolution of Breast Cancer Metastasis and Relapse. *Cancer Cell*, 2017 (32), 169
- **URAMOTO et al.** Recurrence after surgery in patients with NSCLC. *Transl Lung Cancer Res*, 2014 (3), 242
- **TAUNK et al.** Immunotherapy and radiation therapy for operable early stage and locally advanced non-small cell lung cancer. *Transl Lung Cancer Res*, 2017 (6), 178
- **COOMBES et al.** Personalized Detection of Circulating Tumor DNA Antedates Breast Cancer Metastatic Recurrence. *Clin Cancer Res*, 2019 (25), 4255
- **TIE et al.** Circulating Tumor DNA Analyses as Markers of Recurrence Risk and Benefit of Adjuvant Therapy for Stage III Colon Cancer. *JAMA Oncol*, 2019
- **MCEVOY et al.** Monitoring melanoma recurrence with circulating tumor DNA: a proof of concept from three case studies. *Oncotarget*, 2019 (10), 113
- **CHRISTENSEN et al.** Early Detection of Metastatic Relapse and Monitoring of Therapeutic Efficacy by Ultra-Deep Sequencing of Plasma Cell-Free DNA in Patients With Urothelial Bladder Carcinoma. *J Clin Oncol*, 2019 (37), 1547

- **ISAKSSON et al.** Pre-operative plasma cell-free circulating tumor DNA and serum protein tumor markers as predictors of lung adenocarcinoma recurrence. *Acta Oncol*, 2019 (58), 1079
- **ADALSTEINSSON et al.** Scalable whole-exome sequencing of cell-free DNA reveals high concordance with metastatic tumors. *Nature Communications*, 06 November 2017, vol. 8 (1), 1324
- **BAICHOO ; OUZOUNIS**. *BioSystems*, 2017, 156-157
- **BENT et al.** *Cancer Chemother Pharmacol.*, 2017, vol. 80 (6), 1209-17
- **PART et al.** Clinical relevance of TNM staging system according to breast cancer subtypes. *Annals of Oncology*, 2011, vol. 22 (7), 1554-1560
- **CHOMCZYNSKI ; SACCHI**. *Nat Protoc*, 2006, vol. 1 (2), 581-85
- **POECKH et al.** *Anal Biochem.*, 2008, vol. 373 (2), 253-62
- **KIVIOJA et al.** *Nat. Methods*, 2011, vol. 9 (1), 72-74
- **ISLAM et al.** *Nat. Methods*, 2014, vol. 11 (2), 163-66
- **JIANG et al.** *BMC Bioinformatics*, 2014, vol. 15 (182), 1-12
- **HATEM et al.** Benchmarking short sequence mapping tools. *BMC Bioinformatics*, 2013, vol. 14, 184
- **FLICEK ; BIRNEY**. Sense from sequence reads: methods for alignment and assembly. *Nat Methods*, 2009, vol. 6 (11), S6-S12
- **LI ; HOMER**. A survey of sequence alignment algorithms for next-generation sequencing. *Brief Bioinformatics*, 2010, vol. 11, 473-483
- **HAI et al.** Whole-genome circulating tumor DNA methylation landscape reveals sensitive biomarkers of breast cancer. *MedComm*, 03 September 2022 (3), e134
- **MARTIN**. Cutadapt removes adapter sequences from high-throughput sequencing reads. *EMBnet.journal*, 2011, vol. 17 (1), 10-12
- **LEVIE**. Curve Fitting with Least Squares. Critical Reviews. *Analytical Chemistry*, 2000, vol. 30 (1), 59-74
- **SGOUROPOULOS et al.** Matching a Distribution by Matching Quantiles Estimation. *Journal of the American Statistical Association*, 2015, vol. 110 (510), 742-759
- **KOCH**. Robust estimation by expectation maximization algorithm. *Journal of Geodesy*, 2013, vol. 87, 107-116
- **CHEN et al.** A tutorial on kernel density estimation and recent advances. *Biostatistics & Epidemiology*, 2017, vol. 1 (1), 161-187
- **BEAUBIER et al.** Clinical validation of the tempus xT next-generation targeted oncology sequencing assay. *Oncotarget*, 2019, vol. 10 (24), 2384-2396